# EUROPÄISCHE PATENTSCHRIFT

(11) **EP 1 463 810 B1**
(45) Veröffentlichungstag und Bekanntmachung des Hinweises auf die Patenterteilung: **14.05.2014**
(21) Anmeldenummer: 02793003.1
(22) Anmeldetag: 13.12.2002
(51) Int. Cl.: C12N 9/26, C12N 15/56, C12P 7/06, C12P 19/14, C11D 3/386, A23K 1/165

(54) **Glykosylhydrolase**
Glycosyl hydrolase
Glycosyl hydrolase

(30) Priorität: 21.12.2001 DE 10163748
(43) Veröffentlichungstag der Anmeldung: 06.10.2004
(73) Patentinhaber: B.R.A.I.N. Biotechnology Research And Information Network AG, 64673 Zwingenberg (DE)
(72) Erfinder: BREVES, Roland, 40822 Mettmann (DE); MAURER, Karl-Heinz, 40699 Erkrath (DE); ECK, Jürgen, 64646 Heppenheim (DE); LORENZ, Patrick, 69493 Hirschberg (DE); ZINKE, Holger, 64673 Zwingenberg (DE)
(74) Vertreter: Vossius & Partner
(86) Internationale Anmeldenummer: PCT/EP2002/014210
(87) Internationale Veröffentlichungsnummer: WO 2003/054177

(56) Entgegenhaltungen:
- EP-A1- 0 644 260
- WO-A-98/15617
- WO-A2-01/14532
- DATABASE SWISS-PROT [Online] Alpha-amylase precursor, 1. Januar 1998 (1998-01-01) retrieved from EBI, accession no. O24781 XP002247832
- DATABASE EMBL/GENBANK/DDBJ [Online] 4. September 1997 (1997-09-04) ARAI, M.: "Bacillus sp. DNA for alpha-amylase" retrieved from EBI, accession no. AB006823 XP002247833
- SUMITANI JUN-ICHI ET AL: "New type of starch-binding domain: The direct repeat motif in the C-terminal region of Bacillus sp. no. 195 alpha-amylase contributes to starch binding and raw starch degrading." BIOCHEMICAL JOURNAL, Bd. 350, Nr. 2, 2000, Seiten 477-484, XP009013936 ISSN: 0264-6021
- DATABASE EMBL/GENBANK/DDBJ [Online] 1. Oktober 2000 (2000-10-01) SEEGER, K.J. AND HARRIS, D.: "Putative bi-functional protein (Secreted alpha-amylase/dextrinase)" retrieved from EBI Database accession no. Q9KZ11 XP002271703
- RONDON M.R. ET AL: "Cloning the soil metagenome: a strategy for accessing the genetic and functional diversity of uncultured microorganisms", APPLIED AND ENVIRONMENTAL MICROBIOLOGY, vol. 66, no. 6, June 2000 (2000-06), pages 2541-2547,
- KURIKI T. ET AL: "The concept of the alpha-amylase family: structural similarity and common catalytic mechanism", JOURNAL OF BIOSCIENCE AND BIOENGINEERING, vol. 87, no. 5, 1999, pages 557-565,

## Beschreibung

Die vorliegende Erfindung betrifft eine neue Glykosylhydrolase mit amylolytischer Aktivität und die für diese Glykosylhydrolase codierenden Nukleinsäuren, sowie diverse technische Anwendungsmöglichkeiten hierfür wie in den Ansprüchen definiert. Hierunter sind Wasch- und Reinigungsmittel mit solchen Enzymen sowie entsprechende Verfahren und Verwendungsmöglichkeiten von besonderem Interesse.

Glykosylhydrolasen stellen eine weitverzweigte Gruppe von Enzymen dar, die glykosidische Bindungen zwischen zwei oder mehr Kohlenhydraten, oder zwischen einem Kohlenhydrat und einer Nicht-Kohlenhydrat-Funktion hydrolysieren.

Glykosylhydrolasen von besonderem industriellen und technischen Interesse sind die α-Amylasen (E.C. 3. 2. 1. 1), die im Polymerinneren gelegene α-1,4-glycosidische Bindungen von Stärke und stärkeähnlichen Polymeren hydrolysieren, wie beispielsweise Amylose, Amylopektin oder Glykogen, unter Bildung von Dextrinen und β-1,6-verzweigten Oligosacchariden. Sie gehören zu den wichtigsten industriell genutzten Enzymen überhaupt. Dies aus zwei Gründen: Zum einen werden sie zumeist wie viele substratabbauende Enzyme von Mikroorganismen in das umgebende Medium abgegeben, so daß sie durch Fermentation und Aufreinigung aus dem Kulturmedium mit vergleichsweise geringem Aufwand in industriellem Maßstab gewonnen werden können. Zum anderen werden Amylasen für ein breites Anwendungsspektrum benötigt.

An erster Stelle der technischen Verwendungen von α-Amylase steht die Herstellung von Glucosesirup. Andere Verwendungen sind beispielsweise die als aktive Komponenten in Wasch- und Reinigungsmitteln, zur Behandlung von Rohmaterialien in der Textilherstellung, zur Herstellung von Klebstoffen oder zur Herstellung von zuckerhaltigen Lebensmitteln oder Lebensmittelbestandteilen.

Ein Beispiel für eine technisch besonders intensiv eingesetzte Amylase ist die α-Amylase aus *Bacillus licheniformis,* die von der Fa. Novozymes A/S, Bagsværd, Dänemark, unter dem Handelsnamen Termamyl^{®} angeboten wird. Die aus B. *subtilis,* beziehungsweise *B. amyloliquefaciens* gewonnene und in dem US-Patent US 1 227 374 offenbarte Amylase wird von derselben Firma unter dem Namen BAN^{®} vertrieben.

Dieses Amylase-Molekül, beziehungsweise dessen nahen Verwandte, sind in zahlreichen Erfindungen weiterentwickelt worden, denen die Aufgabe zugrunde gelegen hat, mithilfe diverser molekularbiologischer Modifikationen ihre enzymatischen Eigenschaften auf spezifische Anwendungen hin zu optimieren. Solche Optimierungen können beispielsweise die Substratspezifitäten, die Stabilität des Enzyms unter verschiedenen Reaktionsbedingungen oder die enzymatische Aktivität selbst betreffen. Beispielhaft für solche Optimierungen seien folgende Schutzrechte genannt: EP 0410498 B1 für das Schlichten von Textilien und WO 96/02633 A1 zur Stärkeverflüssigung.

Da Entwicklungen, die lediglich in Optimierungen von nur wenigen bekannten Ausgangsenzymen bestehen, möglicherweise in den erzielbaren Ergebnissen beschränkt sind, findet parallel dazu eine intensive Suche nach vergleichbaren Enzymen aus anderen natürlichen Quellen statt. Identifiziert wurden stärkespaltende Enzyme beispielsweise aus *Pimelobacter, Pseudomonas* und *Thermus* für die Lebensmittelherstellung, Kosmetik und Pharmaka (EP 0 636 693 A2), ebensolche aus *Rhizobium, Arthrobacter, Brevibacterium* und *Micrococcus* (EP 0 628 630 A2), aus *Pyrococcus* (WO 94/19454 A2) und *Sulfolobus* zur Stärkeverflüssigung bei hohen Temperaturen, beziehungsweise, stark sauren Reaktionsbedingungen (EP 0 727 485 A1 und WO 96/02633 A1). In der europäischen Patenanmeldung, die als EP 0644260 veröffentlicht wurde, wird eine α-Amylase aus *Bacillus licheniformis* beschrieben, die zur Stärkeverflüssigung in einer sauren Umgebung mit einem pH-Wert von ungefähr 4.0 bis 5.5 bei Temperaturen von 90 bis 110 °C geeignet ist. Für den Einsatz bei alkalischen pH-Werten sind Amylasen aus *Bacillus sp.* (WO 95/26397 A1 und WO 97/00324 A1) gefunden worden. Wegen ihrer geringen Empfindlichkeit gegenüber Detergenzien eignen sich andere Amylasen aus verschiedenen *Bacilli* (EP 0 670 367 A1) zur Verwendung in Wasch- oder Reinigungsmitteln. Weitere Amylasen aus *Bacillus sp.* für den Einsatz in alkalischen Lösungen, insbesondere in alkalischen Waschmittellösungen mit einem pH-Wert von ungefähr 9 bis 11, sind in der internationalen Patentanmeldung, die als WO 01/14532 veröffentlicht wurde, beschrieben.

Weitere Optimierungen der aus natürlichen Quellen isolierten Enzyme für das jeweilige Anwendungsgebiet können beispielsweise über molekularbiologische Methoden (etwa gemäß US 5171673 oder WO 99/20768 A1) oder über chemische Modifikationen vorgenommen werden (DE 4013142 A1). In der Patentanmeldung

WO 99/43793 A1, beispielsweise, wird eine Weiterentwicklung der bekannten Novamyl^{®}-α-Amylase beschrieben. Darin werden Sequenzähnlichkeiten zwischen Novamyl^{®} und bekannten Cyclodextringlucanotransferasen (CGTasen) ausgenutzt, um mithilfe molekularbiologischer Techniken eine Schar verwandter Moleküle zu konstruieren. Bei diesen handelt es sich um α-Amylasen mit zusätzlichen CGTase-spezifischen Consensus-Sequenzen (Boxen) und Funktionen oder, umgekehrt, um CGTasen mit zusätzlichen für α-Amylasen typischen Bereichen und Funktionen oder um Chimären beider Moleküle. Der Sinn dieser Entwicklung besteht darin, Novamyl^{®} für diese Anwendungen zu optimieren.

Die aus der Optimierung bekannter Glykosylhydrolasen und insbesondere Amylasen hervorgehenden neuen Enzyme sind naturgemäß in ihren Eigenschaften limitiert, da sie lokale Optima in der "Fitneß-Landschaft" der Glykosylhydrolasen darstellen. Um weitere, möglicherweise noch bessere lokale Optima aufzufinden, muß von anderen Ursprungsenzymen ausgegangen werden, die entweder selbst ein solches lokales Optimum darstellen, oder sich mit vertretbarem Aufwand in Richtung eines solchen optimieren lassen. Die Auffindung neuer Glykosyltransferasen, die solche Ursprungsenzyme sein können, erfordert jedoch auf dem Wege des klassischen mikrobiologischen Screenings die Isolierung definierter Stämme. Über 95 % aller vorkommenden Mikroorganismen sind jedoch nicht kultivierbar, so daß die in diesen Mikroorganismen vorkommenden neuen Glykosylhydrolasen, einschließlich der Amylasen, einer Charakterisierung bislang nicht zugänglich waren.

**α-Amylasen sind typischerweise monomere Enzyme mit einem Molekulargewichte von ca 55 kDa. Die zentrale Domäne besitzt eine α/β-Barrel-Struktur (TIM-barrel), die nach dem heutigen Kenntnisstand das am weitesten verbereitet Proteinfaltungsmotiv darstellt.** Der lineare Proteinstrang besteht aus je 8 α-Helices und 8 *β*-Faltblatt-Abschnitten (*β*-Sheets), die in alternierender Reihenfolge vorkommen und sich dreidimensional derart falten, daß die *β*-Faltblätter in paralleler Orientierung kreisförmig angeordnet sind und von den α-Helices außen umgeben sind.

Die enzymatische Aktivität wird wahrscheinlich von Aminosäureresten hervorgerufen, die am C-terminalen Ende der einzelnen Helices und *β*-Faltblättern lokalisiert sind.

Im Beispiel der als Termamyl bekannten.Amylase aus *Bacillus licheniformis* sind dies die Aminosäurereste Aspartat 231 (general base catalyst) und Glutamat 261 (general acid catalyst). Dem ursprünglich als general acid catalyst angesehene Rest Asp 328 wird nach neueren Untersuchungen ein essentieller Einfluß auf die elektrostatischen Verhältnisse in der Active site zugeschrieben. Weitere Reste wie zum Beispiel His 210 sollen an der Bindung des Substrates oder weiterer Faktoren, wie zum Beispiel Ca²⁺-Ionen beteiligt sein.

Neben den eigentlichen Amylasen werden weitere Enzyme der Amylase-Super-familie zugerechnet, so zum Beispiel α-1,6-Glucosidasen, Cyclodextringlucanotransferasen (CGTasen), Isoamylasen, Neopullulanasen, Glycogen debranching enzyme, Dextranglucosidasen und Glycosyltransferasen.

Die z.T. recht unterschiedliche Substrabindungs-Spezifität wird durch Proteinloops bewirkt, die zwischen bestimmte Strukturelemente eingelassen sind und kleinere, eigenständige Domänen bilden.

So existiert eine B-Domäne, die zwischen *β*-Faltblatt 3 und Helix 3 angeordnet ist. Sie umfaßt im Falle der α-Amylasen und damit eng verwandter α-1,6-Glucosidasen und Cyclodextrin-Glucanotransferasen (CGTasen) ca. 40 AminoSäuren, kann aber auch, wie zum Beispiel beim Glucan-debranching enzyme 270 Aminosäuren umfassen (Jespersen, H.M; E.A. MacGregor, B. Henrissat, M, Sierks and B. Svensson: "Starch- and Glycogen-Debranching and Branching Enzymes", J. of Protein Chemistry, Band 12, S. 791-805, 1993).

Weitere Domänen sind C-terminal angeordnet, so die C-Domäne bei allen Enzymen und eine oder mehrere weitere D- beziehungsweise E-Domänen (zum Beispiel bei CGTasen) Diesen wird ebenfalls eine Mitwirkung in der Substratbindung zugeschrieben. Sumitani et al. (Biochem. J. (2000) 350, 477-484) beschreibt beispielsweise einen bestimmten C-terminalen Bereich einer α-Amylase aus *Bacillus sp.,* die verantwortlich für die Stärkebindung als auch -degradierung sein soll.

Trotz dieses allgemein ähnlichen Aufbaus variiert die Sequenzhomologie des linearen Proteinstranges stark, wodurch die Definition charakteristischer Sequenzabschnitte als Sonden, mittels derer in effizienter Weise nach neuen Amylasen gesucht werden könnte, außerordentlich erschwert wird.

Die für die Faltung verantwortlichen Strukturelemente können auch ohne Homologie in der Primärstruktur ausgebildet werden. So sind die Amylasen aus Bacillus licheniformis und Bacillus amyloliquefaciens zu über 80 % identisch. Die Homologie über alle Amylasen bakterieller, tierischer und pflanzlicher Herkunft hingegen beträgt weniger als 10 % (Nakajima et al., Appl. Microbiol. Biotechnol., Band 23, S. 355-360 (1986)).

Weitere TIM-Barrel-Proteine sind trotz einer identischen Proteinfaltungsstruktur überhaupt nicht mehr homolog.

Somit können also hinsichtlich der Homologie wenig ähnliche Proteine trotzdem eine gleiche oder nah verwandte Enzymaktivität aufweisen, sofern ein ähnliches Proteinfaltungsmuster und die entsprechende, für die katalytische Reaktion erforderliche topologische Anordnung der beteiligten Aminosäurereste gegeben ist.

Zusammenfassend ist festzustellen, daß sich das Auffinden neuer Glykosylhydrolasen sowohl durch die mangelhafte Kultivierbarkeit vieler Mikroorganismen, die potentiell interessante Glykosylhydrolasen herstellen, als auch durch die oft nur geringe Sequenzhomologie innerhalb der Familie der Glykoylhydrolasen sehr problematisch gestaltet.

Überraschenderweise wurde gefunden, daß neue Glykosylhydrolasen durch Screening von DNA aus Umweltproben, sogenannter Metagenom-DNA, charakterisiert und in technisch nutzbarer Weise bereitgestellt werden können. Rondon et al. (Applied and Environmental Microbiology, June 2000, p. 2541 - 2547) beschreibt beispielhaft die Möglichkeit, DNA-Banken genomischer DNA, die aus Erdproben gewonnen wurde, mithilfe von BAC-Vektoren herzustellen.

Gegenstand der vorliegenden Erfindung ist daher die neue Glykosylhydrolase, die im nachfolgenden Sequenzprotokoll unter der Sequenz-Identifikationsnummer (Seq.-Id.) 1 aufgeführt ist.

Weitere Gegenstände der Erfindung sind Nukleinsäuren (DNA oder RNA), die für eine der zuvor genannten Glykosylhydrolasen kodieren.

Im folgenden bedeutet ein Ausdruck der Form "mindestens X %" "X % bis 100 % einschließlich der Eckwerte X und 100 und aller ganzzahligen und nicht ganzzahligen Prozentwerte dazwischen".

Unter einem Protein ist im Sinne der vorliegenden Anmeldung ein aus den natürlichen Aminosäuren zusammengesetztes, weitgehend linear aufgebautes, zur Ausübung seiner Funktion zumeist dreidimensionale Struktur annehmendes Polymer zu verstehen. In der vorliegenden Anmeldung werden die 19 proteinogenen, natürlich vorkommenden L-Aminosäuren mit den international gebräuchlichen 1- und 3-Buchstaben-Codes bezeichnet.

Unter einem Enzym ist im Sinne der vorliegenden Anmeldung ein Protein zu verstehen, das eine bestimmte biochemische Funktion ausübt. Unter Glykosylhydrolasen sind Proteine zu verstehen, die glykosidische Bindungen hydrolysieren. Bevorzugte und technisch bedeutsame Glykosylhydrolasen sind amylolytische Proteine oder Enzyme mit amylolytischer Funktion, also solche, die α-1,4-glykosidische Bindungen von Polysacchariden hydrolysieren, insbesondere solche Bindungen, die im inneren der Polysaccharide liegen. Sie werden auch als α-1,4-Amylasen (E.C. 3.2.1.1) oder kurz: α-Amylasen bezeichnet.

Zahlreiche Proteine werden als sogenannte Präproteine, also zusammen mit einem Signalpeptid gebildet. Darunter ist dann der N-terminale Teil des Proteins zu verstehen, dessen Funktion zumeist darin besteht, die Ausschleusung des gebildeten Proteins aus der produzierenden Zelle in das Periplasma oder das umgebende Medium und/oder dessen korrekte Faltung zu gewährleisten. Anschließend wird das Signal peptid unter natürlichen Bedigungen durch eine Signalpeptidase vom übrigen Protein abgespalten, so daß dieses seine eigentliche katalytische Aktivität ohne die zunächst vorhandenen N-terminalen Aminosäuren ausübt.

Für technische Anwendungen sind aufgrund ihrer enzymatischen Aktivität die maturen Peptide, das heißt die nach ihrer Herstellung prozessierten Enzyme gegenüber den Präproteinen bevorzugt.

Pro-Proteine sind inaktive Vorstufen von Proteinen. Deren Vorläufer mit Signalsequenz werden als Prä-Pro-Proteine bezeichnet.

Unter Nukleinsäuren sind im Sinne der vorliegenden Anmeldung die natürlicherweise aus Nukleotiden aufgebauten als Informationsträger dienenden Moleküle zu verstehen, die für die lineare Aminosäureabfolge in Proteinen oder Enzymen codieren. Sie können als Einzelstrang, als ein zu diesem Einzelstrang komplementärer Einzelstrang oder als Doppelstrang vorliegen. Als der natürlicherweise dauerhaftere Informationsträger ist die Nukleinsäure DNA für molekularbiologische Arbeiten bevorzugt. Demgegenüber wird für die Realisierung der Erfindung in natürlicher Umgebung, wie beispielsweise in einer exprimierenden Zelle, eine RNA gebitdet.

Bei DNA sind die Sequenzen beider komplementärer Stränge in jeweils allen drei möglichen Leserastern zu berücksichtigen. Ferner ist zu berücksichtigen, daß verschiedene Codon-Triplets für dieselben Aminosäuren codieren können, so daß eine bestimmte Aminosäure-Abfolge von mehreren unterschiedlichen und möglicherweise nur geringe Identität aufweisenden Nukleotidsequenzen abgeleitet werden kann (Degeneriertheit des genetischen Codes). Außerdem weisen verschiedene Organismen Unterschiede im Gebrauch dieser Codons auf. Aus diesen Gründen müssen sowohl Aminosäuresequenzen als auch Nukleotidsequenzen in die Betrachtung des Schutzbereichs einbezogen werden und angegebene Nukleotidsequenzen sind jeweils nur als eine beispielhafte Codierung für eine bestimmte Aminosäurefolge anzusehen.

Die einem Protein entsprechende Informationseinheit wird auch im Sinne der vorliegenden Anmeldung als Gen bezeichnet.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind beispielsweise aus dem "Lexikon der Biochemie", Spektrum Akademischer Verlag, Berlin, 1999, Band 1, S. 267-271 und Band 2, S. 227-229, bekannt.

Änderungen der Nukleotidsequenz, wie sie beispielsweise durch an sich bekannte molekularbiologische Methoden herbeigeführt werden können, werden als Mutationen bezeichnet. Je nach Art der Änderung kennt man beispielsweise Deletions-, Insertions- oder Substitutionsmutationen oder solche, bei denen verschiedene Gene oder Teile von Genen miteinander fusioniert (shuffling) werden; dies sind Genmutationen. Die zugehörigen Organismen werden als Mutanten bezeichnet. Die von mutierten Nukleinsäuren abgeleiteten Proteine werden als Varianten bezeichnet. So führen beispielsweise Deletions-, Insertions-, Substitutionsmutationen oder Fusionen zu deletions-, insertions-, substitutionsmutierten oder Fusionsgenen und auf Proteinebene zu entsprechenden Deletions-, Insertions- oder Substitutionsvarianten, beziehungsweise Fusionsproteinen.

Unter Fragmenten werden alle Proteine oder Peptide verstanden, die kleiner sind als natürliche Proteine oder solche, die vollständig translatierten Genen entsprechen, und beispielsweise auch synthetisch erhalten werden können. Aufgrund ihrer Aminosäuresequenzen können sie den betreffenden vollständigen Proteinen zugeordnet werden. Sie können beispielsweise gleiche Strukturen annehmen oder proteolytische Aktivitäten oder Teilaktivitäten ausüben, wie beispielsweise die Komplexierung eines Substrats. Fragmente und Deletionsvarianten von Ausgangsproteinen sind prinzipiell gleichartig; während Fragmente eher kleinere Bruchstücke darstellen, fehlen den Deletionsmutanten eher nur kurze Bereiche, und damit nur einzelne Teilfunktionen.

Den Fragmenten entsprechen auf Nukleinsäure-Ebene die Teilsequenzen.

Unter chimären oder hybriden Proteinen sind im Sinne der vorliegenden Anmeldung solche Proteine zu verstehen, die aus Elementen zusammengesetzt sind, die natürlicherweise von verschiedenen Polypeptidketten aus demselben Organismus oder aus verschiedenen Organismen stammen. Dieses Vorgehen wird auch Shuffling oder Fusionsmutagenese genannt. Der Sinn einer solchen Fusion kann beispielsweise darin bestehen, mithilfe des heranfusionierten Proteinteils eine bestimmte enzymatische Funktion herbeizuführen oder zu modifizieren. Es ist dabei im Sinne der vorliegenden Erfindung unwesentlich, ob solch ein chimäres Protein aus einer einzelnen Polypeptidkette oder mehreren Untereinheiten besteht, auf welche sich unterschiedliche Funktionen verteilen können. Zur Realisierung der letztgenannten Alternative ist es beispielsweise möglich, posttranslational oder erst nach einem Aufreinigungsschritt durch eine gezielte proteolytische Spaltung eine einzelne chimäre Polypeptidkette in mehrere zu zerlegen.

Unter durch Insertionsmutation erhaltene Proteinen sind solche Varianten zu verstehen, die über an sich bekannte Methoden durch Einfügen eines Nukleinsäure-, beziehungsweise Proteinfragments in die Ausgangssequenzen erhalten worden sind. Sie sind ihrer prinzipiellen Gleichartigkeit wegen den chimären Proteinen zuzuordnen. Sie unterscheiden sich von jenen lediglich im Größenverhältnis des unveränderten Proteinteils zur Größe des gesamten Proteins. In solchen insertionsmutierten Proteinen ist der Anteil an Fremdprotein geringer als in chimären Proteinen.

Inversionsmutagenese, also eine partielle Sequenzumkehrung, kann als Sonderform sowohl der Deletion, als auch der Insertion angesehen werden. Dasselbe gilt für eine von der ursprünglichen Aminosäureabfolge abweichende Neugruppierung verschiedener Molekülteile. Sie kann sowohl als Deletionsvariante, als Insertionsvariante, als auch als Shuffling-Variante des ursprünglichen Proteins angesehen werden.

Unter Derivaten werden im Sinne der vorliegenden Anmeldung solche Proteine verstanden, deren reine Aminosäurekette chemisch modifiziert worden ist. Solche Derivatisierungen können beispielsweise biologisch im Zusammenhang mit der Proteinbiosynthese durch den Wirtsorganismus erfolgen. Hierfür können molekularbiologische Methoden eingesetzt werden. Sie können aber auch chemisch durchgeführt werden, etwa durch die chemische Umwandlung einer Seitenkette einer Aminosäure oder durch kovalente Bindung einer anderen Verbindung an das Protein. Bei solch einer Verbindung kann es sich beispielsweise auch um andere Proteine handeln, die beispielsweise über bifunktionelle chemische Verbindungen an erfindungsgemäße Proteine gebunden werden. Derartige Modifizierungen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Ebenso ist unter Derivatisierung die kovalente Bindung an einen makromolekularen Träger zu verstehen.

Proteine können auch über die Reaktion mit einem Antiserum oder einem bestimmten Antikörper zu Gruppen immunologisch verwandter Proteine zusammengefaßt werden. Die Angehörigen einer Gruppe zeichnen sich dadurch aus, daß sie dieselbe, von einem Antikörper erkannte antigene Determinante aufweisen.

Im Sinne der vorliegenden Erfindung werden alle Enzyme, Proteine, Fragmente und Derivate, sofern sie nicht explizit als solche angesprochen zu werden brauchen, unter dem Oberbegriff Proteine zusammengefaßt.

Unter Vektoren werden im Sinne der vorliegenden Erfindung aus Nukleinsäuren bestehende Elemente verstanden, die als kennzeichnenden Nukleinsäurebereich ein interessierendes Gen enthalten. Sie vermögen dieses in einer Spezies oder einer Zellinie über mehrere Generationen oder Zellteilungen hinweg als vom übrigen Genom unabhängig replizierendes, stabiles genetisches Element zu etablieren. Vektoren sind insbesondere bei der Verwendung in Bakterien spezielle Plasmide, also zirkulare genetische Elemente. Man unterscheidet in der Gentechnik einerseits zwischen solchen Vektoren, die der Lagerung und somit gewissermaßen auch der gentechnischen Arbeit dienen, den sogenannten Klonierungsvektoren, und andererseits denen, die die Funktion erfülllen, das interessierende Gen in der Wirtszelle zu realisieren, das heißt, die Expression des betreffenden Proteins zu ermöglichen. Diese Vektoren werden als Expressionsvektoren bezeichnet. Durch Vergleich mit bekannten Enzymen, die beispielsweise in allgemein zugänglichen Datenbanken hinterlegt sind, läßt sich aus der Aminosäure- oder NukleotidSequenz die enzymatische Aktivität eines betrachteten Enzyms folgern. Diese kann durch andere Bereiche des Proteins, die nicht an der eigentlichen Reaktion beteiligt sind, qualitativ oder quantitativ modifiziert werden. Dies könnte beispielsweise die Enzymstabilität, die Aktivität, die Reaktionsbedingungen oder die Substratspezifität betreffen.

Solch ein Vergleich geschieht dadurch, daß ähnliche Abfolgen in den Nukleotid- oder Aminosäuresequenzen der betrachteten Proteine einander zugeordnet werden. Dies nennt man Homologisierung. Eine tabellarische Zuordnung der betreffenden Positionen wird als Alignment bezeichnet. Bei der Analyse von Nukleotidsequenzen sind wiederum beide komplementären Stränge und jeweils allen drei möglichen Leserastern zu berücksichtigen; ebenso die Degeneriertheit des genetischen Codes und die organismenspezifische Codon-Usage. Inzwischen werden Alignments über Computerprogramme erstellt, wie beispielsweise durch die Algorithmen FASTA oder BLAST; dieses Vorgehen wird beispielsweise von D. J. Lipman und W. R. Pearson (1985) in Science, Band 227, S. 1435-1441 beschrieben.

Eine Zusammenstellung aller in den verglichenen Sequenzen übereinstimmenden Positionen wird als Consensus-Sequenz bezeichnet.

Solch ein Vergleich erlaubt auch eine Aussage über die Ähnlichkeit oder Homologie der verglichenen Sequenzen zueinander. Diese wird in Prozent Identität, das heißt dem Anteil der identischen Nukleotide oder Aminosäurereste an denselben Positionen widergegeben. Ein weiter gefaßter Homologiebegriff bezieht die konservierten Aminosäure-Austausche in diesen Wert mit ein. Es ist dann von Prozent Ähnlichkeit die Rede. Solche Aussagen können über ganze Proteine oder Gene oder nur über einzelne Bereiche getroffen werden.

Die Erstellung eines Alignments ist der erste Schritt zur Definition eines Sequenzraums. Dieser hypothetische Raum umfaßt sämtliche durch Permutation in Einzelpositionen abzuleitenden Sequenzen, die sich unter Berücksichtigung aller in den betreffenden Einzelpositionen des Alignments auftretenden Variationen ergeben. Jedes hypothetisch mögliche Proteinmolekül bildet einen Punkt in diesem Sequenzraum. Beispielsweise begründen zwei Aminosäuresequenzen, die bei weitgehender Identität an lediglich zwei verschiedenen Stellen jeweils zwei verschiedene Aminosäuren aufweisen, somit einen Sequenzraum von vier verschiedenen Aminosäuresequenzen. Ein sehr großer Sequenzraum wird erhalten, wenn zu einzelnen Sequenzen eines Raums jeweils weitere homologe Sequenzen gefunden werden. Über solche, jeweils paarweise bestehenden hohen Homologien können auch sehr niedrig homologe Sequenzen als einem Sequenzraum zugehörig erkannt werden.

Homologe Bereiche von verschiedenen Proteinen sind solche mit gleichen Funktionen, die sich durch Übereinstimmungen in der primären Aminosäuresequenz erkennen lassen. Sie geht bis zu völligen Identitäten in kleinsten Bereichen, sogenannten Boxen, die nur wenige Aminosäuren umfassen und meist für die Gesamtaktivität essentielle Funktionen ausüben. Unter den Funktionen der homologen Bereiche sind kleinste Teilfunktionen der vom gesamten Protein ausgeübten Funktion zu verstehen, wie beispielsweise die Ausbildung einzelner Wasserstoffbrückenbindungen zur Komplexierung eines Substrats oder Übergangskomplexes.

Im Rahmen der vorliegenden Anmeldung ist zwischen Screening (Hybridisierungs-Screening oder DNA-Screening) und Aktivitätstest zu unterscheiden. Im allgemeinen wird unter dem "Screening" von Transformanten eine Nachweisreaktion verstanden, die dazu geeignet ist, solche Klone zu erkennen, bei denen das gewünschte Transformationsereignis stattgefunden hat. Es richtet sich, wie beispielsweise bei der geläufigen Blau-weiß-Selektion meist auf den Nachweis einer biochemischen Aktivität, die die Transformanten erhalten haben oder die nach erfolgter Rekombination nicht mehr vorhanden ist. Diese Art von biochemischer Nachweisreaktion wird im Sinne der vorliegenden Anmeldung auch als Aktivitätstest bezeichnet.

Screening bezeichnet aber auch das Durchsuchen einer Genbank mit bestimmten Nukleinsäuren und das damit mögliche Identifizieren von hinreichend ähnlichen Nukleinsäure-Sequenzen. Dies geschieht beispielsweise über Southem- oder Northem-Blot-Hybridisierungen, wie sie aus dem Stand der Technik hinlänglich bekannt sind. Dieser Begriff schließt beispielsweise auch erfindungsgemäße PCRbasierte Verfahren zur Identifizierung und/oder Gewinnung neuer Gene aus einer Sammlung von Organismen oder Nukleinsäuren ein, die dadurch gekennzeichnet sind, daß PCR-Primer mit einer variablen 3'-Region und einer 5'-Region mit hoher Homologie zu entsprechenden Bereichen aus bekannten Genen verwendet werden.

Unter der Leistung eines Enzyms wird dessen Wirksamkeit im jeweils betrachteten technischen Bereich verstanden. Diese basiert auf der eigentlichen enzymatischen Aktivität, hängt darüberhinaus aber von weiteren, für den jeweiligen Prozeß relevanten Fatoren ab. Dazu gehören beispielsweise Stabilität, Substratbindung, Wechselwirkung mit dem das Substrat tragenden Material oder Wechselwirkungen mit anderen Inhaltsstoffen, insbesondere Synergien. So wird beispielsweise bei der Untersuchung, ob sich ein Enzym für den Einsatz in Wasch- oder Reinigungsmitteln eignet, dessen Beitrag zur Wasch- oder Reinigungsleistung eines mit weiteren Bestandteilen formulierten Mittels betrachtet. Für verschiedene technische Anwendungen kann ein Enzym über an sich bekannte molekularbiologische Techniken, insbesondere die oben genannten weiterentwickelt und optimiert werden.

Ein erster Gegenstand der vorliegenden Erfindung ist eine Glykosylhydrolase, deren Aminosäuresequenz mit der in Seq. 1 angegebenen Aminosäuresequenz zu 100 % übereinstimmt.

Weiterhin beschrieben ist eine Glykosylhydrolase, deren Aminosäuresequenz mit der in Seq. 2 angegebenen Aminosäuresequenz zu mindestens 50 %, zu mindestens 60 %, vorzugsweise mindestens 70 %, insbesondere mindestens 80 %, besonders bevorzugt mindestens 90 % und ganz besonders bevorzugt zu 100 % übereinstimmt.

Außerdem beschrieben ist eine Glykosylhydrolase, deren Aminosäuresequenz mit der in Seq. 3 angegebenen Aminosäuresequenz zu mindestens 60 %, zu mindestens 70 %, vorzugsweise mindestens 80 %, insbesondere mindestens 90 %, besonders bevorzugt mindestens 95 % und ganz besonders bevorzugt zu 100 % übereinstimmt.

Ein weiterer Gegenstand der vorliegenden Erfindung ist eine Nukleinsäure, kodierend für eine Glykosylhydrolase, deren Aminosäuresequenz mit der in Seq. 1 angegebenen Aminosäuresequenz zu 100 % identisch ist.

Ein weiterer Gegenstand der vorliegenden Erfindung ist eine für eine Glykosylhydrolase kodierende Nukleinsäure, deren Nukleotidsequenz mit der in Seq. 4 angegebenen Nukleotidsequenz zu 100 % übereinstimmt.

Außerdem beschrieben ist eine für eine Glykosylhydrolase kodierende Nukleinsäuren, deren Nukleotidsequenz mit der in Seq. 5 zugt mindestens 90 % und ganz besonders bevorzugt zu 100 % übereinstimmt.

Weiterhin beschrieben ist eine für eine Glykosylhydrolase kodierende Nukleinsäure, deren Nukleotidsequenz mit der in Seq. 6 angegebenen Nukleotidsequenz zu mindestens 60 %, zu mindestens 70 %, vorzugsweise mindestens 80 %, insbesondere mindestens 90 %, besonders bevorzugt mindestens 95 % und ganz besonders bevorzugt zu 100 % übereinstimmt.

Ebenfalls beschrieben ist eine Glykosylhydrolase, die durch ein- oder mehrfachen konservativen Aminosäurenaustausch aus einer der oben genannten Glykosylhydrolasen erhältlich ist.

Auch beschrieben ist ein Verfahren zur Identifikation und Gewinnung von neuen Glykosylhydrolasen, daß dadurch gekennzeichnet ist, daß man
a) Metagenom-DNA aus verschiedenen Habitaten gewinnt,
b) die Metagenom-DNA in ein geeignetes, insbesondere bakterielles, Kloniersystem subkloniert und
c) die so erzeugte Genbank einem Screening nach neuen Glykosylhydrolasen unterzieht.

In einer wichtigen Ausführungsform wird die Biodiversität der Metagenom-DNA durch PCR-Typing mittels Glykosylhydrolasespezifischer PCR-Primer und nachfolgende Sequenzierung bestimmt, wodurch eine Schar von Genfragmenten (PCR-Fragmenten) generiert wird, die einen oder mehrere Sequenzräume aufspannen.

Die erfindungsgemäße Glykosylhydrolase wurde mittels des beschriebenen Verfahrens bereitgestellt.

Ein bevorzugtes Habitat zur Gewinnung von Metagenom-DNA ist Erdreich (Boden). Die Bodenproben werden in dem Fachmann bekannter Weise aufbereitet, beispielsweise getrocknet und mechanisch zerkleinert. Die Probe wird dann chemisch aufgeschlossen und die in der Probe vorhandene DNA extrahiert, z. B. wie in der Publikation von Zhou, J., Bruns, M. A., Tiedje, J. M. (1996): "DNA recovery from soils of diverse composition", Appl. Environ. Microbiol., Band 62, S. 316-322, beschrieben.

Vorzugsweise führt man das Screening in Schritt c) durch Selektion auf Glykosylhydrolase-Aktivität durch, beispielsweise durch Selektion solcher Klone, die auf stärkehaltigen Agarplatten einen Klarhof bilden.

Für die Durchführung des Schrittes b) geeignete Kloniersysteme sind dem Fachmann bekannt, beispielsweise *Escherichia coli* und *Streptomyces lividans,* aber auch *Bacillus;* außerdem Hefen und Pilze, z. B. *Saccharomyces, Candida, Pichia, Aspergillus* und andere.

Das Screening in Schritt c) kann jedoch auch mittels Koloniehybrididsierung unter Verwendung von Sonden, die auf den in oben genannten PCR-Fragmenten basieren, durchgeführt werden. Die Sonden können dabei von einem einzigen Genfragment oder aber von mehreren, im Extremfall von einem nicht differenzierten Pool von PCR-Fragmenten abgeleitet sein.

Für eine positive Reaktion ist eine von den Hybridisierungsbedingungen abhängige Mindesthomologie erfoderlich. Das Durchmustern einer Genbank mit einer Sonde, die von einem einzelnen, schon bekannten Enzym-Gen abgeleitet ist, schränkt die Breite der Diversität der aufzufindenden positiven Klone stark ein. Um die tatsächlich in der Genbank vorhandenen, auch weniger stark verwandten Gene auffinden zu können, ist es sinnvoll, eine Sondenschar zu verwenden, die in ihrer Zusammensetzung nicht näher charakterisiert zu sein braucht, aber einen möglichst großen Teil des Sequenzraumes umspannt.

Die Auswahl geeigneter Sonden zur Erfassung eines möglichst großen Sequenzraumes erfolgt, wie in der DE-A-10131441.8-41 beziehungsweise PCT/EP02/06842 beschrieben, auf die hiermit in vollem Umfang Bezug genommen wird.

Anschließend erfolgt gegebenenfalls eine Sequenzanalyse einzelner gefundener Klone, die Überprüfung des Open Reading Frame (ORF) auf Vollständigkeit sowie die Bestimmung der Sequenzhomologie.

Einzelne, überexprimierte Klone können biochemisch charakterisiert und auf ihre Stabilität gegenüber beispielsweise pH, Temperatur und wasch- beziehungsweise reinigungsmitteltypischen Rezepturbestandteilen untersucht werden.

In dem Verfahren einsetzbare Glykosylhydrolase-spezifische PCR-Primer sind solche, die an hochkonservierte Bereiche der für Glykosylhydrolasen kodierenden Nukleinsäuren binden. Die Primerbindungsstellen sollten außerdem einen Bereich flankieren, der für ein Proteinsegment mit möglichst wenig unterschiedlich langen Loops kodiert.

Geeignete Glykosylhydrolase-spezifische PCR-Primer sind insbesondere solche, die an Nukleinsäurebereiche binden, die für jene Regionen der Glykosylhydrolasen kodieren, die in unmittelbarer Nähe um die katalytisch aktiven Reste herum gruppiert sind. So zum Beispiel für die Regionen am C-terminalen Ende des *β-*Faltblatts 3, des *β*-Faltblatts 4 und des *β*-Faltblatts 7.

In dem Verfahren besonders bevorzugt einsetzbare Glykosyl-hydrolase-spezifische PCR-Primer sind solche, die in Tabelle 1 aufgeführt sind, wobei die Primerpaare GEX 24/26 und GEX 23/25 ganz besonders bevorzugt sind.

Mithilfe des Verfahrens wurde eine Zahl von PCR-Fragmenten bereitgestellt, die einen Sequenzraum von Glykosylhydrolasen aufspannen.

Mittels des Primerpaares GEX 24/26 wurden 50 DNA-Sequenzen bereitgestellt, die im Sequenzprotokoll unter den IDs 57 bis 106 aufgeführt sind. Die diesen DNA-Sequenzen entsprechenden Aminosäuresequenzen sind im Sequenzprotokoll unter den IDs 7 bis 56 aufgeführt.

Mittels der Primerpaare GEX 23/25, GEX 36/38 sowie 210-211/25 wurden 99 DNA-Sequenzen bereitgestellt, die im Sequenzprotokoll unter den IDs 206 bis 304 aufgeführt sind. Da die mit den drei Primerkombinationen erzeugten Sequenzen im wesentlichen denselben DNA-Abschnitt überdecken, werden sie im folgenden zusammen behandelt und ggf. vereinfachend mit GEX23/25 bezeichnet. Die diesen DNA-Sequenzen entsprechenden Aminosäuresequenzen sind im Sequenz-protokoll unter den IDs 107 bis 205 aufgeführt.

Durch Einstellung von Sequenz-Alignments der PCR-Fragmente wurden Consensussequenzen gewonnen, die diesen Sequenzraum, beziehungsweise Teilräume davon, repräsentieren.

Ebenfalls beschrieben sind daher Glykosylhydrolasen, die als Vertreter der in den Consensussequenzen A und B dargestellten Sequenzräume beziehungsweise der in den Consensussequenzen D und C dargestellten Subsequenzräume anzusehen sind.

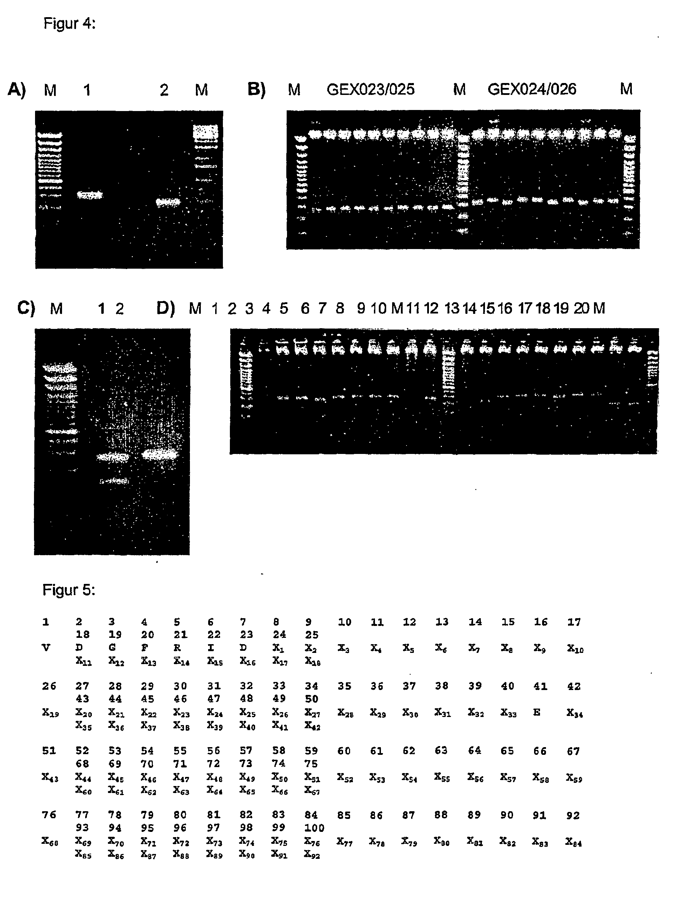

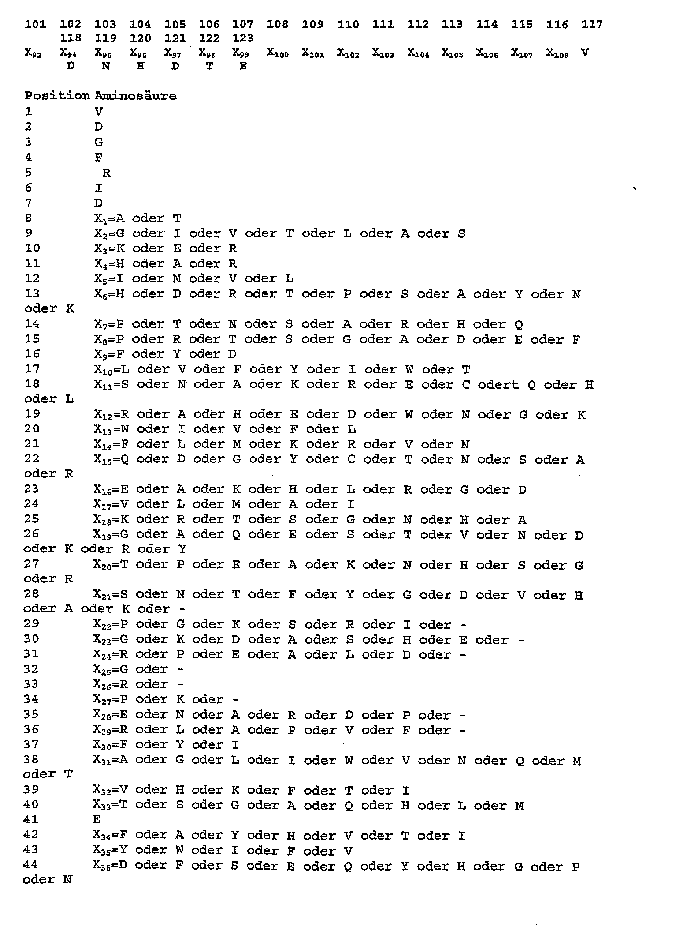

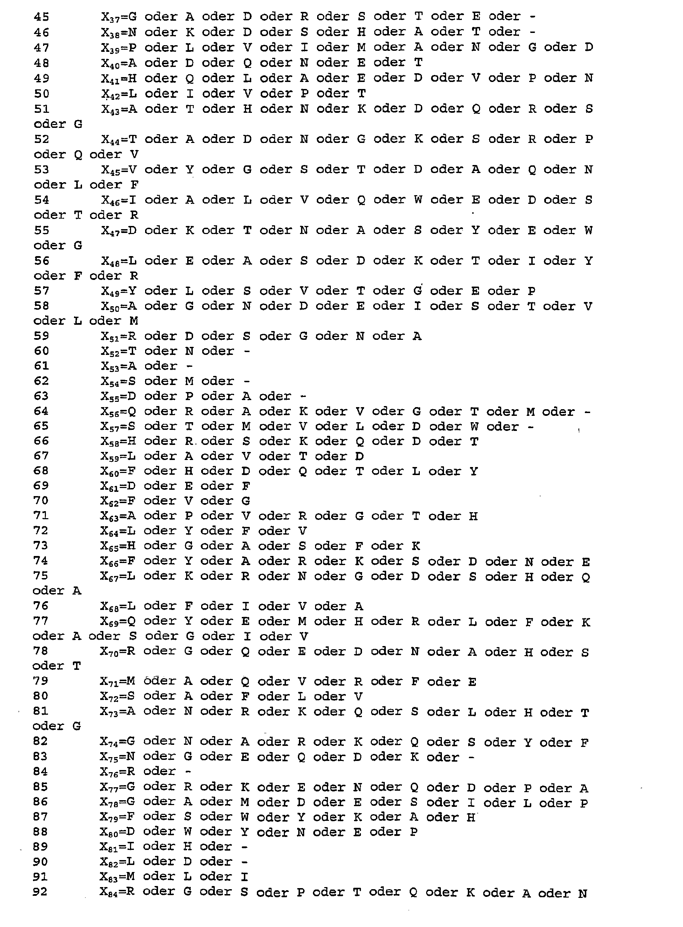

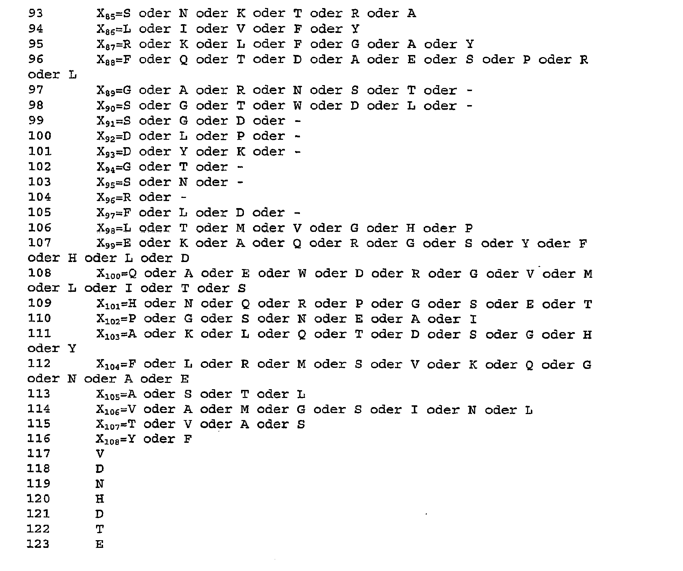

Die Consensussequenzen A, D, B und C sind in den Figuren 5 bis 8 und im Sequenzprotokoll unter SEQ ID NO. 305 bis 308 dargestellt.

Die gezeigten Consensus-Sequenzen verfügen in den Positionen 1-8 und 116-123 (A), 1-8, 114-121 (D), 1-12, 102-109 (B) und 1-12, 72-78 (C) nur Ober eine sehr geringe Varianz. Diese ergibt sich aus der weiter unten und in den Beispielen beschriebenen, PCR-basierten Methode, die zur Identifizierung dieser Sequenz eingesetzt worden ist: Diese Teilsequenzen entsprechen den DNA-Bereichen, an die die zur Amplifizierung eingesetzten Primer gebunden haben. Je nach Stringenz der Bedingungen, unter denen diese Bindung stattfindet, können auch abweichende Nukleotidfolgen gebunden und amplifiziert werden: die als Produkt erhaltene DNA stimmt somit bei vergleichsweise niedriger Selektivität an diesen Stellen nicht völlig mit der Matrize überein. Aus diesem Grund richtet sich das Schutzbegehren insbesondere auf den inneren Teilbereich der Consensussequenzen, der den Positionen zwischen den oben angegebenen Sequenzabschnitten entspricht.

Ein Vergleich der von den PCR-Fragmenten abgeleiteten 149 Aminosäuresequenzen mit den Eintragungen in der Enzym-Datenbank GenBank (National Center for Biotechnology Information NCBI, National Institutes of Health, Bethesda, MD, USA) bestätigt, daß es sich bei allen um Teilsequenzen von Glykosylhydrolasen handelt.

Das Ergebnis dieses Vergleichs ist in Tabelle 2 dargestellt. Dort werden jeweils auch die zu den gefundenen Teilsequenzen nächstähnlichen Datenbankeinträge sowie der Grad der Homologie zwischen diesen beiden angegeben. Der Vergleich mit den in der Datenbank hinterlegten Sequenzen zeigt, daß die nächstähnlichen Proteine Homologiewerte von 95,1 % (Primerpaar 23/25) Identität, beziehungsweise 91 % (Primerpaar 24/26) aufweisen.

Diese Aminosäuresequenzen 107 bis 205 definieren einen Sequenzraum, der einen Teilraum des durch die Consensus-Sequenzen mit den SEQ ID NO. 307 und 308 beschriebenen Sequenzraums darstellt.

Anders formuliert, werden alle Glykosylhydrolasen beschrieben deren Aminosäuresequenzen in einem Teilbereich mit den Consensus-Sequenzen mit den SEQ ID NO. 307 und 308 homologisiert werden können und In diesem Bereich nur solche Aminosäuren aufweisen, die an der entsprechenden Position in einer dieser zwei Sequenzen liegen. In diesem Sinne können sie in jeder Position dieses Teils auf eine dieser Sequenzen zurückgeführt werden.

Beschrieben sind daher Glykosylhydrolasen, deren Aminosäuresequenzen einen Teil enthalten, der mit einer dieser Consensus-Sequenzen mit den SEQ ID NO. 307 (B) und 308 (C) zu 96 % und zunehmend bevorzugt zu 97 %, zu 98 %, zu 99 %, zu 99,25%, zu 99,5%, zu 99,75% oder besonders bevorzugt zu 100% identisch ist oder in jeder homologen Position unmittelbar auf eine dieser Sequenzen zurückgeführt werden kann. Dies gilt, wie oben dargelegt, insbesondere über den Teilbereich, der den Positionen 13 bis 101 der Consensussequenz B, oder 13 bis 71 der Consensussequenz C entspricht.

Zudem beschrieben sind daher Glykosylhydrolasen, deren Aminosäuresequenzen einen Teil enthalten, der mit einer dieser Consensus-Sequenzen mit den SEQ ID NO. 305 (A) und 306 (D) zu 92 % und zunehmend bevorzugt zu 93 %, zu 94 %, zu 95 %, zu 96 %, zu 97 %, zu 98 %, zu 99 % oder besonders bevorzugt zu 100% identisch ist oder in jeder homologen Position unmittelbar auf eine dieser Sequenzen zurückgeführt werden kann. Dies gilt, wie oben dargelegt, insbesondere über den Teilbereich, der den Positionen 9 bis 115 der Consensussequenz A, oder 9 bis 113 der Consensussequenz D entspricht.

Beschrieben ist eine Glykosylhydrolase, deren Aminosäuresequenz einen Teil enthält, der mit einer der unter SEQ ID NO. 7 bis 56 angegebenen Aminosäuresequenzen zu mindestens 92 %, insbesondere zu mindestens 94 %, vorzugsweise zu mindestens 96 %, bevorzugt zu mindestens 99 %, besonders bevorzugt zu 100 % identisch ist, insbesondere über den Teilbereich, der den Positionen 9 bis 113 gemäß der Consensus-Sequenz von SEQ ID NO.306 entspricht.

Weiterhin beschrieben ist eine Glykosylhydrolase, deren Aminosäuresequenz einen Teil enthält, der mit einer der unter SEQ ID NO. 7 bis 56 angegebenen Aminosäuresequenzen zu mindestens 92 %, insbesondere zu mindestens 94 %, vorzugsweise zu mindestens 96 %, bevorzugt zu mindestens 99 %, besonders bevorzugt zu 100 % identisch ist, insbesondere über den Teilbereich, der den Positionen 9 bis 115 gemäß der Consensus-Sequenz von SEQ ID NO.305 entspricht.

Ebenfalls beschrieben ist eine Glykosylhydrolase, deren Aminosäuresequenz einen Teil enthält, der mit einer der unter SEQ ID NO. 107 bis 205 angegebenen Aminosäuresequenzen zu 96 %, insbesondere zu mindestens 97 %, vorzugsweise zu mindestens 98 %, bevorzugt zu mindestens 99 %, besonders bevorzugt zu 100 % identisch ist, insbesondere über den Teilbereich, der den Positionen 13 bis 71 gemäß der Consensus-Sequenz von SEQ ID NO.308 entspricht.

Auch beschrieben ist eine Glykosylhydrolase, deren Aminosäuresequenz einen Teil enthält, der mit einer der unter SEQ ID NO. 107 bis 205 angegebenen Aminosäuresequenzen zu 96 %, insbesondere zu mindestens 97 %, vorzugsweise zu mindestens 98 %, bevorzugt zu mindestens 99 %, besonders bevorzugt zu 100 % identisch ist, insbesondere über den Teilbereich, der den Positionen 13 bis 101 gemäß der Consensus-Sequenz von SEQ ID NO.307 entspricht.

Beschrieben ist eine Glykosylhydrolase, die eine Glykosylhydrolase, die durch ein- oder mehrfachen konservativen Aminosäurenaustausch aus einer erfindungsgemäßen Glykosylhydrolase erhältlich ist.

Weiterhin beschrieben ist eine Glykosylhydrolase, die durch Derivatisierung, Fragmentierung, Deletionsmutation oder Insertionsmutation einer Glykosylhydrolase gemäß einem der Ansprüche 1 bis 6, oder 14 bis 21 erhalten werden kann.

Auch beschrieben ist eine Nukleinsäure, kodierend für eine Glykosylhydrolase, deren Aminosäuresequenz einen Teil enthält, der mit der Consensus-Sequenz von SEQ ID NO.308 zu mindestens 96 %, insbesondere zu mindestens 97 %, vorzugsweise zu mindestens 98 %, bevorzugt zu mindestens 99 %, besonders bevorzugt zu 100 % identisch ist, insbesondere über den Teilbereich, der den Positionen 13 bis 71 entspricht.

Ebenfalls beschrieben ist eine Nukleinsäure, kodierend für eine Glykosylhydrolase, deren Aminosäuresequenz einen Teil enthält, der mit der Consensus-Sequenz von SEQ ID NO.307 zu mindestens 96 %, insbesondere zu mindestens 97 %, vorzugsweise zu mindestens 98 %, bevorzugt zu mindestens 99 %, besonders bevorzugt zu 100 % identisch ist, insbesondere über den Teilbereich, der den Positionen 13 bis 101 entspricht.

Beschrieben ist eine Nukleinsäure, kodierend für eine Glykosylhydrolase, deren Aminosäuresequenz einen Teil enthält, der mit der Consensus-Sequenz von SEQ ID NO.305 zu mindestens 92 %, insbesondere zu mindestens 94 %, vorzugsweise zu mindestens 96 %, bevorzugt zu mindestens 99 %, besonders bevorzugt zu 100 % identisch ist, insbesondere über den Teilbereich, der den Positionen 9 bis 115 entspricht.

Beschrieben ist eine Nukleinsäure, kodierend für eine Glykosylhydrolase, deren Aminosäuresequenz einen Teil enthält, der mit der Consensus-Sequenz von SEQ ID NO.306 zu mindestens 92 %, insbesondere zu mindestens 94 %, vorzugsweise zu mindestens 96 %, bevorzugt zu mindestens 99 %, besonders bevorzugt zu 100 % identisch ist, insbesondere über den Teilbereich, der den Positionen 9 bis 113 entspricht.

Ebenfalls beschrieben ist eine Nukleinsäure, kodierend für eine Glykosylhydrolase, deren Aminosäuresequenz einen Teil enthält, der mit einer der unter SEQ ID NO. 7 bis 56 angegebenen Aminosäuresequenzen zu mindestens 92 %, insbesondere zu mindestens 94 %, vorzugsweise zu mindestens 96 %, bevorzugt zu mindestens 99 %, besonders bevorzugt zu 100 % identisch ist, insbesondere über den Teilbereich, der den Positionen 9 bis 113 gemäß der Consensus-Sequenz von SEQ ID NO.306 entspricht.

Weiterhin beschrieben ist eine Nukleinsäure, kodierend für eine Glykosylhydrolase, deren Aminosäuresequenz einen Teil enthält, der mit einer der unter SEQ ID NO. 7 bis 56 angegebenen Aminosäuresequenzen zu mindestens 92 %, insbesondere zu mindestens 94 %, vorzugsweise zu mindestens 96 %, bevorzugt zu mindestens 99 %, besonders bevorzugt zu 100 % identisch ist, insbesondere über den Teilbereich, der den Positionen 9 bis 115 gemäß der Consensus-Sequenz von SEQ ID NO.305 entspricht

Außerdem beschrieben ist eine Nukleinsäure, kodierend für eine Glykosylhydrolase, deren Aminosäuresequenz einen Teil enthält, der mit einer der unter SEQ ID NO. 107 bis 205 angegebenen Aminosäuresequenzen zu 96 %, insbesondere zu mindestens 97 %, vorzugsweise zu mindestens 98 %, bevorzugt zu mindestens 99 %, besonders bevorzugt zu 100 % identisch ist, insbesondere über den Teilbereich, der den Positionen 13 bis 71 gemäß der Consensus-Sequenz von SEQ ID NO.308 entspricht.

Beschrieben ist eine Nukleinsäure, kodierend für eine Glykosylhydrolase, deren Aminosäuresequenz einen Teil enthält, der mit einer der unter SEQ ID NO. 107 bis 205 angegebenen Aminosäuresequenzen zu 96 %, insbesondere zu mindestens 97 %, vorzugsweise zu mindestens 98 %, bevorzugt zu mindestens 99 %, besonders bevorzugt zu 100 % identisch ist, insbesondere über den Teilbereich, der den Positionen 13 bis 101 gemäß der Consensus-Sequenz von SEQ ID NO.307 entspricht.

Ein weiterer spezieller Gegenstand der Erfindung ist eine Nukleinsäure, kodierend für eine erfindungsgemäße Glykosylhydrolase.

Auch beschrieben ist eine für eine Glykosylhydrolase kodierende Nukleinsäure, deren Sequenz mit einer der unter SEQ ID NO. 57 bis 106, oder 206 bis 304 angegebenen Nukleotidsequenz mindestens zu 80 %, vorzugsweise mindestens zu 90 %, besonders bevorzugt zu 100 % identisch ist, vorzugsweise in den Positionen, die nicht durch die PCR-Primersequenzen vorgegeben sind.

Ebenfalls beschrieben ist ein Oligonukleotid, insbesondere ein PCR-Primer, mit einer der in SEQ ID NO. 309 bis 316 angegebenen Sequenzen.

Nukleinsäuren, die für die nach der Consensus-Sequenz möglichen Varianten codieren, können nach allgemein bekannten Methoden hergestellt werden. Beispielsweise in dem "Lexikon der Biochemie", Spektrum Akademischer Verlag, Berlin, 1999 werden in Band 1, S. 267-271 Methoden zur De-novo-Synthese von DNA und in Band 2, S. 227-229, die Polymerasekettenreaktion (PCR) vorgestellt. Die Sequenzen können nach dem allgemein bekannten Codierungssystem für Aminosäuren vorgegeben werden, gegebenenfalls in einer Codon-Usage, die für bestimmte Gattungen charakteristisch ist.

Hierzu können alle gängigen und zweckmäßigen Methoden eingesetzt werden, wie sie beispielsweise aus dem Handbuch Fritsch, Sambrook und Maniatis "Molecular cloning: a laboratory manual", Cold Spring Harbour Laboratory Press, New York, 1989, bekannt sind. Es kann auch eine PCR auch zum Einführen von einzelnen Basenaustauschen in DNA verwendet werden.

Die erfindungsgemäße Glykosylhydrolase kann weiter optimiert werden, beispielsweise durch chemische Modifikation, gerichtete Evolution oder "Sequence-shuffling" der Enzyme oder Enzymfragmente zu neuartigen Proteinen oder Fusionsproteinen (Hybriden, chimären Proteinen). Hierzu bekannte Methoden sind beispielsweise die Methoden zur gerichteten Evolution von Enzymen wie "In vivo homologous recombination" (Zhang, Y.M., Buchholz, F., Muyrers, J.P.P., Stewart, A.F. (1998): "A new logic for DNA engineering using recombination in Escherichia coli", Nat. Genetics, Band 20, S. 123-128), "Staggered extension process StEP" (Zhao, H., Giver, L., Shao, Z., Affholter, J.A., Amold, F.H. (1998): "Molecular evolution by staggered extension process (StEP) in vitro recombination", Nat. Biotechnol., Band 16, S. 258-261), "Random priming recombination" (Shao, Z., Zhao, H., Giver, L., Amold, F.H. (1998): "Random-priming in vitro recombination: an effective tool for directional evolution", Nucleic Acids Res., Band 26, S. 681-683), "DNA shuffling" (Stemmer, W.P.C. (1994): "Rapid evolution of a protein in vitro by DNA shuffling", Nature, Band 370, S. 389-391) und "Molecular breeding" (Ness, J.E., Welch, M., Giver, L., Bueno, M., Cherry, J.R., Borchert, T.V., Stemmer, W.P.C., Minshull, J. (1999): "DNA shuffling of subgenomic sequences of subtilisin", Nat. Biotechnol., Band 17, S. 893-896).

Die erfindungsgemäße Glykosylhydrolase kann vor allem während der Lagerung durch Stabilisatoren beispielsweise vor Denaturierung, Zerfall oder Inaktivierung, etwa durch physikalische Einflüsse, Oxidation oder Proteolyse geschützt werden. Vielfach werden auch sich ergänzende oder gegenseitig steigernde Kombinationen von Stabilisatoren verwendet.

Eine Gruppe von Stabilisatoren sind reversible Proteaseinhibitoren, wie beispielsweise Benzamidin-Hydrochlorid und Leupeptin, Borax, Borsäuren, Boronsäuren, deren Salze oder Ester, Peptidaldehyde oder rein peptidische Inhibitoren wie Ovomucoid oder spezifische Subtilisin-Inhibitoren. Weitere geläufige Enzymstabilisatoren sind Aminoalkohole wie Mono-, Di-, Triethanol- und -propanolamin, aliphatische Carbonsäuren bis zu C₁₂, Dicarbonsäuren, niedere aliphatische Alkohole, v.a. aber Polyole, wie beispielsweise Glycerin, Ethylenglykol, Propylenglykol oder Sorbit. Ebenso werden Calciumsalze verwendet, wie beispielsweise Calciumacetat oder Calcium-Formiat, Magnesiumsalze, verschiedenste Polymere wie beispielsweise Lignin, Cellulose-Ether, Polyamide oder wasserlösliche Vinyl-Copolymere, um die Enzym-Präparation v.a. gegenüber physikalischen Einflüssen oder pH-Wert-Schwankungen zu stabilisieren. Reduktionsmittel und Antioxidantien, wie beispielsweise Natrium-Sulfit oder reduzierende Zucker, erhöhen die Stabilität der Proteine gegenüber oxidativem Zerfall.

Zahlreiche in der Technik etablierte Anwendungsmöglichkeiten für Glykosylhydrolasen werden in Handbüchern, wie beispielsweise dem Buch "industrial enyzmes and their applications" von H. Uhlig, Wiley-Verlag, New York, 1998, ausgeführt. Folgende Zusammenstellung ist nicht als abschließende Aufzählung zu verstehen, sondern stellt eine Auswahl der technischen Verwendungsmöglichkeiten dar.

Eine Ausführungsform dieses Erfindungsgegenstands sind Wasch- und Reinigungsmittel, die dadurch gekennzeichnet sind, daß sie eine erfindungsgemäße Glykosylhydrolase enthalten.

Ein wichtiges Einsatzgebiet für Glykosylhydrolasen und insbesondere für Amylasen ist das als aktive Komponenten in Wasch- oder Reinigungsmitteln zur Reinigung von Textilien oder von festen Oberflächen, wie beispielsweise Geschirr, Fußböden oder Arbeitsgeräten. In diesen Anwendungen dient die amylolytische Aktivität dazu, kohlenhydrathaltige Verunreinigungen und insbesondere solche auf Stärkebasis hydrolytisch aufzulösen oder vom Untergrund anzulösen. Dabei können sie allein, in geeigneten Medien oder auch in Wasch- oder Reinigungsmitteln zur Anwendung gebracht werden. Die hierfür zu wählenden Bedingungen, wie beispielsweise Temperatur, pH-Wert, Ionenstärke, Redox-Verhältnisse oder mechanische Einflüsse sollten für das jeweilige Reinigungsproblem optimiert sein, also in Bezug auf die Anschmutzung und auf das Trägermaterial. So liegen übliche Temperaturen für Wasch- und Reinigungsmittel in Bereichen von 10°C bei manuellen Mitteln über 40°C und 60°C bis hin zu 95° bei maschinellen Mitteln oder bei technischen Anwendungen. Vorzugsweise werden auch die Inhaltsstoffe der betreffenden Mittel aufeinander abgestimmt. Da bei modernen Wasch- und Spülmaschinen die Temperatur meist stufenlos einstellbar ist, sind auch alle Zwischenstufen der Temperatur eingeschlossen. Die übrigen Bedigungen können über die übrigen, unten ausgeführten Bestandteile der Mittel ebenfalls sehr spezifisch auf den jeweiligen Reinigungszweck ausgelegt werden.

Bevorzugte erfindungsgemäße Mittel zeichnen sich dadurch aus, daß unter irgendwelchen der auf diese Weise definierbaren Bedingungen die Wasch- oder Reinigungsleistung dieses Mittels durch Zugabe eines erfindungsgemäßen amylolytischen Enzyms gegenüber der Rezeptur ohne dieses Enzym verbessert wird. Insofern werden bevorzugt solche amylolytischen Proteine in erfindungsgemäße Mittel eingearbeitet, die die Wasch- und/oder Reinigungsleistung eines Waschoder Reinigungsmittels zu verbessern vermögen.

Weiterhin bevorzugte Mittel zeichnen sich dadurch aus, daß die amylolytischen Enzyme und die übrigen Komponenten synergistisch die Beseitigung der Verunreinigungen bewirken. Das geschieht beispielsweise so, daß die Hydrolyseprodukte der amylolytischen Proteine durch andere Bestandteile der Mittel wie etwa Tenside solubilisiert werden. Ein erfindungsgemäßes Protein kann sowohl in Mitteln für Großverbraucher oder technische Anwender als auch in Produkten für den Privatverbraucher Anwendung finden, wobei alle im Stand der Technik etablierten und/oder zweckmäßigen Darreichungsformen auch Ausführungsformen der vorliegenden Erfindung darstellen.

Mit den erfindungsgemäßen Wasch- oder Reinigungsmitteln sind alle denkbaren Reinigungsmittelarten gemeint, sowohl Konzentrate, als auch unverdünnt anzuwendende Mittel; zum Einsatz im kommerziellen Maßstab, in der Waschmaschine oder bei der Hand-Wäsche, beziehungsweise -Reinigung. Dazu gehören beispielsweise Waschmittel für Textilien, Teppiche, oder Naturfasern, für die nach der vorliegenden Erfindung die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder; für solche wird nach der vorliegenden Erfindung die Bezeichnung Reinigungsmittel verwendet. Jegliche Wasch- oder Reinigungsmittelart stellt eine Ausführungsform der vorliegenden Erfindung dar, sofern sie um ein erfindungsgemäßes Protein bereichert ist.

Ausführungsformen der vorliegenden Erfindung umfassen alle nach den Stand der Technik etablierten und/oder alle zweckmäßigen Darreichungsformen der erfindungsgemäßen Mittel. Dazu zählen beispielsweise feste, pulverförmige, flüssige, gelförmige oder pastöse Mittel, gegebenenfalls auch aus mehreren Phasen, komprimiert oder nicht komprimiert; ferner gehören beispielsweise dazu: Extrudate, Granulate, Tabletten oder Pouches, sowohl in Großgebinden als auch portionsweise abgepackt.

Die Glykosylhydrolasen, insbesondere Amylasen, werden in erfindungsgemäßen Mitteln beispielsweise mit einzelnen oder mehreren der folgenden Inhaltsstoffe kombiniert: nichtionische, anionische und/oder kationische Tenside, Bleichmittel, Bleichaktivatoren, Bleichkatalysatoren, Builder und/oder Cobuilder, Lösungsmittel, Verdicker, Sequestrierungsmittel, Elektrolyte, optische Aufheller, Vergrauungsinhibitoren, Korrosionsinhibitoren, insbesondere Silberschutzmittel, Soil-Release-Wirkstoffe, Farbtransfer( oder -Übertragungs) -Inhibitoren, Schauminhibitoren, Abrasivstoffe, Farbstoffe, Duftstoffe, antimikrobielle Wirkstoffe, UV-Schutzmittel, Enzyme wie beispielsweise Proteasen, (gegebenenfalls andere) Amylasen, Lipasen, Cellulasen, Hemicellulasen oder Oxidasen, Stabilisatoren, insbesondere Enzymstabilisatoren, , und andere Komponenten, die aus dem Stand der Technik bekannt sind.

Bevorzugte Mittel sind dadurch gekennzeichnet, daß sie 0,000001 GewichtsProzent bis 5 Gew.-%, und zunehmend bevorzugt 0,00001 bis 4 Gew.-%, 0,0001 bis 3 Gew.-%, 0,001 bis 2 Gew.-% oder 0,01 bis 1 Gew.-% des amylolytischen Proteins oder Derivats enthalten.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann, beziehungsweise lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester.

Eine weitere Klasse von nichtionischen Tensiden, die vorteilhafterweise eingesetzt werden können, sind die Alkylpolyglycoside (APG). Einsetzbare Alkypolyglycoside genügen der allgemeinen Formel RO(G)_{z}, in der R für einen linearen oder verzweigten, insbesondere in 2-Stellung methylverzweigten, gesättigten oder ungesättigten, aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Glycosylierungsgrad z liegt dabei zwischen 1,0 und 4,0, vorzugsweise zwischen 1,0 und 2,0 und insbesondere zwischen 1,1 und 1,4. Bevorzugt eingesetzt werden lineare Alkylpolyglucoside, also Alkylpolyglycoside, in denen der Polyglycosylrest ein Glucoserest und der Alkylrest ein n-Alkylrest ist.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Der Anteil dieser nichtionischen Tenside liegt vorzugsweise nicht über dem der ethoxylierten Fettalkohole, insbesondere bei nicht mehr als der Hälfte davon.

Weitere geeignete Tenside sind Polyhydroxyfettsäureamide der Formel (II), 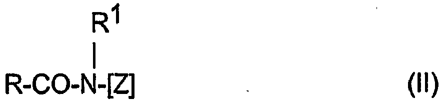 in der RCO für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel (III), 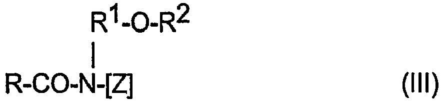 in der R für einen linearen oder verzweigten Alkyl- oder Alkenylrest mit 7 bis 12 Kohlenstoffatomen, R¹ für einen linearen, verzweigten oder cyclischen Alkylrest oder einen Arylrest mit 2 bis 8 Kohlenstoffatomen und R² für einen linearen, verzweigten oder cyclischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes.

[Z] wird vorzugsweise durch reduktive Aminierung eines reduzierenden Zuckers erhalten, beispielsweise Glucose, Fructose, Maltose, Lactose, Galactose, Mannose oder Xylose. Die N-Alkoxy- oder N-Aryloxy-substituierten Verbindungen können beispielsweise durch Umsetzung mit Fettsäuremethylestern in Gegenwart eines Alkoxids als Katalysator in die gewünschten Polyhydroxyfettsäureamide überführt werden.

Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse beziehungsweise Neutralisation gewonnen werden. Ebenso sind auch die Ester von α-Sulfofettsäuren (Estersulfonate), zum Beispiel die α-sulfonierten Methylester der hydrierten Kokos-, Palmkern- oder Talgfettsäuren geeignet.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate sind geeignete Aniontenside.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet. Sie werden in Reinigungsmitteln aufgrund ihres hohen Schaumverhaltens nur in relativ geringen Mengen, beispielsweise in Mengen bis 5 Gew.-%, üblicherweise von 1 bis 5 Gew.%, eingesetzt.

Weitere geeignete Aniontenside sind auch die Salze der Alkylsulfobemsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C₈₋₁₈-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen (Beschreibung siehe unten). Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Als weitere anionische Tenside kommen insbesondere Seifen in Betracht. Geeignet sind gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, zum Beispiel Kokos-, Palmkern- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Die Tenside können in den erfindungsgemäßen Reinigungs- oder Waschmitteln insgesamt in einer Menge von vorzugsweise 5 Gew.-% bis 50 Gew.-%, insbesondere von 8 Gew.-% bis 30 Gew.%, bezogen auf das fertige Mittel, enthalten sein.

Erfindungsgemäße Mittel können Bleichmittel enthalten. Unter den als Bleichmittel dienenden, in Wasser H₂O₂ liefernden Verbindungen haben das Natriumpercarbonat, das Natriumperborattetrahydrat und das Natriumperboratmonohydrat besondere Bedeutung. Weitere brauchbare Bleichmittel sind beispielsweise Peroxopyrophosphate, Citratperhydrate sowie H₂O₂ liefernde persaure Salze oder Persäuren, wie Persulfate beziehungsweise Perschwefelsäure. Brauchbar ist auch das Harnstoffperoxohydrat Percarbamid, das durch die Formel H₂N-CO-NH₂·H₂O₂ beschrieben werden kann. Insbesondere beim Einsatz der Mittel für das Reinigen harter Oberflächen, zum Beispiel beim maschinellen Geschirrspülen, können sie gewünschtenfalls auch Bleichmittel aus der Gruppe der organischen Bleichmittel enthalten, obwohl deren Einsatz prinzipiell auch bei Mitteln für die Textilwäsche möglich ist. Typische organische Bleichmittel sind die Diacylperoxide, wie zum Beispiel Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind die Peroxybenzoesäure und ihre ringsubstituierten Derivate, wie Alkylperoxybenzoesäuren, aber auch Peroxy-α-Naphthoesäure und Magnesium-monoperphthalat, die aliphatischen oder substituiert aliphatischen Peroxysäuren, wie Peroxylaurinsäure, Peroxystearinsäure, ε-Phthalimidoperoxycapronsäure (Phthalimidoperoxyhexansäure, PAP), o-Carboxybenzamidoperoxycapronsäure, N-Nonenylamidoperadipinsäure und N-Nonenylamidopersuccinate, und aliphatische und araliphatische Peroxydicarbonsäuren, wie 1,12-Diperoxycarbonsäure, 1,9-Diperoxyazelainsäure, Diperoxysebacinsäure, Diperoxybrassylsäure, die Diperoxyphthalsäuren, 2-Decyldiperoxybutan-1,4-disäure, N,N-Terephthaloyl-di(6-aminopercapronsäure) können eingesetzt werden.

Der Gehalt der Mittel an Bleichmittel kann 1 bis 40 Gew.-% und insbesondere 10 bis 20 Gew.%, betragen, wobei vorteilhafterweise Perboratmonohydrat oder Percarbonat eingesetzt wird. Eine synergistische Verwendung von Amylase mit Percarbonat oder von Amylase mit Percarbonsäure wird mit den Anmeldungen WO 99/63036, beziehungsweise WO 99/63037 offenbart.

Um beim Waschen bei Temperaturen von 60°C und darunter, und insbesondere bei der Wäschevorbehandlung eine verbesserte Bleichwirkung zu erreichen, können die Mittel auch Bleichaktivatoren enthalten. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glycolurile, insbesondere 1,3,4,6-Tetraacetylglycoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- beziehungsweise iso-NOBS), acylierte Hydroxycarbonsäuren, wie Triethyl-O-acetylcitrat (TEOC), Carbonsäureanhydride, insbesondere Phthalsäureanhydrid, Isatosäureanhydrid und/oder Bernsteinsäureanhydrid, Carbonsäureamide, wie N-Methyldiacetamid, Glycolid, acylierte mehrwertige Alkohole, insbesondere Triacetin, Ethylenglycoldiacetat, Isopropenylacetat, 2,5-Diacetoxy-2,5-dihydrofuran und die aus den deutschen Patentanmeldungen DE 196 16 693 und DE 196 16 767 bekannten Enolester sowie acetyliertes Sorbitol und Mannitol beziehungsweise deren in der europäischen Patentanmeldung EP 0 525 239 beschriebene Mischungen (SORMAN), acylierte Zuckerderivate, insbesondere Pentaacetylglucose (PAG), Pentaacetylfructose, Tetraacetylxylose und Octaacetyllactose sowie acetyliertes, gegebenenfalls N-alkyliertes Glucamin beziehungsweise Gluconolacton, Triazol beziehungsweise Triazolderivate und/oder teilchenförmige Caprolactame und/oder Caprolactamderivate, bevorzugt N-acylierte Lactame, beispielsweise N-Benzoylcaprolactam und N-Acetylcaprolactam, die aus den internationalen Patentanmeldungen WO 94/27970, WO 94/28102, WO 94/28103, WO 95/00626, WO 95/14759 und WO 95/17498 bekannt sind. Die aus der deutschen Patentanmeldung DE 196 16 769 bekannten hydrophil substituierten Acylacetale und die in der deutschen Patentanmeldung DE 196 16 770 sowie der internationalen Patentanmeldung WO 95/14075 beschriebenen Acyllactame werden ebenfalls bevorzugt eingesetzt. Auch die aus der deutschen Patentanmeldung DE 44 43 177 bekannten Kombinationen konventioneller Bleichaktivatoren können eingesetzt werden. Ebenso können Nitrilderivate wie Cyanopyridine, Nitrilquats, zum Beispiel N-Alkylammoniumacetonitrile, und/oder Cyanamidderivate eingesetzt werden. Bevorzugte Bleichaktivatoren sind Natrium-4-(octanoyloxy)-benzolsulfonat, n-Nonanoyl- oder Isononanoyloxybenzolsulfonat (n- beziehungsweise iso-NOBS), Undecenoyloxybenzolsulfonat (UDOBS), Natriumdodecanoyloxybenzolsulfonat (DOBS), Decanoyloxybenzoesäure (DOBA, OBC 10) und/oder Dodecanoyloxybenzolsulfonat (OBS 12), sowie N-Methylmorpholinum-acetonitril (MMA). Derartige Bleichaktivatoren können im üblichen Mengenbereich von 0,01 bis 20 Gew.%, vorzugsweise in Mengen von 0,1 bis 15 Gew.%, insbesondere 1 Gew.-% bis 10 Gew.%, bezogen auf die gesamte Zusammensetzung, enthalten sein.

Zusätzlich zu den konventionellen Bleichaktivatoren oder an deren Stelle können auch sogenannte Bleichkatalysatoren enthalten sein. Bei diesen Stoffen handelt es sich um bleichverstärkende Übergangsmetallsalze beziehungsweise Übergangsmetallkomplexe wie beispielsweise Mn-, Fe-, Co-, Ru - oder Mo-Salenkomplexe oder -carbonylkomplexe. Auch Mn-, Fe-, Co-, Ru-, Mo-, Ti-, V- und Cu-Komplexe mit N-haltigen Tripod-Liganden sowie Co-, Fe-, Cu- und Ru-Aminkomplexe sind als Bleichkatalysatoren geeignet, wobei solche Verbindungen bevorzugt eingesetzt werden, die in der DE 197 09 284 A1 beschrieben sind. Gemäß WO 99/63038 vermögen auch Acetonitril-Derivate und gemäß WO 99/63041 bleichaktivierende Übergangsmetallkomplexverbindungen in Kombination mit Amylasen eine bleichaktivierende Wirkung zu entfalten.

Erfindungsgemäße Mittel enthalten in der Regel einen oder mehrere Builder, insbesondere Zeolithe, Silikate, Carbonate, organische Cobuilder und - wo keine ökologischen Gründe gegen ihren Einsatz sprechen - auch die Phosphate. Letztere sind insbesondere in Reinigungsmitteln für das maschinelle Geschirrspülen bevorzugt einzusetzende Gerüststoffe.

Zu nennen sind hier kristalline, schichtförmige Natriumsilicate der allgemeinen Formel NaMSiₓO₂ₓ₊₁·yH₂O, wobei M Natrium oder Wasserstoff bedeutet, x eine Zahl von 1,6 bis 4, vorzugsweise 1,9 bis 4,0 und y eine Zahl von 0 bis 20 ist und bevorzugte Werte für x 2, 3 oder 4 sind. Derartige kristalline Schichtsilicate werden beispielsweise in der europäischen Patentanmeldung EP 0 164 514 beschrieben. Bevorzugte kristalline Schichtsilicate der angegebenen Formel sind solche, in denen M für Natrium steht und x die Werte 2 oder 3 annimmt. Insbesondere sind sowohl β- als auch δ-Natriumdisilicate Na₂Si₂O₅·yH₂O bevorzugt. Im Handel befinden sich derartige Verbindungen beispielsweise unter der Bezeichnung SKS^{®} (Fa. Clariant). So handelt es sich bei SKS-6^{®} vorwiegend um ein δ-Natriumdisilicat mit der Formel Na₂Si₂O₅·yH₂O, bei SKS-7^{®} vorwiegend um das β-Natriumdisilicat. Durch Reaktion mit Säuren (zum Beispiel Citronensäure oder Kohlensäure) entsteht aus dem δ-Natriumdisilicat Kanemit NaHSi₂O₅·yH₂O, im Handel unter den Bezeichnungen SKS-9^{®} beziehungsweise SKS-10^{®} (Fa. Clariant). Von Vorteil kann es auch sein, chemische Modifikationen dieser Schichtsilicate einzusetzen. So kann beispielsweise die Alkalität der Schichtsilicate geeignet beeinflußt werden. Mit Phosphat beziehungsweise mit Carbonat dotierte Schichtsilicate weisen im Vergleich zu dem δ-Natriumdisilicat veränderte Kristallmorphologien auf, lösen sich schneller und zeigen im Vergleich zu δ-Natriumdisilicat ein erhöhtes Calciumbindevermögen. So sind Schichtsilicate der allgemeinen Summenformel x Na₂O • y SiO₂ • z P₂O₅, in der das Verhältnis x zu y einer Zahl 0,35 bis 0,6, das Verhältnis x zu z einer Zahl von 1,75 bis 1200 und das Verhältnis y zu z einer Zahl von 4 bis 2800 entsprechen, in der Patentanmeldung DE 196 01 063 beschrieben. Die Löslichkeit der Schichtsilicate kann auch erhöht werden, indem besonders feinteilige Schichtsilicate eingesetzt werden. Auch Compounds aus den kristallinen Schichtsilicaten mit anderen Inhaltsstoffen können eingesetzt werden. Dabei sind insbesondere Compounds mit Cellulosederivaten, die Vorteile in der desintegrierenden Wirkung aufweisen und insbesondere in Waschmitteltabletten eingesetzt werden, sowie Compounds mit Polycarboxylaten, zum Beispiel Citronensäure, beziehungsweise polymeren Polycarboxylaten, zum Beispiel Copolymeren der Acrylsäure, zu nennen.

Einsetzbar sind auch amorphe Natriumsilikate mit einem Modul Na₂O : SiO₂ von 1:2 bis 1:3,3, vorzugsweise von 1:2 bis 1:2,8 und insbesondere von 1:2 bis 1:2,6, welche löseverzögert sind und Sekundärwascheigenschaften aufweisen. Die Löseverzögerung gegenüber herkömmlichen amorphen Natriumsilikaten kann dabei auf verschiedene Weise, beispielsweise durch Oberflächenbehandlung, Compoundierung, Kompaktierung/ Verdichtung oder durch Übertrocknung hervorgerufen worden sein. Im Rahmen dieser Erfindung wird unter dem Begriff "amorph" auch "röntgenamorph" verstanden. Dies heißt, daß die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führen, wenn die Silikatpartikel bei Elektronenbeugungsexperimenten verwaschene oder sogar scharfe Beugungsmaxima liefern. Dies ist so zu interpretieren, daß die Produkte mikrokristalline Bereiche der Größe 10 bis einige Hundert nm aufweisen, wobei Werte bis max. 50 nm und insbesondere bis max. 20 nm bevorzugt sind. Insbesondere bevorzugt sind verdichtete/kompaktierte amorphe Silikate, compoundierte amorphe Silikate und übertrocknete röntgenamorphe Silikate.

Ein gegebenenfalls einsetzbarer, feinkristalliner, synthetischer und gebundenes Wasser enthaltender Zeolith ist vorzugsweise Zeolith A und/oder P. Als Zeolith P wird Zeolith MAP^{®} (Handelsprodukt der Firma Crosfield) besonders bevorzugt. Geeignet sind jedoch auch Zeolith X sowie Mischungen aus A, X und/oder P. Kommerziell erhältlich und im Rahmen der vorliegenden Erfindung bevorzugt einsetzbar ist beispielsweise auch ein Co-Kristallisat aus Zeolith X und Zeolith A (ca. 80 Gew.-% Zeolith X), das von der Firma CONDEA Augusta S.p.A. unter dem Markennamen VEGOBOND AX^{®} vertrieben wird und durch die Formel

nNa₂O · (1-n)K₂O · Al₂O₃ · (2 - 2,5)SiO₂ · (3,5 - 5,5) H₂O

beschrieben werden kann. Geeignete Zeolithe weisen eine mittlere Teilchengröße von weniger als 10 µm (Volumenverteilung; Meßmethode: Coulter Counter) auf und enthalten vorzugsweise 18 bis 22 Gew.-%, insbesondere 20 bis 22 Gew.% an gebundenem Wasser.

Selbstverständlich ist auch ein Einsatz der allgemein bekannten Phosphate als Buildersubstanzen möglich, sofern ein derartiger Einsatz nicht aus ökologischen Gründen vermieden werden sollte. Unter der Vielzahl der kommerziell erhältlichen Phosphate haben die Alkalimetallphosphate unter besonderer Bevorzugung von Pentanatrium- beziehungsweise Pentakaliumtriphosphat (Natrium- beziehungsweise Kaliumtripolyphosphat) in der Wasch- und Reinigungsmittel-Industrie die größte Bedeutung.

Alkalimetallphosphate ist dabei die summarische Bezeichnung für die Alkalimetall-(insbesondere Natrium- und Kalium-) -Salze der verschiedenen Phosphorsäuren, bei denen man Metaphosphorsäuren (HPO₃)ₙ und Orthophosphorsäure H₃PO₄ neben höhermolekularen Vertretern unterscheiden kann. Die Phosphate vereinen dabei mehrere Vorteile in sich: Sie wirken als Alkaliträger, verhindern Kalkbeläge auf Maschinenteilen beziehungsweise Kalkinkrustationen in Geweben und tragen überdies zur Reinigungsleistung bei.

Natriumdihydrogenphosphat, NaH₂PO₄, existiert als Dihydrat (Dichte 1,91 gcm⁻³, Schmelzpunkt 60°) und als Monohydrat (Dichte 2,04 gcm⁻³). Beide Salze sind weiße, in Wasser sehr leicht lösliche Pulver, die beim Erhitzen das Kristallwasser verlieren und bei 200°C in das schwach saure Diphosphat (Dinatriumhydrogendiphosphat, Na₂H₂P₂O₇), bei höherer Temperatur in Natiumtrimetaphosphat (Na₃P₃O₉) und Maddrellsches Salz (siehe unten), übergehen. NaH₂PO₄ reagiert sauer; es entsteht, wenn Phosphorsäure mit Natronlauge auf einen pH-Wert von 4,5 eingestellt und die Maische versprüht wird. Kaliumdihydrogenphosphat (primäres oder einbasiges Kaliumphosphat, Kaliumbiphosphat, KDP), KH₂PO₄, ist ein weißes Salz der Dichte 2,33 gcm⁻³, hat einen Schmelzpunkt 253° [Zersetzung unter Bildung von Kaliumpolyphosphat (KPO₃)ₓ] und ist leicht löslich in Wasser.

Dinatriumhydrogenphosphat (sekundäres Natriumphosphat), Na₂HPO₄, ist ein farbloses, sehr leicht wasserlösliches kristallines Salz. Es existiert wasserfrei und mit 2 Mol. (Dichte 2,066 gcm⁻³, Wasserverlust bei 95°), 7 Mol. (Dichte 1,68 gcm⁻³, Schmelzpunkt 48° unter Verlust von 5 H₂O) und 12 Mol. Wasser (Dichte 1,52 gcm⁻³, Schmelzpunkt 35° unter Verlust von 5 H₂O), wird bei 100° wasserfrei und geht bei stärkerem Erhitzen in das Diphosphat Na₄P₂O₇ über. Dinatriumhydrogenphosphat wird durch Neutralisation von Phosphorsäure mit Sodalösung unter Verwendung von Phenolphthalein als Indikator hergestellt. Dikaliumhydrogenphosphat (sekundäres od. zweibasiges Kaliumphosphat), K₂HPO₄, ist ein amorphes, weißes Salz, das in Wasser leicht löslich ist.

Trinatriumphosphat, tertiäres Natriumphosphat, Na₃PO₄, sind farblose Kristalle, die als Dodecahydrat eine Dichte von 1,62 gcm⁻³ und einen Schmelzpunkt von 73-76°C (Zersetzung), als Decahydrat (entsprechend 19-20% P₂O₅) einen Schmelzpunkt von 100°C und in wasserfreier Form (entsprechend 39-40% P₂O₅) eine Dichte von 2,536 gcm⁻³ aufweisen. Trinatriumphosphat ist in Wasser unter alkalischer Reaktion leicht löslich und wird durch Eindampfen einer Lösung aus genau 1 Mol Dinatriumphosphat und 1 Mol NaOH hergestellt. Trikaliumphosphat (tertiäres oder dreibasiges Kaliumphosphat), K₃PO₄, ist ein weißes, zerfließliches, körniges Pulver der Dichte 2,56 gcm⁻³, hat einen Schmelzpunkt von 1340° und ist in Wasser mit alkalischer Reaktion leicht löslich. Es entsteht zum Beispiel beim Erhitzen von Thomasschlacke mit Kohle und Kaliumsulfat. Trotz des höheren Preises werden in der Reinigungsmittel-Industrie die leichter löslichen, daher hochwirksamen, Kaliumphosphate gegenüber entsprechenden Natrium-Verbindungen vielfach bevorzugt.

Tetranatriumdiphosphat (Natriumpyrophosphat), Na₄P₂O₇, existiert in wasserfreier Form (Dichte 2,534 gcm⁻³, Schmelzpunkt 988°, auch 880° angegeben) und als Decahydrat (Dichte 1,815-1,836 gcm⁻³, Schmelzpunkt 94° unter Wasserverlust). Beide Substanzen sind farblose, in Wasser mit alkalischer Reaktion lösliche Kristalle. Na₄P₂O₇ entsteht beim Erhitzen von Dinatriumphosphat auf >200°C oder indem man Phosphorsäure mit Soda im stöchiometrischem Verhältnis umsetzt und die Lösung durch Versprühen entwässert. Das Decahydrat komplexiert Schwermetall-Salze und Härtebildner und verringert daher die Härte des Wassers. Kaliumdiphosphat (Kaliumpyrophosphat), K₄P₂O₇, existiert in Form des Trihydrats und stellt ein farbloses, hygroskopisches Pulver mit der Dichte 2,33 gcm⁻³ dar, das in Wasser löslich ist, wobei der pH-Wert der 1 %igen Lösung bei 25° 10,4 beträgt.

Durch Kondensation des NaH₂PO₄ beziehungsweise des KH₂PO₄ entstehen höhermolekulare Natrium- und Kaliumphosphate, bei denen man cyclische Vertreter, die Natrium- beziehungsweise Kaliummetaphosphate und kettenförmige Typen, die Natrium- beziehungsweise Kaliumpolyphosphate, unterscheiden kann. Insbesondere für letztere sind eine Vielzahl von Bezeichnungen in Gebrauch: Schmelzoder Glühphosphate, Grahamsches Salz, Kurrolsches und Maddrellsches Salz. Alle höheren Natrium- und Kaliumphosphate werden gemeinsam als kondensierte Phosphate bezeichnet.

Das technisch wichtige Pentanatriumtriphosphat, Na₅P₃O₁₀ (Natriumtripolyphosphat), ist ein wasserfrei oder mit 6 H₂O kristallisierendes, nicht hygroskopisches, weißes, wasserlösliches Salz der allgemeinen Formel NaO-[P(O)(ONa)-O]ₙ-Na mit n=3. In 100 g Wasser lösen sich bei Zimmertemperatur etwa 17 g, bei 60° ca. 20 g, bei 100° rund 32 g des kristallwasserfreien Salzes; nach zweistündigem Erhitzen der Lösung auf 100° entstehen durch Hydrolyse etwa 8% Orthophosphat und 15% Diphosphat. Bei der Herstellung von Pentanatriumtriphosphat wird Phosphorsäure mit Sodalösung oder Natronlauge im stöchiometrischen Verhältnis zur Reaktion gebracht und die Lösung durch Versprühen entwässert. Ähnlich wie Grahamsches Salz und Natriumdiphosphat löst Pentanatriumtriphosphat viele unlösliche Metall-Verbindungen (auch Kalkseifen usw.). Pentakaliumtriphosphat, K₅P₃O₁₀ (Kaliumtripolyphosphat), kommt beispielsweise in Form einer 50 Gew.-%-igen Lösung (> 23% P₂O₅, 25% K₂O) in den Handel. Die Kaliumpolyphosphate finden in der Wasch- und Reinigungsmittel-Industrie breite Verwendung. Weiter existieren auch Natriumkaliumtripolyphosphate, welche ebenfalls im Rahmen der vorliegenden Erfindung einsetzbar sind. Diese entstehen beispielsweise, wenn man Natriumtrimetaphosphat mit KOH hydrolysiert:

(NaPO₃)₃ + 2 KOH → Na₃K₂P₃O₁₀ + H₂O

Diese sind erfindungsgemäß genau wie Natriumtripolyphosphat, Kaliumtripolyphosphat oder Mischungen aus diesen beiden einsetzbar; auch Mischungen aus Natriumtripolyphosphat und Natriumkaliumtripolyphosphat oder Mischungen aus Kaliumtripolyphosphat und Natriumkaliumtripolyphosphat oder Gemische aus Natriumtripolyphosphat und Kaliumtripolyphosphat und Natriumkaliumtripolyphosphat sind erfindungsgemäß einsetzbar.

Als organische Cobuilder können in den erfindungsgemäßen Wasch- und Reinigungsmitteln insbesondere Polycarboxylate oder Polycarbonsäuren, polymere Polycarboxylate, Polyasparaginsäure, Polyacetale, gegebenenfalls oxidierte Dextrine, weitere organische Cobuilder (siehe unten) sowie Phosphonate eingesetzt werden. Diese Stoffklassen werden nachfolgend beschrieben.

Brauchbare organische Gerüstsubstanzen sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu vermeiden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen.

Auch die Säuren an sich können eingesetzt werden. Sie besitzen neben ihrer Builderwirkung typischerweise auch die Eigenschaft einer Säuerungskomponente und dienen somit auch zur Einstellung eines niedrigeren und milderen pH-Wertes von Wasch- oder Reinigungsmitteln, sofern nicht der sich durch die Mischung der übrigen Komponenten ergebende pH-Wert gewünscht ist. Insbesondere sind hierbei system- unbd umweltverträgliche Säuren wie Citronensäure, Essigsäure, Weinsäure, Äpfelsäure, Milchsäure, Glykolsäure, Bernsteinsäure, Glutarsäure, Adipinsäure, Gluconsäure und beliebige Mischungen aus diesen zu nennen. Aber auch Mineralsäuren, insbesondere Schwefelsäure oder Basen, insbesondere Ammonium- oder Alkalihydroxide können als pH-Regulatoren dienen. Derartige Regulatoren sind in den erfindungemäßen Mitteln in Mengen von vorzugsweise nicht über 20 Gew.-%, insbesondere von 1,2 Gew.-% bis 17 Gew.-%, enthalten.

Als Builder sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70 000 g/mol.

Bei den für polymere Polycarboxylate angegebenen Molmassen handelt es sich im Sinne dieser Schrift um gewichtsmittlere Molmassen M_{w} der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Geeignete Polymere sind insbesondere Polyacrylate, die bevorzugt eine Molekülmasse von 2 000 bis 20 000 g/mol aufweisen. Aufgrund ihrer überlegenen Löslichkeit können aus dieser Gruppe wiederum die kurzkettigen Polyacrylate, die Molmassen von 2 000 bis 10 000 g/mol, und besonders bevorzugt von 3 000 bis 5 000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.% Acrylsäure und 50 bis 10 Gew.% Maleinsäure enthalten. Ihre relative Molekülmasse, bezogen auf freie Säuren, beträgt im allgemeinen 2 000 bis 70 000 g/mol, vorzugsweise 20 000 bis 50 000 g/mol und insbesondere 30 000 bis 40 000 g/mol. Die (co-) polymeren Polycarboxylate können entweder als Pulver oder als wässerige Lösung eingesetzt werden. Der Gehalt der Mittel an (co-)polymeren Polycarboxylaten kann von 0,5 bis 20 Gew.-%, insbesondere 1 bis 10 Gew.%, betragen.

Zur Verbesserung der Wasserlöslichkeit können die Polymere auch Allylsulfonsäuren, wie beispielsweise Allyloxybenzolsulfonsäure und Methallylsulfonsäure, als Monomer enthalten.

Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol beziehungsweise Vinylalkohol-Derivate oder die als Monomere Salze der Acrylsäure und der 2-Alkylallylsulfonsäure sowie Zucker-Derivate enthalten.

Weitere bevorzugte Copolymere sind solche, die als Monomere vorzugsweise Acrolein und Acrylsäure/Acrylsäuresalze beziehungsweise Acrolein und Vinylacetat aufweisen.

Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren beziehungsweise deren Salze und Derivate.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere beziehungsweise Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500 000 g/mol. Dabei ist ein Polysaccharid mit einem

Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose ist, welche ein DE von 100 besitzt. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2 000 bis 30 000 g/mol.

Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Besonders bevorzugte organische Builder für erfindungsgemäße Mittel sind oxidierte Stärken, beziehungsweise deren Derivate aus den Anmeldungen EP 472 042, WO 97/25399, und EP 755 944.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate. Geeignete Einsatzmengen liegen in zeolithhaltigen und/oder silicathaltigen Formulierungen zwischen 3 und 15 Gew.-%.

Weitere brauchbare organische Cobuilder sind beispielsweise acetylierte Hydroxycarbonsäuren beziehungsweise deren Salze, welche gegebenenfalls auch in Lactonform vorliegen können und welche mindestens 4 Kohlenstoffatome und mindestens eine Hydroxygruppe sowie maximal zwei Säuregruppen enthalten.

Eine weitere Substanzklasse mit Cobuildereigenschaften stellen die Phosphonate dar. Dabei handelt es sich insbesondere um Hydroxyalkan- beziehungsweise Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, zum Beispiel als Hexanatriumsalz der EDTMP beziehungsweise als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Darüberhinaus können alle Verbindungen, die in der Lage sind, Komplexe mit Erdalkaliionen auszubilden, als Cobuilder eingesetzt werden.

Buildersubstanzen können in den erfindungsgemäßen Mitteln gegebenenfalls in Mengen bis zu 90 Gew.% enthalten sein. Sie sind vorzugsweise in Mengen bis zu 75 Gew.% enthalten. Erfindungsgemäße Waschmittel weisen Buildergehalte von insbesondere 5 Gew.% bis 50 Gew.% auf. In erfindungsgemäßen Mitteln für die Reinigung harter Oberflächen, insbesondere zur maschinellen Reinigung von Geschirr, beträgt der Gehalt an Buildersubstanzen insbesondere 5 Gew.% bis 88 Gew.-%, wobei in derartigen Mitteln vorzugsweise keine wasserunlöslichen Buildermaterialien eingesetzt werden. In einer bevorzugten Ausführungsform erfindungsgemäßer Mittel zur insbesondere maschinellen Reinigung von Geschirr sind 20 Gew.-% bis 40 Gew.-% wasserlöslicher organischer Builder, insbesondere Alkalicitrat, 5 Gew.-% bis 15 Gew.-% Alkalicarbonat und 20 Gew.-% bis 40 Gew.-% Alkalidisilikat enthalten.

Lösungsmittel, die in den flüssigen bis gelförmigen Zusammensetzungen von Wasch- und Reinigungsmitteln eingesetzt werden können, stammen beispielsweise aus der Gruppe ein- oder mehrwertigen Alkohole, Alkanolamine oder Glycolether, sofern sie im angegebenen Konzentrationsbereich mit Wasser mischbar sind. Vorzugsweise werden die Lösungsmittel ausgewählt aus Ethanol, n- oder i-Propanol, Butanolen, Ethylenglykolmethylether, Ethylenglykolethylether, Ethylenglykolpropylether, Ethylenglykolmono-n-butylether, Diethylenglykol-methylether, Diethylenglykolethylether, Propylenglykolmethyl-, -ethyl- oder -propyl-ether, Dipropylenglykolmonomethyl-, oder -ethylether, Di-isopropylenglykolmonomethyl-, oder -ethylether, Methoxy-, Ethoxy- oder Butoxytriglykol, 1-Butoxyethoxy-2-propanol, 3-Methyl-3-methoxybutanol, Propylen-glykol-t-butylether sowie Mischungen dieser Lösungsmittel.

Lösungsmittel können in den erfindungsgemäßen flüssigen bis gelförmigen Wasch- und Reinigungsmitteln in Mengen zwischen 0,1 und 20 Gew.%, bevorzugt aber unter 15 Gew.-% und insbesondere unterhalb von 10 Gew.-% eingesetzt werden.

Zur Einstellung der Viskosität können erfindungsgemäßen Zusammensetzungen ein oder mehrere Verdicker, beziehungsweise Verdickungssysteme zugesetzt werden. Diese hochmolekularen Stoffe, die auch Quell(ungs)mittel genannt werden, saugen meist die Flüssigkeiten auf und quellen dabei auf, um schließlich in zähflüssige echte oder kolloide Lösungen überzugehen.

Geeignete Verdicker sind anorganische oder polymere organische Verbindungen. Zu den anorganischen Verdickem zählen beispielsweise Polykieselsäuren, Ton-mineralien wie Montmorillonite, Zeolithe, Kieselsäuem und Bentonite. Die organischen Verdicker stammen aus den Gruppen der natürlichen Polymere, der abgewandelten natürlichen Polymere und der vollsynthetischen Polymere. Solche aus der Natur stammenden Polymere sind beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine und Casein. Abgewandelte Naturstoffe, die als Verdicker verwendet werden, stammen vor allem aus der Gruppe der modifizierten Stärken und Cellulosen. Beispielhaft seien hier Carboxymethylcellulose und andere Celluloseether, Hydroxyethyl- und -propylcellulose sowie Kernmehlether genannt. Vollsynthetische Verdicker sind Polymere wie Polyacryl- und Polymethacryl-Verbindungen, Vinylpolymere, Polycarbonsäuren, Polyether, Polyimine, Polyamide und Polyurethane.

Die Verdicker können in einer Menge bis zu 5 Gew.-%, vorzugsweise von 0,05 bis 2 Gew.-%, und besonders bevorzugt von 0,1 bis 1,5 Gew.-%, bezogen auf die fertige Zusammensetzung, enthalten sein.

Das erfindungsgemäße Wasch- oder Reinigungsmittel kann gegebenenfalls als weitere übliche Inhaltsstoffe Sequestrierungsmittel, Elektrolyte und weitere Hilfsstoffe enthalten.

Erfindungsgemäße Textilwaschmittel können als optische Aufheller Derivate der Diaminostilbendisulfonsäure beziehungsweise deren Alkalimetallsalze enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten optischen Aufheller können verwendet werden.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt werden Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf die Mittel, eingesetzt.

Um einen Silberkorrosionsschutz zu bewirken, können in erfindungsgemäßen Reinigungsmitteln für Geschirr Silberkorrosionsinhibitoren eingesetzt werden. Solche sind aus dem Stand der Technik bekannt, beispielsweise Benzotriazole, Eisen(III)-chlorid oder CoSO₄. Wie beispielsweise aus der europäischen Patentschrift EP 0 736 084 B1 bekannt ist, sind für die gemeinsame Verwendung mit Enzymen besonders geeignete Silberkorrosionsinhibitoren Mangan-, Titan-, Zirkonium-, Hafnium-, Vanadium-, Cobalt- oder Cersalze und/oder -komplexe, in denen die genannten Metalle in einer der Oxidationsstufen II, III, IV, V oder VI vorliegen. Beispiele für derartige Verbindungen sind MnSO₄, V₂O₅, V₂O₄, VO₂, TiOSO₄, K₂TiF₆, K₂ZrF₆, Co(NO₃)₂, Co(NO₃)₃, sowie deren Gemische.

"Soil-Release"-Wirkstoffe oder "Soil-Repellents" sind zumeist Polymere, die bei der Verwendung in einem Waschmittel der Wäschefaser schmutzabstoßende Eigenschaften verleihen und/oder das Schmutzablösevermögen der übrigen Waschmittelbestandteile unterstützen. Ein vergleichbarer Effekt kann auch bei deren Einsatz in Reinigungsmitteln für harte Oberflächen beobachtet werden.

Besonders wirksame und seit langer Zeit bekannte Soil-Release-Wirkstoffe sind Copolyester mit Dicarbonsäure-, Alkylenglykol- und Polyalkylenglykoleinheiten. Beispiele dafür sind Copolymere oder Mischpolymere aus Polyethylenterephthalat und Polyoxyethylenglykol (DT 16 17 141, beziehungsweise DT 22 00 911). In der deutschen Offenlegungsschrift DT 22 53 063 sind saure Mittel genannt, die unter anderem ein Copolymer aus einer dibasigen Carbonsäure und einem Alkylenoder Cycloalkylenpolyglykol enthalten. Polymere aus Ethylenterephthalat und Polyethylenoxid-terephthalat und deren Einsatz in Waschmitteln sind in den deutschen Schriften DE 28 57 292 und DE 33 24 258 und der Europäischen Patentschrift EP 0 253 567 beschrieben. Das europäische Patent EP 066 944 betrifft Mittel, die einen Copolyester aus Ethylenglykol, Polyethylenglykol, aromatischer Dicarbonsäure und sulfonierter aromatischer Dicarbonsäure in bestimmten Molverhältnissen enthalten. Aus dem europäischen Patent EP 0 185 427 sind Methyloder Ethylgruppen-endverschlossene Polyester mit Ethylen- und/oder Propylenterephthalat- und Polyethylenoxid-terephthalat-Einheiten und Waschmitel, die derartiges Soil-release-Polymer enthalten, bekannt. Das europäische Patent EP 0 241 984 betrifft einen Polyester, der neben Oxyethylen-Gruppen und Terephthalsäureeinheiten auch substituierte Ethyleneinheiten sowie Glycerineinheiten enthält. Aus dem europäischen Patent EP 0 241 985 sind Polyester bekannt, die neben Oxyethylen-Gruppen und Terephthalsäureeinheiten 1,2-Propylen-, 1,2-Butylen- und/oder 3-Methoxy-1,2-propylengruppen sowie Glycerineinheiten enthalten und mit C₁- bis C₄-Alkylgruppen endgruppenverschlossen sind. Aus der europäischen Patentanmeldung EP 0 272 033 sind zumindest anteilig durch C₁₋₄-Alkyl- oder Acylreste endgruppenverschlossene Polyester mit Polypropylenterephthalat- und Polyoxyethylenterephthalat-Einheiten bekannt. Das europäische Patent EP 0 274 907 beschreibt sulfoethyl-endgruppenverschlossene terephthalathaltige Soil-release-Polyester. Gemäß der europäischen Patentanmeldung EP 0 357 280 werden durch Sulfonierung ungesättigter Endgruppen Soil-Release-Polyester mit Terephthalat-, Alkylenglykol- und Poly-C₂₋₄-Glykol-Einheiten hergestellt. Die internationale Patentanmeldung WO 95/32232 betrifft saure, aromatische schmutzablösevermögende Polyester. Aus der internationalen Patentanmeldung WO 97/31085 sind nicht polymere soil-repellent-Wirkstoffe für Materialien aus Baumwolle mit mehreren funktionellen Einheiten bekannt: Eine erste Einheit, die beispielsweise kationisch sein kann, ist zur Adsorption auf die Baumwolloberfläche durch elektrostatische Wechselwirkung befähigt, und eine zweite Einheit, die hydrophob ausgebildet ist, ist verantwortlich für das Verbleiben des Wirkstoffs an der Wasser/ Baumwolle-Grenzfläche.

Zu den für den Einsatz in erfindungsgemäßen Textilwaschmitteln in Frage kommenden Farbübertragungsinhibitoren gehören insbesondere Polyvinylpyrrolidone, Polyvinylimidazole, polymere N-Oxide wie Poly-(vinylpyridin-N-oxid) und Copolymere von Vinylpyrrolidon mit Vinylimidazol.

Beim Einsatz in maschinellen Reinigungsverfahren kann es von Vorteil sein, den Mitteln Schauminhibitoren zuzusetzen. Als Schauminhibitoren eignen sich beispielsweise Seifen natürlicher oder synthetischer Herkunft, die einen hohen Anteil an C₁₈-C₂₄-Fettsäuren aufweisen. Geeignete nichttensidartige Schauminhibitoren sind beispielsweise Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure oder Bistearylethylendiamid. Mit Vorteilen werden auch Gemische aus verschiedenen Schauminhibitoren verwendet, zum Beispiel solche aus Silikonen, Paraffinen oder Wachsen. Vorzugsweise sind die Schauminhibitoren, insbesondere Silikonund/oder Paraffin-haltige Schauminhibitoren, an eine granulare, in Wasser lösliche, beziehungsweise dispergierbare Trägersubstanz gebunden. Insbesondere sind dabei Mischungen aus Paraffinen und Bistearylethylendiamiden bevorzugt.

Ein erfindungsgemäßes Reinigungsmittel für harte Oberflächen kann darüber hinaus abrasiv wirkende Bestandteile, insbesondere aus der Gruppe umfassend Quarzmehle, Holzmehle, Kunststoffmehle, Kreiden und Mikroglaskugeln sowie deren Gemische, enthalten. Abrasivstoffe sind in den erfindungsgemäßen Reinigungsmitteln vorzugsweise nicht über 20 Gew.-%, insbesondere von 5 Gew.-% bis 15 Gew.-%, enthalten.

Farb- und Duftstoffe werden Wasch- und Reinigungsmitteln zugesetzt, um den ästhetischen Eindruck der Produkte zu verbessern und dem Verbraucher neben der Wasch- und Reinigungsleistung ein visuell und sensorisch "typisches und unverwechselbares" Produkt zur Verfügung zu stellen. Als Parfümöle beziehungsweise Duftstoffe können einzelne Riechstoffverbindungen, zum Beispiel die synthetischen Produkte vom Typ der Ester, Ether, Aldehyde, Ketone, Alkohole und Kohlenwasserstoffe verwendet werden. Riechstoffverbindungen vom Typ der Ester sind zum Beispiel Benzylacetat, Phenoxyethylisobutyrat, p-tert.-Butylcyclohexylacetat, Linalylacetat, Dimethylbenzyl-carbinylacetat, Phenylethylacetat, Linalylbenzoat, Benzylformiat, Ethylmethylphenyl-glycinat, Allylcyclohexylpropionat, Styrallylpropionat und Benzylsalicylat. Zu den Ethern zählen beispielsweise Benzylethylether, zu den Aldehyden zum Beispiel die linearen Alkanale mit 8-18 C-Atomen, Citral, Citronellal, Citronellyloxyacetaldehyd, Cyclamenaldehyd, Hydroxycitronellal, Lilial und Bourgeonal, zu den Ketonen zum Beispiel die Jonone, α-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, zum Beispiel Pine-, Citrus-, Jasmin-, Patchouly-, Rosen- oder Ylang-Ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl. Üblicherweise liegt der Gehalt von Wasch- und Reinigungsmitteln an Farbstoffen unter 0,01 Gew.-%, während Duftstoffe bis zu 2 Gew.-% der gesamten Formulierung ausmachen können.

Die Duftstoffe können direkt in die Wasch- und Reinigungsmittel eingearbeitet werden, es kann aber auch vorteilhaft sein, die Duftstoffe auf Träger aufzubringen, die die Haftung des Parfüms auf dem Reinigungsgut verstärken und durch eine langsamere Duftfreisetzung für langanhaltenden Duft, insbesondere von behandelten Textilien sorgen. Als solche Trägermaterialien haben sich beispielsweise Cyclodextrine bewährt, wobei die Cyclodextrin-Parfüm-Komplexe zusätzlich noch mit weiteren Hilfsstoffen beschichtet werden können. Ein weiter bevorzugter Träger für Duftstoffe ist der beschriebene Zeolith X, der anstelle von oder in Mischung mit Tensiden auch Duftstoffe aufnehmen kann. Bevorzugt sind daher Wasch- und Reinigungsmittel, die den beschriebenen Zeolith X und Duftstoffe, die vorzugsweise zumindest teilweise an dem Zeolithen absorbiert sind, enthalten.

Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Mittel und gegen Licht sowie keine ausgeprägte Substantivität gegenüber Textilfasern, um diese nicht anzufärben.

Zur Bekämpfung von Mikroorganismen können Wasch- oder Reinigungsmittel antimikrobielle Wirkstoffe enthalten. Hierbei unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppen sind beispielsweise Benzalkoniumchloride, Alkylarylsulfonate, Halogenphenole und Phenolmercuriacetat. Die Begriffe antimikrobielle Wirkung und antimikrobieller Wirkstoff haben im Rahmen der erfindungsgemäßen Lehre die fachübliche Bedeutung, die beispielsweise von K. H. Wallhäußer in "Praxis der Sterilisation, Desinfektion - Konservierung : Keimidentifizierung - Betriebshygiene" (5. Aufl. - Stuttgart; New York : Thieme, 1995) wiedergegeben wird, wobei alle dort beschriebenen Substanzen mit antimikrobieller Wirkung eingesetzt werden können. Geeignete antimikrobielle Wirkstoffe sind vorzugsweise ausgewählt aus den Gruppen der Alkohole, Amine, Aldehyde, antimikrobiellen Säuren beziehungsweise deren Salze, Carbonsäureester, Säureamide, Phenole, Phenolderivate, Diphenyle, Diphenylalkane, Harnstoffderivate, Sauerstoff-, Stickstoff-acetale sowie -formale, Benzamidine, Isothiazoline, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-dicyanobutan, lodo-2-propyl-butyl-carbamat, Iod, lodophore, Peroxoverbindungen, Halogenverbindungen sowie beliebigen Gemischen der voranstehenden.

Der antimikrobielle Wirkstoff kann dabei ausgewählt sein aus Ethanol, n-Propanol, i-Propanol, 1,3-Butandiol, Phenoxyethanol, 1,2-Propylenglykol, Glycerin, Undecylensäure, Benzoesäure, Salicylsäure, Dihydracetsäure, o-Phenylphenol, N-Methylmorpholin-acetonitril (MMA), 2-Benzyl-4-chlorphenol, 2,2'-Methylen-bis-(6-brom-4-chlorphenol), 4,4'-Dichlor-2'-hydroxydiphenylether (Dichlosan), 2,4,4'-Trichlor-2'-hydroxydiphenylether (Trichlosan), Chlorhexidin, N-(4-Chlorphenyl)-N-(3,4-dichlorphenyl)-harnstoff, N,N'-(1,10-decan-diyldi-1-pyridinyl-4-yliden)-bis-(1-octanamin)-dihydrochlorid, N,N'-Bis-(4-chlorphenyl)-3,12-diimino-2,4,11,13-tetraaza-tetradecandiimidamid, Glucoprotaminen, antimikrobiellen oberflächenaktiven quaternären Verbindungen, Guanidinen einschl. den Bi- und Polyguanidinen, wie beispielsweise 1,6-Bis-(2-ethylhexyl-biguanido-hexan)-dihydrochlorid, 1,6-Di-(N₁,N₁'-pheny)diguanido-N₅,N₅')-hexan-tetrahydochlorid, 1,6-Di-(N₁,N₁'-phenyl-N₁,N₁-methyldiguanido-N₅,N₅')-hexan-dihydrochlorid, 1,6-Di-(N₁,N₁'-o-chlorophenyldiguanido- N₅,N₅')-hexan-dihydrochlorid, 1,6-Di-(N₁,N₁'-2,6-dichlorophenyldiguanido-N₅,N₅')hexan-dihydrochlorid, 1,6-Di-[N₁,N₁'-beta-(p-methoxyphenyl) diguanido-N₅,N₅']-hexane-dihydrochlorid, 1,6-Di-(N₁,N₁'-alphamethyl-.beta.-phenyldiguanido-N₅,N₅')-hexan-dihydrochlorid, 1,6-Di-(N₁,N₁'-p-nitrophenyldiguanido-N₅,N₅')hexan-dihydrochlorid, omega:omega-Di-( N₁,N₁'-phenyldiguanido-N₅,N₅')-di-n-propylether-dihydrochlorid, omega:omega'-Di-(N₁,N₁'-p-chlorophenyldiguanido-N₅,N₅')-di-n-propylether-tetrahydrochlorid, 1,6-Di-(N₁,N₁'-2,4- dichlorophenyldiguanido-N₅,N₅')hexan-tetrahydrochlorid, 1,6-Di-(N₁,N₁'-p-methylphenyldiguanido- N₅,N₅')hexan-dihydrochlorid, 1,6-Di-(N₁,N₁'-2,4,5-trichlorophenyldiguanido-N₅,N₅')hexan-tetrahydrochlorid, 1,6-Di-[N₁,N₁'-alpha-(p-chlorophenyl) ethyldiguanido-N₅,N₅'] hexan-dihydrochlorid, omega:omega-Di-(N₁,N₁'-p-chlorophenyldiguanido-N₅,N₅')m-xylene-dihydrochlorid, 1,12-Di-(N₁,N₁'-p-chlorophenyldiguanido-N₅,N₅') dodecan-dihydrochlorid, 1,10-Di-(N₁,N₁'-phenyldiguanido-N₅,N₅')-decan-tetrahydrochlorid, 1,12-Di-(N₁,N₁'-phenyldiguanido- N₅,N₅')dodecan-tetrahydrochlorid, 1,6-Di-(N₁,N₁'-o-chlorophenyldiguanido- N₅,N₅') hexan-dihydrochlorid, 1,6-Di-(N₁,N₁'-o-chlorophenyldiguanido- N₅,N₅') hexan-tetrahydrochlorid, Ethylen-bis-(1-tolyl biguanid), Ethylen-bis-(p-tolyl biguanide), Ethylen-bis-(3,5-dimethylphenylbiguanid), Ethylen-bis-(p-tert-amylphenylbiguanid), Ethylen-bis-(nonylphenylbiguanid), Ethylen-bis-(phenylbiguanid), Ethylen-bis-(N-butylphenylbiguanid), Ethylen-bis (2,5-diethoxyphenylbiguanid), Ethylen-bis (2,4-dimethylphenyl biguanid), Ethylen-bis (o-diphenylbiguanid), Ethylen-bis (mixed amyl naphthylbiguanid), N-Butyl-ethylen-bis-(phenylbiguanid), Trimethylen bis (o-tolylbiguanid), N-Butyl-trimethyle- bis-(phenyl biguanide) und die entsprechenden Salze wie Acetate, Gluconate, Hydrochloride, Hydrobromide, Citrate, Bisulfite, Fluoride, Polymaleate, N-Cocosalkylsarcosinate, Phosphite, Hypophosphite, Perfluorooctanoate, Silicate, Sorbate, Salicylate, Maleate, Tartrate, Fumarate, Ethylendiamintetraacetate, Iminodiacetate, Cinnamate, Thiocyanate, Arginate, Pyromellitate, Tetracarboxybutyrate, Benzoate, Glutarate, Monofluorphosphate, Perfluorpropionate sowie beliebige Mischungen davon. Weiterhin eignen sich halogenierte Xylol- und Kresolderivate, wie p-Chlormetakresol oder p-Chlor-meta-xylol, sowie natürliche antimikrobielle Wirkstoffe pflanzlicher Herkunft (zum Beispiel aus Gewürzen oder Kräutern), tierischer sowie mikrobieller Herkunft. Vorzugsweise können antimikrobiell wirkende oberflächenaktive quaternäre Verbindungen, ein natürlicher antimikrobieller Wirkstoff pflanzlicher Herkunft und/oder ein natürlicher antimikrobieller Wirkstoff tierischer Herkunft, äußerst bevorzugt mindestens ein natürlicher antimikrobieller Wirkstoff pflanzlicher Herkunft aus der Gruppe, umfassend Coffein, Theobromin und Theophyllin sowie etherische Öle wie Eugenol, Thymol und Geraniol, und/oder mindestens ein natürlicher antimikrobieller Wirkstoff tierischer Herkunft aus der Gruppe, umfassend Enzyme wie Eiweiß aus Milch, Lysozym und Lactoperoxidase, und/oder mindestens eine antimikrobiell wirkende oberflächenaktive quaternäre Verbindung mit einer Ammonium-, Sulfonium-, Phosphonium-, lodonium- oder Arsoniumgruppe, Peroxoverbindungen und Chlorverbindungen eingesetzt werden. Auch Stoffe mikrobieller Herkunft, sogenannte Bakteriozine, können eingesetzt werden.

Die als antimikrobielle Wirkstoffe geeigneten quaternären Ammoniumverbindungen (QAV) weisen die allgemeine Formel (R¹)(R²)(R³)(R⁴) N⁺ X⁻ auf, in der R¹ bis R⁴ gleiche oder verschiedene C₁-C₂₂-Alkylreste, C₇-C₂₈-Aralkylreste oder heterozyklische Reste, wobei zwei oder im Falle einer aromatischen Einbindung wie im Pyridin sogar drei Reste gemeinsam mit dem Stickstoffatom den Heterozyklus, zum Beispiel eine Pyridinium- oder Imidazoliniumverbindung, bilden, darstellen und X⁻ Halogenidionen, Sulfationen, Hydroxidionen oder ähnliche Anionen sind. Für eine optimale antimikrobielle Wirkung weist vorzugsweise wenigstens einer der Reste eine Kettenlänge von 8 bis 18, insbesondere12 bis 16, C-Atomen auf.

QAV sind durch Umsetzung tertiärer Amine mit Alkylierungsmitteln, wie zum Beispiel Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxy-substituierte Alkyl-Reste verfügen, sind wenig reaktiv und werden bevorzugt mit Dimethylsulfat quaterniert.

Geeignete QAV sind beispielweise Benzalkoniumchlorid (N-Alkyl-N,N-dimethylbenzyl-ammoniumchlorid, CAS No. 8001-54-5), Benzalkon B (m,p-Dichlorbenzyl-dimethyl-C12-alkylammoniumchlorid, CAS No. 58390-78-6), Benzoxoniumchlorid (Benzyl-dodecyl-bis-(2-hydroxyethyl)-ammonium-chlorid), Cetrimoniumbromid (N-Hexadecyl-N,N-trimethyl-ammoniumbromid, CAS No. 57-09-0), Benzetoniumchlorid (N,N-Dimethyl-N-[2-[2-[*p*-(1,1,3,3-tetramethylbutyl)-pheno-xy]ethoxy]ethyl]-benzylammoniumchlorid, CAS No. 121-54-0), Dialkyldimethylammonium-chloride wie Di-n-decyl-dimethyl-ammoniumchlorid (CAS No. 7173-51-5-5), Didecyldimethylammoniumbromid (CAS No. 2390-68-3), Dioctyl-dimethyl-ammoniumchloric, 1-Cetylpyridiniumchlorid (CAS No. 123-03-5) und Thiazoliniodid (CAS No. 15764-48-1) sowie deren Mischungen. Besonders bevorzugte QAV sind die Benzalkoniumchloride mit C₈-C₁₈-Alkylresten, insbesondere C₁₂-C₁₄-Aklyl-benzyl-dimethyl-ammoniumchlorid.

Benzalkoniumhalogenide und/oder substituierte Benzalkoniumhalogenide sind beispielsweise kommerziell erhältlich als Barquat^{®} ex Lonza, Marquat^{®} ex Mason, Variquat^{®} ex Witco/ Sherex und Hyamine^{®} ex Lonza, sowie Bardac^{®} ex Lonza. Weitere kommerziell erhältliche antimikrobielle Wirkstoffe sind N-(3-Chlorallyl)-hexaminiumchlorid wie Dowicide^{®} und Dowicil^{®} ex Dow, Benzethoniumchlorid wie Hyamine^{®} 1622 ex Rohm & Haas, Methylbenzethoniumchlorid wie Hyamine^{®} 10X ex Rohm & Haas, Cetylpyridiniumchlorid wie Cepacolchlorid ex Merrell Labs.

Die antimikrobiellen Wirkstoffe werden in Mengen von 0,0001 Gew.-% bis 1 Gew.%, bevorzugt von 0,001 Gew.-% bis 0,8 Gew.-%, besonders bevorzugt von 0,005 Gew.-% bis 0,3 Gew.% und insbesondere von 0,01 bis 0,2 Gew.-% eingesetzt.

Die Mittel können UV-Absorbenzien (UV-Absorber) enthalten, die auf die behandelten Textilien aufziehen und die Lichtbeständigkeit der Fasern und/oder die Lichtbeständigkeit sonstiger Rezepturbestandteile verbessern. Unter UV-Absorber sind organische Substanzen (Lichtschutzfilter) zu verstehen, die in der Lage sind, ultraviolette Strahlen zu absorbieren und die aufgenommene Energie in Form längerwelliger Strahlung, zum Beispiel Wärme wieder abzugeben.

Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung Phenylsubstituierte Acrylate (Zimtsäurederivate, gegebenenfalls mit Cyanogruppen in 2-Stellung), Salicylate, organische Ni-Komplexe sowie Naturstoffe wie Umbelliferon und die körpereigene Urocansäure geeignet. Besondere Bedeutung haben Biphenyl- und vor allem Stilbenderivate wie sie beispielsweise in der EP 0728749 A beschrieben werden und kommerziell als Tinosorb^{®} FD oder Tinosorb^{®} FR ex Ciba erhältlich sind. Als UV-B-Absorber sind zu nennen: 3-Benzylidencampher beziehungsweise 3-Benzylidennorcampher und dessen Derivate, zum Beispiel 3-(4-Methylbenzy-liden)campher, wie in der EP 0693471 B1 beschrieben; 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester; Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene); Ester der Salicylsäure, vorzugsweise Salicylsäure-2-ethylhexylester, Salicylsäure-4-isopropylbenzylester, Salicylsäurehomomenthylester; Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon; Ester der Benzalmalonsäure, vorzugsweise 4-Methoxybenzmalonsäuredi-2-ethylhexylester; Triazinderivate, wie zum Beispiel 2,4,6-Trianilino-(p-carbo-2'-ethy)-1'-hexyloxy)-1,3,5-triazin und Octyl Triazon, wie in der EP 0818450 A1 beschrieben oder Dioctyl Butamido Triazone (Uvasorb® HEB); Propan-1,3-dione, wie zum Beispiel 1-(4-tert.Butylphenyl)-3-(4'methoxyphenyl)propan-1,3-dion; Ketotricyclo(5.2.1.0)decan-Derivate, wie in der EP 0694521 B1 beschrieben. Weiterhin geeignet sind 2-Phenylbenzimidazol-5-sulfonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hydroxy-4-methoxybenzophenon-5-sulfonsäure und ihre Salze; Sulfonsäurederivate des 3-Benzylidencamphers, wie zum Beispiel 4-(2-Oxo-3-bornylidenmethyl)benzol-sulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der DE 19712033 A1 (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse, vorzugsweise nanoisierte Metalloxide beziehungsweise Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente bereits für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind ummantelte Titandioxide, wie zum Beispiel Titandioxid T 805 (Degussa) oder Eusolex® T2000 (Merck; als hydrophobe Coatingmittel kommen dafür bevorzugt Silicone und besonders bevorzugt Trialkoxy-octylsilane oder Simethicone in Frage. Vorzugsweise wird mikronisiertes Zinkoxid verwendet. Weitere geeignete UV-Lichtschutzfilter sind der Übersicht von P. Finkel in SÖFW-Journal, Band 122 (1996), S. 543 zu entnehmen.

Die UV-Absorbenzien werden üblicherweise in Mengen von 0,01 Gew.% bis 5 Gew.-%, vorzugsweise von 0,03 Gew.-% bis 1 Gew.-%, eingesetzt.

Enzyme wie Proteasen, Amylasen, Lipasen oder Cellulasen werden seit Jahrzehnten als aktive Komponenten in Wasch- und Reinigungsmitteln eingesetzt. Ihr jeweiliger Beitrag zur Wasch-, beziehungsweise Reinigungsleistung des betreffenden Mittels ist im Fall von Protease die Fähigkeit zum Abbau proteinhaltiger Verunreinigungen, im Fall von Amylase der Abbau von stärkehaltigen Verunreinigungen und im Fall von Lipase die fettspaltende Aktivität. Cellulasen werden insbesondere wegen ihres Beitrags zur Sekundärwaschleistung eines Waschmittels und wegen ihrer Faserwirkung auf Textilien bevorzugt in Waschmitteln eingesetzt. Die jeweiligen Hydrolyseprodukte werden von den übrigen Wasch- oder Reinigungsmittel-Bestandteilen angegriffen, gelöst, emulgiert oder suspendiert oder aufgrund ihrer höheren Löslichkeit mit der Waschflotte ausgeschwemmt, so daß es günstigenfalls zu Synergieeffekten zwischen den Enzymen und den übrigen Bestandteilen kommt.

Erfindungsgemäße Mittel können zusätzlich zu dem erfindungswesentlichen Protein andere amylolytische Enzyme, insbesondere α-Amylasen enthalten. Darunter können sich auch die für die Verwendung in Wasch- und Reinigungsmitteln etablierten Enzyme befinden. Beispiele für kommerziell erhältliche Amylasen sind BAN^{®}, Termamyl^{®}, Purastar^{®}, Amylase-LT^{®}, Maxamyl^{®}, Duramyl^{®} und/oder Purafect^{®} OxAm. Dies ist dann angebracht, wenn sich die verschiedenen Enzyme einander zu ergänzen vermögen. Solch eine Ergänzung kann beispielsweise in regulatorischer Hinsicht erfolgen, etwa durch gegenseitige Aktivierung oder durch Inaktivierung. Sie kann sich beispielsweise dadurch ergeben, daß mindestens ein nicht zu den bekannten α-Amylasen homologer Teil des erfindungswesentlichen Enzyms Einfluß auf die nicht erfindungswesentlichen amylolytischen Aktivitäten nimmt. Die gemeinsame Verwendung kann aber auch aufgrund abweichender Substratspezifitäten sinnvoll sein. Beides sind hier beschriebene Ausführungsformen

Insbesondere an chemisch diversen Anschmutzungen kann es vorteilhaft sein, amylolytische Enzyme in Wasch- und Reinigungsmitteln zusammen mit anderen wasch- und/oder reinigungsaktiven Enzymen einzusetzen. Somit stellen Waschoder Reinigungsmittel, die über ein erfindungsgemäßes Protein hinaus durch zusätzlich weitere Enzyme gekennzeichnet sind, bevorzugte Ausführungsformen dar.

Dazu gehören neben weiteren Amylasen beispielsweise Proteasen, aber auch Lipasen, Cutinasen, Esterasen, Pullulanasen, Cellulasen, Hemicellulasen und/oder Xylanasen, sowie deren Gemische. Besonders bevorzugt sind Proteasen, Lipasen, β-Glucanasen und/oder Cellulasen. Weitere Enzyme erweitern die Reinigunsgleistung entsprechender Mittel um ihre jeweils spezifische enzymatische Leistung. Dazu gehören beispielsweise Oxidoreductasen oder Peroxidasen als Komponenten von enzymatischen Bleichsystemen, zum Beispiel Laccasen (WO 00/39306), β-Glucanasen (WO 99/06515 und WO 99/06516) oder Pektinlösende Enzyme (WO 00/42145), die insbesondere in Spezialwaschmitteln zum Einsatz kommen.

Beispiele für kommerziell erhältliche Enzyme zum Gebrauch in erfindungsgemäßen Mitteln sind Proteasen wie Subtilisin BPN', Properase^{®}, BLAP^{®}, Optimase^{®}, Opticlean^{®}, Maxatase^{®}, Maxacal^{®}, Maxapem^{®}, Alcalase^{®}, Esperase^{®}, Savinase^{®}, Durazym^{®}, Everlase^{®} und/oder Purafect^{®}G oder Purafect^{®} OxP und Lipasen wie Lipolase^{®}, Lipomax^{®}, Lumafast^{®} und/oder Lipozym^{®}.

Die Proteaseaktivität in derartigen Mitteln kann nach der in Tenside, Band 7 (1970), S. 125-132 beschriebenen Methode ermittelt werden. Sie wird dementsprechend in PE (Protease-Einheiten) angegeben. Die Proteaseaktivität bevorzugter Mittel kann bis zu 1.500.000 Proteaseeinheiten pro Gramm Zubereitung betragen.

Hinsichtlich ihrer Gewinnung kommen unter den verwendbaren Enzymen in erster Linie die aus Mikroorganismen, wie Bakterien oder Pilzen, gewonnenen in Frage, beispielsweise aus *Bacillus subtilis, B. licheniformis, Streptomyces griseus, Humicola lanuginosa, H. insolens, Pseudomonas pseudoalcaligenes* oder *P. cepacia,* insbesondere die von diesen Stämmen natürlicherweise gebildeten Enzymgemische, beziehungsweise Gemische mit denen anderer Stämme. Sie werden in bekannter Weise durch Fermentationsprozesse aus geeigneten Mikroorganismen gewonnen, die zum Beispiel in den deutschen Offenlegungsschriften DE 19 40 488 und DE 21 21 397, den US-amerikanischen Patentschriften US 3 623 957 und US 4 264 738, der europäischen Patentanmeldung EP 006 638, sowie der internationalen Patentanmeldung WO 91/02792 beschrieben sind.

Auch diese gegebenenfalls zusätzlich verwendeten Enzyme können, wie zum Beispiel in der europäischen Patentschrift EP 0 564 476 oder in der internationalen Patentanmeldung WO 94/23005 beschrieben, an Trägerstoffen adsorbiert und/oder in Hüllsubstanzen eingebettet sein, um sie gegen vorzeitige Inaktivierung zu schützen. Sie sind in Waschmitteln vorzugsweise in Mengen bis zu 10 Gew.-%, insbesondere von 0,2 Gew.% bis 2 Gew.-%, enthalten, wobei besonders bevorzugt gegen oxidativen Abbau stabilisierte Enzyme, wie zum Beispiel aus den internationalen Patentanmeldungen WO 94/18314 bekannt, eingesetzt werden.

Ein erfindungsgemäßes Protein kann besonders während der Lagerung durch Stabilisatoren beispielsweise vor Denaturierung, Zerfall oder Inaktivierung, etwa durch physikalische Einflüsse, Oxidation oder proteolytische Spaltung geschützt werden. Bei Proteinen, die aus Mikroorganismen erhalten werden, ist eine Inhibierung der Proteolyse besonders kritisch, weil die meisten Mikroorganismen verschiedene Proteasen als Verdauungsenzyme in die umgebenden Medien sekretieren. Diese können die interessierenden Proteine während der nachfolgenden Aufreinigungstufen erheblich schädigen. Auch in Wasch- und Reinigungsmitteln können erfindungswesentliche Proteine mit Proteasen vergesellschaftet sein, und bedürfen deshalb eines besonderen Schutzes.

Auch erfindungsgemäße Mittel können zu diesem Zweck Stabilisatoren enthalten. Eine Gruppe von Stabilisatoren sind reversible Proteaseinhibitoren, die bei Verdünnung des Mittels in der Waschflotte abdissozieren. Benzamidin-Hydrochlorid und Leupeptin sind für diesen Zweck etabliert. Häufig werden Borax, Borsäuren, Boronsäuren oder deren Salze oder Ester verwendet, darunter vor allem Derivate mit aromatischen Gruppen, etwa ortho-, meta- oder para-substituierte Phenylboronsäuren, insbesondere 4-Formylphenyl-Boronsäure, beziehungsweise die Salze oder Ester der genannten Verbindungen. Auch Peptidaldehyde, das heißt Oligopeptide mit reduziertem C-Terminus, insbesondere solche aus 2-50 Monomeren werden zur reversiblen Inhibierung von Wasch- und Reinigungsmittelproteasen eingesetzt. Zu den peptidischen reversiblen Proteaseinhibitoren gehört unter anderem Ovomucoid. Auch spezifische, reversible Peptid-Inhibitoren für die Protease Subtilisin sind zur Verwendung in Protease-haltigen Mitteln geeignet sowie entsprechende Fusionsproteine aus Protease und Inhibitor.

Weitere Enzymstabilisatoren sind Aminoalkohole wie Mono-, Di-, Triethanol- und -Propanolamin und deren Mischungen, aliphatische Carbonsäuren bis zu C₁₂, wie beispielsweise Bernsteinsäure, andere Dicarbonsäuren oder Salze der genannten Säuren. Auch endgruppenverschlossene Fettsäureamidalkoxylate sind für diesen Zweck geeignet. Bestimmte als Builder eingesetzte organische Säuren vermögen, wie in WO 97/18287 offenbart, zusätzlich ein enthaltenes Enzym zu stabilisieren.

Niedere aliphatische Alkohole, vor allem aber Polyole, wie beispielsweise Glycerin, Ethylenglykol, Propylenglykol oder Sorbit sind weitere häufig eingesetzte Enzymstabilisatoren. Auch Di-Glycerinphosphat schützt gegen Denaturierung durch physikalische Einflüsse. Ebenso werden Calcium- und/oder Magnesiumsalze eingesetzt, wie beispielsweise Calciumacetat, oder Calcium-Formiat.

Polyamid-Oligomere oder polymere Verbindungen wie Lignin, wasserlösliche Vinyl-Copolymere oder Cellulose-Ether, Acryl-Polymere und/oder Polyamide stabilisieren die Enzym-Präparation unter anderem gegenüber physikalischen Einflüssen oder pH-Wert-Schwankungen. Polyamin-N-Oxid-enthaltende Polymere wirken gleichzeitig als Enzymstabilisatoren und als Farbübertragungsinhibitoren. Andere polymere Stabilisatoren sind lineare C₈-C₁₈ Polyoxyalkylene. Auch Alkylpolyglycoside können die enzymatischen Komponenten des erfindungsgemäßen Mittels stabilisieren und vermögen vorzugsweise, diese zusätzlich in ihrer Leistung zu steigern. Vernetzte N-haltige Verbindungen erfüllen vorzugsweise eine Doppelfunktion als Soll-release-Agentien und als Enzym-Stabilisatoren. Hydrophobes, nichtionisches Polymer wirkt vorzugsweise in einer Mischung mit anderen Stabilisatoren stabilisierend auf eine gegebenenfalls enthaltene Cellulase und bevorzugt auch auf das erfindungsgemäße Enzym.

Reduktionsmittel und Antioxidantien erhöhen die Stabilität der Enzyme gegenüber oxidativem Zerfall; hierfür sind beispielsweise schwefelhaltige Reduktionsmittel geläufig. Andere Beispiele sind Natrium-Sulfit und reduzierende Zukker.

Insbesondere werden Kombinatonen von Stabilisatoren eingesetzt, beispielsweise eine Kombination von Polyolen, Borsäure und/oder Borax, die Kombination von Borsäure oder Borat, reduzierenden Salzen und Bernsteinsäure oder anderen Dicarbonsäuren oder die Kombination von Borsäure oder Borat mit Polyolen oder Polyaminoverbindungen und mit reduzierenden Salzen. Die Wirkung von Peptid-Aldehyd-Stabilisatoren wird günstigerweise durch die Kombination mit Borsäure und/oder Borsäurederivaten und Polyolen gesteigert und noch weiter durch die zusätzliche Verwendung von Calcium-Ionen.

Mittel mit stabilisierten Enzymaktivitäten stellen bevorzugte Ausführungsformen dar. Besonders bevorzugt sind solche mit Enzymen, die auf mehrere der dargestellten Weisen stabilisiert sind.

Erfindungsgemäße Mittel sind dadurch gekennzeichnet, daß sie, beispielsweise um die enthaltenen Wirkstoffe zeitlich oder räumlich voneinander getrennt freizusetzen, aus mehr als einer Phase bestehen. Dabei kann es sich um Phasen in verschiedenen, insbesondere aber um Phasen in denselben Aggregatzuständen handeln.

Erfindungsgemäße Mittel, die aus mehr als einer festen Komponente zusammengesetzt sind, können auf einfache Weise dadurch hergestellt werden, daß verschiedene feste Komponenten, insbesondere Pulver, Granulate oder Extrudate mit verschiedenen Inhaltsstoffen und/oder unterschiedlichem Freisetzungsverhalten in insgesamt loser Form miteinander vermischt werden. Die Herstellung erfindungsgemäßer fester Mittel aus einer oder mehreren Phasen kann auf bekannte Weise, zum Beispiel durch Sprühtrocknen oder Granulation erfolgen, wobei die Enzyme und eventuelle weitere thermisch empfindliche Inhaltsstoffe wie zum Beispiel Bleichmittel gegebenenfalls später separat zugesetzt werden. Zur Herstellung erfindungsgemäßer Mittel mit erhöhtem Schüttgewicht, insbesondere im Bereich von 650 g/l bis 950 g/l, ist ein aus der europäischen Patentschrift EP 0 486 592 bekanntes, einen Extrusionschritt aufweisendes Verfahren bevorzugt. Eine weitere bevorzugte Herstellung mit Hilfe eines Granulationsverfahrens ist in der europäischen Patentschrift EP 0 642 576 beschrieben.

Proteine können für feste Mittel beispielsweise in getrockneter, granulierter, verkapselter oder verkapselter und zusätzlich getrockneter Form eingesetzt werden. Sie können separat, das heißt als eigene Phase, oder mit anderen Bestandteilen zusammen in derselben Phase, mit oder ohne Kompaktierung zugesetzt werden. Sollen mikroverkapselte Enzyme in fester Form verarbeitet werden, so kann das Wasser mit aus dem Stand der Technik bekannten Verfahren aus den sich aus der Aufarbeitung ergebenden wäßrigen Lösungen entfernt werden, wie Sprühtrocknung, Abzentrifugieren oder durch Umsolubilisieren. Die auf diese Weise erhaltenen Teilchen haben üblicherweise eine Teilchengröße zwischen 50 und 200 µm.

Die verkapselte Form bietet sich an, um die Enzyme vor anderen Bestandteilen, wie beispielsweise Bleichmitteln, zu schützen oder um eine kontrollierte Freisetzung (controlled release) zu ermöglichen. Je nach der Größe dieser Kapseln wird nach Milli-, Mikro- und Nanokapseln unterschieden, wobei Mikrokapseln für Enzyme besonders bevorzugt sind. Solche Kapseln werden beispielsweise mit den Patentanmeldungen WO 97/24177 und DE 199 18 267 offenbart. Eine weitere mögliche Verkapselungsmethode besteht darin, daß die für die Verwendung in Wasch- oder Reinigungsmitteln geeigneten Enzyme, ausgehend von einer Mischung der Enzymlösung mit einer Lösung oder Suspension von Stärke oder einem Stärkederivat, in Stärke, beziehungsweise dem Stärkederivat verkapselt werden. Ein solches Verkapselungsverfahren wird mit der deutschen Anmeldung DE 199 56 382 beschrieben.

Es können auch mindestens zwei feste Phasen miteinander verbunden vorliegen. So besteht eine Möglichkeit, ein festes erfindungsgemäßes Mittel zur Verfügung zu stellen, in dem Verpressen oder Kompaktieren zu Tabletten. Solche Tabletten können ein- oder mehrphasig sein. Damit bietet auch diese Darreichungsform die Möglichkeit, ein festes erfindungsgemäßes Mittel mit zwei festen Phasen vorzulegen. Zur Herstellung von erfindungsgemäßen Mitteln in Tablettenform, die einphasig oder mehrphasig, einfarbig oder mehrfarbig sein können und/oder aus einer mehreren Schichten bestehen können, werden vorzugsweise alle Bestandteile - gegebenenfalls je einer Schicht - in einem Mischer miteinander vermischt und das Gemisch mittels herkömmlicher Tablettenpressen, beispielsweise Exzenterpressen oder Rundläuferpressen, mit Preßkräften im Bereich von etwa 50 bis 100 kN/cm², vorzugsweise bei 60 bis 70 kN/cm² verpreßt. Insbesondere bei mehrschichtigen Tabletten kann es von Vorteil sein, wenn mindestens eine Schicht vorverpreßt wird. Dies wird vorzugsweise bei Preßkräften zwischen 5 und 20 kN/cm², insbesondere bei 10 bis 15 kN/cm² durchgeführt. Vorzugsweise weist eine derart hergestellte Tablette ein Gewicht von 10 g bis 50 g, insbesondere von 15 g bis 40 g auf. Die Raumform der Tabletten ist beliebig und kann rund, oval oder eckig sein, wobei auch Zwischenformen möglich sind.

Besonders vorteilhaft ist es, wenn in mehrphasigen Mitteln wenigstens eine der Phasen ein Amylase-sensitives Material, insbesondere Stärke enthält oder von diesem zumindest teilweise umgeben oder beschichtet ist. Auf diese Weise wird diese Phase mechanisch stabilisiert und/oder gegen Einflüsse von außen geschützt und gleichzeitig über eine in der Waschflotte wirksame Amylase angegriffen, so daß die Freisetzung der Inhaltsstoffe erleichtert wird.

Ebenfalls bevorzugte erfindungsgemäße Mittel sind dadurch gekennzeichnet, daß sie insgesamt flüssig, gelförmig oder pastös vorliegen. Die enthaltenen Proteine, vorzugsweise ein erfindungsgemäßes Protein, werden solchen Mitteln bevorzugt ausgehend von einer nach dem Stand der Technik durchgeführten Proteingewinnung und Präparation in konzentrierter wäßriger oder nichtwäßriger Lösung, beispielsweise in flüssiger Form, etwa als Lösung, Suspension oder Emulsion, aber auch in Gelform oder verkapselt oder als getrocknetes Pulver zugesetzt. Derartige erfindungsgemäße Wasch- oder Reinigungsmittel in Form von Lösungen in üblichen Lösungsmitteln werden in der Regel durch einfaches Mischen der Inhaltsstoffe hergestellt, die in Substanz oder als Lösung in einen automatischen Mischer gegeben werden können.

Eine Ausführungsform sind solche flüssigen, gelförmigen oder pastösen Mittel, denen ein erfindungswesentliches Protein und/oder eines der anderen enthaltenen Proteine und/oder einer der anderen enthaltenene Inhaltsstoffe verkapselt, vorzugsweise in Form von Mikrokapseln zugesetzt worden ist. Besonders bevorzugt sind darunter solche mit Kapseln aus amylasesensitivem Material. Solch eine gemeinsame Verwendung von Amylase-sensitiven Materialien und dem erfindungswesentlichen amylolytischen Enzym in einem Wasch- oder Reinigungsmittel kann Synergieeffekte zeigen, etwa dergestalt daß das stärkespaltende Enzym die Spaltung der Mikrokapseln unterstützt und somit den Freisetzungsprozeß der verkapselten Inhaltsstoffe steuert, so daß deren Freisetzung nicht während der Lagerung und/oder nicht zu Beginn des Reinigungsvorgangs, sondern erst zu einem bestimmten Zeitpunkt erfolgt. Auf diesem Mechanismus können komplexe Wasch- und Reinigungsmittelsysteme mit verschiedensten Inhaltsstoffen und verschiedensten Kapseltypen beruhen, die besonders bevorzugte Ausführungsformen darstellen.

Ein vergleichbarer Effekt ist dann gegeben, wenn sich die Inhaltsstoffe des Wasch- oder Reinigungsmittels auf mindestens zwei unterschiedliche Phasen verteilen, beispielsweise zwei oder mehr feste, miteinander verbundene Phasen eines tablettenförmigen Wasch- oder Reinigungsmittels, oder verschiedene Granulate innerhalb desselben pulverförmigen Mittels. Zwei- oder Mehrphasenreiniger sind für die Anwendung sowohl in maschinellen Geschirrspülern als auch in Waschmitteln Stand der Technik. Die Aktivität eines amylolytischen Enzyms in einer früher aktivierten Phase ist Voraussetzung für die Aktivierung einer späteren Phase, wenn diese von einer Amylase-sensitiven Hülle oder Beschichtung umgeben ist oder das Amylase-sensitive Material einen integralen Bestandteil der festen Phase darstellt, bei dessen teilweiser oder vollständiger Hydrolyse die betreffende Phase desintegriert. Der Einsatz des erfindungswesentlichen Enzyms für diesen Zweck stellt somit eine bevorzugte Ausführungsform der vorliegenden Erfindung dar.

Die Inhaltsstoffe von Wasch- und Reinigungsmitteln vermögen sich geeigneterweise gegenseitig in ihrer Leistung zu unterstützen. Die synergistische Verwendung von Amylase und Farbübertragungsinhibitoren zur Steigerung der Reinigungsleistung wird beispielsweise mit der Anmeldung WO 99/63035 offenbart. Es ist auch bekannt, daß Polymere, die gleichzeitig als Cobuilder eingesetzt werden können, wie beispielsweise Alkyl-Poly-Glykoside, die Aktivität und die Stabilität von enthaltenen Enzymen stabilisieren und steigern können, so aus der Anmeldung WO 98/45396. Somit ist es bevorzugt, wenn ein erfindungsgemäßes, Protein oder Derivat, vorzugsweise mit amylolytischer Aktivität, durch einen der übrigen, oben aufgeführten Bestandteile modifiziert, insbesondere stabilisiert und/oder in seinem Beitrag zur Wasch-, beziehungsweise Reinigungsleistung des Mittels gesteigert wird. Entsprechend abgestimmte Rezepturen für erfindungsgemäße Mittel stellen somit besonders bevorzugte Ausführungsformen.

Entsprechend den bisherigen Ausführungen können erfindungsgemäß Verfahren zur Reinigung von Textilien oder von harten Oberflächen, dadurch verbessert werden, daß in wenigstens einem der Verfahrensschritte ein erfindungsgemäßes, Protein oder Derivat, vorzugsweise mit amylolytischer Aktivität zum Einsatz kommt.

Vorzugsweise sind derartige Verfahren dadurch gekennzeichnet, daß in wenigstens einem der Verfahrensschritte ein Mittel gemäß der bisherigen Beschreibung eingesetzt wird.

Besonders bevorzugt sind derartige Verfahren dadurch gekennzeichnet, daß das erfindungsgemäße, Protein oder Derivat, vorzugsweise mit amylolytischer Aktivität in Maschinen, beispielsweise in gängigen Haushaltsgeschirrspülmaschinen oder Haushaltswaschmaschinen eingesetzt wird, vorzugsweise von 0,01 mg bis 400 mg, bevorzugt von 0,02 mg bis 200 mg, besonders bevorzugt von 0,02 mg bis 100 mg des erfindungsgemäßen Enzyms in dem durch dieses Enzym gekennzeichneten Verfahrensschritt.

Günstigenfalls ergeben sich dabei Konzentrationswerte von 0,0005 bis 20 mg pro I, bevorzugt 0,005 bis 10 mg pro I, besonders bevorzugt 0,005 bis 8 mg des amylolytischen Proteins pro I Waschflotte. Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem. 177 (1948), S. 751-766) bestimmt werden.

Entsprechend den bisherigen Ausführungen stellt auch die Verwendung eines erfindungsgemäßen, Proteins oder Derivats, vorzugsweise mit amylolytischer Aktivität allein oder zusammen mit mindestens einem anderen reinigungsaktiven oder die Reinigungswirkung unterstützenden Wirkstoff zur Reinigung von Textilien oder von harten Oberflächen eine Ausführungsform der vorliegenden Erfindung dar.

Vorzugsweise geschieht dies durch Verwendung eines erfindungsgemäßen Mittels.

Das erfindungsgemäße Mittel oder ein erfindungsgemäßes, Protein oder Derivat, vorzugsweise mit amylolytischer Aktivität wird bevorzugt in den oben angegebenen Mengenbereichen eingesetzt, so daß sich günstigerweise in der Waschflotte die oben angegebenen Konzentrationswerte für das amylolytische Protein ergeben. Diese Dosierung kann, je nach Reinigungsproblem vom Hersteller des Mittels oder vom Endverbraucher vorgenommen werden.

Eine weitere Verwendungsmöglichkeit für ein erfindungsgemäßes, Protein oder Derivat, vorzugsweise mit amylolytischer Aktivität stellt die zur Aktivierung der eigenen oder anderer Phasen dar, wenn sie allein oder zusammen mit mindestens einem anderen reinigungsaktiven oder die Reinigungswirkung unterstützenden Wirkstoff in einem aus mehr als einer Phase bestehenden Wasch- oder Reinigungsmittel bereitgestellt wird.

Eine weitere Verwendungsmöglichkeit für ein erfindungsgemäßes, Protein oder Derivat, vorzugsweise mit amylolytischer Aktivität stellt die zur Freisetzung der Inhaltsstoffe aus den Kapseln dar, wenn sie allein oder zusammen mit mindestens einem anderen reinigungsaktiven oder die Reinigungswirkung unterstützenden Wirkstoff in einem Wasch- oder Reinigungsmittel mit verkapselten Inhaltsstoffen bereitgestellt wird.

Eine weitere Ausführungsform stellt die Verwendung eines erfindungsgemäßen Proteins oder Derivats zur Behandlung von Rohmaterialien oder Zwischenprodukten in der Textilherstellung dar, insbesondere zum Entschlichten von Baumwolle.

Rohmaterialien und Zwischenprodukte der Textilherstellung, beispielsweise für solche auf Baumwollbasis, werden im Rahmen ihrer Herstellung und Weiterverarbeitung mit Stärke ausgerüstet, um eine bessere Verarbeitung zu ermöglichen. Dieses sowohl auf Garne, auf Zwischenprodukte, als auch auf Textilien angewendete Verfahren nennt man Schlichten (sizing). Zur Entfernung der Schlichte, also der stärkehaltigen Schutzschicht (Entschlichten, desizing) sind erfindungsgemäße amylolytische Proteine geeignet.

Eine weitere Ausführungsform stellen Verfahren zur Stärkeverflüssigung, insbesondere zur Ethanolproduktion dar, die dadurch gekennzeichnet sind, daß darin ein erfindungsgemäßes Protein oder Derivat eingesetzt wird.

Zur Stärkeverflüssigung wird in Wasser oder Puffer gequollene Stärke mit amylolytischen Enzymen inkubiert, wodurch das Polysaccharid in kleinere Bestandteile, zuletzt überwiegend in Maltose gespalten wird. Erfindungsgemäße Enzyme werden bevorzugt für ein solches Verfahren oder einen Teilschritt davon eingesetzt, wenn sie sich aufgrund ihrer biochemischen Eigenschaften gut in ein entsprechendes Produktionsverfahren einpassen lassen. Dies kann beispielsweise dann der Fall sein, wenn sie in einem Schritt zusätzlich zu anderen Enzymen eingebracht werden sollen, die die gleichen Reaktionsbedingungen benötigen. Besonders bevorzugt sind erfindungsgemäße amylolytische Proteine, wenn gerade die von ihnen selbst gebildeten Produkte im Zentrum des Interesses stehen. Die Stärkeverflüssigung kann auch einen Schritt in einem mehrstufigen Verfahren zur Gewinnung von Ethanol oder davon abgeleiteten Folgeprodukten, beispielsweise Essigsäure darstellen.

Eine weitere Ausführungsform stellt die Verwendung eines erfindungsgemäßen Proteins oder Derivats zur Herstellung von linearen und/oder kurzkettigen Oligosacchariden dar.

Aufgrund ihrer enzymatischen Aktivität bilden erfindungsgemäße amylolytische Proteine aus stärkeähnlichen Polymeren nach kürzerer Inkubationszeit überwiegend höhermolekulare Oligosaccharide, wie beispielsweise Maltohexaose, Maltoheptaose oder Maltooctaose. Nach längerer Inkubationszeit steigt unter den Reaktionsprodukten der Anteil niedrigerer Oligosaccharide, wie beispielsweise Maltose oder Maltotriose an. Bei besonderem Interesse an bestimmten Reaktionsprodukten können entsprechende Varianten erfindungsgemäßer Proteine verwendet und/oder die Reaktionsbedingungen entsprechend gestaltet werden. Dies ist insbesondere dann attraktiv, wenn es nicht auf reine Verbindungen, sondern auf Gemische ähnlicher Verbindungen ankommt, wie beispielsweise bei der Bildung von Lösungen, Suspensionen oder Gelen mit lediglich bestimmten physikalischen Eigenschaften.

Eine weitere Ausführungsform stellt die Verwendung eines erfindungsgemäßen Proteins oder Derivats zur Hydrolyse von Cyclodextrinen dar.

Cyclodextrine sind α-1,4-giycosidisch verknüpfte, cyclische Oligosaccharide, von denen die aus 6, 7 oder 8 Glucose-Monomeren, die α-, β-, beziehungsweise γ-Cyclodextrine (oder Cyclohexa-, -hepta-, beziehungsweise -octa-Amylosen), wirtschaftlich die größte Bedeutung besitzen. Sie können mit hydrophoben Gastmolekülen, wie beispielsweise Duftstoffen, Geschmacksstoffen oder pharmazeutischen Wirkstoffen, Einschlußverbindungen bilden, aus welchen die Gastmoleküle bei Bedarf wieder freigesetzt werden können. Solche Einschlußverbindungen sind je nach Einsatzgebiet der Inhaltsstoffe beispielsweise für die Herstellung von Nahrungsmitteln, die Pharmazie oder die Kosmetik, beispielsweise in entsprechenden Produkten für den Endverbraucher von Bedeutung. Die Freisetzung von Inhaltsstoffen aus Cyclodextrinen stellt somit eine Verwendungsmöglichkeit für erfindungsgemäße Proteine dar.

Eine weitere Ausführungsform stellt die Verwendung eines erfindungsgemäßen Proteins oder Derivats zur Freisetzung von niedermolekularen Verbindungen aus Polysaccharidträgem oder Cyclodextrinen dar.

Aufgrund ihrer enzymatischen Aktivität können erfindungsgemäße amylolytische Proteine niedermolekulare Verbindungen auch aus anderen α-1,4-glycosidisch verknüpften Polysacchariden freisetzen. Dieses kann sich wie bei den Cyclodextrinen auf molekularer Ebene abspielen als auch an größeren Systemen, wie beispielsweise Inhaltsstoffen, die in Form von Mikrokapseln verkapselt sind. Beispielsweise Stärke ist ein im Stand der Technik etabliertes Material, um Verbindungen wie beispielsweise Enzyme, die in definierten Mengen in Reaktionsansätze eingebracht werden sollen, während der Lagerung einzukapseln. Der kontrollierte Freisetzungsprozeß aus derartigen Kapseln kann von erfindungsgemäßen amylolytischen Enzymen unterstützt werden.

Eine weitere Ausführungsform stellt die Verwendung eines erfindungsgemäßen Proteins oder Derivats zur Herstellung von Lebensmitteln und/oder Lebensmittelbestandteilen dar.

Ebenso stellt die Verwendung eines erfindungsgemäßen Proteins oder Derivats zur Herstellung von Tierfutter und/oder Tierfutterbestandtellen eine Ausführungsform dieses Erfindungsgegenstands dar.

Wo immer Stärke oder von Stärke abgeleitete Kohlenhydrate als Lebensmittel- oder Tiernahrungsbestandteil eine Rolle spielen, kann eine amylolytische Aktivität zur Herstellung dieser Artikel zum Einsatz kommen. Sie erhöht den Anteil von Mono-, oder Oligomeren gegenüber dem polymeren Zucker, was beispielsweise dem Geschmack, der Verdaubarkeit oder der Konsistenz des Lebensmittels zugute kommen kann. Dies kann zur Herstellung bestimmter Tierfutter erforderlich sein, beispielsweise aber auch bei der Herstellung von Fruchtsäften, Wein oder anderer Lebensmittel, wenn der Anteil polymerer Zucker verringert und der von süßen und/oder leichter löslichen Zuckern erhöht werden soll. Die weiter oben ausgeführte Verwendungsmöglichkeit zur Stärkeverflüssigung und/oder Ethanolproduktion kann als großtechnische Variante dieses Prinzips angesehen werden.

Amylasen wirken darüberhinaus auch dem unter Altbacken-Werden bekannten Geschmacksverlust von Backwaren (Anti staling-Effekt) entgegen. Dafür werden sie geeigneterweise dem Teig vor dem Backen zugesetzt. Bevorzugte Ausführungsformen der vorliegenden Erfindung sind also solche, bei denen erfindungsgemäße Proteine zur Herstellung von Backwaren verwendet werden.

Eine weitere Ausführungsform stellt die Verwendung eines erfindungsgemäßen Proteins oder Derivats zur Auflösung stärkehaltiger Klebeverbindungen dar.

Ebenso stellen temporäre Klebeverfahren, die dadurch gekennzeichnet sind, daß darin ein erfindungsgemäßes Protein oder Derivat eingesetzt wird, eine Ausführungsform dieses Erfindungsgegenstands dar.

Neben anderen Naturstoffen wird auch Stärke bereits seit Jahrhunderten als Bindemittel in der Papierherstellung und dem Verkleben unterschiedlicher Papiere und Pappen verwendet. Dies betrifft beispeilsweise Graphiken und Bücher. Im Laufe langer Zeiträume können solche Papiere unter ungünstigen Einflüssen, wie beispielsweise Feuchtigkeit, Wellen aufwerfen oder brechen, was bis zu deren völliger Zerstörung führen kann. Bei der Restaurierung solcher Papiere und Pappen kann das Auflösen der Klebeschichten erforderlich sein und durch Verwendung eines erfindungsgemäßen amylolytischen Proteins erheblich erleichtert werden.

Pflanzliche Polymere wie Stärke oder Cellulose und deren wasserlösliche Derivate werden unter anderem als Klebstoffe oder Kleister verwendet. Dafür müssen sie in Wasser zunächst quellen und nach Aufbringen auf dem Klebegut trocknen, wodurch dieses am Untergrund fixiert wird. Das erfindungsgemäße Enzym kann solch einer wäßrigen Suspension zugesetzt werden, um die Klebeeigenschaften des entstehenden Kleisters zu beeinflussen. Es kann aber auch stattdessen oder zusätzlich zu dieser Funktion dem Kleister zugesetzt werden, um nach dem Antrocknen für lange Zeit, beispielsweise einige Jahre, inaktiv auf dem Klebegut zu verharren. Gezieltes Ändern der Umgebungsbedingungen, beispielsweise durch Anfeuchten, kann dann dazu verwendet werden, um das Enzym zu einem späteren Zeitpunkt zu aktivieren und damit ein Auflösen des Kleisters herbeizuführen. Auf diese Weise ist das Klebegut leichter wieder vom Untergrund abzulösen. In diesem Verfahren fungiert das erfindungsgemäße Enzym aufgrund seiner amylolytischen Aktivität als scheidendes Agens in einem temporären Klebeverfahren oder als sogenannter "Schalter" zum Ablösen des Klebeguts.

Die folgenden Beispiele erläutern die Erfindung, ohne sie jedoch darauf einzuschränken:

### Beispiel 1: Isolierung von Metagenom - DNA aus mikrobiellen Habitaten

### Gewinnung von Bodenproben

Die Bodenproben wurden von verschiedenen Standorten in Deutschland (u.a.) gewonnen und zunächst mechanisch trocken aufbereitet (Sieben durch 4mm und 1mm Siebe).

### DNA Isolierung aus Boden

Für die DNA-Präparation wurden 5 g Boden unter Zugabe von 13,5 ml Extraktionspuffer (100 mM Tris-HCl, pH 8.0; 100 mM EDTA, pH 8.0; 100 mM Natriumphosphat, pH 8.0; 1.5 M NaCl; 1 % Hexadecyltrimethylammoniumbromid) aufgeschlämmt. Die Suspension wurde 3 mal unter Zugabe von Flüssigstickstoff (N₂) schockgefroren, im Mörser zermahlen und in der Mikrowelle aufgekocht. Nach Umfüllen in ein 50 ml Schraubdeckelgefäß und Zugabe von 1.5 ml Lysozymlösung (50 mg/ml) wurde die Suspension 30 min bei 37°C inkubiert und in 5 min Intervallen invertiert. Nach Zugabe von 200 µl einer Proteinase K Lösung (10 mg/ml) erfolgte eine 30-minütige Inkubation bei 37°C mit Invertieren wie zuvor. Anschließend fand eine zweistündige Behandlung mit SDS (durch Zugabe von 3 ml einer 10%-Lösung) bei 65°C statt (Invertieren des Gefäßes alle 20 min).

Nach Zentrifugation (6000xg, 10 min) und Aufschlämmen mit 4.5 ml Extraktionspuffer plus 1 ml 10% SDS wurde das Sediment erneut extrahiert und 10 min bei 65°C inkubiert sowie zentrifugiert wie zuvor. Die vereinten Überstände aus beiden Zentrifugationen wurden mit 1 Volumen Phenol/Chloroform (1:1) versetzt und wie zuvor zentrifugiert. Die Fällung der DNA aus der oberen wäßrigen Phase erfolgte unter Zugabe von 0.6 Volumen Isopropanol und einstündiger Inkubation bei Raumtemperatur sowie einer 20-minütigen Zentrifugation bei 16000xg. Das DNA-Pellet wurde nach Waschen mit 70% Ethanol und Trocknen an der Luft in 200 µl TE-Puffer (100 mM Tris-HCl pH 8.0; 1 mM EDTA pH 8.0) gelöst.

Die hochmolekulare genomische DNA wurde zuletzt über eine präparative Gelelektrophorese (0.7% Agarose) und anschließende Extraktion (QIAex II Gel Extraction Kit; Fa. Qiagen, Hilden) von gelösten inhibitorischen Huminsäuren abgetrennt.

### Beispiel 2: Anlage der Expressions-Genbanken und Screening auf amylolytische Aktivität

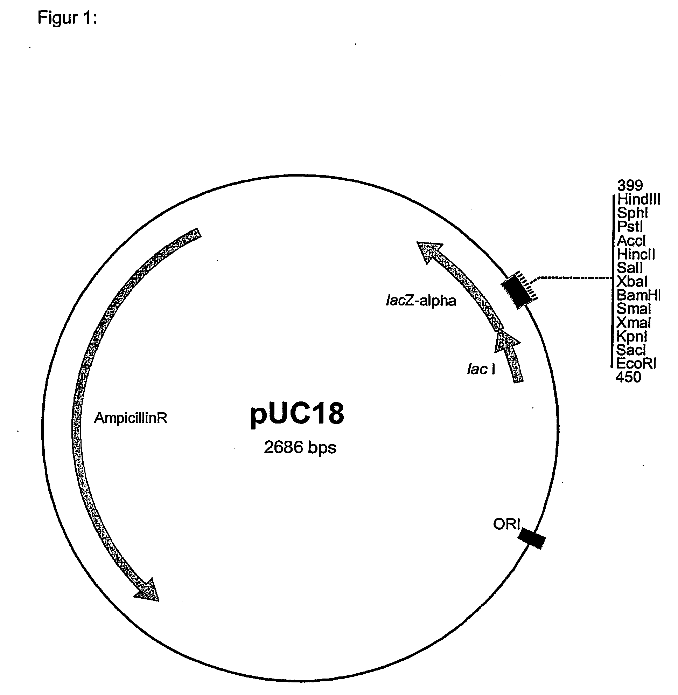

Zur Klonierung und Isolation vollständiger α-Amylasegene aus Metagenom wurde eine Expressionsklonierung im heterologen Wirtsorganismus *Escherischia coli* gewählt. Da keine heterologe Erkennung der Promotoren unbekannter Donororganismen im Wirtsorganismus *E. coli* vorausgesetzt werden kann, wurde ein in bekannter Weise mit IPTG (Isopropylthiogalactosid) induzierbarer *E*. *coli* Promotor, der β-Galactosidase-Promotor des lac-Operons (*lac*-Promotor) auf dem pUC18 Plasmidvektor (GenBank NCBI/NIH, Bethesda, Acc.-No. L08752; Abb. 1) genutzt. In Wirtsstämmen mit einem *lac*Iq-Genotyp (zum Beispiel JM109) kann so durch Zugabe von IPTG die Expression der unter Kontrolle dieses Promotors stehenden Gene induziert werden.

Die *E*. *coli* Stämme JM 109, DH 10B und DH 12S erwiesen sich als geeignete Systeme für die Klonierung der aus Metagenom stammenden α-Amylasen und ihrem aktivitätsabhängigen Nachweis. Denn obwohl sie über die von *mal*S codierten periplamatischen Enzyme verfügen (Freundlieb, S., Boos, W. (1986): "Alpha-amylase of Escherichia coli, mapping and cloning of the structural gene, malS, and identification of its product as a periplasmic protein"; J. Biol. Chem. 261 (6), S. 2946-2953), zeigten sie unter den Versuchsbedingungen keine nennenswerte Amylasefreisetzung.

Gereinigte Metagenom-DNA aus Boden wurde partiell mit Restriktionsenzymen, vorzugsweise *Sau*3AI gespalten, und der Fragmentgrößenbereich von vorzugsweise 8 bis 12 kb durch Gelelektrophorese aufgetrennt und isoliert. Diese DNA Fragmente wurden in geeignete Vektoren, vorzugsweise mit dem Restriktionsenzym *Bam*HI linearisierten pUC18-Plasmidvektor (Abb. 1) ligiert.

Zur Ermittlung der optimalen Restriktionsparameter für den präparativen Partialverdau von Metagenom DNA wurden zunächst Restriktionskinetiken unter Einsatz von ca. 4 µg DNA und Restriktionsenzym (*Sau*3AI) in Konzentrationen von 0,3 bis 0,4 U pro µg DNA in 1 x-Puffer, gegebenenfalls laut Herstellerangaben mit Rinderserumalbumin (BSA) in einem Gesamtvolumen von 20 µl durchgeführt. Die Ansätze wurden dafür zunächst ohne Enzym auf 37°C temperiert. Dann wurde die jeweilige Reaktion durch Zugabe von Restriktionsendonuklease gestartet. In festgelegten Intervallen zwischen 0 und 10 min wurden jeweils 2 µl Reaktionsansatz zu 1 x-Stop-Puffer (6X: 10 mM Tris, pH 7,0; 20 % Glycerin; 0,1% SDS) auf Eis gegeben und auf einem 0,7 %-igen Agarosegel analysiert. Dadurch wurde individuell für jede DNA-Präparation die optimale Restriktionsdauer für einen partiellen Verdau ermittelt.

Der präparative Partialverdau erfolgte über die absolut identische Durchführung wie bei der Restriktionskinetik in 15 bis 20 einzelnen Ansätzen. Nach Abstoppen der Reaktion durch Zugabe von 1 x-Stopp-Puffer wurde der Ansatz auf einem 0,7 %igen Agarosegel elektrophoretisch aufgetrennt, der Gelbereich mit DNA der Größe von 8 - 12 kb ausgeschnitten und dieser über zweistündige Elektroelution in Dialyseschläuchen bei 4°C isoliert. Die mit 1/10 Volumen von 3 M Natriumacetat und dem 2,5-fachen Volumen Ethanol gefällte und in einem kleineren Volumen aufgenommene DNA wurde zur weiteren Abtrennung von kleineren Fragmenten einer zweiten Gelelektrophorese mit anschließender Elektroelution und erneuter Aufkonzentrierung unterzogen.

Die Ligation mit dem Plasmidvektor pUC18 erfolgte über Nacht bei 16°C in einem Gesamtvolumen von 20 µl unter Einsatz von 100 ng mit *Bam*HI linearisiertem, nach Herstellerangaben mit CIAP (Alkalische Phosphatase aus Kalbsthymus) dephosphoryliertem pUC18-Vektor und 800 ng partiell gespaltener genomischer DNA, sowie einer angemessenen Ligase-Menge (hier 600 NEB-Units) in 1 x-Ligase-Puffer.

Die Transformation kompetenter *E.coli* DH12S Zellen (Gibco Life Technologies, Karlsruhe) erfolgte über Elektro-Transformation. Hierfür wurden 1 µl Ligationsansatz und 25 µl Zellen gemischt, in einer Elektroporationsküvette 1 min lang auf Eis inkubiert und im Elektroporator (BTX^{®} ECM630, Genetronics Inc. San Diego, USA) nach Herstellerangaben behandelt. Nach sofortiger Überführung in 1 ml SOC-Medium (2% Bacto-Trypton; 0,5% Hefeextrakt; 10 mM NaCl; 2,5 mM KCl; pH 7,0, eingestellt mit NaOH; autoklaviert; supplementiert mit 10 mM MgSO₄ und MgCl₂ sowie 20 mM D(+)Glucose) folgte vor der Plattierung eine Erholungsphase von 1 h bei 37°C.

Zur Untersuchung der Qualität der Genbank wurde die Anzahl der insgesamt erzeugten Primärtransformanten und die Anzahl Insert-tragender Klone über Blau/Weiß-Selektion in einer Testplattierung bestimmt. Hierfür wurden je 1 und 10 µl des Ligationsansatzes auf LB-Medium mit Ampicillin, IPTG, X-Gal (wie oben beschrieben) ausplattiert und über Nacht bei 37°C inkubiert. Zur Bestätigung der tatsächlich klonierten Insertgrößen erfolgte die Isolation der Plasmide von mindestens 10 weißen Kolonien der Testplattierung und ein geeigneter Restriktionsverdau mit nachfolgender gelelektrophoretischer Größenanalyse.

Beim Screening der Genbanken wurde die Fähigkeit einzelner rekombinanter E. *coli* Klone in Metagenom-Banken zum Abbau von Stärke ermittelt. Hierfür wurde LB-Agar mit 1 % löslicher Stärke (Fa. Merck, Darmstadt, Best.-Nr. #1252) verwendet, auf welcher Klone, die zum Abbau von Stärke befähigt sind, durch Abbauhöfe zu identifizieren sind. Dafür war zunächst eine zwei- bis vierwöchige Lagerung der Platten bei 4°C bis zur Eintrübung notwendig, wonach ein Abbau der Stärke als Aufklaren der Platten um die aktiven Kolonien herum sichtbar war. Vor Gebrauch wurden den Platten IPTG (100 mM) zur Induktion des Promotors und Ampicillin (100 µg/ml) zur Ausübung des Selektionsdrucks auf die Transformanten zugesetzt.

Entsprechend des Titers der jeweils erzeugten Bank wurde ein definiertes Volumen des Transformationsansatzes mit ca. 10 000 Kolonien je Platte (14 cm Durchmesser) mittels Glaskugeln gleichmäßig ausplattiert (primäre Plattierung). Nach 16-stündiger Inkubation bei 37°C wurden die Platten weitere 24 - 48 h bei 28°C zur Amylase-Expression und Freisetzung inkubiert. Dabei war die Inkubation bei 28°C in der Regel für einen sichtbaren Abbau von Stärke unabdingbar. Hierbei mögen eine verbesserte Proteinfaltung und eine Permeabilisierung der äußeren Zellmembran eine Rolle spielen (Stathopoulos, C., Georgiou, G., Earhart, C. F. (1996): "Characterization of Escherichia coli expressing an Lpp'OmpA(46-159)-PhoA fusion protein localized in the outer membran"; Appl. Microbiol. Biotechnol., 45 (1-2), 112-119). Damit ging also eine Freisetzung der jeweils gebildeten α-Amylase einher, und eine zusätzlich durchzuführende Zell-Lyse zum Nachweis nicht exportierter cytoplasmatischer Amylasen war nicht nötig.

Nach einer Vereinzelung der Kolonien aus den Hofbereichen der primären Plattierung durch erneute (sekundäre) Plattierung auf den gleichen Stärkeplatten konnten Amylase-bildende Einzelkolonien anhand erneuter Hofbildung selektiert werden. Für fotodokumentarische Zwecke hat sich nach Sicherung der Klone eine Färbung der Stärkeplatten mit 50% Lugol-Lösung (Fa. Merck) zur eindeutigen Visualisierung von Stärke-freien Bereichen (Abbauhöfen) bewährt.

Die nach Minipräparation (Kit der Fa. Qiagen, Hilden, Deutschland, verwendet nach Herstellerangaben) erhaltene Plasmid-DNA aktiver Klone wurde in einer Restriktionsanalyse mit *Eco* R I oder *Sac*I / *Hind* III auf die Insertgröße hin untersucht und anschließend sequenziert. Hierbei kamen zunächst die Insertflankierenden M13-Primer (M13 forward: 5'-GTAAAACGACGGCCAG-3'; M13 reverse: 5'-CAGGAAACAGCTATGAC-3') zum Einsatz, um schließlich die Gesamtsequenz über sogenanntes Primer-walking zu erhalten, wie es aus dem Stand der Technik bekannt ist.

### Beispiel 3: Charakterisierung des Metagenompools mittels PCR

Zur Abschätzung des Gehalts von Metagenom-DNA aus Boden an Gensequenzen für Glykosylhydrolasen erfolgte eine DNA-Amplifikation durch die Polymerase Kettenreaktion (PCR). Diese wurde unter Verwendung von HotStar Taq-Polymerase (Fa. Qiagen, Hilden) und der Glykosylhydrolase-spezifischen Primer durchgeführt (Tabelle 1). Hierfür wurden jeweils 0,1 µl der nach obigem Verfahren isolierten Metagenom DNA in Gegenwart von 200 µM dNTPs (jeweils dATP, dTTP, dCTP, dGTP), 20 pmol jedes Primers eines Paares (forward/reverse), 2,5 U HotStar Taq-Polymerase (Qiagen, Hilden) und 1 x PCR-Puffer in 50 µl Gesamtvolumen eingesetzt. Es wurden Fragmente der Größen 150 bis 300 bp in folgenden Zyklen amplifiziert: 1 Zyklus 15 min bei 95°C; 35 Zyklen mit je 30 s bei 95°C, 30 s bei 57°C (GEX036/038) beziehungsweise 60°C (andere Primerpaare) und 30 s bei 72°C; 1 Zyklus 7 min bei 72°C.

Mit den Primerpaaren GEX024/026 und GEX023/025 wurden jeweils singuläre PCR-Produkte amplifiziert (300 bp beziehungsweise 280 bp; Abb. 4). Dabei war häufig eine Amplifikation nur nach einer 1:10 Verdünnung der Metagenom-DNA möglich. Dies lag vermutlich an inhibitorischen Komponenten in den DNA-Präparationen, die durch Verdünnen unterhalb eines kritischen Schwellenwertes unwirksam wurden. Mit Primerpaar GEX036/038 wurden erst nach Reamplifikation über Einsatz von 4 µl der ursprünglichen Reaktion PCR-Fragmente erhalten (150 und 300 bp).

Alle PCR-Fragmente wurden zur Sequenzierung zunächst mittels TOPO TA-Kit (Invitrogen, Groningen NL) nach Herstellerangaben in den pCR2.1-TOPO^{®}-Vektor zwischenkloniert. Mit den Ligationsprodukten wurde in *E.coli*-TOP^{®} 10F' (Invitrogen, Groningen NL) transformiert. Nach Blau/weiß-Selektion rekombinanter Klone auf LB-Medium mit Ampicillin (100 µg/ml), IPTG (100 mM) und X-Gal (40 mg/ml) wurde die Plasmid-DNA von weißen Klonen nach Minipräparation isoliert und in einer Restriktionsanalyse mit *Eco*RI auf den Gehalt an Insert-DNA überprüft. Hierbei sind insbesondere bei den Klonen aus der PCR mit GEX024/026 und 036/038 Unterschiede der Insertgrößen ersichtlich (Abb. 4), welche die molekulare Diversität der amplifizierten Glykosylhydrolase-Sequenzen im Metagenom wiederspiegeln.

Die Sequenzanalyse von Plasmiden mit Insert-DNA erfolgte routinemäßig auf einem ABI PRISM^{®} 310 (Fa. Perkin Eimer, Weiterstadt) nach Herstellerangaben mittels AmpliTaq-FS-Big-Dye^{®} Terminator-Kit (Fa. Perkin Elmer, Weiterstadt) unter Einsatz von 200 - 500 ng Plasmid-DNA, 10 pmol Primer (TopoF: 5'-GCTCGGATCCACTAGTAACG-3' und TopoR: 5'-CTCTAGATGCATGCTCGAG-3'), 4 µl Premix in einem Gesamtvolumen von 20 µl.

Die erhaltenen DNA-Sequenzen wurden konzeptionell in eine Aminosäuresequenz umgeschrieben, über die BlastX, BlastN und BlastP Algorithmen (Altschul SF, Gish W, Miller W, Myers EW, Lipman DJ (1990) Basic local alignment search tool. J Mol Biol 1990 Oct 5;215(3):403-10) mit dem Programm FASTA (Pearson WR, Lipman DJ. (1988) Improved tools for biological sequence comparison. Proc Natl Acad Sci U S A Apr;85(8):2444-8) mit den vorhandenen Einträgen in der Sequenzdatenbank GenBank (Rel 122.0) verglichen und auf diese Weise als Teilsequenzen von α-Amylasen und anderen Glykosylhydrolasen identifiziert (siehe Tabelle 2).

Teile der konstanten Primerbereiche, welche aufgrund spät einsetzender Sequenzierleseschärfe unklar waren oder von der synthetisch vorgelegten Sequenz unerwartet abwichen, wurden auf nur Aminosäuresequenzniveau korrigiert und so in die entsprechenden Sequenzlisten eingetragen. Daher können in diesen konstanten Primerbereichen DNA-Sequenzen von den konzeptionell translatierten Aminosäuresequenzen abweichen

Die Aminosäuresequenzen werden im Sequenzprotokoll unter den Nummern SEQ ID NO. 7 bis 56 und SEQ ID NO. 107 bis 205 aufgeführt und in den Consensus-Sequenzen A bis D zusammengefaßt.

### Beispiel 4: Sequenzanalyse der gefundenen Amylase-Gene

Ein Klon HA 11, der in dem oben beschriebenen Expressions-Screening Amylase-Aktivität durch Hofbildung anzeigte, wurden vollständig sequenziert. Die Sequenzanalyse ergab die Existenz von zwei offenen Leserastern, wovon eines ORF1 durch Homologievergleiche als Amylase-Gen identifiziert werden konnte. Die abgeleitete Proteinsequenz ist in Seq_ID 1 wiedergegeben. Das Ergebnis eines Fasta3-Searches ergab als nächstähnliches Enzym mit einer AS-Homologie von 72, 3 % eine Amylase aus Bacillus sp. Diese Amylase zeigte im carboxyterminalen Bereich der Proteinsequenz starke Homologien zu einer in der nächstähnlichen bekannten Sequenz (Sumitani, J.; Tottori, T., Kawaguchi, T. und Arai, M. ; Biochem J 350, 477-484, (2000)) experimentell verifizierten neuartigen Stärkebindedomäne.

Ein zweites Leseraster ORF 2 mit einer Homologie zum nächstähnlichen Gen (bifunktionelle Amylase-Dextrinase) aus Streptomyces coelicolor A3 (Redenbach, M; Kieser, H.M. ; Depanaite, D.; Eichner, A, Cullum J; Kinashi, H. und Hopwood, D.A., Mol. Microbiol 21, 77-96 (1996)) von 46 % bezogen auf die gesamte vorhandene Gensequenz war nur unvollständig im genomischen Klon vorhanden. Ein Stop-Codon am 3'-Ende fehlte, so daß das eigentliche Gen über die bekannte klonierte Sequenz hinaus geht.

Das Amylase-Gen aus HA 11 wurde aus dem isolierten genomischen Klon heraus durch PCR und den Einsatz geeigneter Primer-Oligonukleotide amplifiziert und im *E. coli-Streptomyces* Shuttle-Vektor pEX601 kloniert. Nach Expression im heterologen Wirt *Streptomyces lividans* TK 24 wurde das im Kulturüberstand gewonnene Enzym biochemisch charakterisiert.

Die Amylase-Domäne des bifunktionellen ORF 2 wurde analog zur Amylase separat kloniert und in *S*. *lividans* TK 24 zur Expression gebracht. Die Analyse der Überstande ergab eindeutig eine mittels des DNSS-Assays messbare Amylase-Aktivität. Der so klonierte und exprimierte Teil des bifunktionellen ORF 2 wird mit HA 13 bezeichnet, die Proteinsequenz ist in Seq_ID 3 angegeben.

### Beispiel 5: Biochemische Charakterisierung der exprimierten Gene

Im Hinblick auf die vielfältigen Anwendungsgebiete von α-Amylasen erfolgt die Charakterisierung der enzymatischen Eigenschaften, welche die Grundlage zur anwendungsbezogenen Auswahl von Enzymen sein kann. Die Darstellung der Enzyme kann hierbei durch Klonierung und heterologe Expression der Gensequenzen entsprechend Beispiel 4 erfolgen. Die amylolytische Aktivität wurde mittels eines modifizierten Dinitrosalicylsäure-Tests (DNSS-Test; Hofstetter, F. & E. Borel (1951); Helv. Chim. Acta, 34, 2133-2139)) bestimmt. Dabei wird die Hydrolyse eines komplexen Stärke-Substrates anhand der Zunahme reduzierender Enden des Polysaccharides bestimmt, wobei die Absorption bei 540 nm an umgesetztem DNSS-Reagenz als Maß für die hydrolytische Aktivität dient.

Für die Durchführung des Tests wurden in einer 96-Well-Platte für PCR-Thermocycler (0,2 ml "thin-wall plate" #3416, Molecular BioProducts MßP, San Diego, USA) in die Proben- und Blank-Kavitäten jeweils 25 µl Substratlösung (1 % lösliche Stärke Merck p.a. in 180 mM Tris-Maleat-Puffer, pH 8,6, beziehungsweise in 180 mM des entsprechenden Test-Puffers) vorgelegt und 5 min bei 50°C, beziehungsweise der jeweiligen Test-Temperatur in einem Thermocycler-Block vorinkubiert. Nach Zugabe von 20 µl Enzymlösung je Well erfolgte die Substratumsetzung für 15 min bei 50°C, beziehungsweise der jeweiligen Test-Temperatur. Nach Zugabe von 65 µl DNSS-Reagenz (8,8 g Dinitrosalicylsäure, 250 g Kalium-Natrium-Tartrat, 6,3 g Natriumdisulfit, 334 ml 4,5% (w/v) NaOH, 915 ml H₂O) in die Proben- und Blank-Kavitäten sowie der Zugabe von 20 µl Enzymlösung in die Blank-Kavitäten wurde die Testplatte sofort in einen auf 100°C vorgewärmten Thermoblock transferiert und 20 min bei 100°C inkubiert. Die Messung der Proben erfolgte durch Überführen von je 60 µl des Testansatzes in 200 µl H₂O, welche in einer 96-Well-Meßplatte (PS-Microplate, 96 Well #655101, Greiner) vorgelegt worden waren, und anschließender Bestimmung der Absorption bei 540 nm mittels Mikrotiterplatten-Spektralphotometer (Spectramax 190, Molecular Devices, Sunnivale USA) mit H₂O als Referenz. Es wurden jeweils drei Messungen vorgenommen; die Auswertung erfolgte durch Subtraktion des Mittelwertes der 3 Blank-Kavitäten vom Mittelwert der 3 Proben- Kavitäten.

### Temperaturprofil

Zur Bestimmung des Temperaturprofils erfolgte die Umsetzung gemäß der oben beschriebenen Vorgehensweise bei verschiedenen Temperaturen und 100 mM Tris-Maleat, pH 8,6. Für die alpha-Amylase HA11 (SEQ ID NO. 1) ergab sich eine maximale Aktivität bei 50,4°C. Temperaturen von über 60° führten bei diesem Enzym zu Aktivitätsverlusten (siehe Tabelle 3).

Für die Amylasedomäne HA13 ergab sich das Maximum bei 55,5°C. (siehe Tabelle 4)

### Temperaturstabilität der Amylasedomäne HA13

Nach einer Vorinkubation für 15 min im Temperaturgradienten von 50°C bis 69.7°C erfolgte die Substratumsetzung und der DNSS-Assay wie oben beschrieben, bei 50°C in Tris-HCl pH8,6. Tabelle 5 zeigt, dass die Stabilität ab 60 °C deutlich abnimmt.

### Stabilität gegenüber pH-Wert-Schwankungen

Zur Bestimmung der Aktivität der α-Amylase-Amylasen unter verschiedenen pH-Wert-Bedingungen wurde die Stärke-Substratlösung im pH-Bereich von 4 bis 12 mit dem für den jeweiligen pH geeigneten Puffer angesetzt. Die Pufferkonzentration wurde jeweils auf 180 mM eingestellt, so dass sich nach Verdünnung unter Testbedingungen eine Konzentrationvon 100 mM ergab. Die Bestimmung der amylolytischen Aktivität unter den jeweiligen pH-Werten erfolgte wie oben angegeben bei 50°C. Die jeweiligen Puffer sowie die relativen Aktivitätswerte für beide Amylasen (HA 11 und HA 13) sind in Tabelle 6 zu finden. Zu erkennen ist, dass beide Amylasen das Maximum im Bereich pH 5 bis 6,5 aufweisen, dass sich Amylase HA 13 durch ein breites Maximum auszeichnet und auch bei 12 noch eine beträchtlicghe Restaktivität besitzt.

### Stabilität gegenüber Tensiden :

Für die Bestimmung der Tensid-Stabilität wurde eine Endkonzentration von SDS zwischen 0 und 1 % eingestellt. Die Substratumsetzung erfolgte mit 100mM Tris-HCl pH 8,6 bei 50°C für 15 min. (Tabelle 7= HA11; Tab. 8= HA13).

### Einfluß von Bleichmitteln

Der Einfluss von Bleichmitteln wurde über die Einwirkung von bis zu 0,7 % H₂O₂ in 100mM Glycin-NaOH pH 9,5 bestimmt. Nach Vorinkubation in Gegenwart von H₂O₂ bei Raumtemperatur unter entsprechenden Bedingungen erfolgte eine weitere Inkubation bel 50°C für 15 min. Danach wurde die Substratumsetzung im DNSS-Assay gemessen. (Tabelle 9: HA 11; Tabelle10: HA13)

### Einfluß von Komplexbildnern:

Der Einfluss von Komplexbildnern wurde durch Einstellen einer Konzentration von 2mM CaCl₂ bzw 1 mM EDTA in 100mM Tris-HCl pH 8,6 bei 50°C für 15 min unter-sucht. (Tabelle 11 = HA11; Tabelle 12 = HA13)

### Beispiel 6: Untersuchung der Waschleistung

Die Bestimmung der Waschleistung präparierter Amylasemuster erfolgte in Minwaschversuchen unter Verwendung standardisierter Testanschmutzungen. Diese bestanden aus auf Gewebe immobilisierter Stärke sowie einem Farbindikator. (EMPA 162, Stärke auf Polyester-Baumwolle-Mischgewebe sowie CFT PCS 26, Maisstärke auf Baumwolle)

Dazu wurden Gewebeproben von 19,6 cm² in 20 ml Waschlauge mit bzw ohne Bleichezusatz inkubiert. Es wurde jeweils die Enyzmmenge eingesetzt, die in der Lage ist, aus löslicher Stärke bei 37 °C und pH 6,5 pro Minute eine Menge an reduzierenden Zuckern zu generieren, die 12 µmol Glucose entspricht. Die Inkubation der Gewebeproben erfolgte bei 40 °C und 80 upm für 90 Minuten. Danach wurde mit H₂O abgespült, getrocknet und die Färbung (L-Wert) mittels eines Minolta-Oberflächen - Photometers bestimmt. Die Aufhellung als Maß für die Ablösung der Stärke und somit der Waschleistung wird dann durch die Differenzbildung mit der unbehandelten Gewebeprobe bestimmt. Die Ergebnisse sind in Tabelle 13 dargestellt.

Daraus geht hervor, dass die Waschleistung von HA 11 (SEQ ID NO. 1) bei gleicher eingesetzter Enzymaktivität jeweils am höchsten war. Termamyl® war schwächer, aber jeweils besser als BAN®. Diese Enzyme zeigten eine Abnahme bei Vorliegen einer bleichehaltigen Rezeptur. HA13 (SEQ ID NO. 3) dagegen erwies sich als relativ unempfindlich gegenüber Bleiche, sodass die Waschleistung in der bleichefreien Rezeptur unter der der anderen Enzyme lag, in der bleichehaltigen dagegen vergleichbar mit Termamyl wurde.

Verzeichnis der Figuren (Abbildungen):
Figur 1: Schematische Darstellung des für die Expressionsgenbanken verwendeten Plasmidvektors pUC18 (GenBank Acc. L08752). Der Vektor wurde zur Aufnahme von mit *Sau*3AI verdauter Metagenom-DNA mit *Bam*HI linearisiert. ORI: Replikationsursprung; *lac*I: Gen für *lac*-Repressor; *lacZ*-alpha: Gen für alpha-Peptid der beta-Galaktosidase; AmpicillinR: beta-lactamase.

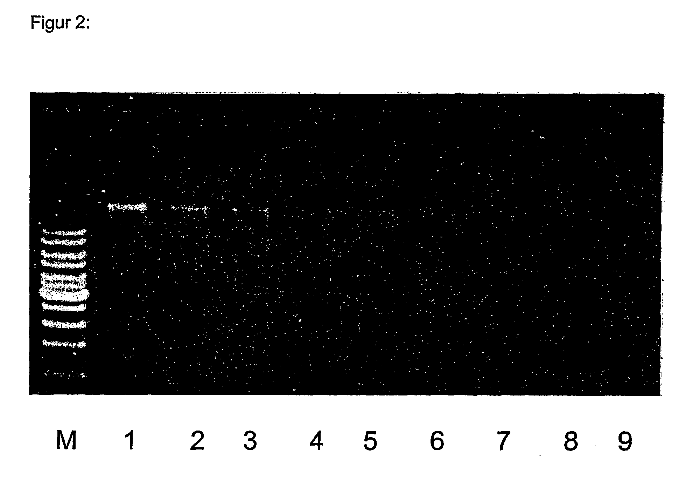

Figur 2: Analyse einer *Sau*3AI-Restriktionskinetik von Metagenom-DNA aus Boden zur Ermittlung der optimalen Restriktionsdauer bei vorgegebener Enzymkonzentration. Aus 20 µl Gesamtansatz mit 0.3 U *Sau*3AI pro µg DNA wurden nach 0, 1, 2, 3, 4, 5, 6, 8 und 10 min je 2 µl in 1x Stopp-Puffer überführt und auf einem 0.7 % Agarosegel aufgetrennt (von links nach rechts Spuren 1-9). Marker (M): 1 kb DNA-Leiter (in kb von oben nach unten: 10; 8; 6; 5; 4; 3.5; 3; 2.5; 2; 1.5; 1. Spur 7 repräsentativ für 5 min Restriktion weist hier einen optimalen Partialverdau im Größenbereich 8 - 12 kb auf.

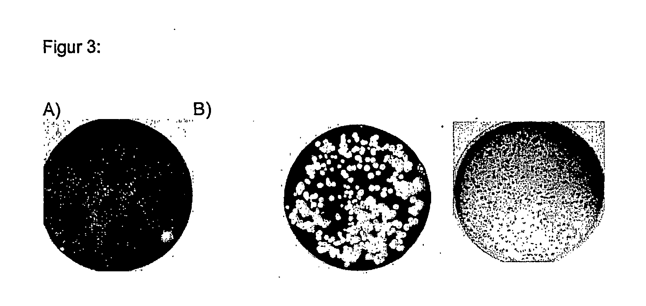

Figur 3: Aktivitätsassay zum Amylasenachweis in einer Metagenom-Expressionsgenbank. Lugol-gefärbte LB-Agarplatten mit 1% löslicher Stärke. Amylase-exprimierende rekombinante *E. coli* Klone weisen einen Stärkeabbauhof um die Kolonien auf, der nach Lugolfärbung hell erscheint.
   A) Beispielhaft eine "Mutterplatte" (Primärplattierung) eines Hof-bildenden Klons aus der Gesamtplattierung einer Plasmid-Expressionsgenbank nach Transformation von *E. coli.*
   B) Vereinzelte Kolonien nach Replattierung (sekundäre Plattierung) aus einem Stärkehofbereich einer Mutterplatte. Es sind einzelne Kolonien mit und ohne Hof zu erkennen. Die Plasmide von Kolonien mit Hof wurden analysiert und ihre Insert-DNA sequenziert.
   Links: Lugol-gefärbte Platte mit hellen Abbauhöfen
   Rechts: Aufklärungshöfe aktiver Klone auf trüber Stärke ohne Lugol-Färbung
Figur 4: Größenanalyse der PCR-Reaktionsprodukte mit Primern GEX023/025, GEX024/026 und GEX036/038 auf Boden Metagenom-DNA. Beispielhafte Größenanalyse der PCR Reaktionsprodukte von Boden S196 (A) und Boden S131 (C). Die PCR wurde mit den Amylase-spezifischen Primern GEX024/026 (A, Spur 1), GEX023/025 (A, Spur 2) und GEX036/038 (C, Spur 1) durchgeführt und die Reaktionsprodukte (1/10 des Reaktionsvolumens) in einem 2% Agarosegel aufgetrennt. Parallel erfolgte als Positivkontrolle eine PCR mit GEX024/026 auf genomischer DNA aus *Streptomyces* sp. Stamm B124 (C, Spur 2). PCR-Bedingungen siehe Text (oben).
   Marker (M): 100 bp DNA Leiter (in bp von oben nach unten: 3000, 2000, 1500, 1200, 1031, 900, 800, 700, 600, 500, 400, 300, 200, 100).
   Mit Primerpaar GEX024/026 wird eine 300bp Bande amplifiziert, mit Primerpaar GEX023/025 ein 280 bp Fragment., während mit GEX036/038 erst nach Reamplifikation des ursprünglichen PCR-Ansatzes 2 Produkte der Größe 300 und 150 bp amplifiziert werden. Sämtliche PCR-Produkte wurden kloniert sowie die DNA von bis zu 18 Klonen nach Exzision der Inserts über EcoRI-Restriktion auf einem 2% Agarosegel aufgetrennt (B). Die Klone für das PCR-Produkt GEX023/025 weisen durchgehend Insert-DNA Größen von ca. 280 bp auf (B, links), die Fragmentgrößen für die Klone aus der PCR GEX024/026 variieren zwischen 300 und 350 bp (B, rechts). Die Klone aus der PCR GEX036/038 weisen Fragmente um 300 bp beziehungsweise 150 bp auf (D).
Figur 5: Consensus-Sequenz -A- und Aminosäureaustausche von 50 Glykosylhydrolasen aus Bodenmikroorganismen. Der Consensussequenz liegt ein Alignment mit Clustal X Version 1.64b zugrunde (Standardeinstellungen). Aminosäurepositionen 1-8 und 116-123 sind zum Teil durch die PCR-Primersequenzen (GEX024/026) vorgegeben.

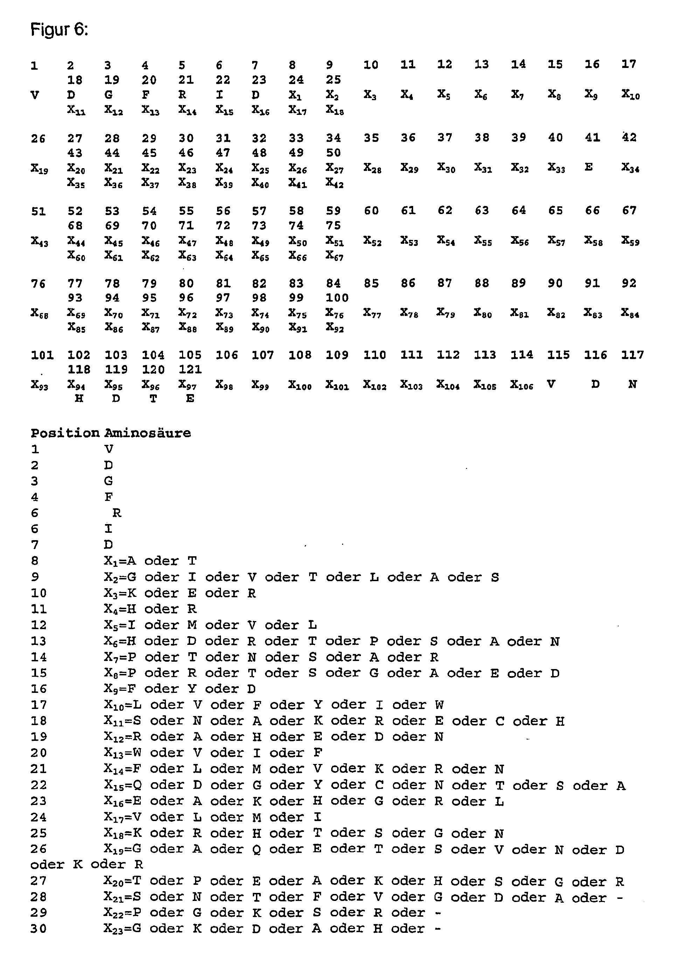

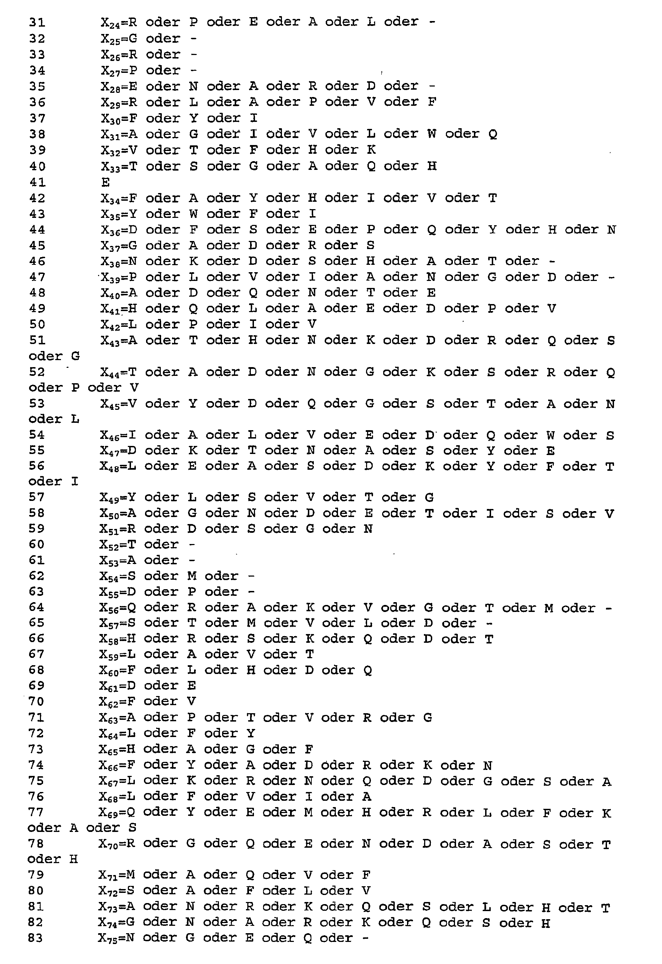

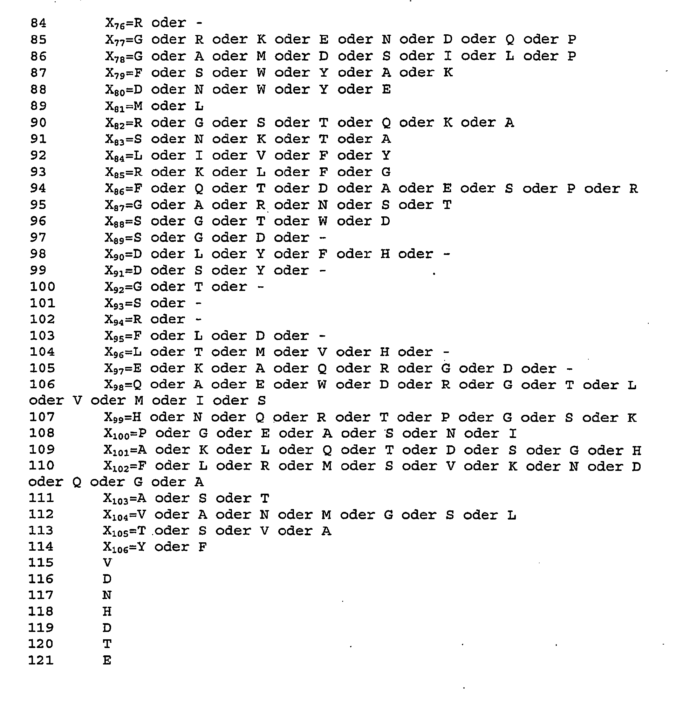

Figur 6: Consensus-Sequenz -D- (Subsequenzraum zu A) und Aminosäureaustausche von 42 Amylasen aus Bodenmikroorganismen. Der Consensussequenz liegt ein Alignment mit Clustal X Version 1.64b zugrunde (Standardeinstellungen). Aminosäurepositionen 1-8 und 114-121 sind zum Teil durch die PCR-Primersequenzen (GEX024/026) vorgegeben.

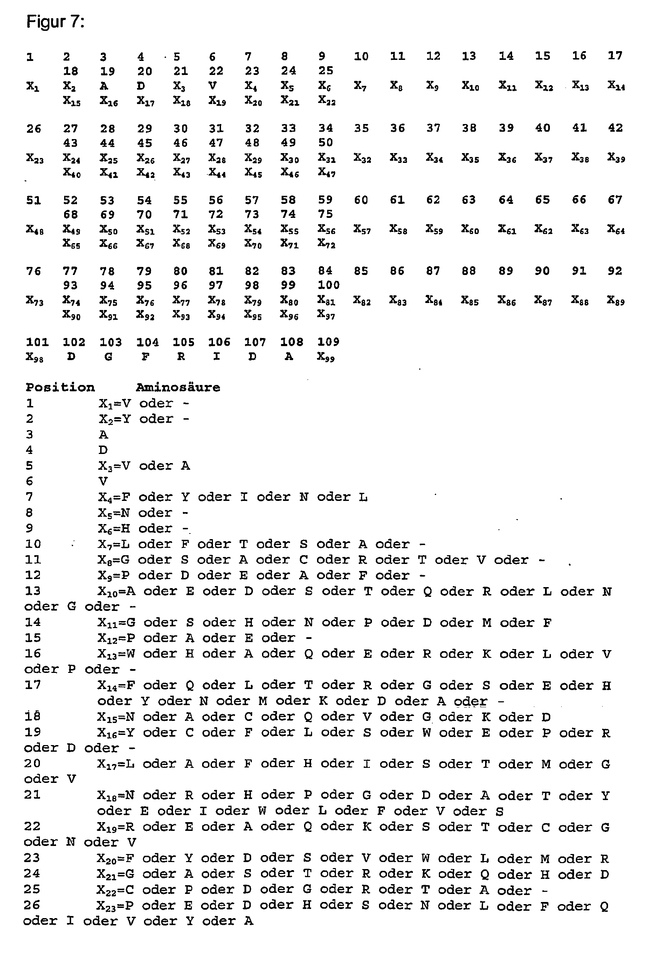

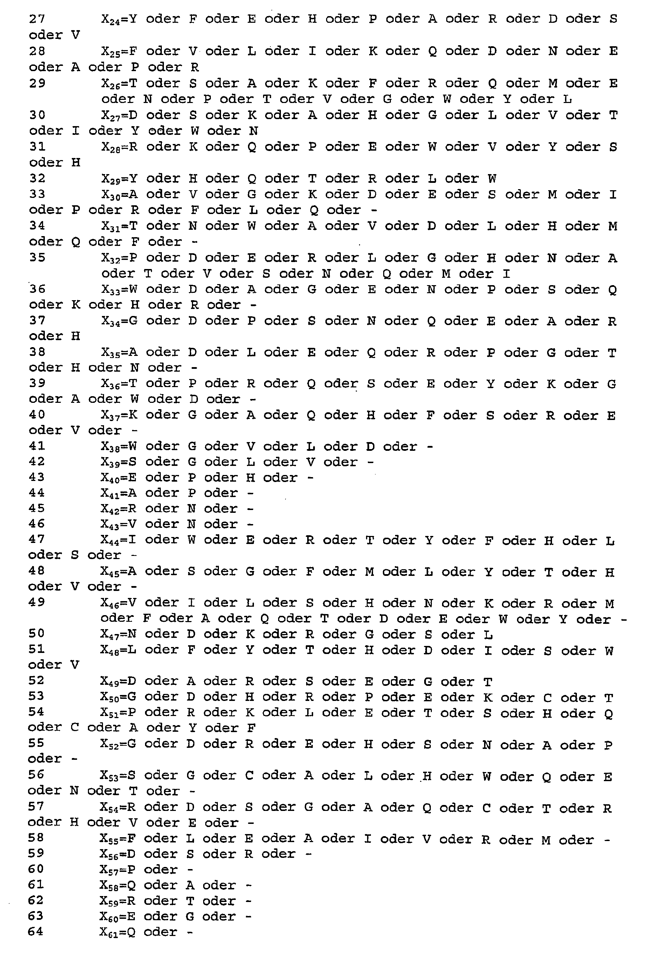

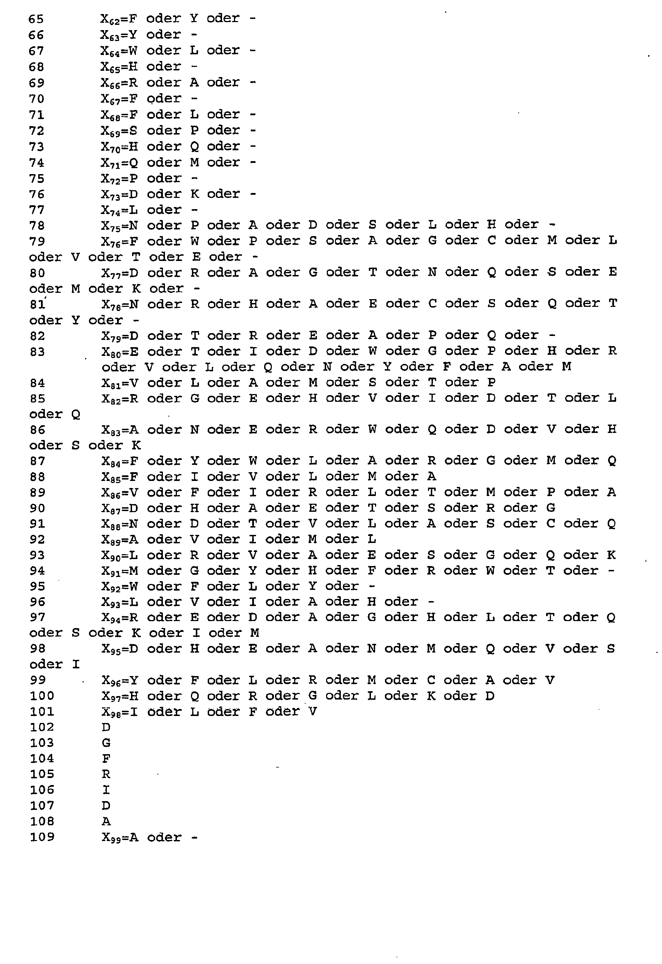

Figur 7: Consensus-Sequenz -B- und Aminosäureaustausche von 99 Glykosylhydrolasen aus Bodenmikroorganismen. Der Consensussequenz liegt ein Alignment mit Clustal X Version 1.64b zugrunde (Standardeinstellungen). Aminosäurepositionen 1-12 und 102-109 sind zum Teil durch die PCR-Primersequenzen (GEX023/210/211/036 und GEX25/038) vorgegeben.

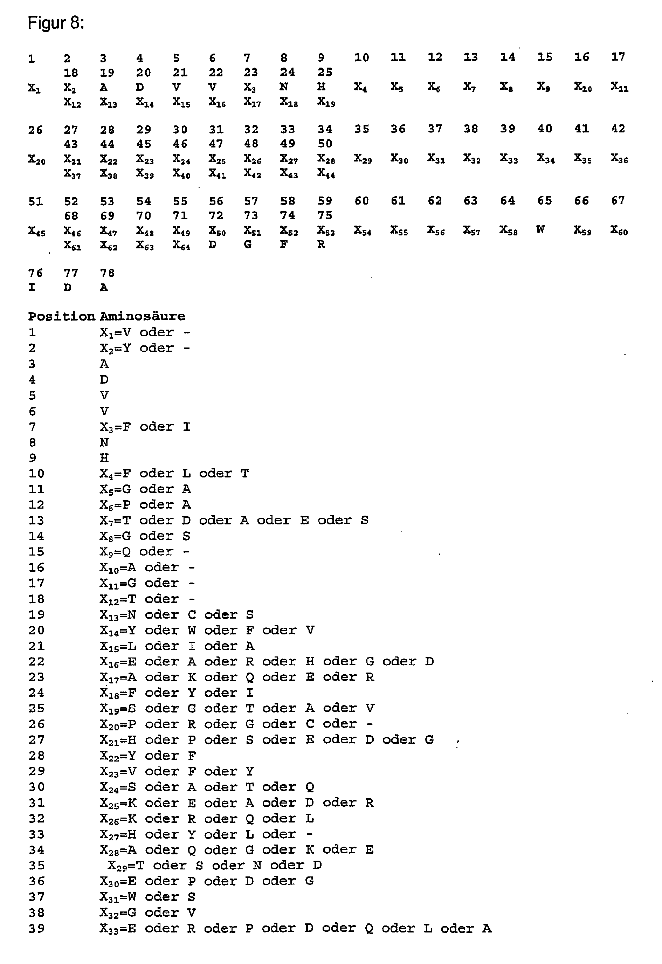

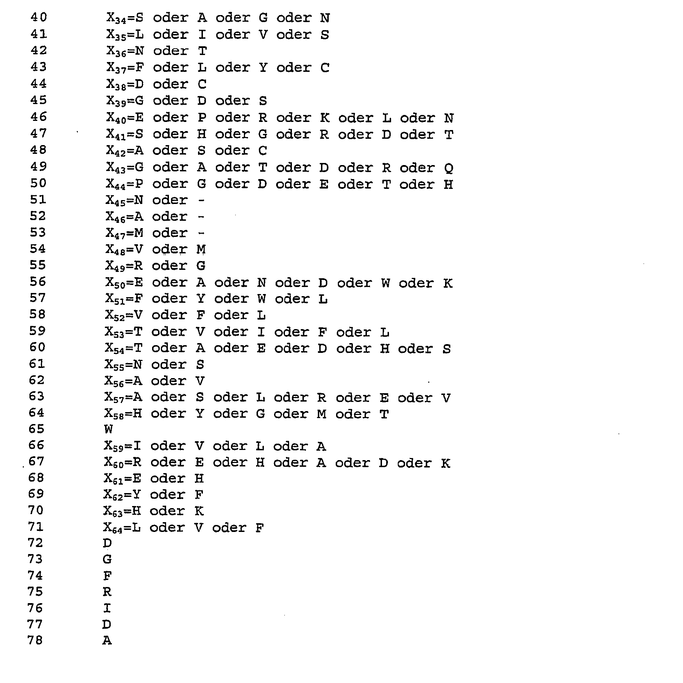

Figur 8: Consensus-Sequenz -C- (Subsequenzraum zu B) und Aminosäureaustausche von 10 Amylasen aus Bodenmikroorganismen. Der Consensussequenz liegt ein Alignment mit Clustal X Version 1.64b zugrunde (Standardeinstellungen). Aminosäurepositionen 1-12 und 72-78 sind zum Teil durch die PCR-Primersequenzen (GEX023/210/211 und GEX25) vorgegeben.

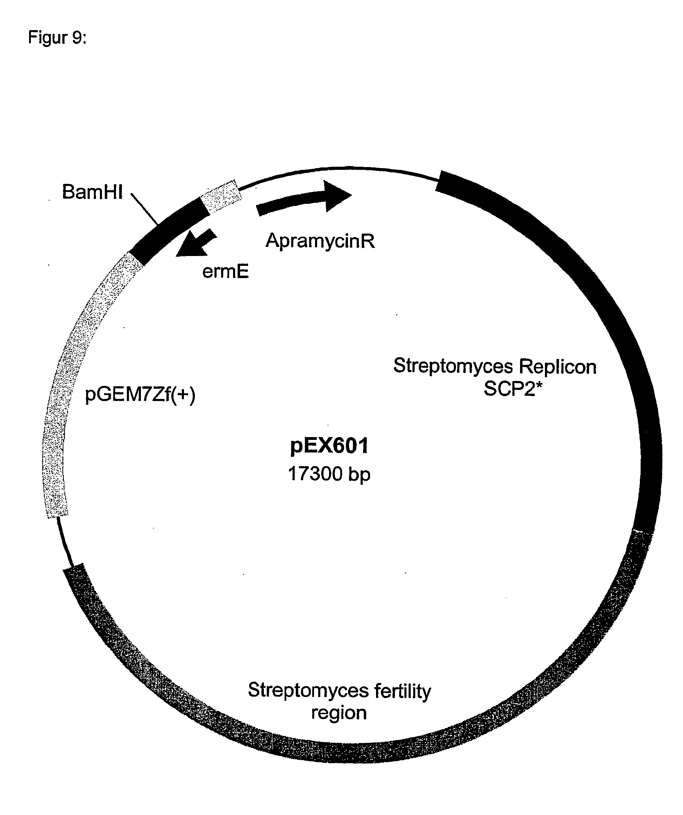

Figur 9: Schematische Darstellung des für die Expression der Metagenom-Amylasen in *Streptomyces lividans* TK 24 verwendeten Plasmid Shuttle-Vektors pEX601.
   Der Vektor wurde durch Einfügen der ermE/MCS/Terminator Kassette (BgIII/EcoRI) aus pWHM680pro in den BamHI/EcoRI verdauten Vektor pWHM601 erzeugt. Die in der Multi-Cloning-Site vorhandene BamHI Erkennungssequenz wurde zur Klonierung der Amylasegene verwendet. Sowohl pWHM601 als auch pWHM680pro stammen aus dem Labor von Dr. C.R. Hutchinson (University of Wisconsin, pers.Kommunikation).
   Die Expression inserierter Gene erfolgt ausgehend vom konstitutiven *erm*E Promotor.
   Die angegebenen Größen des Vektors und der darin enthaltenen Elemente sind Schätzwerte. SCP2* Replicon Replikationsursprung in Streptomyces; *erm*E, *erm*E up Promotor von *S. erythrea;* ApramycinR Apramycin Resistenzgen.
   Die Expression der Amylasegene in *Streptomyces lividans* kann auch mit anderen geeigneten Shuttle-Vektoren wie zum Beispiel pAX5a (Faß S.H., Engels, J.W. 1996; Influence of Specific Signal Peptide Mutations on the Expression and Secretion of the alpha-Amylase Inhibitor Tendamistat in Streptomyces lividans. J. Biol. Chem. 271, Number 25, 15244-15252) erzielt werden.

**Tabelle 1: PCR-Primer zur Amplifikation von Teilsequenzen von für Glykosylhydrolasen kodierenden Sequenzen aus Metagenom-DNA. Symbole für degenerierte Basenpositionen (Mischungen verschiedener Nukleotide): R=AG; Y=CT; M=AC; K=GT; W=AT; S=CG; B=CGT; D=AGT; H=ACT; V=ACG; N=ACGT**

| Name | Sequenz (5'-3') | Orientierung |
|---|---|---|
| GEX 023 | GGTCTACGCCGACGTCGTSWWCAACCA | forward |
| GEX 024 | CGTCGACGGCTTCCGSATCGACRC | forward |
| GEX 025 | GGCGTCGATGCGGAASCCGTC | reverse |
| GEX 026 | GCTCGGTGTCGTGGTTGTCSACGWA | reverse |
| GEX 036 | GTACGCCGACGCCGTNWTHAAYCA | forward |
| GEX 038 | GGCGGCGTCGATCCKRAANCCRTC | reverse |
| GEX 210 | CGACGTGGTGTTCAACCAYYTNGGNCC | forward |
| GEX 211 | GCCGACGTGGTGTTCAAYCAYYTNGG | forward |

**Tabelle 2: Aufstellung der 149 Einzelsequenzen mit den Identitäten zu ihren nächsten Verwandten in GenBank (NCBI; Release 121.0); ermittelt mit dem Programm FASTA am 2.2.2001.**

| Primer | SEQ ID NO. gem. Seq. Prot. | Nächster Datenbankhit | Gen/Produkt | Identität (%) | Organismus |
|---|---|---|---|---|---|
| 23/25 | 107 | AB003697 | oligo-1,6-glucosidase | 74,5 | Bacillus flavocaldarius |
| 23/25 | 108 | AB015615 | isoamylase | 65 | Bacillus flavocaldarius |
| 23/25 | 109 | AB015615 | isoamylase | 61,7 | Orysa sativum |
| 23/25 | 110 | AB015615 | isoamylase | 66,2 | Orysa sativum |
| 23/25 | 111 | AB015615 | isoamylase | 64,2 | Orysa sativum |
| 23/25 | 112 | AB015615 | isoamylase | 68,8 | Orysa sativum |
| 23/25 | 113 | AB031392 | glycogen debranching enzyme | 77,8 | Arthrobacter sp. |
| 23/25 | 114 | AB031392 | glycogen debranching enzyme | 63 | Arthrobacter sp. |
| 23/25 | 115 | AB031392 | glycogen debranching enzyme | 80,2 | Arthrobacter sp. |
| 23/25 | 116 | AB031392 | glycogen debranching enzyme | 77,8 | Arthrobacter sp. |
| 23/25 | 117 | AB031392 | glycogen debranching enzyme | 79 | Arthrobacter sp. |
| 23/25 | 118 | AB031392 | glycogen debranching enzyme | 77,8 | Arthrobacter sp. |
| 23/25 | 119 | AB031392 | glycogen debranching enzyme | 81,5 | Arthrobacter sp. |
| 23/25 | 120 | AB031392 | glycogen debranching enzyme | 76,5 | Arthrobacter sp. |
| 23/25 | 121 | AB031392 | glycogen debranching enzyme | 86,4 | Arthrobacter sp. |
| 23/25 | 122 | AB031392 | glycogen debranching enzyme | 86,8 | Arthrobacter sp. |
| 23/25 | 123 | AB031392 | glycogen debranching enzyme | 80,2 | Arthrobacter sp. |
| 23/25 | 124 | AE001888 | glycogen operon protein GlgX | 76,5 | Deinococcus radiodurans |
| 23/25 | 125 | AE001888 | glycogen operon protein GlgX | 37,8 | Deinococcus radiodurans |
| 23/25 | 126 | AE001888 | glycogen operon protein GlgX | 72,8 | Deinococcus radiodurans |
| 23/25 | 127 | AE001888 | glycogen operon protein GlgX | 77,8 | Deinococcus radiodurans |
| 23/25 | 128 | AE001888 | glycogen operon protein GlgX | 69,1 | Deinococcus radiodurans |
| 23/25 | 129 | AE001888 | glycogen operon protein GlgX | 76,5 | Deinococcus radiodurans |
| 23/25 | 130 | AE001888 | glycogen operon protein GlgX | 76,5 | Deinococcus radiodurans |
| 23/25 | 131 | AE001888 | glycogen operon protein GlgX | 71,6 | Deinococcus radiodurans |
| 23/25 | 132 | AE001888 | glycogen operon protein GlgX | 77,8 | Deinococcus radioduransv |
| 23/25 | 133 | AE001888 | glycogen operon protein GlgX | 77,8 | Deinococcus radiodurans |
| 23/25 | 134 | AE001905 | maltooligosyltrehalose trehalohydrolase | 45,6 | Deinococcus radiodurans |
| 23/25 | 135 | AE001905 | maltooligosyltrehalose trehalohydrolase | 50,7 | Deinococcus radiodurans |
| 23/25 | 136 | AE004643 | probable glycosyl hydrolase | 38,9 | Pseudomonas aeruginosa |
| 23/25 | 137 | AE004643 | probable glycosyl hydrolase | 53,8 | Pseudomonas aeruginosa |
| 23/25 | 138 | AF002109 | putative isoamylase | 71,2 | Arabidopsis thaliana |
| 23/25 | 139 | AF002109 | putative isoamylase | 62 | Arabidopsis thaliana |
| 23/25 | 140 | AF002109 | putative isoamylase | 60,8 | Arabidopsis thaliana |
| 23/25 | 141 | AF002109 | putative isoamylase | 62 | Arabidopsis thaliana |
| 23/25 | 142 | AF002109 | putative isoamylase | 64,6 | Arabidopsis thaliana |
| 23/25 | 143 | AF002109 | putative isoamylase | 65,4 | Arabidopsis thaliana |
| 23/25 | 144 | AF002109 | putative isoamylase | 60,8 | Arabidopsis thaliana |
| 23/25 | 145 | AF002109 | putative isoamylase | 62 | Arabidopsis thaliana |
| 23/25 | 146 | AF142590 | isoamylase 1 | 66,7 | Triticum aestivum |
| 23/25 | 147 | AF142590 | isoamylase 1 | 53,2 | Triticum aestivum |
| 23/25 | 148 | AF201335 | maltooligosyl trehalose trehalohydrolase | 47,1 | Sulfolobus shibatae |
| 23/25 | 149 | AJ001206 | putative glycogen debranching enzyme | 82,7 | Streptomyces coelicolor |
| 23/25 | 150 | AJ133789 | cyclomaltodextrinase | 91 | Alicyclobacillus acidocaldarius |
| 23/25 | 151 | AJ133789 | cyclomaltodextrinase | 93,3 | Alicyclobacillus acidocaldarius |
| 23/25 | 152 | AJ133789 | cyclomaltodextrinase | 92,1 | Alicyclobacillus acidocaldarius |
| 23/25 | 153 | AJ291603 | glycogen debranching enzyme | 70,4 | Rhizobium tropici |
| 23/25 | 154 | AJ291603 | glycogen debranching enzyme | 44,4 | Rhizobium tropici |
| 23/25 | 155 | AJ291603 | glycogen debranching enzyme | 41,9 | Rhizobium tropici |
| 23/25 | 156 | AL157916 | putative glycosyl hydrolase | 60 | Streptomyces coelicolor |
| 23/25 | 157 | AL355752 | secreted alpha-amylase/dextrinas e | 93,5 | Streptomyces coelicolor |
| 23/25 | 158 | AL356932 | glycogen debranching enzyme | 79 | Streptomyces coelicolor |
| 23/25 | 159 | AL356932 | putative glycogen debranching enzyme | 95,1 | Streptomyces coelicolor |
| 23/25 | 160 | AL512975 | glycogen operon protein GlgX | 71,6 | Sulfolobus solfataricus |
| 23/25 | 161 | AL512975 | glycogen operon protein GlgX | 75,3 | Sulfolobus solfataricus |
| 23/25 | 162 | AL512975 | alpha-amylase | 52,9 | Sulfolobus solfataricus |
| 23/25 | 163 | AL512975 | glycogen operon protein GlgX | 68,8 | Sulfolobus solfataricus |
| 23/25 | 164 | AL512975 | alpha-amylase | 50 | Sulfolobus solfataricus |
| 23/25 | 165 | AL512975 | glycogen operon protein GlgX | 75,3 | Sulfolobus solfataricus |
| 23/25 | 166 | AL512975 | glycogen operon protein GlgX | 70,4 | Sulfolobus solfataricus |
| 23/25 | 167 | AL512975 | glycogen operon protein GlgX | 70,4 | Sulfolobus solfataricus |
| 23/25 | 168 | AL512975 | glycogen operon protein GlgX | 72,8 | Sulfolobus solfataricus |
| 23/25 | 169 | AL512975 | alpha-amylase | 62,7 | Sulfolobus solfataricus |
| 23/25 | 170 | AL512975 | alpha-amylase | 50 | Sulfolobus solfataricus |
| 23/25 | 171 | AL512975 | alpha-amylase | 50 | Sulfolobus solfataricus |
| 23/25 | 172 | AL512975 | glycogen operon protein GlgX | 69,1 | Sulfolobus solfataricus |
| 23/25 | 173 | AL512975 | glycogen operon protein GlgX | 75,3 | Sulfolobus solfataricus |
| 23/25 | 174 | AL512975 | glycogen operon protein GlgX | 56,8 | Sulfolobus solfataricus |
| 23/25 | 175 | AL589708 | glycogen debranching enzyme | 74,1 | Streptomyces coelicolor |
| 23/25 | 176 | AX063741 | putative ORF | 72 | Corynebacterium glutamicum |
| 23/25 | 177 | D63343 | maltooligosyl trehalose trehalohydrolase | 62,9 | Arthrobacter sp. |
| 23/25 | 178 | D63343 | maltooligosyl trehalose trehalohydrolase | 62,7 | Arthrobacter sp. |
| 23/25 | 179 | D78001 | maltooligosyl trehalose trehalohydrolase | 50,7 | Rhizobium sp |
| 23/25 | 180 | D83245 | glycogen debranching enzyme | 56,8 | Sulfolobus acidocaldarius |
| 23/25 | 181 | D83245 | glycogen debranchinq enzyme | 74,1 | Sulfolobus acidocaldarius |
| 23/25 | 182 | D83245 | glycogen debranching enzyme | 61,7 | Sulfolobus acidocaldarius |
| 23/25 | 183 | D83245 | glycogen debranching enzyme | 74,1 | Sulfolobus acidocaldarius |
| 23/25 | 184 | D83245 | glycogen debranching enzyme | 80,2 | Sulfolobus acidocaldarius |
| 23/25 | 185 | D83245 | glycogen debranching enzyme | 69,1 | Sulfolobus acidocaldarius |
| 23/25 | 186 | D83245 | glycogen debranching enzyme | 70,4 | Sulfolobus acidocaldarius |
| 23/25 | 187 | D83245 | glycogen debranching enzyme | 71,6 | Sulfolobus acidocaldarius |
| 23/25 | 188 | D90900 | glycogen operon protein GlgX | 64,2 | Synechocystis sp |
| 23/25 | 189 | D90900 | glycogen operon protein GlgX | 64,2 | Synechocystis sp |
| 23/25 | 190 | D90908 | glycogen operon protein GlgX | 55,6 | Synechocystis sp |
| 210-211/2 5 | 191 | D90908 | glycogen operon protein GlgX | 91,1 | Synechocystis sp |
| 210-211/2 5 | 192 | D90908 | glycogen operon protein GlgX | 88,6 | Synechocystis sp |
| 210-211/2 5 | 193 | D90908 | glycogen operon protein GlgX | 56,8 | Synechocystis sp |
| 210-211/2 5 | 194 | U18997 | glgX | 69,6 | E. coli |
| 210-211/2 5 | 195 | Y08256 | glycogen operon protein GlgX | 71,6 | Sulfolobus solfataricus |
| 210-211/2 5 | 196 | Y08256 | glycogen operon protein GlgX | 72,8 | Sulfolobus solfataricus |
| 210-211/2 5 | 197 | Y08256 | glycogen operon protein GlgX | 58 | Sulfolobus solfataricus |
| 210-211/2 5 | 198 | Y08256 | alpha-amylase precursor | 51,5 | Sulfolobus solfataricus |
| 210-211/2 5 | 199 | Y08256 | alpha-amylase precursor | 59,7 | Sulfolobus solfataricus |
| 210-211/2 5 | 200 | Y08256 | alpha-amylase precursor | 52,9 | Sulfolobus solfataricus |
| 36/38 | 201 | Y08256 ' | alpha-amylase precursor | 44,8 | Sulfolobus solfataricus |
| 36/38 | 202 | Y08256 | glycogen operon protein GlgX | 70,4 | Sulfolobus solfataricus |
| 36/38 | 203 | Z74020 | glgX | 36,4 | Mycobacterium tuberculosis |
| 36/38 | 204 | Z74020 | glgX | 74,1 | Mycobacterium tuberculosis |
| 36/38 | 205 | Z74020 | glgX | 77,8 | Mycobacterium tuberculosis |
| 24/26 | 7 | A20154 | alpha-amylase | 42 | Bacillus amyloliquefaciens |
| 24/26 | 8 | AE006357 | alpha-amylase | 44,3 | Lactococcus lactis |
| 24/26 | 9 | AF153911 | beta-agarase | 60 | Pseudomonas sp. |
| 24/26 | 10 | AF153911 | beta-agarase | 58 | Pseudomonas sp. |
| 24/26 | 11 | AF153911 | beta-agarase | 59 | Pseudomonas sp. |
| 24/26 | 12 | AF153911 | beta-agarase | 60 | Pseudomonas sp. |
| 24/26 | 13 | AF153911 | beta-agarase | 58 | Pseudomonas sp. |
| 24/26 | 14 | AF208003 | alpha-amylase | 45,4 | Diabrotica virgifera |
| 24/26 | 15 | AL352956 | secreted alpha-amylase | 74 | Streptomyces coelicolor |
| 24/26 | 16 | AL352956 | secreted alpha-amylase | 89 | Streptomyces coelicolor |
| 24/26 | 17 | AL352956 | secreted alpha-amylase | 81 | Streptomyces coelicolor |
| 24/26 | 18 | AL352956 | secreted alpha-amylase | 88 | Streptomyces coelicolor |
| 24/26 | 19 | AL352956 | secreted alpha-amylase | 80 | Streptomyces coelicolor |
| 24/26 | 20 | AL352956 | secreted alpha-amylase | 80 | Streptomyces coelicolor |
| 24/26 | 21 | AL352956 | secreted alpha-amylase | 80 | Streptomyces coelicolor |
| 24/26 | 22 | AL352956 | secreted alpha-amylase | 82 | Streptomyces coelicolor |
| 24/26 | 23 | AX036894 | unnamed protein product | 54,2 | Bacillus stearothermophilus |
| 24/26 | 24 | J01542 | alpha-amylase protein precursor | 42,9 | Bacillus amyloliquefaciens |
| 24/26 | 25 | L01642 | alpha-amylase | 56,6 | E.coli |
| 24/26 | 26 | L01642 | alpha-amylase | 52,8 | E.coli |
| 24/26 | 27 | M13255 | amyS | 52,3 | B.stearothermop hilus |
| 24/26 | 28 | M13255 | amyS | 57 | B.stearothermop hilus |
| 24/26 | 29 | M13255 | amyS | 50,5 | B.stearothermop hilus |
| 24/26 | 30 | M13255 | amyS | 52,3 | B.stearothermop hilus |
| 24/26 | 31 | M13255 | amyS | 50,5 | B.stearothermop hilus |
| 24/26 | 32 | M18244 | alpha-amylase | 55,4 | Streptomyces limosus |
| 24/26 | 33 | M25263 | alpha-amylase | 65 | Streptomyces venezuelae |
| 24/26 | 34 | M25263 | alpha-amylase | 91 | Streptomyces venezuelae |
| 24/26 | 35 | U51129 | amylase | 85 | Streptomyces albus |
| 24/26 | 36 | U51129 | amylase | 85 | Streptomyces albus |
| 24/26 | 37 | U75445 | alpha-amylase | 56,1 | Bacillus sp. MK 716 |
| 24/26 | 38 | U75445 | alpha-amylase | 54,2 | Bacillus sp. MK 717 |
| 24/26 | 39 | U75445 | alpha-amylase | 55,9 | Bacillus sp. MK 718 |
| 24/26 | 40 | X15752 | cyclomaltodextrin glucanotransferase | 33,9 | Bacillus licheniformis |
| 24/26 | 41 | X57568 | alpha-amylase | 64 | Streptomyces griseus |
| 24/26 | 42 | X57568 | alpha-amylase | 56,4 | Streptomyces griseus |
| 24/26 | 43 | X57568 | alpha-amylase | 75 | Streptomyces qriseus |
| 24/26 | 44 | X57568 | alpha-amylase | 45,2 | Streptomyces qriseus |
| 24/26 | 45 | X57568 | alpha-amylase | 47,1 | Streptomyces griseus |
| 24/26 | 46 | X59279 | putative alphaglucanotransferase /hydrolase | 35,1 | Anabaena variabilis |
| 24/26 | 47 | X70255 | alpha-amylase | 43,1 | Streptomyces lividans |
| 24/26 | 48 | X70255 | alpha-amylase | 44 | Streptomyces lividans |
| 24/26 | 49 | Y13332 | alpha-amylase | 58,4 | Streptomyces sp. |
| 24/26 | 50 | Y13332 | alpha-amylase | 94 | Streptomyces sp. |
| 24/26 | 51 | Y13332 | alpha-amylase | 89 | Streptomyces sp. |
| 24/26 | 52 | Y13332 | alpha-amylase | 93 | Streptomyces sp. |
| 24/26 | 53 | Y13332 | alpha-amylase | 86 | Streptomyces sp. |
| 24/26 | 54 | Y13332 | alpha-amylase | 91 | Streptomyces sp. |
| 24/26 | 55 | Y13332 | alpha-amylase | 92 | Streptomyces sp. |
| 24/26 | 56 | Y13332 | alpha-amylase | 92 | Streptomyces sp. |

**Tabelle 3: Temperaturprofil der α-Amylase aus HA11**

| **Temperatur** | Aktivität relativ zu der von 45°C |
|---|---|
| **[°C]** | |
| | **[%]** |
| 45 | 100 |
| 46,4 | 123 |
| 50,4 | 146 |
| 55,8 | 121 |
| 61 | 38 |
| 64,7 | 28 |

**Tabelle 4: Ha13 Amylase**

| Bestimmt wurde das Temperaturprofil (analog HA11) sowie die Temperaturstabilität nach Vorinkubation für 15 min. Danach wurde die Restaktivität bei 50°C in 100 mM Tris HCl pH8,6 bestimmt. Substratumsetzung im Temperaturgradienten von 50°C bis 69.7°C im DNSS-Assay (Tris-HCl pH8,6). | |
|---|---|
| Temperatur | Prozent Restaktivität |
| 50°C | 100 |
| 51.4°C | 110 |
| 55.5°C | 139 |
| 60.8°C | 118 |
| 66°C | 98 |
| 69.7°C | 41 |

**Tabelle 5: Temperaturstabilität der Amylasedomäne aus HA13**

| Substratumsetzung im DNSS-Assay nach Vorinkubation für 15 min. im Temperaturgradienten von 50°C bis 69.7°C, anschliessend Substratumsetzung bei 50°C in Tris-HCl pH8,6. | |
|---|---|
| Temperatur | Prozent Restaktivität |
| 50°C | 100 |
| 51.4°C | 97 |
| 55.5°C | 82 |
| 60.8°C | 38 |
| 66°C | 2 |
| 69.7°C | 2 |

**Tabelle 6: pH-Wert Stabilität der Amylasen HA11 und HA 13**

| Substratumsetzung im jeweiligen Puffersystem im DNSS-Assay bei 50°C für 15 min. | | | |
|---|---|---|---|
| pH-Wert | Puffer | Prozent Restaktivität | |
| | | HA 11 | HA 13 |
| 4,0 | Glycin-HCl | 60 | 90 |
| 4,5 | Glycin-HCl | 59 | 96 |
| 5,0 | Na-Acetat | 110 | 107 |
| 6,5 | Na-Acetat | 104 | 106 |
| 6,5 | Tris-Maleat | 100 | 100 |
| 8,6 | Tris-HCl | 64 | 99 |
| 10 | Glycin-NaOH | 41 | 83 |
| 12 | Glycin-NaOH | 6 | 74 |

**Tabelle 7: Einfluß von Tensiden auf Aktivität der Amylase HA 11**

| Substratumsetzung im DNSS-Assay mit 100mM Tris-HCl pH 8,6 bei 50°C für 15 min. | |
|---|---|
| Zusatz Tenside | Prozent Restaktivität |
| + 0,05 % SDS | 100 |
| + 0,10 % SDS | 86 |
| + 0,50 % SDS | 66 |
| + 1,00 % SDS | 49 |

**Tabelle 8: Einfluß von Tensiden auf die Aktivität der AmylaseHA 13**

| Substratumsetzung im DNSS-Assay mit 100mM Tris-HCl pH 8,6 bei 50°C für 15 min. | |
|---|---|
| Zusatz Tenside | Prozent Restaktivität |
| ohne SDS | 100 |
| + 0,05 % SDS | 90 |
| + 0,10 % SDS | 74 |
| + 0,50 % SDS | 71 |
| + 1,00 % SDS | 67 |

**Tabelle 9: Einfluß von Bleichmitteln auf die HA11 Amylase**

| Substratumsetzung im DNSS-Assay mit 100mM Glycin-NaOH pH 9,5. Nach Vorinkubation bei Raumtemperatur unter entsprechenden Bedingungen erfolgte eine weitere Inkubation bei 50°C für 15 min. | |
|---|---|
| Bleichmittel | Prozent Restaktivität |
| ohne H2O2 | 100 |
| + 0,05 % H2O2 | 99 |
| + 0,10 % H2O2 | 87 |
| + 0,20 % H2O2 | 4 |
| + 0,35 % H2O2 | 1 |
| + 0,70 % H2O2 | 1 |

**Tabelle 10: Einfluß von Bleichmitteln auf die Amylase HA13**

| Substratumsetzung im DNSS-Assay mit 100mM Glycin-NaOH pH 9,5. Nach Vorinkubation bei Raumtemperatur unter entsprechenden Bedingungen erfolgte eine weitere Inkubation bei 50°C für 15 min. | |
|---|---|
| Bleichmittel | Prozent Restaktivität |
| ohne H2O2 | 100 |
| + 0,05 % H2O2 | 81 |
| + 0,10 % H202 | 85 |
| + 0,20 % H2O2 | 80 |
| + 0,35 % H2O2 | 68 |
| + 0,70 % H2O2 | 60 |

**Tabelle 11: Calciumabhängigkeit der HA11Amylase**

| Substratumsetzung im DNSS-Assay mit 100mM Tris-HCl pH 8,6 bei 50°C für 15 min. | |
|---|---|
| Zusatz | Prozent Restaktivität |
| 2 mM CaCl2 | 100 |
| 1 mM EDTA | 44 |

**Tabelle 12: Calciumabhängigkeit der Amylasedomäne HA13**

| Substratumsetzung im DNSS-Assay mit 100mM Tris-HCl pH 8,6 bei 50°C für 15 min. | |
|---|---|
| Zusatz | Prozent Restaktivität |
| 2 mM CaCl2 | 100 |
| 1 mM EDTA | 104 |

**Tabelle 13: Untersuchung der Waschleistung**

| | mit Bleiche | | ohne Bleiche | |
|---|---|---|---|---|
| | CFT PCS 26 | CFT PCS 26 | CFT PCS 26 | CFT PCS 26 |
| ohne Enzym | 3,8 | 0,4 | 3,7 | 0,2 |
| HA11 (S213) | 7,7 | 3 | 9,4 | 4,5 |
| HA 13 (S213Pb) | 5,7 | 2 | 5,9 | 1,8 |
| BAN | 4,2 | 0,7 | 7,3 | 2,9 |
| Termamyl | 5,6 | 1,7 | 8,1 | 3,5 |

### SEQUENZPROTOKOLL - SEQUENCE LISTING

<110> Henkel Kommanditgesellschaft auf Aktien
<120> Neue Glykosylhydrolasen
<130> H 05206 PCT
<150> 101 63 748.9-41
   <151> 2001-12-21
<160> 316
<170> SeqWin99, version 1.02
<210> 1
   <211> 700
   <212> PRT
   <213> Unknown
<220>
   <223> Metagenom
<400> 1
   Met Thr Gly Leu Pro Asp Ser Gly Thr Leu Arg Arg Pro Gly Pro Arg
   1               5                   10                  15
   Lys Arg Leu Arg Thr Leu Ile Ala Val Thr Ala Ala Ala Val Leu Ala
               20                  25                  30
   Gly Leu Ala Ala Ile Gly Pro Gly Ala Pro Ala Gly Pro Ala Glu Ala
           35                  40                  45
   Ala Ala Pro Gly Pro Lys Asp Ala Thr Ala Val Leu Phe Ser Tyr Thr
       50                  55                  60
   Trp Asn Ala Ile Ala Arg Glu Cys Thr Glu Asn Leu Gly Pro Ala Gly
   65                  70                  75                  80
   Tyr Gly Tyr Val Gln Thr Ser Pro Pro Gln Glu His Val Gln Gly Pro
                   85                  90                  95
   Gln Trp Trp Ile His Tyr Gln Pro Val Ser Tyr Arg Val Glu Ser Arg
               100                 105                 110
   Leu Gly Thr Arg Ala Glu Phe Lys Ala Met Val Asp Thr Cys His Ala
           115                 120                 125
   Ala Gly Val Lys Val Ile Ala Asp Ala Val Ile Asn His Met Ser Gly
       130                 135                 140
   Tyr Ser Ala Gly Gly Val Gly Trp Ala Gly Ser Ser Phe Gln His Tyr
   145                 150                 155                 160
   Ser Tyr Pro Gly Ile Tyr Gln Ser Gln Asp Phe His Ser Cys Arg Arg
                   165                 170                 175
   Asp Ile Ala Asn Tyr Gln Asp Arg Trp Glu Val Gln Glu Cys Asn Leu
               180                 185                 190
   Val Asn Leu Ala Asp Leu Asn Thr Gly Ser Ala Tyr Val Gln Gly Arg
           195                 200                 205
   Ile Ala Ala Tyr Leu Asn Asp Leu Val Ser Leu Gly Val Asp Gly Phe
       210                 215                 220
   Arg Ile Asp Ala Val Lys His Ile Ser Ala Ala Asp Met Asn Gly Ile
   225                 230                 235                 240
   Leu Ser Arg Val Asn Asp Arg Ala Arg Leu Tyr Leu Val Gln Glu Val
                   245                 250                 255
   Ile Arg Ala Asn Glu Pro Val Gln Pro Glu Glu Tyr Leu Gly Leu Gly
               260                 265                 270
   Asp Ile His Glu Phe Ala Tyr Ala Arg Lys Leu Lys Glu Ala Phe Gly
           275                 280                 285
   Gly Arg Thr Leu Asn Trp Leu Ile Ser Gly Ala Gly Ile Gly Pro Ser
       290                 295                 300
   Trp Ala Gly Phe Leu Gln Asn Ala Asp Ala Ala Val Phe Val Asp Asn
   305                 310                 315                 320
   His Asp Thr Glu Arg Asn Gly Glu Thr Leu Ser Tyr Arg Asp Gly Ala
                   325                 330                 335
   Ala Tyr Asp Leu Ala Gln Val Phe Thr Leu Ala Trp Asn Tyr Gly Ser
               340                 345                 350
   Pro Ser Ile His Ser Gly Tyr Gln Phe Ser Asn Lys Asp Ala Gly Pro
           355                 360                 365
   Ala Leu Ala Gly Asn Gly Arg Val Val Asp Pro Val Cys Gly Gln Asn
       370                 375                 380
   Gly Trp Thr Cys Lys His Ala Gln Thr Asn Ile Glu Asn Met Val Gly
   385                 390                 395                 400
   Phe Arg Thr Ala Thr Tyr Gly Thr Ala Ile Thr Asn Lys Trp Asp Asn
                   405                 410                 415
   Gly Ser Ser Ala Ile Ala Phe Gly Arg Gly Asp Lys Gly Phe Val Ala
               420                 425                 430
   Ile Asn Arg Gly Thr Ala Ala Val Asp Arg Thr Trp Gln Thr Ser Leu
           435                 440                 445
   Pro Ala Gly Arg Tyr Cys Asn Val Ile Val Gly Leu Pro Thr Ala Ser
       450                 455                 460
   Gly Cys Ser Ala Gly Gly Val Ile Thr Val Asp Ser Ser Gly Arg Phe
   465                 470                 475                 480
   Ala Ala Ser Val Ala Ala Asp Thr Ala Leu Ala Leu His Val Gly Ala
                   485                 490                 495
   Lys Ala Gly Gly Thr Gly Thr Thr Pro Pro Pro Thr Gly Ser Thr Met
               500                 505                 510
   Thr Val Tyr Phe Ala Thr Thr Lys Gly Trp Thr Asn His Tyr Val His
           515                 520                 525
   His Arg Val Gly Ser Gly Ala Trp Thr Ala Leu Pro Gly Ala Ala Met
       530                 535                 540
   Ala Ala Ala Cys Thr Gly Trp Val Ser Arg Thr Ile Asp Leu Gly Ser
   545 550 555 560
   Ala Thr Gly Ile Thr Ala Ala Phe Thr Asn Gly Ala Gly Ala Trp Asp
   565 570 575
   Asn Asn Gly Gly Arg Asp Tyr Ala Leu Thr Gly Ser Val Ala Ala Val
   580 585 590
   Lys Asp Gly Val Val Thr Ala Thr Asn Pro Cys Ala Ala Gln Pro Thr
   595 600 605
   Ser Thr Thr Val Tyr Tyr Ala Thr Gly Trp Ser Thr Ala Asn Ile His
   610 615 620
   Tyr Arg Val Gly Ser Gly Ala Trp Thr Ala Val Pro Gly Val Ala Met
   625 630 635 640
   Ala Asn Ala Cys Ala Gly Trp Lys Ser Lys Thr Ile Glu Leu Gly Ala
   645 650 655
   Ala Ser Gly Ile Thr Ala Ala Phe Asn Asn Gly Ala Gly Thr Trp Asp
   660 665 670
   Asn Asn Gly Gly Lys Asp Tyr Ala Ile Gly Ala Gly Ala Met Lys Val
   675 680 685
   Gln Asn Gly Thr Val Thr Ala Gly Asn Pro Cys Ser
   690 695 700
<210> 2
   <211> 2013
   <212> PRT
   <213> Unknown
<220>
   <223> Metagenom
<400> 2
   Met Thr His Arg Ala Arg Ala Arg Ser Trp Leu Ala Leu Gly Val Thr
   1               5                   10                  15
   Gly Ala Val Ala Ala Ser Val Leu Ala Val Val Pro Ile Gly Ala Ala
               20                  25                  30
   Ala Glu Glu Gly Asp Thr Phe Ala Leu Val Gly Ser Leu Gln Ser Glu
           35                  40                  45
   Leu Gly Cys Ser Glu Asp Trp Gln Pro Ser Cys Glu Ala Thr Glu Leu
       50                  55                  60
   Leu Pro Thr Asp Thr Ala Gly Leu Tyr Ala Ala Glu Phe Thr Val Pro
   65                  70                  75                  80
   Ala Gly Ser Tyr Glu Tyr Lys Val Ala Ala Asn Asp Ser Trp Asp Ala
                   85                  90                  95
   Ser Trp Gly Leu Asp Gly Gly Gly Asp Asn Ile Pro Leu Thr Val Gly
               100                 105                 110
   Gly Asp Thr Asp Val Arg Val Val Phe Asp Asp Thr Gln Lys Arg Val
           115                 120                 125
   Gly Leu Glu Leu Leu Ser Thr Arg Gly Ala Tyr Asp Glu Gln Ala Asp
       130                 135                 140
   Ala Ala Leu Ala Leu Pro Pro Val Arg Gln Pro Gly Ser Ala Glu Asn
   145                 150                 155                 160
   Phe Tyr Phe Val Met Thr Asp Arg Phe Ala Asn Gly Asp Glu Ser Asn
                   165                 170                 175
   Asp Thr Ala Gly Ile Glu Gly Asp Arg Leu Ala His Gly Phe Asp Pro
               180                 185                 190
   Thr Asp Lys Gly Phe Tyr His Gly Gly Asp Ile Gln Gly Ile Arg Asp
           195                 200                 205
   His Leu Asp Tyr Ile Glu Gly Leu Gly Thr Thr Ala Ile Trp Phe Thr
       210                 215                 220
   Pro Ser Phe Lys Asn Lys Pro Val Gln Gly Glu Gly Ala Asn Ala Ser
   225                 230                 235                 240
   Ala Gly Tyr His Gly Tyr Trp Ile Thr Asp Phe Thr Gln Ile Asp Pro
                   245                 250                 255
   His Leu Gly Thr Asn Ala Glu Leu Glu Ala Leu Ile Asp Glu Ala His
               260                 265                 270
   Glu Arg Gly Ile Lys Val Tyr Phe Asp Ile Ile Thr Asn His Thr Ala
           275                 280                 285
   Asp Val Ile Ser Tyr Glu Glu Gly Gln Tyr Ser Tyr Ile Asp Lys Ala
       290                 295                 300
   Thr Ser Pro Tyr Arg Asp Ala Asp Gly Asn Val Phe Asp Pro Ser Thr
   305                 310                 315                 320
   Ile Ala Gly Ser Ala Asp Phe Pro Ala Leu Asp Pro Ala Thr Ser Phe
                   325                 330                 335
   Pro Tyr Thr Pro Thr Val Ala Pro Glu Glu Ala Asp Val Lys Val Pro
               340                 345                 350
   Ala Trp Leu Asn Asp Pro Thr Leu Tyr His Asn Arg Gly Asp Ser Thr
           355                 360                 365
   Trp Ser Gly Glu Ser Val Thr Phe Gly Asp Phe Val Gly Leu Asp Asp
       370                 375                 380
   Leu Met Thr Glu His Pro Thr Val Val Asp Gly Phe Ile Glu Val Tyr
   385                 390                 395                 400
   Gln Asp Trp Val Asp Leu Gly Ile Asp Gly Phe Arg Ile Asp Thr Ala
                   405                 410                 415
   Lys His Val Asn Phe Glu Phe Trp Glu Glu Trp Ser Thr Glu Val Leu
               420                 425                 430
   Asp Tyr Ala His Ala Gln Gly Lys Pro Asp Phe Phe Met Phe Gly Glu
           435                 440                 445
   Val Tyr Asp Ala Asp Pro Val Lys Leu Ser Pro Tyr Val Arg Lys Thr
       450                 455                 460
   Asp Met Asn Ser Val Leu Asp Phe Thr Phe Gln Ser Gln Ala Val Ser
   465 470 475 480
   Phe Ala Ala Gly Asn Ser Ala Lys Asn Leu Gln Ser Leu Phe Ala Gly
   485 490 495
   Asp Asp Tyr Tyr Thr Thr Pro Asp Ser Ser Ala Thr Ala Leu Pro Thr
   500 505 510
   Phe Leu Gly Asn His Asp Met Gly Arg Ile Gly Tyr Phe Leu Ala Asn
   515 520 525
   Ser Gly Ala Thr Ala Val Gln Arg Asn Glu Leu Ala His Glu Leu Met
   530 535 540
   Tyr Leu Thr Arg Gly Gln Pro Val Val Tyr Tyr Gly Asp Glu Gln Gly
   545 550 555 560
   Phe Ala Gly Thr Gly Gly Asp Lys Asp Ala Arg Gln Thr Leu Phe Ala
   565 570 575
   Ser Gln Val Asp Glu Tyr Val Asn Gln Pro Leu Val Thr Gly Glu Asn
   580 585 590
   Ala Gly Ser Val Asp Arg Tyr Gly Thr Asp Ala Pro Leu Tyr Glu His
   595 600 605
   Ile Ala Gly Leu Ala Glu Leu Arg Lys Ala His Pro Ala Leu Glu Gln
   610 615 620
   Gly Ala Gln Ile Glu Arg Phe Val Ala Asp Gly Ala Ser Val Tyr Ala
   625 630 635 640
   Phe Ser Arg Val Asp Arg Asp Glu Lys Val Glu Tyr Leu Val Ala Val
   645 650 655
   Asn Asn Ala Asn Glu Ala Lys Ala Val Glu Val Ser Thr Leu Thr Ala
   660 665 670
   Asp Gly Ala Phe Glu Val Leu Tyr Gly Asp Gly Gln Ala Leu Thr Ala
   675 680 685
   Asn Ala Asp Gly Val Ala Ser Val Thr Ala Pro Ala Leu Gly Ala Val
   690 695 700
   Val Trp Lys Ala Asp Arg Glu Val Thr Ala Pro Glu Ala Ala Ser Ala
   705 710 715 720
   Ile Ala Val Asp Val Pro Val Ala Gly Ala Gly Val Thr Gly Ile Ala
   725 730 735
   Pro Val Ser Ala Asp Val Ala Asp Ala Thr Trp Gln Glu Thr Ser Phe
   740 745 750
   Ala Trp Arg Val Ala Gly Ser Asp Glu Trp Thr Pro Leu Gly Thr Ala
   755 760 765
   Glu Asp Thr Thr Pro Arg Val Tyr His Asp Thr Ala Gly Leu Ala Lys
   770 775 780
   Gly Thr Leu Val Glu Tyr Arg Ala Val Ser Thr Asp Ala Ala Gly Asn
   785 790 795 800
   Arg Ala Ala Ala Ser Thr Tyr Ala Ser Val Gly Asn Ala Val Asn Leu
   805 810 815
   Ala Val Val Glu Glu Pro Glu Thr Asp Ile Glu Leu Val Thr Val Pro
   820 825 830
   Gly Ser His Asn Ser Glu Met Gly Cys Pro Gly Asp Trp Gln Pro Gly
   835 840 845
   Cys Glu Ala Ala Lys Leu Thr Lys Arg Ala Asp Gly Ile Tyr Ala Gly
   850 855 860
   Thr Phe Gln Ile Pro Ala Gly Thr Tyr Glu Tyr Lys Val Ala Ile Asn
   865 870 875 880
   Gly Ser Trp Ala Leu Asn Tyr Gly Ala Asn Gly Val Gln Asp Gly Ala
   885 890 895
   Asn Ala Thr Tyr Thr Thr Ser Gly Gly Ala Val Thr Phe Tyr Trp Asp
   900 905 910
   Pro Arg Ser Lys Val Phe Ser Ser Thr Ala Glu Gly Pro Ile Val Thr
   915 920 925
   Leu Pro Gly Ser Met Gln Ser Glu Leu Gly Cys Pro Gly Asp Trp Gln
   930 935 940
   Pro Asp Cys Met Val Thr Phe Ala Gln Asp Gly Asp Lys Asp Gly Val
   945 950 955 960
   Tyr Glu Phe Ser Thr Asn Gly Leu Pro Ala Gly Ser Tyr Glu Leu Lys
   965 970 975
   Val Ala His Gly Leu Ser Trp Asp Glu Asn Tyr Gly Val Asp Gly Val
   980 985 990
   Arg Asn Gly Gly Asn Ile Ser Phe Val Val Gly Lys Ala Gly Glu Phe
   995 1000 1005
   Val Thr Phe Arg Tyr Thr Leu Glu Thr His Val Leu Glu Val Val Val
   1010 1015 1020
   Ala Asp Pro Pro Leu Ala Gly Thr Gly Gln Gln Arg Ala Tyr Trp Leu
   1025 1030 1035 1040
   Asp Ala Glu Thr Leu Ala Trp Pro Ala Ser Leu Leu Gly Gly Ala Asn
   1045 1050 1055
   Ala Ala Asp Arg Ala Tyr Thr Leu Glu Phe Ala Ala Asp Gly Gly Leu
   1060 1065 1070
   Gly Thr Ala Asp Gly Ala Val Thr Gly Ala Asp Arg Thr Val Glu Leu
   1075 1080 1085
   Ala His Glu Pro Ala Gly Ile Gly Ala Glu Leu Thr Ala Arg Phe Pro
   1090 1095 1100
   His Leu Ala Ser Tyr Val Ala Leu Arg Pro Val Gly Leu Asp Arg Ala
   1105 1110 1115 1120
   Ala Ile Ala Glu Leu Val Thr Gly Glu Leu Gln Val Ala Gln Arg Ala
   1125 1130 1135
   Gly Asp Glu Leu Thr Ala Met Thr Gly Val Gln Leu Pro Gly Val Leu
   1140 1145 1150
   Asp Asp Leu Tyr Ala Gln Gly Val Ala Ser Ala Ser Leu Gly Ala Thr
   1155 1160 1165
   Val Asp Gly Asp Ala Ala Ser Leu Ser Val Trp Ala Pro Thr Ala Lys
   1170 1175 1180
   Ser Val Ser Ala Gln Val Trp Ala Ala Gly Ala Thr Gly Asp Pro Glu
   1185 1190 1195 1200
   Val Leu Pro Ala Glu Phe Asp Ala Glu Ser Gly Ala Trp Ser Val Asp
   1205 1210 1215
   Glu Gly Ile Glu Ala Gly Asp Glu Tyr Arg Trp Leu Val Glu Val Tyr
   1220 1225 1230
   Ala Pro Thr Thr Gly Lys Val Glu Arg Asn Ser Val Thr Asp Pro Tyr
   1235 1240 1245
   Ser Val Ala Leu Thr Val Asn Ser Ala Arg Thr Val Val Val Asp Leu
   1250 1255 1260
   Asp Asp Pro Ala Leu Ala Pro Glu Leu Trp Ala Glu Thr Pro Ala Pro
   1265 1270 1275 1280
   Val Val Glu Arg Gln Val Asp Arg Ala Ile Tyr Glu Leu His Val Arg
   1285 1290 1295
   Asp Phe Ser Ile Thr Asp Glu Thr Val Pro Glu Ala Glu Arg Gly Thr
   1300 1305 1310
   Tyr Arg Ala Phe Thr Arg Asn Ser Ala Gly Ser Ala Gln Leu Arg Glu
   1315 1320 1325
   Leu Ala Ala Ala Gly Ile Asn Thr Val His Leu Leu Pro Thr Phe Asp
   1330 1335 1340
   Ile Ala Thr Ile Glu Glu Arg Arg Asp Gln Gln Ala Thr Pro Asp Cys
   1345 1350 1355 1360
   Asp Leu Glu Ser Tyr Gly Pro Ala Ser Glu Glu Gln Gln Ala Cys Ile
   1365 1370 1375
   Glu Ala Ile Arg Asp Leu Asp Gly Phe Asn Trp Gly Tyr Asp Pro Tyr
   1380 1385 1390
   His Phe Gln Ala Pro Glu Gly Ser Tyr Ala Val Asp Pro Asp Gly Gly
   1395 1400 1405
   Ala Arg Val Ala Glu Phe Arg Glu Met Val Gly Ala Leu His Ala Ala
   1410 1415 1420
   Gly Met Gln Val Val Leu Asp Glu Val Tyr Asn His Thr Ala Glu Ser
   1425 1430 1435 1440
   Gly Gln Gly Gln Lys Ser Val Leu Asp Lys Val Val Pro Gly Tyr Tyr
   1445 1450 1455
   His Arg Leu Asn Ala Thr Gly Gly Val Glu Thr Ser Thr Cys Cys Gln
   1460 1465 1470
   Asn Val Ala Thr Glu His Ala Val Ala Glu Lys Leu Met Val Asp Ser
   1475 1480 1485
   Thr Val Leu Trp Val Lys Glu Tyr Lys Val Asp Gly Phe Arg Phe Asp
   1490 1495 1500
   Leu Met Gly His His Ser Lys Ala Asn Leu Leu Ala Val Arg Ala Ala
   1505 1510 1515 1520
   Leu Asp Glu Leu Thr Leu Ala Glu Asp Gly Val Asp Gly Ser Lys Val
   1525 1530 1535
   Phe Leu Tyr Gly Glu Gly Trp Asn Phe Gly Glu Val Ala Asn Asn Ala
   1540 1545 1550
   Arg Phe Glu Gln Ala Ser Gln Gly Gln Leu Gly Gly Thr Gly Ile Ala
   1555 1560 1565
   Thr Phe Asn Asp Arg Leu Arg Asp Gly Val His Gly Gly Ser Pro Val
   1570 1575 1580
   Ala Gly Asp Ser Lys Tyr Glu Gln Gly Phe Gly Thr Gly Leu Ala Gly
   1585 1590 1595 1600
   Glu Pro Asn Gly Leu Pro Gln Arg Asp Gly Ile Arg Asn Leu Gly Gln
   1605 1610 1615
   Gln Thr Asp Leu Val Lys Ile Gly Leu Ala Gly Asn Leu Arg Asp Phe
   1620 1625 1630
   Arg Phe Thr Gly Tyr Asp Gly Val Leu Lys Ser Gly Ala Glu Val Asp
   1635 1640 1645
   Tyr Asn Gly Ser Pro Ala Gly Tyr Ala Asp His Pro Glu Glu Val Ile
   1650 1655 1660
   Asn Tyr Val Asp Ala His Asp Asn Glu Thr Leu Phe Asp Leu Gly Val
   1665 1670 1675 1680
   Leu Lys Leu Pro Gln Glu Thr Ser Met Ala Asp Arg Ile Arg Met Asn
   1685 1690 1695
   Thr Leu Ser Leu Ala Thr Val Thr Phe Ser Gln Ser Pro Ser Phe Trp
   1700 1705 1710
   His Ala Gly Thr Glu Leu Leu Arg Ser Lys Ser Leu Asp Arg Asn Ser
   1715 1720 1725
   Tyr Asn Ser Gly Asp Trp Phe Asn Arg Ile Asp Trp Thr Gly Gln Glu
   1730 1735 1740
   Ser Thr Phe Gly Ser Gly Leu Pro Met Lys Ser Asp Asn Glu Asp His
   1745 1750 1755 1760
   Trp Pro Thr Met Ala Thr Leu Leu Ala Asp Pro Ala Leu Lys Pro Ala
   1765 1770 1775
   Pro Ala Asp Ile Ala Ala Ala Glu Ala Ala Ala Leu Asp Leu Leu Arg
   1780 1785 1790
   Val Arg Asp Ser Val Asp Leu Leu Lys Leu Gly Ser Ala Glu Leu Ile
   1795 1800 1805
   Thr Gln Lys Val Ser Phe Pro Asn Ser Gly Ala Asp Ala Thr Asp Gly
       1810                1815                1820
   Leu Ile Val Met Leu Ile Asp Asp Leu Val Gly Asp Asp Val Asp Pro
   1825                1830                1835                1840
   Glu Leu Glu Gly Ala Leu Val Val Phe Asn Ala Ser Ala Glu Pro Ile
                   1845                1850                1855
   Thr Glu Ala Val Asp Gly Leu Ala Gly Arg Glu Phe Ala Leu Ala Pro
               1860                1865                1870
   Ala Leu Ala Asn Gly Thr Asp Ala Val Val Lys Gln Thr Ala Trp Glu
           1875                1880                1885
   Ala Ala Thr Gly Thr Leu Thr Ile Pro Ala Arg Thr Ala Ala Val Phe
       1890                1895                1900
   Val Asp Asp Gln Ala Lys Pro Gly Gly Gly Lys Pro Asp Lys Pro Gly
   1905                1910                1915                1920
   Lys Pro Glu Lys Pro Gly Lys Pro Asp Lys Pro Gly Lys Pro Asp Lys
                   1925                1930                1935
   Pro Glu Lys Pro Gly Lys Pro Glu Lys Pro Gly Lys Pro Glu Lys Pro
               1940                1945                1950
   Gly Lys Pro Glu Gln Pro Gly Lys Pro Val Val Arg Leu Asp Ala Ala
           1955                1960                1965
   Ser Val Gln Thr Gly Ser Thr Leu Ala Val Ser Gly Lys Gly Phe Ala
       1970                1975                1980
   Lys Gly Glu Leu Val Gln Val Trp Leu Glu Ser Thr Pro Thr Leu Met
   1985                1990                1995                2000
   Glu Ala Gln Pro Ala Ser Ala Asp Gly Thr Val Glu Phe
                   2005                2010
<210> 3
   <211> 952
   <212> PRT
   <213> Unknown
<220>
   <223> Metagenom
<400> 3
   Met Thr His Arg Ala Arg Ala Arg Ser Trp Leu Ala Leu Gly Val Thr
   1               5                   10                  15
   Gly Ala Val Ala Ala Ser Val Leu Ala Val Val Pro Ile Gly Ala Ala
               20                  25                  30
   Ala Glu Glu Gly Asp Thr Phe Ala Leu Val Gly Ser Leu Gln Ser Glu
           35                  40                  45
   Leu Gly Cys Ser Glu Asp Trp Gln Pro Ser Cys Glu Ala Thr Glu Leu
       50                  55                  60
   Leu Pro Thr Asp Thr Ala Gly Leu Tyr Ala Ala Glu Phe Thr Val Pro
   65                  70                  75                  80
   Ala Gly Ser Tyr Glu Tyr Lys Val Ala Ala Asn Asp Ser Trp Asp Ala
   85 90 95
   Ser Trp Gly Leu Asp Gly Gly Gly Asp Asn Ile Pro Leu Thr Val Gly
   100 105 110
   Gly Asp Thr Asp Val Arg Val Val Phe Asp Asp Thr Gln Lys Arg Val
   115 120 125
   Gly Leu Glu Leu Leu Ser Thr Arg Gly Ala Tyr Asp Glu Gln Ala Asp
   130 135 140
   Ala Ala Leu Ala Leu Pro Pro Val Arg Gln Pro Gly Ser Ala Glu Asn
   145 150 155 160
   Phe Tyr Phe Val Met Thr Asp Arg Phe Ala Asn Gly Asp Glu Ser Asn
   165 170 175
   Asp Thr Ala Gly Ile Glu Gly Asp Arg Leu Ala His Gly Phe Asp Pro
   180 185 190
   Thr Asp Lys Gly Phe Tyr His Gly Gly Asp Ile Gln Gly Ile Arg Asp
   195 200 205
   His Leu Asp Tyr Ile Glu Gly Leu Gly Thr Thr Ala Ile Trp Phe Thr
   210 215 220
   Pro Ser Phe Lys Asn Lys Pro Val Gln Gly Glu Gly Ala Asn Ala Ser
   225 230 235 240
   Ala Gly Tyr His Gly Tyr Trp Ile Thr Asp Phe Thr Gln Ile Asp Pro
   245 250 255
   His Leu Gly Thr Asn Ala Glu Leu Glu Ala Leu Ile Asp Glu Ala His
   260 265 270
   Glu Arg Gly Ile Lys Val Tyr Phe Asp Ile Ile Thr Asn His Thr Ala
   275 280 285
   Asp Val Ile Ser Tyr Glu Glu Gly Gln Tyr Ser Tyr Ile Asp Lys Ala
   290 295 300
   Thr Ser Pro Tyr Arg Asp Ala Asp Gly Asn Val Phe Asp Pro Ser Thr
   305 310 315 320
   Ile Ala Gly Ser Ala Asp Phe Pro Ala Leu Asp Pro Ala Thr Ser Phe
   325 330 335
   Pro Tyr Thr Pro Thr Val Ala Pro Glu Glu Ala Asp Val Lys Val Pro
   340 345 350
   Ala Trp Leu Asn Asp Pro Thr Leu Tyr His Asn Arg Gly Asp Ser Thr
   355 360 365
   Trp Ser Gly Glu Ser Val Thr Phe Gly Asp Phe Val Gly Leu Asp Asp
   370 375 380
   Leu Met Thr Glu His Pro Thr Val Val Asp Gly Phe Ile Glu Val Tyr
   385 390 395 400
   Gln Asp Trp Val Asp Leu Gly Ile Asp Gly Phe Arg Ile Asp Thr Ala
   405 410 415
   Lys His Val Asn Phe Glu Phe Trp Glu Glu Trp Ser Thr Glu Val Leu
   420 425 430
   Asp Tyr Ala His Ala Gln Gly Lys Pro Asp Phe Phe Met Phe Gly Glu
   435 440 445
   Val Tyr Asp Ala Asp Pro Val Lys Leu Ser Pro Tyr Val Arg Lys Thr
   450 455 460
   Asp Met Asn Ser Val Leu Asp Phe Thr Phe Gln Ser Gln Ala Val Ser
   465 470 475 480
   Phe Ala Ala Gly Asn Ser Ala Lys Asn Leu Gln Ser Leu Phe Ala Gly
   485 490 495
   Asp Asp Tyr Tyr Thr Thr Pro Asp Ser Ser Ala Thr Ala Leu Pro Thr
   500 505 510
   Phe Leu Gly Asn His Asp Met Gly Arg Ile Gly Tyr Phe Leu Ala Asn
   515 520 525
   Ser Gly Ala Thr Ala Val Gln Arg Asn Glu Leu Ala His Glu Leu Met
   530 535 540
   Tyr Leu Thr Arg Gly Gln Pro Val Val Tyr Tyr Gly Asp Glu Gln Gly
   545 550 555 560
   Phe Ala Gly Thr Gly Gly Asp Lys Asp Ala Arg Gln Thr Leu Phe Ala
   565 570 575
   Ser Gln Val Asp Glu Tyr Val Asn Gln Pro Leu Val Thr Gly Glu Asn
   580 585 590
   Ala Gly Ser Val Asp Arg Tyr Gly Thr Asp Ala Pro Leu Tyr Glu His
   595 600 605
   Ile Ala Gly Leu Ala Glu Leu Arg Lys Ala His Pro Ala Leu Glu Gln
   610 615 620
   Gly Ala Gln Ile Glu Arg Phe Val Ala Asp Gly Ala Ser Val Tyr Ala
   625 630 635 640
   Phe Ser Arg Val Asp Arg Asp Glu Lys Val Glu Tyr Leu Val Ala Val
   645 650 655
   Asn Asn Ala Asn Glu Ala Lys Ala Val Glu Val Ser Thr Leu Thr Ala
   660 665 670
   Asp Gly Ala Phe Glu Val Leu Tyr Gly Asp Gly Gln Ala Leu Thr Ala
   675 680 685
   Asn Ala Asp Gly Val Ala Ser Val Thr Ala Pro Ala Leu Gly Ala Val
   690 695 700
   Val Trp Lys Ala Asp Arg Glu Val Thr Ala Pro Glu Ala Ala Ser Ala
   705 710 715 720
   Ile Ala Val Asp Val Pro Val Ala Gly Ala Gly Val Thr Gly Ile Ala
   725 730 735
   Pro Val Ser Ala Asp Val Ala Asp Ala Thr Trp Gln Glu Thr Ser Phe
   740 745 750
   Ala Trp Arg Val Ala Gly Ser Asp Glu Trp Thr Pro Leu Gly Thr Ala
   755 760 765
   Glu Asp Thr Thr Pro Arg Val Tyr His Asp Thr Ala Gly Leu Ala Lys
   770 775 780
   Gly Thr Leu Val Glu Tyr Arg Ala Val Ser Thr Asp Ala Ala Gly Asn
   785 790 795 800
   Arg Ala Ala Ala Ser Thr Tyr Ala Ser Val Gly Asn Ala Val Asn Leu
   805 810 815
   Ala Val Val Glu Glu Pro Glu Thr Asp Ile Glu Leu Val Thr Val Pro
   820 825 830
   Gly Ser His Asn Ser Glu Met Gly Cys Pro Gly Asp Trp Gln Pro Gly
   835 840 845
   Cys Glu Ala Ala Lys Leu Thr Lys Arg Ala Asp Gly Ile Tyr Ala Gly
   850 855 860
   Thr Phe Gln Ile Pro Ala Gly Thr Tyr Glu Tyr Lys Val Ala Ile Asn
   865 870 875 880
   Gly Ser Trp Ala Leu Asn Tyr Gly Ala Asn Gly Val Gln Asp Gly Ala
   885 890 895
   Asn Ala Thr Tyr Thr Thr Ser Gly Gly Ala Val Thr Phe Tyr Trp Asp
   900 905 910
   Pro Arg Ser Lys Val Phe Ser Ser Thr Ala Glu Gly Pro Ile Val Thr
   915 920 925
   Leu Pro Gly Ser Met Gln Ser Glu Leu Gly Cys Pro Gly Asp Trp Gln
   930 935 940
   Pro Asp Cys Met Val Thr Phe Ala
   945 950
<210> 4
   <211> 2103
   <212> DNA
   <213> Unknown
<220>
   <223> Metagenom
<400> 4
   atgacaggcc ttcccgactc cgggacgctt cgacgccccg gcccgcgcaa gcgtttgcga 60
   acgctgatcg cggtcaccgc cgccgcggtc ctcgccggcc tcgccgccat cggtcccggc 120
   gcgcccgccg gccccgccga ggcggccgcc cccggcccga aggacgccac cgcggtgctc 180
   ttctcgtaca cctggaacgc catcgcccgc gagtgcaccg agaacctcgg acccgccggc 240
   tacggctacg tgcagacctc gccgccgcag gagcacgtgc agggccccca gtggtggatc 300
   cactaccagc ccgtcagcta ccgcgtcgag tcgcgcctcg gcacgcgcgc cgagttcaag 360
   gccatggtcg acacgtgcca cgcggccggc gtgaaggtca tcgccgacgc cgtcatcaac 420
   cacatgagcg ggtacagcgc gggcggcgtc ggctgggccg gctcgtcgtt ccagcactac 480
   tcgtaccccg gcatctacca gtcgcaggac ttccactcgt gccgacgcga catcgcgaac 540
   taccaggacc ggtgggaggt gcaggagtgc aacctcgtga acctcgccga cctgaacacc 600
   ggctcggcct acgtgcaggg ccgcatcgcc gcgtacctca acgacctcgt ctcgctcggc 660
   gtcgacggat tccggatcga cgccgtcaag cacatctcgg cggccgacat gaacggcatc 720
   ctctcgcgcg tcaacgaccg ggcgcgtctc tacctcgttc aggaggtcat ccgcgccaac 780
   gagcccgtgc agcccgagga gtacctgggc ctcggcgaca tccacgagtt cgcgtatgcg 840
   cgcaagctca aggaggcgtt cggcggccgc accctgaact ggctcatcag cggcgcgggc 900
   atcggcccga gctgggccgg cttcctgcag aacgcggatg ccgcggtgtt cgtcgacaac 960
   cacgacaccg agcgcaacgg cgagacgctg agctaccgcg acggcgcggc gtacgacctc 1020
   gcgcaggtgt tcacgctcgc gtggaactac ggttcgccgt cgatccactc cgggtaccag 1080
   ttctcgaaca aggacgccgg accggcgctc gcgggcaacg gacgcgtggt cgatcccgtg 1140
   tgcgggcaga acggctggac ctgcaagcac gcccagacga acatcgagaa catggtcggc 1200
   ttccgcacgg ccacctacgg caccgccatc acgaacaagt gggacaacgg ttcgtcggcg 1260
   atcgcgttcg gtcgcggtga caagggcttc gtcgccatca accgcggcac cgcggcggtc 1320
   gaccgcacct ggcagacgtc cctgccggcc gggcgctact gcaacgtgat cgtcgggctg 1380
   ccgacggcga gcggatgcag cgccggaggc gtcatcacgg tcgactcgag cggccggttc 1440
   gcggcatccg tcgccgccga caccgcgctc gccctccacg tcggcgcgaa ggccggcggc 1500
   accggcacga ccccgccgcc gaccggatcg acgatgaccg tgtacttcgc gaccacgaag 1560
   ggctggacga accactacgt gcaccaccgc gtcggctcgg gcgcatggac cgccctgccc 1620
   ggcgcagcga tggcggcggc ctgcaccggt tgggtgtcca ggaccatcga cctcggatcg 1680
   gcgacgggca tcaccgcggc cttcaccaac ggcgcgggcg cgtgggacaa caacggcggg 1740
   cgcgactacg cgctcaccgg atccgtcgcc gccgtgaagg acggggtcgt caccgcgacg 1800
   aacccgtgcg ccgcgcagcc cacctcgacc acggtgtact acgcgaccgg atggagcacc 1860
   gcgaacatcc actaccgcgt cgggtcgggc gcctggacgg cggtgccggg cgtcgcgatg 1920
   gcgaacgcct gcgcgggctg gaagtcgaag accatcgagc tcggcgcggc atccggcatc 1980
   accgcggcct tcaacaacgg cgccggcacg tgggacaaca acggcgggaa ggactacgcg 2040
   atcggggcgg gcgcgatgaa ggtgcagaac ggcaccgtca ccgccggcaa cccgtgtagc 2100
   tga                                                               2103
<210> 5
   <211> 6040
   <212> DNA
   <213> Unknown
<220>
   <223> Metagenom
<400> 5
   atgacgcatc gagcgcgcgc tcgcagctgg ttggcattgg gggtgacggg cgccgtcgcg 60
   gcatccgtgc tcgccgtcgt tcccatcggg gctgcagccg aggagggcga cacgttcgct 120
   ctcgtcggtt cgctgcagag cgaactgggc tgttccgagg actggcagcc gagctgcgag 180
   gccaccgagc tgctgcccac cgacaccgcg ggcctctacg ccgccgagtt caccgtcccg 240
   gcgggctcgt acgagtacaa ggtcgcggcg aacgactcgt gggacgcctc gtggggtctc 300
   gacgggggcg gcgacaacat cccgctcacg gtcggcggcg acaccgacgt gcgcgtcgtg 360
   ttcgacgaca cccagaagcg cgtcggcctc gagctgctga gcacccgtgg cgcgtacgac 420
   gagcaggcgg atgccgcgct cgcgctgccg cccgtccgcc agccgggcag cgccgagaac 480
   ttctacttcg tcatgaccga ccgcttcgcc aacggcgacg agtcgaacga caccgccggc 540
   atcgagggag accgcctcgc gcacggcttc gaccccaccg acaagggctt ctaccacggc 600
   ggcgacatcc agggcatccg cgatcacctc gactacatcg agggcctcgg cacgaccgcg 660
   atctggttca cgccgagctt caagaacaag cccgtgcagg gcgagggtgc gaacgccagc 720
   gccggctacc acggctactg gatcaccgac ttcacgcaga tcgacccgca cctcggcacg 780
   aacgccgagc tcgaggcgct catcgacgag gcgcacgagc gcggcatcaa ggtctacttc 840
   gacatcatca cgaaccacac ggccgacgtg atctcgtacg aggagggcca gtactcgtac 900
   atcgacaagg ccacgagccc gtaccgcgac gccgacggca acgtgttcga cccgagcacc 960
   atcgcgggca gcgccgactt cccggcgctc gatccggcca cgagcttccc gtacacgccg 1020
   accgtcgcgc ccgaggaggc cgacgtcaag gtccccgcgt ggctcaacga cccgacgctg 1080
   taccacaacc gcggcgactc gacctggtcg ggcgaatcgg tgacgttcgg cgacttcgtc 1140
   ggcctcgacg acctgatgac cgagcacccg accgtcgtcg acggcttcat cgaggtgtac 1200
   caggactggg tcgacctcgg catcgacggc ttccggatcg acaccgccaa gcacgtgaac 1260
   ttcgagttct gggaggagtg gtcgaccgag gtcctcgact acgcccacgc ccagggcaag 1320
   cccgacttct tcatgttcgg cgaggtctac gacgccgacc cggtgaagct ctcgccgtac 1380
   gtgcgcaaga ccgacatgaa ctcggtcctc gacttcacct tccagtcgca ggccgtgtcg 1440
   ttcgccgccg gcaactcggc gaagaacctg cagtcgctgt tcgccggtga cgactactac 1500
   acgacgcccg actcgtcggc gacggccctg ccgaccttcc tcggcaacca cgacatgggt 1560
   cgcatcggct acttcctcgc caactcgggc gccaccgcgg tgcagcgcaa cgagctcgcg 1620
   cacgagctca tgtacctcac gcgcggccag ccggtcgtct actacggcga cgagcagggc 1680
   ttcgcaggca cgggcggcga caaggacgcc cgccagacgc tcttcgcgag ccaggtcgac 1740
   gagtacgtca accagccgct cgtcaccggc gagaacgccg gcagcgtcga ccgctacggc 1800
   accgacgcgc cgctctacga gcacatcgcc ggactcgccg agctgcgcaa ggcgcacccg 1860
   gcgctcgagc agggcgcgca gatcgagcgc ttcgtcgccg acggcgccag cgtgtacgcg 1920
   ttcagccgcg tcgaccgcga cgagaaggtc gagtacctgg tcgccgtgaa caacgcgaac 1980
   gaggccaagg ccgtggaggt ctccaccctc accgccgacg gcgcgttcga ggtgctgtac 2040
   ggcgacgggc aggccctgac cgcgaacgcc gacggcgtgg catccgtcac cgcgccggcg 2100
   ctcggcgccg tggtgtggaa ggccgaccgc gaggtgaccg cccccgaggc ggcatccgcc 2160
   atcgccgtcg acgtgcccgt cgccggggcc ggcgtcacgg gcatcgcacc cgtctcggcc 2220
   gacgtcgccg acgccacctg gcaggagacg agcttcgcct ggcgcgtggc cggttccgac 2280
   gagtggacgc cgctcggcac cgccgaggac accaccccgc gcgtctacca cgacaccgcg 2340
   ggcctcgcga agggcacgct ggtcgagtac cgggccgtgt cgacggatgc cgcgggcaac 2400
   cgcgccgccg catcgaccta cgcctcggtg ggcaacgccg tgaacctcgc ggtcgtcgag 2460
   gagcccgaga ccgacatcga gctggtcacc gtccccggca gccacaactc ggagatgggc 2520
   tgcccgggcg actggcagcc cggctgcgag gccgcgaagc tcaccaagcg cgccgacggc 2580
   atctacgccg gcacgttcca gatcccggcc ggcacgtacg agtacaaggt cgcgatcaac 2640
   ggcagctggg cgctgaacta cggcgccaac ggcgtgcagg acggcgcgaa cgccacctac 2700
   acgacgagtg gcggcgcggt caccttctac tgggacccgc gcagcaaggt gttctcgtcg 2760
   accgccgagg ggccgatcgt cacgctgccc ggcagcatgc agtccgaact cggctgcccc 2820
   ggcgactggc agcccgactg catggtcacc ttcgcgcagg acggcgacaa ggacggcgtc 2880
   tacgagttct cgacgaacgg cctgccggcc ggctcctacg aactgaaggt cgcgcacggc 2940
   ctgtcgtggg atgagaacta cggcgtcgac ggtgtccgca acggcggcaa catctcgttc 3000
   gtcgtcggca aggccggcga gttcgtcacc ttccgctaca cgctcgagac ccacgtgctc 3060
   gaggtcgtcg tggccgaccc gccgctcgcg ggcaccggac agcagcgcgc gtactggctc 3120
   gacgccgaga cgctcgcctg gcccgcctcg ctcctcggcg gagcgaacgc ggcggaccgc 3180
   gcctacacgc tcgagttcgc cgccgacggc ggactcggca ccgccgacgg cgccgtgacc 3240
   ggcgccgacc ggacggtcga gctcgcccac gagcccgcgg gcatcggcgc ggagctcacc 3300
   gcgcggttcc cgcacctggc gagctacgtg gcgctccggc ctgtcggcct cgaccgcgcg 3360
   gcgatcgccg aactcgtcac gggcgagctc caggtggcgc agcgcgccgg cgacgagctc 3420
   accgcgatga ccggcgtgca gctgccgggc gtgctcgacg acctctacgc ccagggcgtg 3480
   gcatccgcct cgctcggcgc cacggtcgac ggcgacgccg cgagcctctc cgtctgggcg 3540
   ccgaccgcga agtcggtgtc ggcccaggtc tgggccgccg gagcgacggg cgaccccgag 3600
   gtgctgccgg ccgagttcga cgccgagtcc ggcgcgtggt cggtcgacga gggcatcgag 3660
   gcgggcgacg agtaccgctg gctcgtcgag gtgtacgccc cgaccaccgg caaggtggag 3720
   cgcaactcgg tgaccgaccc gtactcggtc gcgctcaccg tgaactccgc ccgcaccgtc 3780
   gtcgtcgacc tcgacgaccc ggcgctcgca cccgagctgt gggccgagac gcccgcaccg 3840
   gtcgtcgagc ggcaggtcga ccgggcgatc tacgagctgc acgtgcgcga cttctcgatc 3900
   accgatgaga cggtgcccga ggccgagcgc ggcacctacc gggcgttcac ccgcaacagt 3960
   gcgggcagtg cgcagctgcg cgagctcgcc gcggccggca tcaacacggt gcacctgctg 4020
   cccacgttcg acatcgcgac catcgaggag cgccgcgacc agcaggcgac ccccgactgc 4080
   gacctcgagt cgtacggtcc ggcgtcggag gagcagcagg cctgcatcga ggcgatccgc 4140
   gacctcgacg gcttcaactg gggctacgac ccgtaccact tccaggcgcc cgagggctcc 4200
   tacgcggtcg accccgacgg cggcgcgcgc gtcgcggagt tccgcgagat ggtcggcgcc 4260
   ctgcacgccg cgggcatgca ggtcgtgctc gacgaggtgt acaaccacac cgccgagtcg 4320
   ggccagggcc agaagtcggt gctcgacaag gtcgtgcccg gctactacca ccgcctgaac 4380
   gcgaccggcg gcgtcgagac ctcgacgtgc tgccagaacg tcgcgaccga gcacgccgtc 4440
   gccgagaagc tcatggtcga ctcgaccgtg ctctgggtga aggagtacaa ggtcgacggg 4500
   ttccgcttcg acctgatggg ccaccactcc aaggcgaacc tcctggccgt gcgcgcggcg 4560
   ctcgacgagc tcacgctcgc cgaggacggc gtcgacggct cgaaggtctt cctctacggc 4620
   gagggctgga acttcggcga ggtcgcgaac aacgcgcggt tcgaacaggc ctcgcagggc 4680
   cagctcggcg gcacgggcat cgcgacgttc aacgaccggc tgcgtgacgg cgtgcacggc 4740
   ggcagcccgg tcgcgggcga ctcgaagtac gagcagggct tcggcaccgg cctcgcgggc 4800
   gagccgaacg gcctgcccca gcgcgacggc atccggaacc tcggccagca gaccgacctc 4860
   gtgaagatcg gcctcgcggg caacctgcgc gacttccggt tcaccgggta cgacggggtg 4920
   ctgaagtcgg gcgccgaggt cgactacaac gggtcgccgg ccggctacgc cgaccacccc 4980
   gaggaggtca tcaactacgt cgacgcgcac gacaacgaga cgctgttcga cctcggcgtg 5040
   ctgaagctgc cgcaggagac gtcgatggcc gaccgcatcc gcatgaacac gctgtcgctc 5100
   gcgaccgtga cgttctcgca gagcccgtcg ttctggcacg ccggcaccga gctgctccgc 5160
   tcgaagtcgc tcgaccgcaa tagctacaac tcgggcgact ggttcaaccg gatcgactgg 5220
   accggccagg agtcgacctt cgggtcgggc ctgccgatga agtccgacaa cgaggaccac 5280
   tggccgacga tggcgacgct gctcgccgac ccggcgctga agcccgctcc ggcggacatc 5340
   gccgccgccg aggccgccgc gctcgacctg ctgcgcgtgc gcgacagcgt cgacctgctc 5400
   aagctgggct cggccgagct catcacgcag aaggtgtcgt tcccgaacag cggtgcggat 5460
   gccacggacg ggctcatcgt gatgctcatc gacgacctcg tgggcgacga cgtcgacccc 5520
   gagctcgagg gtgcgctggt cgtgttcaac gcttccgccg agccgatcac cgaggcggtg 5580
   gacggactcg ccggtcgcga gttcgccctc gccccggcgc tcgcgaacgg gaccgacgcg 5640
   gtcgtcaagc agaccgcgtg ggaggccgcg accggcacgc tcacgatccc ggcccgcacg 5700
   gcggcggtgt tcgtcgacga ccaggccaag cccggcggcg gcaagcccga caagccgggc 5760
   aagcccgaga agcccggcaa gccggacaag cccggcaagc cggacaagcc cgagaagccg 5820
   ggcaagccgg agaagcccgg caagccggag aagcccggca agccggagca gcccggcaag 5880
   cccgtcgtcc gcctggacgc ggcgtcggtg cagaccggct cgaccctggc cgtgtccggg 5940
   aagggcttcg cgaagggcga gctcgtgcag gtgtggctgg agtcgacgcc gacgctcatg 6000
   gaggcgcagc ccgcctccgc ggacggcacg gtcgagttcg 6040
<210> 6
   <211> 2859
   <212> DNA
   <213> Unknown
<220>
   <223> Metagenom
<400> 6
   atgacgcatc gagcgcgcgc tcgcagctgg ttggcattgg gggtgacggg cgccgtcgcg   60
   gcatccgtgc tcgccgtcgt tcccatcggg gctgcagccg aggagggcga cacgttcgct  120
   ctcgtcggtt cgctgcagag cgaactgggc tgttccgagg actggcagcc gagctgcgag  180
   gccaccgagc tgctgcccac cgacaccgcg ggcctctacg ccgccgagtt caccgtcccg  240
   gcgggctcgt acgagtacaa ggtcgcggcg aacgactcgt gggacgcctc gtggggtctc  300
   gacgggggcg gcgacaacat cccgctcacg gtcggcggcg acaccgacgt gcgcgtcgtg  360
   ttcgacgaca cccagaagcg cgtcggcctc gagctgctga gcacccgtgg cgcgtacgac  420
   gagcaggcgg atgccgcgct cgcgctgccg cccgtccgcc agccgggcag cgccgagaac  480
   ttctacttcg tcatgaccga ccgcttcgcc aacggcgacg agtcgaacga caccgccggc  540
   atcgagggag accgcctcgc gcacggcttc gaccccaccg acaagggctt ctaccacggc  600
   ggcgacatcc agggcatccg cgatcacctc gactacatcg agggcctcgg cacgaccgcg  660
   atctggttca cgccgagctt caagaacaag cccgtgcagg gcgagggtgc gaacgccagc  720
   gccggctacc acggctactg gatcaccgac ttcacgcaga tcgacccgca cctcggcacg  780
   aacgccgagc tcgaggcgct catcgacgag gcgcacgagc gcggcatcaa ggtctacttc  840
   gacatcatca cgaaccacac ggccgacgtg atctcgtacg aggagggcca gtactcgtac  900
   atcgacaagg ccacgagccc gtaccgcgac gccgacggca acgtgttcga cccgagcacc  960
   atcgcgggca gcgccgactt cccggcgctc gatccggcca cgagcttccc gtacacgccg 1020
   accgtcgcgc ccgaggaggc cgacgtcaag gtccccgcgt ggctcaacga cccgacgctg 1080
   taccacaacc gcggcgactc gacctggtcg ggcgaatcgg tgacgttcgg cgacttcgtc 1140
   ggcctcgacg acctgatgac cgagcacccg accgtcgtcg acggcttcat cgaggtgtac 1200
   caggactggg tcgacctcgg catcgacggc ttccggatcg acaccgccaa gcacgtgaac 1260
   ttcgagttct gggaggagtg gtcgaccgag gtcctcgact acgcccacgc ccagggcaag 1320
   cccgacttct tcatgttcgg cgaggtctac gacgccgacc cggtgaagct ctcgccgtac 1380
   gtgcgcaaga ccgacatgaa ctcggtcctc gacttcacct tccagtcgca ggccgtgtcg 1440
   ttcgccgccg gcaactcggc gaagaacctg cagtcgctgt tcgccggtga cgactactac 1500
   acgacgcccg actcgtcggc gacggccctg ccgaccttcc tcggcaacca cgacatgggt 1560
   cgcatcggct acttcctcgc caactcgggc gccaccgcgg tgcagcgcaa cgagctcgcg 1620
   cacgagctca tgtacctcac gcgcggccag ccggtcgtct actacggcga cgagcagggc 1680
   ttcgcaggca cgggcggcga caaggacgcc cgccagacgc tcttcgcgag ccaggtcgac 1740
   gagtacgtca accagccgct cgtcaccggc gagaacgccg gcagcgtcga ccgctacggc 1800
   accgacgcgc cgctctacga gcacatcgcc ggactcgccg agctgcgcaa ggcgcacccg 1860
   gcgctcgagc agggcgcgca gatcgagcgc ttcgtcgccg acggcgccag cgtgtacgcg 1920
   ttcagccgcg tcgaccgcga cgagaaggtc gagtacctgg tcgccgtgaa caacgcgaac 1980
   gaggccaagg ccgtggaggt ctccaccctc accgccgacg gcgcgttcga ggtgctgtac 2040
   ggcgacgggc aggccctgac cgcgaacgcc gacggcgtgg catccgtcac cgcgccggcg 2100
   ctcggcgccg tggtgtggaa ggccgaccgc gaggtgaccg cccccgaggc ggcatccgcc 2160
   atcgccgtcg acgtgcccgt cgccggggcc ggcgtcacgg gcatcgcacc cgtctcggcc 2220
   gacgtcgccg acgccacctg gcaggagacg agcttcgcct ggcgcgtggc cggttccgac 2280
   gagtggacgc cgctcggcac cgccgaggac accaccccgc gcgtctacca cgacaccgcg 2340
   ggcctcgcga agggcacgct ggtcgagtac cgggccgtgt cgacggatgc cgcgggcaac 2400
   cgcgccgccg catcgaccta cgcctcggtg ggcaacgccg tgaacctcgc ggtcgtcgag 2460
   gagcccgaga ccgacatcga gctggtcacc gtccccggca gccacaactc ggagatgggc 2520
   tgcccgggcg actggcagcc cggctgcgag gccgcgaagc tcaccaagcg cgccgacggc 2580
   atctacgccg gcacgttcca gatcccggcc ggcacgtacg agtacaaggt cgcgatcaac 2640
   ggcagctggg cgctgaacta cggcgccaac ggcgtgcagg acggcgcgaa cgccacctac 2700
   acgacgagtg gcggcgcggt caccttctac tgggacccgc gcagcaaggt gttctcgtcg 2760
   accgccgagg ggccgatcgt cacgctgccc ggcagcatgc agtccgaact cggctgcccc 2820
   ggcgactggc agcccgactg catggtcacc ttcgcgtga                          2859

<210> 7
   <211> 99
   <212> PRT
   <213> Unknown
<220>
   <223> Metagenom
<400> 7
   Val Asp Gly Phe Arg Ile Asp Ala Ala Lys His Ile Pro Pro Ala Asp
   1               5                   10                  15
   Leu Ala Ala Ile Lys Asp Leu Leu Thr Gly Ser Pro Tyr Leu His Gln
               20                  25                  30
   Glu Val Ile Gln Gly Asp Asn Glu Val Ile Gln Pro Gly Gln Tyr Thr
           35                  40                  45
   Gly Ile Gly Asp Val His Glu Phe Val Tyr Gly Arg Lys Leu Lys Glu
       50                  55                  60
   Gln Phe Asn Gly Gln Ile Lys Trp Leu Gly Ser Phe Gly Gln Ser Trp
   65                  70                  75                  80
   Gly Leu Ser Val Pro Gly Asp Lys Ala Val Val Phe Val Asp Asn His
                   85                  90                  95
   Asp Thr Glu
<210> 8
   <211> 100
   <212> PRT
   <213> Unknown
<220>
   <223> Metagenom
<400> 8
   Val Asp Gly Phe Arg Ile Asp Thr Ala Lys His Ile Ala Ala Ser Asp
   1               5                   10                  15
   Leu Ala Ala Ile Arg Ala Arg Val Asn Gly Gly Asp Val Tyr Trp Lys
               20                  25                  30
   Gln Glu Val Ile His Gly Ala Gly Glu Ala Val Gln Pro Gly Glu Tyr
           35                  40                  45
   Thr Gly Val Gly Asp Val Gln Glu Phe Arg Tyr Ala Tyr Asp Leu Lys
       50                  55                  60
   Arg Val Phe Gln Asn Glu Arg Leu Ala Tyr Leu Glu Asn Tyr Gly Glu
   65                  70                  75                  80
   Gly Trp Gly Tyr Leu Pro Ser Gly Gly Ala Ala Val Phe Val Asp Asn
                   85                  90                  95
   His Asp Thr Glu
               100
<210> 9
   <211> 99
   <212> PRT
   <213> Unknown
<220>
   <223> Metagenom
<400> 9
   Val Asp Gly Phe Arg Ile Asp Thr Ser Lys His Met Pro Ala Ala Asp
   1               5                   10                  15
   Ile Ala Ala Ile Lys Ser Lys Leu Ser Arg Pro Ala Tyr Ile Val Gln
               20                  25                  30
   Glu Val Ile Phe Gly Ala Gly Glu Pro Ile Gly Pro Gly Glu Tyr Thr
           35                  40                  45
   Gly Asn Gly Asp Val His Glu Phe Arg Tyr Gly Lys Asp Leu Ala Arg
       50                  55                  60
   Val Phe Arg Ser Glu Arg Leu Ala Tyr Leu Lys Asn Phe Gly Glu Gly
   65                  70                  75                  80
   Trp Gly His Leu Pro Ser Gly Val Ser Ser Val Tyr Val Asp Asn His
                   85                  90                  95
   Asp Thr Glu
<210> 10
   <211> 100
   <212> PRT
   <213> Unknown
<220>
   <223> Metagenom
<400> 10
   Val Asp Gly Phe Arg Ile Asp Ala Val Lys Arg Met Pro Ala Ala Asp
   1               5                   10                  15
   Leu Ala Asn Ile Lys Ser Arg Leu Ser Asn Pro Asp Ala Tyr Trp Lys
               20                  25                  30
   Gln Glu Val Ile His Gly Ala Gly Glu Ala Val Gln Pro Gly Glu Tyr
           35                  40                  45
   Thr Gly Asn Gly Asp Val Gln Glu Phe Arg Tyr Ala Tyr Asp Leu Lys
       50                  55                  60
   Arg Val Phe His Asn Glu Asn Leu Ala Tyr Leu Lys Asn Tyr Gly Glu
   65                  70                  75                  80
   Gly Trp Gly Tyr Leu Lys Asn Ser Ser Ala Gly Val Phe Val Asp Asn
                   85                  90                  95
   His Asp Thr Glu
               100
<210> 11
   <211> 116
   <212> PRT
   <213> Unknown
<220>
   <223> Metagenom
<400> 11
   Val Asp Gly Phe Arg Ile Asp Ala Thr Lys Ala Ile Lys Asn Phe Asp
   1               5                   10                  15
   Thr Leu Lys Leu Leu Thr Asp Ala Ala Tyr Ser Lys Ile Glu Asp Arg
               20                  25                  30
   Lys Pro Phe Ile Thr Ile Gly Glu His Val Pro Glu Asp Pro Glu Val
           35                  40                  45
   Thr Gly Arg Asp Arg Gly Arg Pro Met Asp Ala Ala Trp His Asp Tyr
       50                  55                  60
   Phe Gly His Val Ala Arg Ala Val Val Ala Glu Val Gln Lys Asp Asp
   65                  70                  75                  80
   Leu His Pro His Asp Ile Lys Arg Phe Leu Glu Arg Leu Asp Pro Lys
                   85                  90                  95
   Thr Asn Gly Tyr Gly Ser Ala Tyr Arg Thr Val Thr Phe Val Asp Asn
               100                 105                 110
   His Asp Thr Glu
           115
<210> 12
   <211> 101
   <212> PRT
   <213> Unknown
<220>
   <223> Metagenom
<400> 12
   Val Asp Gly Phe Arg Ile Asp Ala Ala Lys His Ile Pro Asn Glu Asp
   1               5                   10                  15
   Leu Arg Ala Ile Phe Gly Arg Leu Arg Glu Thr Val Ser Gly Ala Arg
               20                  25                  30
   Pro Tyr Val Phe His Glu Val Phe Pro Gly Glu Pro Pro Arg Pro Gln
           35                  40                  45
   Asp Tyr Phe Ser Thr Gly Asp Val Leu Asp Phe Thr Tyr Xaa Asp Lys
       50                  55                  60
   Ile Lys Thr Ala Phe Gln Gly Asp Ile Ala Trp Leu Ser Ser Phe Gly
   65                  70                  75                  80
   Pro Ser Trp Gly Leu Leu Pro Ala Ala Asp Ser Val Ser Tyr Val Asp
                   85                  90                  95
   Asn His Asp Thr Glu
               100
<210> 13
   <211> 96
   <212> PRT
   <213> Unknown
<220>
   <223> Metagenom
<400> 13
   Val Asp Gly Phe Arg Ile Asp Ala Ala Lys His Met Tyr His Gly Asp
   1               5                   10                  15
   Ile Gln Gly Ile Leu Ser Arg Ala Gly Asn Pro Tyr Val Phe Leu Glu
               20                  25                  30
   Val Ile Gly Ser Ala Gly Glu Ala Val Gln Pro Ser Gln Tyr Thr Tyr
           35                  40                  45
   Leu Gly Gln Val Thr Glu Phe Gly Tyr Ser Ser His Ile Gly His Arg
       50                  55                  60
   Phe Lys Tyr Gly Gln Ile Lys Asp Leu Asn Asn Ile Ala Asp Gly Lys
   65                  70                  75                  80
   Leu Pro Ser Thr Ser Ala Ile Val Tyr Val Asp Asn His Asp Thr Glu
                   85                  90                  95
<210> 14
   <211> 99
   <212> PRT
   <213> Unknown
<220>
   <223> Metagenom
<400> 14
   Val Asp Gly Phe Arg Ile Asp Ala Ala Lys His Ile Pro Pro Ala Asp
   1 5 10 15
   Leu Ala Ala Ile Lys Asp Leu Leu Thr Gly Ser Pro Tyr Leu His Gln
   20 25 30
   Glu Val Ile Gln Gly Asp Asn Glu Val Ile Gln Pro Gly Gln Tyr Thr
   35 40 45
   Gly Ile Gly Asp Val His Glu Phe Val Tyr Gly Arg Lys Leu Lys Glu
   50 55 60
   Gln Phe Asn Gly Gln Ile Lys Trp Leu Gly Ser Phe Gly Gln Ser Trp
   65 70 75 80
   Gly Leu Ser Val Pro Gly Asp Lys Ala Val Val Tyr Val Asp Asn His
   85 90 95
   Asp Thr Glu
<210> 15
   <211> 102
   <212> PRT
   <213> Unknown
<220>
   <223> Metagenom
<400> 15
   Val Asp Gly Phe Arg Ile Asp Ala Ile Lys His Met Asp Thr Arg Phe
   1 5 10 15
   Val Asn Ala Trp Leu Asp Ala Val Arg Gly Pro Arg Phe Ala Val Ser
   20 25 30
   Glu Ala Trp Phe Ala Lys Leu Asp Gln Leu Thr Ala Tyr Ala Lys Glu
   35 40 45
   Leu Gly Asp Arg Thr Arg Leu Phe Asp Val Pro Leu His Tyr Leu Phe
   50 55 60
   Tyr Gly Met Ser Asn Gly Asn Gly Ala Trp Asp Met Arg Asn Leu Lys
   65 70 75 80
   Phe Ala Gly Phe Thr Glu Ala Asn Gly Lys Leu Ser Val Pro Tyr Val
   85 90 95
   Asp Asn His Asp Thr Glu
   100
<210> 16
   <211> 96
   <212> PRT
   <213> Unknown
<220>
   <223> Metagenom
<400> 16 <210> 17
   <211> 99
   <212> PRT
   <213> Unknown
<220>
   <223> Metagenom
<400> 17
<210> 18
   <211> 96
   <212 PRT
   <213> Unknown
<220>
   <223> Metagenom
<400> 18
   Val Asp Gly Phe Arg Ile Asp Thr Ala Lys His Met Tyr His Gly Asp
   1               5                   10                  15
   Ile Gln Gly Ile Leu Ser Arg Ala Gly Asn Pro Tyr Val Phe Leu Glu
               20                  25                  30
   Val Ile Gly Ser Ala Gly Glu Ala Val Gln Pro Ser Gln Tyr Thr Tyr
           35                  40                  45
   Leu Gly Gln Val Thr Glu Phe Gly Tyr Ser Ser His Ile Gly His Arg
       50                  55                  60
   Phe Lys Tyr Gly Gln Ile Lys Asp Leu Asn Asn Ile Ala Asp Gly Lys
   65                  70                  75                  80
   Leu Pro Ser Thr Ser Ala Ile Val Tyr Val Asp Asn His Asp Thr Glu
                   85                  90                  95
<210> 19
   <211> 117
   <212> PRT
   <213> Unknown
<220>
   <223> Metagenom
<400> 19
   Val Asp Gly Phe Arg Ile Asp Ala Ala Arg His Val Asn Thr Ala Phe
   1               5                   10                  15
   Trp His Ala Phe Asn Thr Ala Ile Arg Asp Arg Ala Arg Ala Leu Gly
               20                  25                  30
   Arg Pro Asp Phe Ile Gln Phe Gly Glu Val Tyr Asn Asp Thr Pro Glu
           35                  40                  45
   Ala Pro Ala Val Leu Ser Glu Phe Ser Thr Gly Met Pro Met Asp Thr
       50                  55                  60
   Thr Leu Asp Phe Gly Phe Phe Asn Ala Ala Arg His Phe Val Ser Gln
   65                  70                  75                  80
   Gln Arg Pro Ala Ala Glu Leu Ala Ala Phe Phe Arg Thr Asp Asp Leu
                   85                  90                  95
   Tyr Thr Asp His Asp Ser Asn Ile His Ala Thr Leu Thr Tyr Val Asp
               100                 105                 110
   Asn His Asp Thr Glu
           115
<210> 20
   <211> 100
   <212> PRT
   <213> Unknown
<220>
   <223> Metagenom
<400> 20
   Val Asp Gly Phe Arg Ile Asp Thr Ala Lys His Met Pro Ala Ala Asp
   1 5 10 15
   Leu Ala Asn Ile Lys Ser Arg Leu Gly Asp Pro Asn Val Tyr Trp Lys
   20 25 30
   Gln Glu Ala Ile Tyr Gly Ala Gly Glu Ala Val Ser Pro Asp Glu Tyr
   35 40 45
   Ala Gly Thr Gly Asp Val Gln Glu Phe Arg Tyr Ala Arg Gly Leu Lys
   50 55 60
   Gln Val Phe Asn Asn Glu Asn Leu Ala Tyr Leu Lys Asn Tyr Gly Glu
   65 70 75 80
   Gly Trp Gly Phe Met Pro Ser Ser Lys Ala Ala Val Tyr Val Asp Asn
   85 90 95
   His Asp Thr Glu
   100
<210> 21
   <211> 108
   <212> PRT
   <213> Unknown
<220>
   <223> Metagenom
<400> 21
   Val Asp Gly Phe Arg Ile Asp Thr Ile Arg His Met Pro Gln Ala Phe
   1 5 10 15
   Trp His Ala Phe Ala Arg Arg Ile Arg Ala Glu His Pro Gly Phe Phe
   20 25 30
   Met Phe Gly Glu Ala Phe Asp Thr Lys Ala Ala Asn Ile Ala Pro Phe
   35 40 45
   Thr Trp Ala Glu Asn Ala Asn Val Ser Val Leu Asp Phe Pro Leu Lys
   50 55 60
   Glu Gly Leu Ile Glu Val Phe Gly Arg Lys Arg Ala Gly Phe Glu Ile
   65 70 75 80
   Leu Leu Pro Arg Leu Tyr Leu Thr Asp Gly Pro Tyr Ala Asn Pro Tyr
   85 90 95
   Glu Leu Met Thr Tyr Val Asp Asn His Asp Thr Glu
   100 105
<210> 22
   <211> 117
   <212> PRT
   <213> Unknown
<220>
   <223> Metagenom
<400> 22
   Val Asp Gly Phe Arg Ile Asp Ala Ala Arg His Val Asn Thr Ala Phe
   1 5 10 15
   Trp His Ala Phe Asn Thr Ala Ile Arg Asp Arg Ala Arg Ala Leu Gly
   20 25 30
   Arg Pro Asp Phe Ile Gln Phe Gly Glu Val Tyr Asn Asp Thr Pro Glu
   35 40 45
   Ala Pro Ala Val Leu Ser Glu Phe Ser Thr Gly Met Pro Met Asp Thr
   50 55 60
   Thr Leu Asp Phe Gly Phe Phe Asn Ala Ala Arg His Phe Val Ser Gln
   65 70 75 80
   Gln Arg Pro Ala Ala Glu Leu Ala Ala Phe Phe Arg Thr Asp Asp Leu
   85 90 95
   Tyr Thr Asp His Asp Ser Asn Ile His Ala Thr Leu Thr Phe Val Asp
   100 105 110
   Asn His Asp Thr Glu
   115
<210> 23
   <211> 96
   <212> PRT
   <213> Unknown
<220>
   <223> Metagenom
<400> 23
   Val Asp Gly Phe Arg Ile Asp Ala Val Lys His Ile Ala His Gly Asp
   1 5 10 15
   Leu Ala Gly Ile Leu Ser Arg Ala Gly Asn Pro Tyr Asn Phe Met Glu
   20 25 30
   Val Ile Gly Ala Ala Gly Glu Pro Ile Gln Pro Ser Glu Tyr Thr Tyr
   35 40 45
   Leu Gly Gln Val Thr Glu Phe Gly Tyr Ser Ser His Leu Gly His Arg
   50 55 60
   Phe Lys Phe Gly Gln Ile Lys Asp Leu Arg Asn Ile Gly Asp Gly Lys
   65 70 75 80
   Leu Pro Ser Asp Lys Ala Ile Val Phe Val Asp Asn His Asp Thr Glu
   85 90 95
<210> 24
   <211> 96
   <212> PRT
   <213> Unknown
<220>
   <223> Metagenom
<400> 24
   Val Asp Gly Phe Arg Ile Asp Thr Val Lys His Ile Ala His Gly Asp
   1 5 10 15
   Leu Ala Gly Ile Leu Ser Arg Ala Gly Asn Pro Tyr Asn Phe Met Glu
   20 25 30
   Val Ile Gly Ala Ala Gly Glu Pro Ile Gln Pro Ser Glu Tyr Thr Tyr
   35 40 45
   Leu Gly Gln Val Thr Glu Phe Gly Tyr Ser Ser His Leu Gly His Arg
   50 55 60
   Phe Lys Phe Gly Gln Ile Lys Asp Leu Arg Asn Ile Gly Asp Gly Lys
   65 70 75 80
   Leu Pro Ser Asp Lys Ala Met Val Tyr Val Asp Asn His Asp Thr Glu
   85 90 95
<210> 25
   <211> 100
   <212> PRT
   <213> Unknown
<220>
   <223> Metagenom
<400> 25
   Val Asp Gly Phe Arg Ile Asp Ala Ala Lys His Ile Ala Thr Glu Asp
   1 5 10 15
   Leu Ala Ala Val Lys Ala Lys Leu Ser Lys Pro Asp Val Tyr Trp Lys
   20 25 30
   Gln Glu Thr Ile Tyr Ser Ala Gly Glu Ala Val Gln Pro Gln Glu Tyr
   35 40 45
   Leu Gly Asn Gly Asp Val Gln Glu Phe Arg Tyr Ala Arg Asp Leu Lys
   50 55 60
   Arg Val Phe Gln Ser Glu Arg Leu Ala Tyr Leu Lys Asn Tyr Gly Glu
   65 70 75 80
   Gly Trp Gly Tyr Leu Pro Gly Asp Arg Ala Ser Val Tyr Val Asp Asn
   85 90 95
   His Asp Thr Glu
   100
<210> 26
   <211> 100
   <212> PRT
   <213> Unknown
<220>
   <223> Metagenom
<400> 26
   Val Asp Gly Phe Arg Ile Asp Ala Ala Lys His Met Ala Ala Gly Asp
   1 5 10 15
   Leu Ala Asn Ile Lys Ser Arg Leu Thr Asn Pro Asn Val Tyr Trp Lys
   20 25 30
   His Glu Ala Ile Tyr Gly Ala Gly Glu Ala Val Ser Pro Ala Glu Tyr
   35 40 45
   Leu Gly Ser Gly Asp Val Gln Glu Phe Arg Tyr Ala Arg Asp Leu Lys
   50 55 60
   Arg Val Phe Asn Asn Glu Asn Leu Ala Tyr Leu Lys Asn Phe Gly Glu
   65 70 75 80
   Ser Trp Gly Tyr Leu Pro Ser Asp Gln Ala Ala Val Tyr Val Asp Asn
   85 90 95
   His Asp Thr Glu
   100
<210> 27
   <211> 100
   <212> PRT
   <213> Unknown
<220>
   <223> Metagenom
<400> 27
   Val Asp Gly Phe Arg Ile Asp Ala Ala Lys His Met Ala Ala Ser Asp
   1               5                   10                  15
   Leu Ala Asp Ile Lys Ser Arg Leu Ser Asn Pro Ser Val Tyr Trp Lys
               20                  25                  30
   Gln Glu Ala Ile Phe Gly Ala Gly Glu Ala Val Ser Pro Ser Glu Tyr
           35                  40                  45
   Leu Gly Asn Gly Asp Val Gln Glu Phe Arg Tyr Ala Arg Asp Leu Lys
       50                  55                  60
   Arg Val Phe Asn Asn Glu Asn Leu Ala Tyr Leu Lys Asn Tyr Gly Glu
   65                  70                  75                  80
   Gly Trp Gly Tyr Met Ser Ser Gly Ser Ser Ala Val Tyr Val Asp Asn
                   85                  90                  95
   His Asp Thr Glu
               100
<210> 28
   <211> 100
   <212> PRT
   <213> Unknown
<220>
   <223> Metagenom
<400> 28
   Val Asp Gly Phe Arg Ile Asp Thr Ala Lys His Met Ala Ala Gly Asp
   1               5                   10                  15
   Leu Ala Asn Ile Lys Ser Arg Leu Thr Asn Pro Asn Val Tyr Trp Lys
               20                  25                  30
   His Glu Ala Ile Tyr Gly Ala Gly Glu Ala Val Ser Pro Ala Glu Tyr
           35                  40                  45
   Leu Gly Ser Gly Asp Val Gln Glu Phe Arg Tyr Ala Arg Asp Leu Lys
       50                  55                  60
   Arg Val Phe Asn Asn Glu Asn Leu Ala Tyr Leu Lys Asn Phe Gly Glu
   65                  70                  75                  80
   Ser Trp Gly Tyr Leu Pro Ser Asp Gln Ala Ala Val Tyr Val Asp Asn
                   85                  90                  95
   His Asp Thr Glu
               100
<210> 29
   <211> 100
   <212> PRT
   <213> Unknown
<220>
   <223> Metagenom
<400> 29
   Val Asp Gly Phe Arg Ile Asp Thr Ala Lys His Met Pro Ala Ala Asp
   1               5                   10                  15
   Leu Ala Asn Ile Lys Ser Arg Leu Thr Asn Pro Ser Ala Tyr Trp Lys
               20                  25                  30
   Gln Glu Val Ile Tyr Gly Ala Asp Glu Ala Val Gln Pro Gly Glu Tyr
           35                  40                  45
   Thr Gly Thr Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
   65                  70                  75                  80
   Gly Trp Gly Tyr Leu Lys Ser Ser Ser Ala Gly Val Tyr Val Asp Asn
                   85                  90                  95
   His Asp Thr Glu
               100
<210> 30
   <211> 100
   <212> PRT
   <213> Unknown
<220>
   <223> Metagenom
<400> 30
   Val Asp Gly Phe Arg Ile Asp Ala Ala Lys His Met Pro Ala Ala Asp
   1               5                   10                  15
   Leu Ala Asn Ile Lys Ser Arg Leu Thr Asp Pro Gly Ala Tyr Trp Lys
               20                  25                  30
   Gln Glu Ala Ile His Gly Ala Gly Glu Ala Val Ser Pro Ser Glu Tyr
           35                  40                  45
   Leu Gly Ser Gly Asp Val Gln Glu Phe Arg Tyr Ala Arg Asp Leu Lys
       50                  55                  60
   Arg Val Leu Gln Asn Glu Lys Leu Ala Tyr Leu Lys Asn Phe Gly Glu
   65                  70                  75                  80
   Ala Trp Gly His Met Pro Ser Gly Arg Ala Gly Val Phe Val Asp Asn
                   85                  90                  95
   His Asp Thr Glu
               100
<210> 31
   <211> 107
   <212> PRT
   <213> Unknown
<220>
   <223> Metagenom
<400> 31
   Val Asp Gly Phe Arg Ile Asp Ala Val Lys His Ile Arg Asn Thr Tyr
   1               5                   10                  15
   Phe Ala His Trp Leu Gly Lys Leu Arg Ala Glu Asn Pro Gly Arg Glu
               20                  25                  30
   Leu Phe Ala Val Gly Glu Tyr Trp Ser Asp Lys Val Gln Leu Leu His
           35                  40                  45
   Asp Tyr Leu Thr Ala Ser Gly Ser Ala Met Ser Leu Phe Asp Val Pro
       50                  55                  60
   Leu His Tyr Lys Phe Tyr Gln Ala Ser Arg Asn Gly Gly Gly Phe Asp
   65                  70                  75                  80
   Met Gly Ser Ile Leu Gln Arg Thr Leu Met Lys Glu Gln Pro Ala Arg
                   85                  90                  95
   Ala Val Thr Tyr Val Asp Asn His Asp Thr Glu
               100                 105
<210> 32
   <211> 100
   <212> PRT
   <213> Unknown
<220>
   <223> Metagenom
<400> 32
   Val Asp Gly Phe Arg Ile Asp Ala Ala Lys His Ile Pro Ala Ala Asp
   1 5 10 15
   Leu Ala Asn Ile Lys Ser Arg Leu Thr Asn Pro Ser Val Tyr Trp Lys
   20 25 30
   Gln Glu Val Ile Tyr Gly Ala Gly Glu Ala Val Gln Pro Thr Glu Tyr
   35 40 45
   Thr Gly Asn Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Met Ser Ser Ser Val Ala Gly Val Phe Val Asp Asn
   85 90 95
   His Asp Thr Glu
   100
<210> 33
   <211> 107
   <212> PRT
   <213> Unknown
<220>
   <223> Metagenom
<400> 33
   Val Asp Gly Phe Arg Ile Asp Ala Val Lys His Ile Pro Ala Ser Phe
   1 5 10 15
   Phe Glu Asp Trp Phe Asn His Leu Arg Thr His Phe Gly Gly Glu Glu
   20 25 30
   Leu Phe Gly Val Gly Glu Tyr Trp Ser Gly Asn Leu Asp Glu Leu Asn
   35 40 45
   Lys Tyr Leu Ala Asp Thr Ala Gly Val Met Lys Leu Phe Asp Val Pro
   50 55 60
   Leu His Phe Asn Leu Met Asn Ala Ser Arg Gly Gly Arg Asp Tyr Asp
   65 70 75 80
   Leu Ser Lys Val Phe Asp Gly Thr Leu Val Arg Gly Asn Pro Gln Met
   85 90 95
   Ala Val Thr Tyr Val Asp Asn His Asp Thr Glu
   100 105
<210> 34
   <211> 107
   <212> PRT
   <213> Unknown
<220>
   <223> Metagenom
<400> 34
   Val Asp Gly Phe Arg Ile Asp Ala Val Lys His Ile Pro Ala Ser Phe
   1               5                   10                  15
   Tyr Lys Asp Trp Phe Cys His Leu Arg Glu Lys Phe Pro Asp Arg Glu
               20                  25                  30
   Leu Phe Gly Val Ala Glu Tyr Trp Ser Gly Ser Leu Asp Glu Leu Lys
           35                  40                  45
   Gly Tyr Leu Ala Ala Thr Asp Gly Val Val Arg Leu Phe Asp Val Pro
       50                  55                  60
   Leu His Tyr Arg Leu Met Glu Ala Ser Arg Arg Gly Arg Asp Phe Asp
   65                  70                  75                  80
   Leu Ser Lys Ile Phe Asp Gly Thr Leu Val Gln Asp Asn Pro Leu Met
                   85                  90                  95
   Ala Val Thr Phe Val Asp Asn His Asp Thr Glu
               100                 105
<210> 35
   <211> 100
   <212> PRT
   <213> Unknown
<220>
   <223> Metagenom
<400> 35
   Val Asp Gly Phe Arg Ile Asp Ala Ala Lys His Ile Pro Ala Ala Asp
   1 5 10 15
   Leu Ala Asn Ile Lys Ser Arg Leu Ser Asn Pro Gly Val Tyr Trp Lys
   20 25 30
   His Glu Val Ile Tyr Gly Ala Gly Glu Ala Val Gln Pro Thr Glu Tyr
   35 40 45
   Thr Gly Ser Gly Asp Val Gln Glu Phe Arg Tyr Ala Tyr Asp Leu Lys
   50 55 60
   Arg Val Phe Thr Asn Glu Asn Leu Ala Tyr Leu Lys Asn Tyr Gly Glu
   65 70 75 80
   Gly Trp Gly Tyr Leu Asn Ser Gly Val Ser Gly Val Phe Val Asp Asn
   85 90 95
   His Asp Thr Glu
   100
<210> 36
   <211> 100
   <212> PRT
   <213> Unknown
<220>
   <223> Metagenom
<400> 36
   Val Asp Gly Phe Arg Ile Asp Ala Ala Lys His Ile Pro Ala Ala Asp
   1 5 10 15
   Leu Ala Asn Ile Lys Ser Arg Leu Thr Asn Pro Ser Val Tyr Trp Lys
   20 25 30
   Gln Glu Val Ile Tyr Gly Ala Gly Glu Ala Val Gln Pro Thr Glu Tyr
   35 40 45
   Thr Gly Asn Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Met Ser Ser Ser Val Ala Gly Val Tyr Val Asp Asn
   85 90 95
   His Asp Thr Glu
   100
<210> 37
   <211> 100
   <212> PRT
   <213> Unknown
<220>
   <223> Metagenom
<400> 37
   Val Asp Gly Phe Arg Ile Asp Thr Thr Lys His Met Pro Ala Ala Asp
   1               5                   10                  15
   Leu Ala Asn Ile Lys Ser Arg Leu Thr Asp Pro Gly Val Tyr Trp Lys
               20                  25                  30
   Gln Glu Val Ile Phe Gly Ala Gly Glu Ala Val Gln Pro Thr Glu Tyr
           35                  40                  45
   Ile Gly Asn Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
   65                  70                  75                  80
   Gly Trp Gly Tyr Leu Lys Ser Ser Ser Ala Gly Val Tyr Val Asp Asn
                   85                  90                  95
   His Asp Thr Glu
               100
<210> 38
   <211> 100
   <212> PRT
   <213> Unknown
<220>
   <223> Metagenom
<400> 38
   Val Asp Gly Phe Arg Ile Asp Ala Xaa Lys His Ile Pro Ala Ala Asp
   1               5                   10                  15
   Leu Ala Asn Ile Lys Ser Arg Leu Thr Asn Pro Ser Val Tyr Trp Lys
               20                  25                  30
   Gln Glu Val Ile Tyr Gly Ala Gly Glu Ala Val Arg Pro Thr Glu Tyr
           35                  40                  45
   Thr Gly Asn Gly Asp Val Gln Glu Phe Arg Tyr Ala Tyr Asp Leu Lys
       50                  55                  60
   Arg Val Phe Asn Asn Glu Asn Leu Ala Tyr Leu Lys Asn Tyr Gly Glu
   65                  70                  75                  80
   Gly Trp Gly Tyr Met Ser Ser Ser Val Ala Gly Val Tyr Val Asp Asn
                   85                  90                  95
   His Asp Thr Glu
               100
<210> 39
   <211> 100
   <212> PRT
   <213> Unknown
<220>
   <223> Metagenom
<400> 39
   Val Asp Gly Phe Arg Ile Asp Ala Ala Lys His Met Pro Ala Ala Asp
   1               5                   10                  15
   Leu Ala Asn Ile Lys Ser Arg Leu Gly Asp Pro Asn Val Tyr Trp Lys
               20                  25                  30
   Gln Glu Ala Ile Tyr Gly Ala Gly Glu Ala Val Ser Pro Asp Glu Tyr
           35                  40                  45
   Ala Gly Thr Gly Asp Val Gln Glu Phe Arg Tyr Ala Arg Gly Leu Lys
       50                  55                  60
   Gln Val Phe Asn Asn Glu Asn Leu Ala Tyr Leu Lys Asn Tyr Gly Glu
   65                  70                  75                  80
   Gly Trp Gly Phe Met Pro Ser Ser Lys Ala Ala Val Phe Val Asp Asn
                   85                  90                  95
   His Asp Thr Glu
               100
<210> 40
   <211> 100
   <212> PRT
   <213> Unknown
<220>
   <223> Metagenom
<400> 40
   Val Asp Gly Phe Arg Ile Asp Ala Ala Lys His Ile Pro Ala Ala Asp
   1               5                   10                  15
   Leu Ala Asn Ile Lys Ser Arg Leu Thr Asn Pro Ser Val Tyr Trp Lys
               20                  25                  30
   Gln Glu Val Ile Tyr Gly Ala Gly Glu Ala Val Gln Pro Thr Glu Tyr
           35                  40                  45
   Thr Gly Asn Gly Asp Val Gln Glu Phe Arg Tyr Ala Tyr Asp Leu Lys
   50 55 60
   Arg Val Phe Asn Asn Glu Asn Pro Ala Tyr Leu Lys Asn Tyr Gly Glu
   65 70 75 80
   Gly Trp Gly Tyr Met Ser Ser Ser Val Ala Gly Val Tyr Val Asp Asn
   85 90 95
   His Asp Thr Glu
   100
<210> 41
   <211> 111
   <212> PRT
   <213> Unknown
<220>
   <223> Metagenom
<400> 41
   Val Asp Gly Phe Arg Ile Asp Ala Thr Lys His Ile Thr Ser Thr Phe
   1 5 10 15
   Phe Asn Glu Trp Leu Tyr His Val Arg Gln Ala Thr Gly Lys Pro Asn
   20 25 30
   Ala Phe Ala Val Ser Glu Tyr Trp Glu Arg Asp Ile Asn Ala Leu Asn
   35 40 45
   Asn Tyr Val Asn Ser Val Asn Ser Thr Ala Ser Asp Lys Met Ser Ala
   50 55 60
   Phe Asp Val Pro Leu His Ala Lys Phe Glu Gln Ala Ala Asn Ala Asn
   65 70 75 80
   Gly Met Phe Asp Met Gly Ser Leu Leu Thr Asn Thr Leu Val Ala Trp
   85 90 95
   Gln Pro Ala Arg Ala Ala Thr Tyr Val Asp Asn His Asp Thr Glu
   100 105 110
<210> 42
   <211> 107
   <212> PRT
   <213> Unknown
<220>
   <223> Metagenom
<400> 42
   Val Asp Gly Phe Arg Ile Asp Ala Leu Lys His Leu Pro Ala Ser Phe
   1 5 10 15
   Ile Lys Asp Trp Leu Asn Ala Leu Arg Thr His Phe Ser Gly Arg Glu
   20 25 30
   Leu Phe Ala Val Gly Glu Tyr Trp Ser Ala Asp Val Asn Glu Leu His
   35 40 45
   Gly Tyr Leu Ser Lys Val Glu Gly Ala Thr Arg Leu Phe Asp Val Pro
   50 55 60
   Leu His Phe Arg Phe Leu Glu Ala Ser Lys Gln Gly Glu Ser Phe Asp
   65 70 75 80
   Leu Thr Thr Ile Phe Ala Gly Ser Leu Val Ala Glu Asn Pro Leu Ser
   85 90 95
   Ala Val Thr Tyr Val Asp Asn His Asp Thr Glu
   100 105
<210> 43
   <211> 107
   <212> PRT
   <213> Unknown
<220>
   <223> Metagenom
<400> 43
   Val Asp Gly Phe Arg Ile Asp Thr Ile Lys His Ile Pro Ala Ser Phe
   1 5 10 15
   Tyr Arg Glu Trp Phe Asp His Val Arg Gly Ala Phe Pro Ala Arg Glu
   20 25 30
   Leu Phe Gly Val Gly Glu Tyr Trp Ser Gly Asp Val Asn Glu Leu Asp
   35 40 45
   Gly Tyr Leu Ala Ala Thr Gly Gly Val Met Arg Leu Phe Asp Val Pro
   50 55 60
   Leu His Phe Arg Leu His Glu Ala Ser Lys Lys Gly Arg Asp Phe Asp
   65 70 75 80
   Leu Ser Lys Leu Phe Asp Gly Thr Leu Val Gln Arg Asn Pro Leu Met
   85 90 95
   Ala Val Thr Tyr Val Asp Asn His Asp Thr Glu
   100 105
<210> 44
   <211> 107
   <212> PRT
   <213> Unknown
<220>
   <223> Metagenom
<400> 44
   Val Asp Gly Phe Arg Ile Asp Thr Ile Lys His Ile Pro Ala Ser Phe
   1               5                   10                  15
   Phe Lys Asp Trp Leu Thr His Val Arg Ser Thr Phe Lys His Arg Glu
               20                  25                  30
   Leu Phe Ala Val Gly Glu Tyr Trp Ser Gly Ser Leu Asp Glu Leu Lys
           35                  40                  45
   Gly Tyr Leu Ala Ala Val Asp Gly Ala Met Arg Leu Phe Asp Val Pro
       50                  55                  60
   Leu His Phe Asn Phe Arg Asp Ala Ala Gln Gln Gly Lys Ser Phe Asp
   65                  70                  75                  80
   Leu Ser Lys Ile Phe Asp Gly Ser Leu Val Gln Asp Asn Pro Leu Met
                   85                  90                  95
   Ala Val Thr Tyr Val Asp Asn His Asp Thr Glu
               100                 105
<210> 45
   <211> 101
   <212> PRT
   <213> Unknown
<220>
   <223> Metagenom
<400> 45
   Val Asp Gly Phe Arg Ile Asp Ala Ala Glu His Ile Asp Ala Ala Asp
   1               5                   10                  15
   Leu Ser Ala Val Val Ala Gly Leu His Thr Thr Thr Ser Gly Ala Ala
               20                  25                  30
   Pro Tyr Ile Thr Gln Glu Ile Tyr Pro Gly Thr Pro Pro Ala Gln Asp
           35                  40                  45
   Glu Tyr Tyr Gly Thr Gly Asp Val Leu Asp Phe Thr Phe Ala Ala Gln
       50                  55                  60
   Val Lys Ser Gln Phe Gln Gly Asp Ile Ala Asn Leu Ser Ser Phe Gly
   65                  70                  75                  80
   Ser Ser Trp Gly Leu Thr Thr Glu Ala Asn Ser Asn Thr Tyr Val Asp
                   85                  90                  95
   Asn His Asp Thr Glu
               100
<210> 46
   <211> 101
   <212> PRT
   <213> Unknown
<220>
   <223> Metagenom
<400> 46
   Val Asp Gly Phe Arg Ile Asp Ala Ala Lys His Ile Asp Ala Ala Asp
   1               5                   10                  15
   Leu Ser Ala Val Val Ala Gly Leu His Thr Thr Thr Ser Gly Ala Ala
               20                  25                  30
   Pro Tyr Ile Thr Gln Glu Ile Tyr Pro Gly Thr Pro Pro Ala Gln Asp
           35                  40                  45
   Glu Tyr Tyr Gly Thr Gly Asp Val Leu Asp Phe Thr Phe Ala Ala Gln
       50                  55                  60
   Val Lys Ser Gln Phe Gln Gly Asp Ile Ala Asn Leu Ser Ser Phe Gly
   65                  70                  75                  80
   Ser Ser Trp Gly Leu Thr Thr Glu Ala Asn Ser Asn Thr Phe Val Asp
                   85                  90                  95
   Asn His Asp Thr Glu
               100
<210> 47
   <211> 100
   <212> PRT
   <213> Unknown
<220>
   <223> Metagenom
<400> 47
   Val Asp Gly Phe Arg Ile Asp Ala Ala Lys His Ile Pro Ala Ala Asp
   1 5 10 15
   Leu Ala Asn Ile Lys Ser Arg Leu Thr Asn Pro Ser Val Tyr Trp Lys
   20 25 30
   Gln Glu Val Ile Tyr Gly Ala Gly Glu Ala Val Gln Pro Thr Glu Tyr
   35 40 45
   Thr Gly Asp Gly Asp Val Gln Glu Phe Arg Tyr Ala Tyr Asp Leu Lys
   50 55 60
   Arg Val Phe Asn Asn Glu Asn Leu Ala Tyr Leu Lys Asn Tyr Gly Glu
   65 70 75 80
   Gly Trp Gly Tyr Met Ser Ser Ser Val Ala Gly Val Tyr Val Asp Asn
   85 90 95
   His Asp Thr Glu
   100
<210> 48
   <211> 100
   <212> PRT
   <213> Unknown
<220>
   <223> Metagenom
<400> 48
   Val Asp Gly Phe Arg Ile Asp Ala Ala Lys His Met Pro Ala Gly Asp
   1 5 10 15
   Leu Ala Asn Ile Lys Ser Arg Leu Ser Asn Pro Asn Val Tyr Trp Lys
   20 25 30
   His Glu Ala Ile Phe Gly Ala Gly Glu Ala Val Ser Pro Ser Glu Tyr
   35 40 45
   Leu Gly Ser Gly Asp Val Gln Glu Phe Arg Tyr Gly Arg Ser Leu Lys
   50 55 60
   Gln Val Phe Asn Asn Glu Asn Leu Ala Asn Leu Lys Asn Phe Gly Glu
   65 70 75 80
   Gly Trp Gly Phe Met Glu Ser Gly Lys Ser Ala Val Tyr Val Asp Asn
   85 90 95
   His Asp Thr Glu
   100
<210> 49
   <211> 107
   <212> PRT
   <213> Unknown
<220>
   <223> Metagenom
<400> 49
   Val Asp Gly Phe Arg Ile Asp Ala Leu Lys His Leu Pro Ala Gly Phe
   1               5                   10                  15
   Ile Lys Asp Trp Leu Ser Ala Met Arg Thr His Phe Ser Gly Arg Glu
               20                  25                  30
   Leu Phe Ala Val Gly Glu Tyr Trp Ser Ala Asp Val Asn Glu Leu His
           35                  40                  45
   Ser Tyr Leu Thr Lys Val Glu Gly Gly Thr Gln Leu Phe Asp Val Pro
       50                  55                  60
   Leu His Phe Arg Phe Leu Glu Ala Ser Ser Gln Gly Glu Ser Phe Asp
   65                  70                  75                  80
   Leu Thr Lys Ile Phe Glu Gly Ser Leu Val Ala Glu Asn Pro Leu Val
                   85                  90                  95
   Ala Val Thr Phe Val Asp Asn His Asp Thr Glu
               100                 105
<210> 50
   <211> 99
   <212> PRT
   <213> Unknown
<220>
   <223> Metagenom
<400> 50
   Val Asp Gly Phe Arg Ile Asp Thr Ser Lys His Met Pro Ala Ala Asp
   1               5                   10                  15
   Ile Ala Ala Ile Lys Ala Lys Leu Asn Gly Ala Pro Tyr Leu Val Gln
               20                  25                  30
   Glu Val Ile Tyr Gly Ala Gly Glu Pro Ile His Pro Asn Glu Tyr Thr
           35                  40                  45
   Gly Asn Gly Asp Val His Glu Phe Arg Tyr Gly Lys Asp Leu Ser Arg
       50                  55                  60
   Val Phe Arg His Glu Lys Leu Ala Tyr Leu Ala Asn Phe Gly Glu Gly
   65                  70                  75                  80
   Trp Gly His Ile Ser Ser Ala Lys Ala Val Ala Tyr Val Asp Asn His
                   85                  90                  95
   Asp Thr Glu
<210> 51
   <211> 107
   <212> PRT
   <213> Unknown
<220>
   <223> Metagenom
<400> 51
   Val Asp Gly Phe Arg Ile Asp Ala Val Lys His Val Arg Ser Ser Phe
   1               5                   10                  15
   Phe Arg Asp Trp Met Asn His Leu Arg Val His Phe Gly Gly Arg Glu
               20                  25                  30
   Leu Phe Ala Val Gly Glu His Trp Ser Ala His Ala Ala Asp Leu His
           35                  40                  45
   Arg Tyr Ile Thr Ala Thr Glu Gly Val Met Ser Leu Phe Asp Val Ala
       50                  55                  60
   Leu His Tyr Arg Phe Arg Glu Ala Ser Leu Ser Gly Asn Ser Tyr Asp
   65                  70                  75                  80
   Met Arg Thr Ile Leu Asp Gly Thr Leu Val Gly Gln Gln Pro Ala Lys
                   85                  90                  95
   Ala Val Thr Tyr Val Asp Asn His Asp Thr Glu
               100                 105
<210> 52
   <211> 107
   <212> PRT
   <213> Unknown
<220>
   <223> Metagenom
<400> 52
   Val Asp Gly Phe Arg Ile Asp Thr Val Lys His Ile Arg Arg Arg Tyr
   1               5                   10                  15
   Phe Cys Asp Trp Leu Asn His Leu Arg Thr His Phe Gly Gly Arg Glu
               20                  25                  30
   Leu Phe Ala Val Gly Glu Tyr Trp Ser Gly Asp Val Asn Glu Leu His
           35                  40                  45
   Arg Tyr Leu Asp Ala Thr Glu Asn Thr Leu Ser Val Phe Asp Val Pro
       50                  55                  60
   Leu His Tyr Asn Phe Phe Ala Ala Ser His Ala Gly Arg Asp Tyr Asp
   65                  70                  75                  80
   Leu Arg Arg Ile Phe Asp Asn Thr Leu Val Ala Gln Arg Pro Thr Lys
                   85                  90                  95
   Ala Val Thr Phe Val Asp Asn His Asp Thr Glu
               100                 105
<210> 53
   <211> 100
   <212> PRT
   <213> Unknown
<220>
   <223> Metagenom
<400> 53
   Val Asp Gly Phe Arg Ile Asp Thr Ala Lys His Met Ala Ala Ser Asp
   1               5                   10                  15
   Leu Ala Asn Ile Lys Ser Arg Leu Ser Asn Pro Asn Ala Tyr Trp Lys
               20                  25                  30
   Gln Glu Ala Ile Phe Gly Ala Gly Glu Ala Val Ser Pro Ser Glu Tyr
           35                  40                  45
   Leu Gly Asn Gly Asp Val Gln Glu Phe Arg Tyr Ala Arg Asp Leu Lys
       50                  55                  60
   Arg Val Phe Asn Asn Glu Asn Leu Ala Tyr Leu Lys Asn Tyr Gly Glu
   65                  70                  75                  80
   Gly Trp Gly Tyr Met Ser Ser Gly Ser Ser Ala Val Tyr Val Asp Asn
                   85                  90                  95
   His Asp Thr Glu
               100
<210> 54
   <211> 109
   <212> PRT
   <213> Unknown
<220>
   <223> Metagenom
<400> 54
   Val Asp Gly Phe Arg Ile Asp Ala Gly Lys His Ile His Pro Pro Phe
   1 5 10 15
   Leu Ser Arg Trp Phe Gln Glu Val Lys Gly Thr Ser Arg Phe Ala Val
   20 25 30
   Thr Glu Phe Tyr Asp Gly Asn Pro Ala His Leu Ala Thr Val Ile Asp
   35 40 45
   Leu Tyr Ala Arg Gln Ser His Leu Phe Asp Phe Ala Leu His Phe Leu
   50 55 60
   Leu Gln Arg Met Ser Ala Gly Asn Gly Gly Phe Asp Met Arg Ser Leu
   65 70 75 80
   Arg Phe Gly Ser Ser Asp Asp Gly Ser Arg Phe Leu Glu Gln His Pro
   85 90 95
   Ala Phe Ala Val Thr Tyr Val Asp Asn His Asp Thr Glu
   100 105
<210> 55
   <211> 107
   <212> PRT
   <213> Unknown
<220>
   <223> Metagenom
<400> 55
   Val Asp Gly Phe Arg Ile Asp Ala Val Lys His Val Pro Ala Ser Phe
   1 5 10 15
   Tyr Lys Trp Trp Leu Asp Glu Met Arg Gln Asn Tyr Pro Ser Arg Glu
   20 25 30
   Leu Phe Ala Val Gly Glu Tyr Trp Ser Gly Asn Met Gly Glu Leu His
   35 40 45
   Gly Tyr Leu Ala Ala Thr Asp Gly Ala Met Lys Leu Phe Asp Val Pro
   50 55 60
   Leu His Tyr Lys Phe Met Glu Ala Ser Gln Lys Gly Arg Glu Phe Asp
   65 70 75 80
   Leu Pro Thr Ile Phe Asp Gly Thr Leu Val Lys Glu Asn Pro Leu Met
   85 90 95
   Ala Val Thr Phe Val Asp Asn His Asp Thr Glu
   100 105
<210> 56
   <211> 109
   <212> PRT
   <213> Unknown
<220>
   <223> Metagenom
<400> 56
   Val Asp Gly Phe Arg Ile Asp Ala Gly Lys His Ile His Pro Pro Phe
   1 5 10 15
   Leu Ser Arg Trp Phe Gln Glu Val Lys Gly Thr Ser Arg Phe Ala Val
   20 25 30
   Thr Glu Phe Tyr Asp Gly Asn Pro Ala His Leu Ala Thr Val Ile Asp
   35 40 45
   Leu Tyr Ala Arg Gln Ser His Leu Phe Asp Phe Ala Leu His Phe Leu
   50 55 60
   Leu Gln Arg Met Ser Ala Gly Asn Gly Gly Ser Asp Met Arg Asn Leu
   65 70 75 80
   Arg Phe Gly Ser Ser Asp Asp Gly Ser Arg Phe Leu Glu Gln His Pro
   85 90 95
   Ala Phe Ala Val Thr Phe Val Asp Asn His Asp Thr Glu
   100 105
<210> 57
   <211> 302
   <212> DNA
   <213> Unknown
<220>
   <223> Metagenom
<400> 57
   cgtcgacggc ttccggatcg acgccgccaa gcacatcgcg accgaggacc tcgccgccgt 60
   caaggccaag ctgagcaagc cggacgtcta ctggaagcag gagaccatct acagcgccgg 120
   cgaagccgtt cagccgcagg agtacctggg caacggcgac gtccaggagt tccgctacgc 180
   gcgcgacctc aagcgcgtct tccagagcga gcggctggcc tacctgaaga actacggcga 240
   gggctggggc tacctgcccg gcgaccgggc gtccgtctac ggtgacaacc acgacaccga 300
   gc 302
<210> 58
   <211> 302
   <212> DNA
   <213> Unknown
<220>
   <223> Metagenom
<400> 58
   cgtcgacggc ttccggatcg acgcggccaa gcacatggcc gccggcgacc tcgccaacat    60
   caagtcccgg ctcaccaatc cgaacgtgta ctggaagcac gaggcgatct acggcgccgg   120
   cgaggccgtc tccccggccg agtacctcgg cagcggcgac gtacaggagt tccgctacgc   180
   ccgcgacctc aagcgcgtct tcaacaacga gaacctcgcc tacctgaaga acttcggcga   240
   gtcctggggc tacctgccct cggaccaggc cgccgtctac gtggacaacc acgacaccga   300
   gc                                                                  302
<210> 59
   <211> 302
   <212> DNA
   <213> Unknown
<220>
   <223> Metagenom
<400> 59
   cgtcgacggc ttccgcatcg acgccgccaa gcacatggcc gcctctgacc tggccgacat    60
   caagtcccgg ctgagcaacc cgagcgtcta ctggaagcag gaggccatct tcggcgcggg   120
   ggaggcggtc tcgcccagcg aatacctggg caacggcgac gtccaggagt tccgctacgc   180
   ccgtgacctg aagcgggtct tcaacaacga gaacctcgcc tacctgaaga actacggcga   240
   aggttggggc tacatgtcct cgggcagctc ggccgtctac gtcgacaacc acgacaccga   300
   gc                                                                  302
<210> 60
   <211> 302
   <212> DNA
   <213> Unknown
<220>
   <223> Metagenom
<400> 60
   gctcggtgtc gtggttgtcc acgtagacgg cggcctggtc cgagggcagg tagccccagg    60
   actcgccgaa gttcttcagg taggcgaggt tctcgttgtt gaagacgcgc ttgaggtcgc   120
   gggcgtagcg gaactcctgt acgtcgccgc tgccgaggta ctcggccggg gagacggcct   180
   cgccggcgcc gtagatcgcc tcgtgcttcc agtacacgtt cggattggtg agccgggact   240
   tgatgttggc gaggtcgccg gcggccatgt gcttggccgt gtcgatgcgg aagccgtcga   300
   cg                                                                  302
<210> 61
   <211> 302
   <212> DNA
   <213> Unknown
<220>
   <223> Metagenom
<400> 61
   cgtcgacggc ttccggatcg acacggccaa gcacatgccc gccgccgacc tggcgaacat    60
   caagtcccgg ctgaccaacc cgtccgccta ctggaagcag gaggtcatct acggcgcgga   120
   cgaggccgtc cagcccggcg agtacaccgg caccggcgac gtccaggagt tccgctacgc 180
   ctacgacctc aagcgggtct tcaacaacga gaacctcgcc tacctgaaga actacggtga 240
   gggctggggc tacctcaaga gctccagcgc cggcgtctac gtcgacaacc acgacaccga 300
   gc 302
<210> 62
   <211> 302
   <212> DNA
   <213> Unknown
<220>
   <223> Metagenom
<400> 62
   cgtcgacggc ttccgcatcg acgccgccaa gcacatgccg gccgccgacc tcgcgaacat 60
   caagtcgcgg ctcaccgacc ccggcgcgta ctggaagcag gaggccatcc acggcgccgg 120
   cgaggccgtc tcaccgagcg agtacctcgg cagcggcgac gtgcaggagt tccgctacgc 180
   ccgcgacctc aagcgcgtcc tgcagaacga gaagctggcg tacctgaaga acttcggcga 240
   ggcctggggc cacatgccct ccggccgggc cggcgtcttc gtcgacaacc acgacaccga 300
   gc 302
<210> 63
   <211> 323
   <212> DNA
   <213> Unknown
<220>
   <223> Metagenom
<400> 63
   cgtcgacggc ttccggatcg acgcggtgaa gcacatccgc aacacctact tcgcccactg 60
   gctcggcaag ctccgcgccg agaatcccgg gcgcgagctg ttcgcggtgg gggagtattg 120
   gtccgacaag gttcaactgc tgcacgacta cctgaccgcc agcgggagcg cgatgtcgct 180
   cttcgacgtg ccgctgcact acaagttcta ccaggcgagt cgcaacggcg gcgggttcga 240
   catggggtcg atcctccagc gcacgttgat gaaggagcag cccgcgcggg cggtcacgta 300
   cgtcgacaac cacgacaccg agc 323
<210> 64
   <211> 302
   <212> DNA
   <213> Unknown
<220>
   <223> Metagenom
<400> 64
   cgtcgacggc ttccggatcg acgcggccaa gcacatcccg gcggccgacc tcgccaacat 60
   caagtcgcgg ctgaccaatc cgtcggtgta ctggaagcag gaggtcatct acggcgccgg 120
   ggaggcggtc cagccgaccg agtacaccgg caacggcgac gtccaggagt tccggtacgc 180
   ctacgacctc aagcgggtct tcaacaacga gaacctcgcc tacctcaaga actacggcga 240
   gggctggggc tatatgagca gctccgtcgc gggggtcttc gtcgacaacc acgacaccga 300
   gc 302
<210> 65
   <211> 323
   <212> DNA
   <213> Unknown
<220>
   <223> Metagenom
<400> 65
   cgtcgacggc ttccgcatcg acgccgtcaa gcatatcccc gcgagttttt ttgaagactg   60
   gttcaaccac cttcgcaccc atttcggcgg cgaagagctg ttcggcgtcg gtgagtattg  120
   gagcgggaac ctcgacgagt tgaataaata tcttgccgac accgcgggcg tgatgaagct  180
   gttcgacgtg ccgctgcatt ttaatctgat gaacgcctcc aggggcggtc gcgactacga  240
   tctcagcaaa gtcttcgacg gaacgctcgt gcggggcaac ccgcagatgg ccgtcacgta  300
   cgtcgacaac cacgacaccg agc                                          323
<210> 66
   <211> 323
   <212> DNA
   <213> Unknown
<220>
   <223> Metagenom
<400> 66
   cgtcgacggc ttccggatcg acgccgtcaa gcacatcccc gccagcttct acaaggactg   60
   gttctgccac ctccgcgaga agttccccga ccgcgaactg ttcggcgtcg ccgagtactg  120
   gagcggcagc ctcgacgagc tcaaagggta cctcgccgcg accgacggcg tcgtgcgcct  180
   gttcgacgtc cccctccact accgcctgat ggaggcctcg cgccggggcc gcgacttcga  240
   cctctccaag atcttcgacg gcacgctcgt ccaggacaac ccgctcatgg ccgtcacgtt  300
   cgtcgacaac cacgacaccg agc                                          323
<210> 67
   <211> 302
   <212> DNA
   <213> Unknown
<220>
   <223> Metagenom
<400> 67
   cgtcgacggc ttccgcatcg acgcggccaa gcacatcccg gcggccgacc tcgcgaacat   60
   caagtcccgg ctgagcaatc cgggcgtgta ttggaagcac gaggtcatct acggcgccgg  120
   ggaggccgtc cagccgaccg agtacaccgg cagcggcgac gtgcaggagt tccgctacgc  180
   ctacgacctc aagcgcgtct tcaccaacga gaacctggcc tacctgaaga actacggcga  240
   gggctggggg tacctgaaca gcggcgtctc cggtgtcttc gtcgacaacc acgacaccga  300
   gc                                                                 302
<210> 68
   <211> 302
   <212> DNA
   <213> Unknown
<220>
   <223> Metagenom
<400> 68
   cgtcgacggc ttccggatcg acgcggccaa gcacatcccg gcggccgacc tcgccaacat   60
   caagtcgcgg ctgaccaatc cgtcggtgta ctggaagcag gaggtcatct acggcgccgg  120
   ggaggcggtc cagccgaccg agtacaccgg caacggcgac gtccaggagt tccggtacgc  180
   ctacgacctc aagcgggtct tcaacaacga gaacctcgcc tacctcaaga actacggcga  240
   gggctggggc tatatgagca gctccgtcgc gggggtctac gtcgacaacc acgacaccga  300
   gc                                                                  302
<210> 69
   <211> 302
   <212> DNA
   <213> Unknown
<220>
   <223> Metagenom
<400> 69
   gctcggtgtc gtggttgtcc tcgtagacgc ccgcggagga gctcttgagg tagccccagc   60
   cctcgccgta gttcttcagg taggcgaggt tctcgttgtt gaagacccgc ttgaggtcgt  120
   aggcgtaccg gaattcctgg acgtcgccgt tgccgatgta ctcggtgggc tggacggcct  180
   cgcccgcgcc gaagatgacc tcctgcttcc agtacacgcc ggggtcggtg agccgggact  240
   tgatgttcgc caggtcggcg gccggcatgt gcttggtggt gtcgatgcgg aagccgtcga  300
   cg                                                                  302
<210> 70
   <211> 302
   <212> DNA
   <213> Unknown
<220>
   <221> unsure
   <222> 27, 28
   <223> Metagenom
<400> 70
   cgtcgacggc ttccggatcg acgcgannaa gcacatcccg gcggccgacc tcgccaacat   60
   caagtcgcgg ctgaccaatc cgtcggtgta ctggaagcag gaggtcatct acggcgctgg  120
   ggaggcggtc cggccgaccg agtacaccgg caacggcgac gtccaggagt tccggtacgc  180
   ctacgacctc aagcgggtct tcaacaacga gaacctcgcc tacctcaaga actacggcga  240
   gggctggggc tatatgagca gctccgtcgc gggggtctac gtcgacaacc acgacaccga  300
   gc                                                                  302
<210> 71
   <211> 302
   <212> DNA
   <213> Unknown
<220>
   <223> Metagenom
<400> 71
   cgtcgacggc ttccgcatcg acgccgccaa gcacatgccc gccgccgacc tggcgaacat   60
   caagtcacgg ctgggcgacc cgaacgtcta ctggaagcag gaggcgatat acggcgcggg  120
   cgaggcggtc tcgccggacg agtacgccgg caccggggac gtccaggaat tccggtacgc  180
   ccgcggcctc aagcaggtct tcaacaacga gaacctcgcc tatctgaaga actacggcga  240
   gggctggggc ttcatgccct cgtcgaaggc agcggtcttc gtcgacaacc acgacaccga  300
   gc                                                                 302
<210> 72
   <211> 302
   <212> DNA
   <213> Unknown
<220>
   <223> Metagenom
<400> 72
   gctcggtgtc gtggttgtcc acgtagaccc ccgcgacgga gctgctcata tagccccagc   60
   cctcgccgta gttcttgagg taggcggggt tctcgttgtt gaagacccgc ttgaggtcgt  120
   aggcgtaccg gaactcctgg acgtcgccgt tgccggtgta ctcggtcggc tggaccgcct  180
   ccccggcgcc gtagatgacc tcctgcttcc agtacaccga cggattggtc agccgcgact  240
   tgatgttggc gaggtcggcc gccgggatgt gcttggccgc gtcgatccgg aagccgtcga  300
   cg                                                                 302
<210> 73
   <211> 335
   <212> DNA
   <213> Unknown
<220>
   <223> Metagenom <400> 73
<210> 74
   <211> 323
   <212> DNA
   <213> Unknown
<220>
   <223> Metagenom
<400> 74
   cgtcgacggc ttccgcatcg acgcgctcaa gcacctgccg gccagcttca tcaaggattg 60
   gctgaacgcg ctacgcacgc acttcagcgg acgcgagctg tttgcggtgg gcgagtactg 120
   gagtgcagac gtgaacgagc tgcacggcta cttgagcaag gtggagggcg ctacgcggct 180
   gttcgacgtg ccgctgcact ttcgtttcct cgaggccagc aagcaggggg agagcttcga 240
   tctgactacc atctttgcgg gctccctggt ggccgagaac ccgctctcgg ccgtcacgta 300
   cgtggacaac cacgacaccg agc 323
<210> 75
   <211> 323
   <212> DNA
   <213> Unknown
<220>
   <223> Metagenom
<400> 75
   cgtcgacggc ttccgcatcg acacgatcaa gcatatcccg gccagcttct accgcgagtg 60
   gttcgatcac gtccgcggcg cgttccccgc tcgcgaactg ttcggcgtcg gcgagtactg 120
   gagcggcgac gtcaacgagc tcgacggcta cctcgccgcc accggcggcg tcatgcgcct 180
   cttcgacgtc ccgcttcatt tccgcctgca cgaggcatcg aagaagggcc gcgacttcga 240
   cctgtcgaag ctcttcgacg gcacgctcgt gcagcgcaac ccgctgatgg ccgtcacgta 300
   cgtcgacaac cacgacaccg agc 323
<210> 76
   <211> 323
   <212> DNA
   <213> Unknown
<220>
   <223> Metagenom
<400> 76
   cgtcgacggc ttccggatcg acacgatcaa gcacatccca gccagctttt tcaaagactg 60
   gcttacgcat gtgcgctcca cattcaaaca tcgagaacta tttgctgtcg gcgagtactg 120
   gagcggcagc ctggatgaac tgaagggcta cctcgccgcg gtggatggcg cgatgcggct 180
   gttcgacgtg ccgctgcatt tcaatttccg tgacgcggcg cagcagggaa aatcgttcga 240
   cctgagcaag attttcgacg gctccctcgt acaggacaac ccgctgatgg ccgtcaccta 300
   cgtggacaac cacgacaccg agc 323
<210> 77
   <211> 305
   <212> DNA
   <213> Unknown
<220>
   <223> Metagenom
<400> 77
   cgtcgacggc ttccggatcg acgcggccga gcacatcgac gccgccgacc tgagcgctgt   60
   cgtcgccgga ctgcacacca cgacctccgg cgcggcgccg tacatcaccc aggagatcta  120
   ccccggcacc ccgcccgcgc aggacgagta ctacggcacc ggtgacgtcc tcgacttcac  180
   cttcgccgcg caggtgaagt cccagttcca gggcgacatc gccaacctgt cgagcttcgg  240
   cagcagctgg ggcctgacga ccgaggcgaa ctccaacacc tacgtcgaca accacgacac  300
   cgagc                                                              305
<210> 78
   <211> 305
   <212> DNA
   <213> Unknown
<220>
   <223> Metagenom
<400> 78
   cgtcgacggc ttccggatcg acgcggccaa gcacatcgac gccgccgacc tgagcgccgt   60
   cgtcgccgga ctgcacacca cgacctccgg cgcggcgccg tacatcaccc aggagatcta  120
   ccccggcacc ccgcccgcgc aggacgagta ctacggcacc ggtgacgtcc tcgacttcac  180
   cttcgccgcg caggtgaagt cccagttcca gggcgacatc gccaacctgt cgagcttcgg  240
   cagcagctgg ggcctgacga ccgaggcgaa ctccaacacc ttcgtcgaca accacgacac  300
   cgagc                                                              305
<210> 79
   <211> 302
   <212> DNA
   <213> Unknown
<220>
   <223> Metagenom
<400> 79
   cgtcgacggc ttccggatcg acgcggccaa gcacatcccg gcggccgacc tcgccaacat   60
   caagtcgcgg ctgaccaatc cgtcggtgta ctggaagcag gaggtcatct acggcgccgg  120
   ggaggcggtc cagccgaccg agtacaccgg cgacggcgac gtccaggagt tccggtacgc  180
   ctacgacctc aagcgggtct tcaacaacga gaacctcgcc tacctcaaga actacggcga  240
   gggctggggc tacatgagca gctccgtcgc gggggtctac gtcgacaacc acgacaccga  300
   gc                                                                 302
<210> 80
   <211> 302
   <212> DNA
   <213> Unknown
<220>
   <223> Metagenom
<400> 80
   cgtcgacggc ttccgcatcg acgccgccaa gcacatgccc gccggtgacc tcgccaacat   60
   caagtcccgg ctgagcaatc cgaacgtcta ctggaagcac gaggcgatct tcggggccgg  120
   tgaggccgtc tcgccctccg agtacctcgg cagcggtgac gtccaggagt tccgctacgg  180
   ccgaagcctc aagcaggtct tcaacaacga gaacctcgcc aacctgaaga acttcggcga  240
   gggatggggc ttcatggagt ccggcaagtc cgcggtctac gtcgacaacc acgacaccga  300
   gc                                                                 302
<210> 81
   <211> 323
   <212> DNA
   <213> Unknown
<220>
   <223> Metagenom
<400> 81
   cgtcgacggc ttccggatcg acgcgctcaa gcacctgccg gcggggttca tcaaagactg   60
   gctgagcgcc atgcgcacac atttcagtgg ccgcgagctg ttcgcggtcg gtgagtactg  120
   gagtgcagac gtcaacgagc tgcacagcta cctgaccaag gtagagggcg gcacccagct  180
   gttcgacgta ccgctccact ttcgctttct ggaggccagc agccagggcg agagcttcga  240
   cctgaccaag atcttcgagg gctcgctggt ggcggagaat ccgctggtcg cggtcacctt  300
   cgtggacaac cacgacaccg agc                                          323
<210> 82
   <211> 299
   <212> DNA
   <213> Unknown
<220>
   <223> Metagenom
<400> 82
   cgtcgacggc ttccggatcg acaccagcaa gcacatgccg gccgccgaca tcgccgcgat   60
   caaggccaag ctcaacgggg cgccttacct cgtgcaggag gtcatctacg gcgcgggcga  120
   gcccatccac ccgaatgagt acaccggcaa cggcgacgtc cacgagttcc gctacggcaa  180
   ggacctcagc cgggtgttcc gccacgagaa gctcgcctac ctcgccaact tcggcgaagg  240
   gtgggggcac atcagcagtg ccaaggccgt ggcctacgtc gacaaccacg acaccgagc   299
<210> 83
   <211> 323
   <212> DNA
   <213> Unknown
<220>
   <223> Metagenom
<400> 83
   cgtcgacggc ttccgcatcg acgccgtgaa acacgtccgc tcgagcttct tccgtgactg   60
   gatgaaccac ctgcgcgtcc acttcggcgg ccgggagctg ttcgccgtcg gcgagcactg  120
   gtccgcccac gctgctgacc tccaccgcta catcaccgcc accgagggtg tgatgagtct  180
   gttcgatgtg gccctgcact atcgcttccg cgaggcgagc ctctccggaa acagctacga  240
   catgcggacg atcctcgacg ggacgctggt cggccagcaa ccggccaagg ccgtcaccta  300
   cgtggacaac cacgacaccg agc                                          323
<210> 84
   <211> 323
   <212> DNA
   <213> Unknown
<220>
   <223> Metagenom
<400> 84
   cgtcgacggc ttccggatcg acaccgtgaa gcacatccgc cggcggtact tctgcgactg   60
   gctgaaccac cttcgtacgc acttcggcgg gcgcgagctg ttcgcggtgg gggagtactg  120
   gtcgggcgac gtgaacgagc tgcaccgcta tctcgacgcg acggagaaca cgctgtccgt  180
   cttcgacgtc ccgctacact acaacttctt cgccgcgagc catgcggggc gcgattacga  240
   cctccgtcgg atcttcgaca acacgctcgt cgcgcagcgg ccgacgaagg cggtgacgtt  300
   cgtcgacaac cacgacaccg agc                                          323
<210> 85
   <211> 302
   <212> DNA
   <213> Unknown
<220>
   <223> Metagenom
<400> 85
   cgtcgacggc ttccggatcg acaccgccaa gcacatggcc gcctccgacc tggccaacat   60
   caagtcccgg ctgagcaacc cgaacgccta ctggaagcag gaggccatct tcggcgcggg  120
   ggaggcggtc tcgcccagcg agtacctcgg caacggcgac gtccaggagt tccgctacgc  180
   ccgcgacctg aagcgggtct tcaacaacga gaacctcgcc tacctgaaga actacggcga  240
   gggctggggc tacatgtcct cgggcagctc ggccgtctac gtcgacaacc acgacaccga  300
   gc                                                                 302
<210> 86
   <211> 330
   <212> DNA
   <213> Unknown
<220>
   <223> Metagenom
<400> 86
   tcgtcgacgg cttccgcatc gacgccggaa aacacatcca tccgccattt ctctcgcgct   60
   ggttccaaga agtgaagggg acctcccgct ttgccgtgac cgagttttac gacggcaacc  120
   cggcccacct cgccacggtc atcgatctct acgcccggca gagccacctt ttcgacttcg  180
   cgttgcactt tctgctgcag cgcatgagcg ccggcaacgg cggattcgac atgcgcagtc  240
   tgcggttcgg ttcgagtgac gatggctcgc ggtttctcga gcagcatccg gccttcgccg  300
   tcacctacgt cgacaaccac gacaccgagc                                   330
<210> 87
   <211> 323
   <212> DNA
   <213> Unknown
<220>
   <223> Metagenom
<400> 87
   cgtcgacggc ttccggatcg acgccgtcaa gcacgtcccg gcgagctttt acaagtggtg    60
   gctggatgag atgcggcaga actatccgag ccgcgagctg ttcgccgtcg gcgaatactg   120
   gagcggcaac atgggcgagc tgcacggtta tctcgccgcc accgacgggg cgatgaagct   180
   ctttgacgtc ccgctgcact acaagttcat ggaggcgtca cagaaggggc gcgagttcga   240
   cctgcccacg atcttcgacg gcacgctggt gaaggaaaac ccgctgatgg ccgtcacctt   300
   cgtcgacaac cacgacaccg agc                                           323
<210> 88
   <211> 329
   <212> DNA
   <213> Unknown
<220>
   <223> Metagenom
<400> 88
   cgtcgacggc ttccgcatcg acgccggaaa acacatccat ccgccatttc tctcgcgctg    60
   gttccaagaa gtgaagggga cctcccgctt tgccgtgacc gagttttacg acggcaaccc   120
   ggcccacctc gccacggtca tcgatctcta cgcccggcag agccaccttt tcgacttcgc   180
   gttgcacttt ctgctgcagc gcatgagcgc cggcaacggc ggatccgaca tgcgcaatct   240
   gcggttcggt tcgagtgacg atggctcgcg gtttctcgag cagcatccgg ccttcgccgt   300
   caccttcgtg gacaaccacg acaccgagc                                     329
<210> 89
   <211> 299
   <212> DNA
   <213> Unknown
<220>
   <223> Metagenom
<400> 89
   cgtcgacggc ttccgcatcg acgcggccaa gcacatcccg ccggccgacc tggccgcgat    60
   caaggacctc ctcaccggat cgccctacct ccaccaggag gtcatccagg gcgacaacga   120
   ggtgatccag cccggccagt acacgggcat cggcgacgtg cacgagttcg tctacggtcg   180
   caagctcaag gagcagttca acgggcagat caaatggctc ggttccttcg ggcagagctg   240
   ggggctctcc gtccccggcg acaaggccgt ggtgttcgtc gacaaccacg acaccgagc    299
<210> 90
   <211> 302
   <212> DNA
   <213> Unknown
<220>
   <223> Metagenom
<400> 90
   cgtcgacggc ttccggatcg acaccgccaa gcacatcgcc gccagcgacc tggccgccat    60
   cagggcccgg gtgaacggcg gcgacgtcta ctggaagcaa gaggtcatcc acggcgccgg   120
   cgaggcggtc cagcccggcg agtacacggg cgtcggcgac gtgcaggagt tccgctacgc   180
   ctacgacctc aagcgtgtct tccagaacga gcgcctcgcc tacctggaga actacggtga   240
   gggctggggc tacctcccga gcggcggcgc cgccgtcttc gtcgacaacc acgacaccga   300
   gc                                                                  302
<210> 91
   <211> 299
   <212> DNA
   <213> Unknown
<220>
   <223> Metagenom
<400> 91
   cgtcgacggc ttccggatcg acaccagcaa gcacatgccg gccgccgaca tcgcggcgat    60
   caagagcaag ctctcccgcc cggcgtacat cgtgcaggag gtcatcttcg gcgcgggcga   120
   gccgatcggc cccggtgagt acaccggcaa cggcgacgtg cacgagttcc gctacggcaa   180
   ggacctcgcc cgggtcttcc gctccgagcg gctggcgtac ctgaagaact tcggcgaggg   240
   ctggggccac ctgcccagcg gcgtctcctc ggtctacgtc gacaaccacg acaccgagc    299
<210> 92
   <211> 302
   <212> DNA
   <213> Unknown
<220>
   <223> Metagenom
<400> 92
   cgtcgacggc ttccgcatcg acgcggtcaa gcgcatgcct gccgccgacc tggcgaacat    60
   caagtcccgg ctgagcaacc ccgacgcgta ctggaagcag gaggtcatcc acggcgcggg   120
   cgaggccgtc cagcccggcg agtacacggg caacggcgac gtccaggagt tccgctacgc   180
   ctacgacctc aagcgggtct tccacaacga gaacctcgcc tacctgaaga actacggcga   240
   gggctggggc tacctgaaga actcctccgc cggcgtcttc gtggacaacc acgacaccga   300
   gc                                                                  302
<210> 93
   <211> 350
   <212> DNA
   <213> Unknown
<220>
   <223> Metagenom
<400> 93
   cgtcgacggc ttccggatcg acgccaccaa ggcgatcaag aacttcgaca cgctcaagct   60
   gctgaccgat gccgcctaca gcaagatcga agaccgcaag ccgttcatca ccatcggcga  120
   gcacgtgccg gaagatccgg aagtgacagg ccgcgaccgc ggccggccga tggacgcggc  180
   gtggcacgac tacttcggcc acgtcgcgcg ggccgtcgtc gcggaggtgc agaaggatga  240
   tctgcatccg catgacatca agcgatttct tgagcggctc gatccgaaga ccaacggcta  300
   cggcagcgcg tatcgcaccg tcaccttcgt cgacaaccac gacaccgagc             350
<210> 94
   <211> 305
   <212> DNA
   <213> Unknown
<220>
   <223> Metagenom
<220>
   <221> unsure
   <222> 186
<400> 94
   cgtcgacggc ttccgcatcg acgccgccaa gcacatcccc aacgaggacc tgcgggcgat   60
   cttcggccgg ctgcgcgaga ccgtctccgg tgcgcggccg tacgtcttcc acgaggtgtt  120
   ccccggcgag ccgccccgcc cgcaggacta cttcagcacc ggcgacgtgc tggacttcac  180
   ctacgncgac aagatcaaga ccgccttcca gggcgacatc gcctggctgt cgtcgttcgg  240
   cccgagctgg ggcctgctgc cggcggccga ctcggtctcg tacgtggaca accacgacac  300
   cgagc                                                              305
<210> 95
   <211> 290
   <212> DNA
   <213> Unknown
<220>
   <223> Metagenom
<400> 95
   cgtcgacggc ttccgcatcg acgcggccaa gcacatgtat cacggcgata tccagggtat   60
   cctcagccgc gcgggcaatc cctacgtgtt cctcgaggtc atcggctccg cgggcgaggc  120
   cgtgcagccc agccagtata cgtatctcgg ccaggtgacc gagtttggct atagctcgca  180
   catcggccat cgcttcaagt acggccagat caaagatctg aacaacatcg cggacggcaa  240
   gctgcccagc accagcgcga tcgtgtacgt ggacaaccac gacaccgagc             290
<210> 96
   <211> 299
   <212> DNA
   <213> Unknown
<220>
   <223> Metagenom
<400> 96
   cgtcgacggc ttccggatcg acgcggccaa gcacatcccg ccggccgacc tggccgcgat 60
   caaggacctc ctcaccggat cgccctacct ccaccaggag gtcatccagg gcgacaacga 120
   ggtgatccag cccggccagt acacgggcat cggcgacgtg cacgagttcg tctacggtcg 180
   caagctcaag gagcagttca acgggcagat caaatggctc ggttccttcg ggcagagctg 240
   ggggctctcc gtccccggcg acaaggccgt ggtgtacgtg gacaaccacg acaccgagc 299
<210> 97
   <211> 308
   <212> DNA
   <213> Unknown
<220>
   <223> Metagenom
<400> 97
   cgtcgacggc ttccggatcg acgccatcaa gcacatggac acgcgcttcg tgaacgcgtg 60
   gctcgacgcc gtgcgcggcc cgcgtttcgc cgtgagcgag gcatggttcg cgaagctcga 120
   ccagctcacg gcctacgcga aggagctcgg cgaccgcacg cgcctcttcg acgtgccgct 180
   ccactacctg ttttacggga tgagcaacgg caacggggcg tgggacatgc ggaacctcaa 240
   gttcgccggc ttcacggagg cgaacggcaa gctctccgtg ccctacgtgg acaaccacga 300
   caccgagc 308
<210> 98
   <211> 290
   <212> DNA
   <213> Unknown
<220>
   <223> Metagenom
<400> 98
   cgtcgacggc ttccggatcg acacggtcaa gcacatcgcc catggcgacc tcgccggcat 60
   cctgtcgcgc gccggcaatc cgtacaactt catggaggtg atcggcgcgg ccggcgagcc 120
   catccagccg agcgagtaca cgtatctcgg ccaggtgacc gaattcggtt acagctcgca 180
   cctcggccat cgcttcaagt tcggccagat caaggatctg cgcaacatcg gcgacggcaa 240
   gctgcccagc gacaaggcca tcgtgtacgt ggacaaccac gacaccgagc 290
<210> 99
   <211> 299
   <212> DNA
   <213> Unknown
<220>
   <223> Metagenom
<400> 99 <210> 100
   <211> 290
   <212> DNA
   <213> Unknown
<220>
   <223> Metagenom
<400> 100
<210> 101
   <211> 353
   <212> DNA
   <213> Unknown

<220>
   <223> Metagenom
<400> 101
   cgtcgacggc ttccgcatcg acgccgctcg gcacgtcaac accgccttct ggcacgcgtt   60
   caacaccgcg atccgcgacc gcgcgcgcgc cctcggccga cccgacttca tccaattcgg  120
   tgaggtgtac aacgacacac ccgaggctcc cgccgtgctg agcgagttct ccaccggcat  180
   gccgatggac acgacgctcg acttcggctt tttcaacgcc gcccgccact tcgtctcgca  240
   acagcgcccc gccgccgagc tcgccgcctt cttccgcacg gacgacctct acaccgacca  300
   cgacagcaac atccacgcga cgctcaccta cgtcgacaac cacgacaccg agc         353
<210> 102
   <211> 302
   <212> DNA
   <213> Unknown
<220>
   <223> Metagenom
<400> 102
   cgtcgacggc ttccggatcg acaccgccaa gcacatgccc gccgccgacc tggcgaacat   60
   caagtcgcgg ctgggcgacc cgaacgtcta ctggaagcag gaggcgatat acggcgcggg  120
   cgaggcggtc tcgccggacg agtacgccgg caccggggac gtccaggaat tccggtacgc  180
   ccgcggcctc aagcaggtct tcaacaacga gaacctcgcc tatctgaaga actacggcga  240
   gggctggggc ttcatgccct cgtcgaaggc agcggtctac gtcgacaacc acgacaccga  300
   gc                                                                 302
<210> 103
   <211> 326
   <212> DNA
   <213> Unknown
<220>
   <223> Metagenom
<400> 103
   cgtcgacggc ttccgcatcg acaccatccg ccacatgccg caggcgttct ggcacgcgtt   60
   cgcgcgccgc atccgcgccg agcatcccgg tttcttcatg ttcggcgagg cgttcgacac  120
   caaggccgcg aacatcgcgc cgttcacctg ggcggagaac gccaacgtca gcgtgctcga  180
   ttttccgctg aaggaaggtc tgatcgaggt gttcggccgc aagcgcgccg gcttcgagat  240
   cctgctgccg cggctgtacc tgaccgacgg cccgtacgcc aatccgtacg agctgatgac  300
   ctacgtcgac aaccacgaca ccgagc                                        326
<210> 104
   <211> 353
   <212> DNA
   <213> Unknown
<220>
   <223> Metagenom
<400> 104
   cgtcgacggc ttccggatcg acgccgcccg gcacgtcaac accgccttct ggcacgcgtt   60
   caacaccgcg atccgcgacc gcgcccgcgc cctcggccgc cccgacttca tccaatttgg  120
   tgaggtgtac aacgacacgc ccgaggctcc cgccgtgctg agcgagtttt ccaccggcat  180
   gccgatggat acgacgctcg acttcggctt tttcaacgcc gcccgccact tcgtctcgca  240
   acagcgcccc gccgccgagc tcgccgcctt cttccgcacg gacgacctct acaccgacca  300
   cgacagcaac atccacgcga cgctcacctt cgtcgacaac cacgacaccg agc          353
<210> 105
   <211> 290
   <212> DNA
   <213> Unknown
<220>
   <223> Metagenom
<400> 105
   cgtcgacggc ttccgcatcg acgcggtcaa gcacatcgcc catggcgacc tcgccggcat   60
   cctgtcgcgc gccggcaatc cgtacaactt catggaggtg atcggcgcgg ccggcgagcc  120
   catccagccg agcgagtaca cgtatctcgg ccaggtgacc gaattcggtt acagctcgca  180
   cctcggccat cgcttcaagt tcggccagat caaggatctg cgcaacatcg gcgacggcaa  240
   gctgcccagc gacaaggcca tcgtgttcgt ggacaaccac gacaccgagc              290
<210> 106
   <211> 290
   <212> DNA
   <213> Unknown
<220>
   <223> Metagenom
<400> 106
   cgtcgacggc ttccggatcg acacggtcaa gcacatcgcc catggcgacc tcgccggcat   60
   cctgtcgcgc gccggcaatc cgtacaactt catggaggtg atcggcgcgg ccggcgagcc  120
   catccagccg agcgagtaca cgtatctcgg ccaggtgacc gaattcggtt acagctcgca  180
   cctcggccat cgcttcaagt tcggccagat caaggatctg cgcaacatcg gcgacggcaa  240
   gctgcccagc gacaaggcca tggtgtacgt cgacaaccac gacaccgagc             290
<210> 107
   <211> 78
   <212> PRT
   <213> Unknown
<220>
   <223> Metagenom
<400> 107
   Val Tyr Ala Asp Val Val Phe Asn His Thr Gly Glu Gly Asp Ala Thr
   1               5                   10                  15
   Lys Pro Thr Trp Thr Phe Arg Gly Leu Asp Asp Arg Leu Tyr Leu His
               20                  25                  30
   Asp Arg Asp Gly Thr Tyr Asn Asp Asp Ala Gly Cys Gly Asn Val Ala
           35                  40                  45
   Asp Pro Gly Ala Glu Glu Val Arg Arg Leu Val Leu Glu Ala Leu Gln
       50                  55                  60
   Arg Phe Thr Asp Leu Gly Ile Asp Gly Phe Arg Ile Asp Ala
   65                  70                  75
<210> 108
   <211> 81
   <212> PRT
   <213> Unknown
<220>
   <223> Metagenom
<400> 108
   Val Tyr Ala Asp Val Val Phe Asn His Thr Ala Glu Gly Asn Arg Met
   1               5                   10                  15
   Gly Pro Thr Leu Ser Trp Arg Gly Ile Asp Asn Thr His Tyr Tyr Arg
               20                  25                  30
   Leu Thr Glu Asp Lys Arg Tyr His Leu Asp Tyr Thr Gly Thr Gly Asn
           35                  40                  45
   Ala Leu Asn Met Thr Ser Pro Arg Val Leu Gln Met Ile Met Asp Ser
       50                  55                  60
   Leu Arg Tyr Trp Val Met Glu Met His Val Asp Gly Phe Arg Ile Asp
   65                  70                  75                  80
   Ala
<210> 109
   <211> 82
   <212> PRT
   <213> Unknown
<220>
   <223> Metagenom
<400> 109
   Val Tyr Ala Asp Val Val Phe Asn His Thr Gly Glu Gly Asn His Asn
   1 5 10 15
   Gly Pro Thr Ile Ser Phe Arg Gly Gln Ala Asn Glu Ala Tyr Tyr His
   20 25 30
   Leu Val Asp Glu Asp Arg Lys Tyr Tyr Met Asp Tyr Ser Gly Cys Gly
   35 40 45
   Asn Thr Ile Asn Ala Asn His Pro Leu Val Thr Lys Leu Ile Ile Glu
   50 55 60
   Ser Leu Glu Tyr Trp Val Thr Glu His His Val Asp Gly Phe Arg Ile
   65 70 75 80
   Asp Ala
<210> 110
   <211> 80
   <212> PRT
   <213> Unknown
<220>
   <223> Metagenom
<400> 110
   Val Tyr Ala Asp Val Val Phe Asn His Thr Ala Glu Gly Asn Glu Thr
   1 5 10 15
   Gly Pro Thr Phe Cys Phe Lys Gly Leu Glu Asn Glu Val Tyr Tyr Met
   20 25 30
   Leu Asn Glu Asn Gly Thr Tyr Lys Asn Phe Ser Gly Cys Gly Asn Thr
   35 40 45
   Val Asn Gly Asn His Pro Val Met Arg Glu Met Ile Phe His Cys Leu
   50 55 60
   Arg His Trp Val His Asn Tyr His Ile Asp Gly Phe Arg Ile Asp Ala
   65 70 75 80
<210> 111
<211> 82
   <212> PRT
   <213> Unknown
<220>
   <223> Metagenom
<400> 111
   Val Tyr Ala Asp Val Val Phe Asn His Thr Cys Glu Gly Asn Glu Arg
   1               5                   10                  15
   Gly Pro Thr Leu Cys Tyr Arg Gly Leu Asp Ala Pro Val Trp Tyr Trp
               20                  25                  30
   Val Asp Pro Ala Gln Pro Ser Arg His Leu Asp Phe Thr Gly Cys Gly
           35                  40                  45
   Asn Ser Phe Asn Met Arg His Pro Gln Val Leu Lys Leu Ala Met Asp
       50                  55                  60
   Ser Leu Arg Tyr Trp Val Thr Glu Met His Val Asp Gly Phe Arg Ile
   65                  70                  75                  80
   Asp Ala
<210> 112
   <211> 80
   <212> PRT
   <213> Unknown
<220>
   <223> Metagenom
<400> 112
   Val Tyr Ala Asp Val Val Phe Asn His Thr Ala Glu Glu Gly Leu Gly
   1               5                   10                  15
   Gly Pro Thr Ser Ser Leu Arg Gly Ile Asp Asn Arg Asn Tyr Tyr Arg
               20                  25                  30
   Gln Thr Pro Asp Gly Glu Tyr Ile Asp Val Thr Gly Cys Gly Asn Ser
           35                  40                  45
   Val Asn Thr Ala Thr Glu Ala Ser Arg Arg Leu Ile Leu Asp Ser Leu
       50                  55                  60
   Arg Tyr Trp Ala Asn Glu Val Gln Val Asp Gly Phe Arg Ile Asp Ala
   65                  70                  75                  80
<210> 113
   <211> 82
   <212> PRT
   <213> Unknown
<220>
   <223> Metagenom
<400> 113
   Val Tyr Ala Asp Val Val Phe Asn His Thr Ala Glu Gly Asn His Met
   1 5 10 15
   Gly Pro Thr Leu Ser Phe Arg Gly Ile Asp Asn Val Ser Tyr Tyr Arg
   20 25 30
   Val Asp Pro Ala Asn Pro Arg Leu Tyr Val Asp Tyr Thr Gly Cys Gly
   35 40 45
   Asn Thr Leu Asn Met Gln Asn Pro Arg Val Leu Gln Leu Ile Met Asp
   50 55 60
   Ser Leu Arg Tyr Trp Val Thr Glu Met His Val Asp Gly Phe Arg Ile
   65 70 75 80
   Asp Ala
<210> 114
   <211> 82
   <212> PRT
   <213> Unknown
<220>
   <223> Metagenom
<400> 114
   Val Tyr Ala Asp Val Val Ile Asn His Thr Ala Glu Gly Asn Glu Arg
   1 5 10 15
   Gly Pro Thr Leu Ser Phe Arg Gly Ile Asp Asn Ala Ser Tyr Tyr Arg
   20 25 30
   Leu Val Asp Gly Asp Trp Gln His Tyr Tyr Asp Thr Thr Gly Thr Gly
   35 40 45
   Asn Ser Leu Leu Met Arg His Pro Tyr Val Leu Gln Leu Ile Met Asp
   50 55 60
   Ser Leu Arg Tyr Trp Val Thr Glu Met His Val Asp Gly Phe Arg Ile
   65 70 75 80
   Asp Ala
<210> 115
   <211> 81
   <212> PRT
   <213> Unknown
<220>
   <223> Metagenom
<400> 115
   Val Tyr Ala Asp Val Val Tyr Asn His Thr Ala Glu Gly Asn Gln Met
   1 5 10 15
   Gly Pro Thr Leu Ser Phe Arg Gly Ile Asp Asn Ala Ser Tyr Tyr Leu
   20 25 30
   Leu Gly Asp Asp Pro Arg Tyr Tyr Phe Asp Thr Thr Gly Cys Gly Asn
   35 40 45
   Ala Val Asn Leu Arg His Gln Arg Val Leu Gln Met Val Met Asp Ser
   50 55 60
   Leu Arg Tyr Trp Val Gln Glu Cys His Val Asp Gly Phe Arg Ile Asp
   65 70 75 80
   Ala
<210> 116
   <211> 82
   <212> PRT
   <213> Unknown
<220>
   <223> Metagenom
<400> 116
   Val Tyr Ala Asp Val Val Phe Asn His Ser Ala Glu Gly Asn His Met
   1 5 10 15
   Gly Pro Thr Leu Ser Phe Lys Gly Phe Asp Asn Pro Leu Tyr Tyr Arg
   20 25 30
   Leu Val Ala Asp Glu Gln Glu His Tyr Phe Asp Tyr Thr Gly Thr Gly
   35 40 45
   Asn Thr Met Asn Met Arg Ser Pro His Thr Leu Gln Leu Val Met Asp
   50 55 60
   Ser Leu Arg Trp Trp Ala Thr Glu Met His Val Asp Gly Phe Arg Ile
   65 70 75 80
   Asp Ala
<210> 117
   <211> 81
   <212> PRT
   <213> Unknown
<220>
   <223> Metagenom
<400> 117
   Val Tyr Ala Asp Val Val Phe Asn His Thr Cys Glu Gly Asn Glu Glu
   1 5 10 15
   Gly Pro Ile Leu Ser Phe Lys Gly Leu Glu Asn Arg Val Tyr Tyr Met
   20 25 30
   Leu Ala Asn Gly Gly Ser Gln Tyr Met Asn Tyr Ser Gly Cys Gly Asn
   35 40 45
   Thr Val Asn Gly Asn His Pro Ile Val Arg Glu Met Ile Phe His Cys
   50 55 60
   Leu Arg His Trp Val His Asn Tyr His Ile Asp Gly Phe Arg Ile Asp
   65 70 75 80
   Ala
<210> 118
   <211> 82
   <212> PRT
   <213> Unknown
<220>
   <223> Metagenom
<400> 118
   Val Tyr Ala Asp Val Val Phe Asn His Thr Ala Glu Gly Asn His Thr
   1 5 10 15
   Gly Pro Met Leu Ser Phe Lys Gly Ile Asp Asn Gln Ala Tyr Tyr Arg
   20 25 30
   Leu Met Glu Asp Glu Pro Lys Tyr Tyr Arg Asp Tyr Thr Gly Thr Gly
   35 40 45
   Ser Ser Leu Asn Val Arg His Pro His Ser Leu Gln Leu Ile Met Asp
   50 55 60
   Ser Leu Arg Tyr Trp Val Thr Glu Met His Val Asp Gly Phe Arg Ile
   65 70 75 80
   Asp Ala
<210> 119
   <211> 82
   <212> PRT
   <213> Unknown
<220>
   <223> Metagenom
<400> 119
   Val Tyr Ala Asp Val Val Phe Asn His Thr Ala Glu Gly Asn His Leu
   1 5 10 15
   Gly Pro Ser Phe Asn Leu Lys Gly Ile Asp Asn Pro Thr Tyr Tyr Arg
   20 25 30
   Leu Val Pro Asn Glu Pro Arg Tyr Tyr Phe Asp Tyr Thr Gly Thr Gly
   35 40 45
   Asn Thr Leu Asn Val Arg His Pro Gln Val Leu Ala Leu Ile Met Asp
   50 55 60
   Ser Leu Arg Tyr Trp Ala Ile Glu Met His Val Asp Gly Phe Arg Ile
   65 70 75 80
   Asp Ala
<210> 120
   <211> 82
   <212> PRT
   <213> Unknown
<220>
   <223> Metagenom
<400> 120
   Val Tyr Ala Asp Val Val Tyr Asn His Ala Ala Glu Gly Asn His Leu
   1 5 10 15
   Gly Pro Met Leu Ser Phe Lys Gly Tyr Asp Asn Glu Ala Tyr Tyr Arg
   20 25 30
   Leu Gly Pro Asp Asp Pro Arg Tyr Tyr Val Asp Tyr Thr Gly Cys Gly
   35 40 45
   Asn Ser Leu Asn Met Arg Asn Pro His Val Leu Gln Leu Ile Met Asp
   50 55 60
   Ser Leu Arg Tyr Trp Val Glu Glu Met His Val Asp Gly Phe Arg Ile
   65 70 75 80
   Asp Ala
<210> 121
   <211> 82
   <212> PRT
   <213> Unknown
<220>
   <223> Metagenom
<400> 121
   Val Tyr Ala Asp Val Val Phe Asn His Thr Ser Glu Gly Asn His Asp
   1 5 10 15
   Gly Pro Thr Ile Ser Phe Lys Gly Phe Glu Asn Ser Val Tyr Tyr His
   20 25 30
   Leu Thr Pro Arg Asp Arg Gln Tyr Tyr Met Asp Tyr Ser Gly Cys Gly
   35 40 45
   Asn Thr Val Asn Cys Asn His Pro Leu Val Asp Lys Phe Ile Leu Asp
   50 55 60
   Ser Leu Glu Phe Trp Val His Glu Met His Val Asp Gly Phe Arg Ile
   65 70 75 80
   Asp Ala
<210> 122
   <211> 81
   <212> PRT
   <213> Unknown
<220>
   <223> Metagenom
<400> 122
   Val Tyr Ala Asp Val Val Phe Asn His Thr Ala Glu Gly Asn His Leu
   1 5 10 15
   Gly Pro Val Leu Ala Phe Lys Gly Ile Asp Asn Ala Ser Tyr Tyr Arg
   20 25 30
   Leu Ala Asp Asp Ala Arg Tyr Tyr Met Asp Phe Thr Gly Cys Gly Asn
   35 40 45
   Thr Leu Asn Met Arg Ser Pro Gln Val Leu Gln Leu Ile Met Asp Ser
   50 55 60
   Leu Arg Tyr Trp His Leu Asp Met His Val Asp Gly Phe Arg Ile Asp
   65 70 75 80
   Ala
<210> 123
   <211> 82
   <212> PRT
   <213> Unknown
<220>
   <223> Metagenom
<400> 123
   Val Tyr Ala Asp Val Val Phe Asn His Thr Thr Glu Gly Asp Glu His
   1 5 10 15
   Gly Pro Thr Leu Ser Phe Arg Gly Ile Asp Asn Pro Thr His Tyr Leu
   20 25 30
   Leu Asn Pro Asp Asp Arg Ala Arg Tyr Ile Asp Tyr Thr Gly Cys Gly
   35 40 45
   Asn Thr Ile Ser Gly Asn His Pro Ile Val Arg Arg Met Ile Leu Asp
   50 55 60
   Ala Leu Arg Tyr Trp Val Glu His Met His Val Asp Gly Phe Arg Ile
   65 70 75 80
   Asp Ala
<210> 124
   <211> 82
   <212> PRT
   <213> Unknown
<220>
   <223> Metagenom
<400> 124
   Val Tyr Ala Asp Val Val Phe Asn His Thr Ala Glu Gly Asn His Leu
   1 5 10 15
   Gly Pro Val Leu Ser Phe Lys Gly Leu Asp Asn Ala Ala Tyr Tyr Arg
   20 25 30
   Leu Leu Ala Gly Asp Ala Arg Tyr Tyr Met Asp Tyr Thr Gly Thr Gly
   35 40 45
   Asn Ser Leu Asn Met Arg His Pro His Val Leu Gln Leu Leu Met Asp
   50 55 60
   Ser Leu Arg Tyr Trp Val Leu Glu Met His Val Asp Gly Phe Arg Ile
   65 70 75 80
   Asp Ala
<210> 125
   <211> 82
   <212> PRT
   <213> Unknown
<220>
   <223> Metagenom
<400> 125
   Val Tyr Ala Asp Val Val Asn Asn His Thr Ala Glu Gly Asn His Leu
   1               5                   10                  15
   Gly Pro Met Leu Ser Met Lys Gly Ile Asp Asn Ala Ala Tyr Tyr Arg
               20                  25                  30
   Leu Thr Pro Glu Asp Lys Gln Phe Tyr Met Asp Tyr Thr Gly Thr Gly
           35                  40                  45
   Asn Ser Leu Asn Met Arg His Pro His Val Leu Gln Leu Ile Met Asp
       50                  55                  60
   Ser Leu Arg Tyr Trp Val Leu Asp Met His Val Asp Gly Phe Arg Ile
   65                  70                  75                  80
   Asp Ala
<210> 126
   <211> 82
   <212> PRT
   <213> Unknown
<220>
   <223> Metagenom
<400> 126
   Val Tyr Ala Asp Val Val Tyr Asn His Thr Val Glu Gly Asp Gln Arg
   1               5                   10                  15
   Gly Pro Thr Leu Ser Leu Arg Gly Phe Asp Asn Ala Asn Tyr Tyr Arg
               20                  25                  30
   Leu Ser Pro His Asp Arg Ser Leu Tyr Glu Asn Phe Ser Gly Thr Gly
           35                  40                  45
   Asn Thr Val Ser Phe Asp His Pro Ala Val Arg Ser Leu Val Ile Glu
       50                  55                  60
   Cys Leu Arg Tyr Trp Val Ser Asp Met Gly Leu Asp Gly Phe Arg Ile
   65                  70                  75                  80
   Asp Ala
<210> 127
   <211> 80
   <212> PRT
   <213> Unknown
<220>
   <223> Metagenom
<400> 127
   Val Tyr Ala Asp Val Val Phe Asn His Thr Ser Glu Gly Asn Glu Ser
   1 5 10 15
   Gly Pro Thr Phe Gly Phe Lys Gly Leu Glu Asn Arg Val Tyr Tyr Met
   20 25 30
   Leu Asn Ala Asp Gly Thr Tyr Arg Asn Tyr Thr Gly Cys Gly Asn Thr
   35 40 45
   Val Asn Gly Asn His Pro Ile Val Arg Glu Met Ile Phe His Cys Leu
   50 55 60
   Arg His Trp Val His Asn Tyr His Ile Asp Gly Phe Arg Ile Asp Ala
   65 70 75 80
<210> 128
   <211> 80
   <212> PRT
   <213> Unknown
<220>
   <223> Metagenom
<400> 128
   Val Tyr Ala Asp Val Val Phe Asn His Thr Ala Glu Gly Asn Glu Thr
   1 5 10 15
   Gly Pro Thr Phe Cys Phe Lys Gly Leu Glu Asn Lys Val Tyr Tyr Met
   20 25 30
   Leu Asn Asp Asn Gly Thr Tyr Lys Asn Phe Ser Gly Cys Gly Asn Thr
   35 40 45
   Val Asn Gly Asn His Pro Val Met Arg Glu Met Ile Phe His Cys Leu
   50 55 60
   Arg His Trp Val His Asn Tyr His Ile Asp Gly Phe Arg Ile Asp Ala
   65 70 75 80
<210> 129
   <211> 82
   <212> PRT
   <213> Unknown
<220>
   <223> Metagenom
<400> 129
   Val Tyr Ala Asp Val Val Phe Asn His Thr Ala Glu Gly Asn His Leu
   1 5 10 15
   Gly Pro Val Leu Ser Leu Lys Gly Ile Asp Asn Ala Ala Tyr Tyr Arg
   20 25 30
   Leu Val Ala Asp His Arg Arg Tyr Tyr Met Asp Tyr Thr Gly Thr Gly
   35 40 45
   Asn Ser Leu Asn Met Arg His Pro His Val Leu Gln Leu Leu Met Asp
   50 55 60
   Ser Leu Arg Tyr Trp Val Thr Glu Met His Val Asp Gly Phe Arg Ile
   65 70 75 80
   Asp Ala
<210> 130
   <211> 82
   <212> PRT
   <213> Unknown
<220>
   <223> Metagenom
<400> 130
   Val Tyr Ala Asp Val Val Asn Asn His Thr Ala Glu Gly Asn His Leu
   1 5 10 15
   Gly Pro Thr Leu Ser Phe Arg Gly Ile Asp Asn Thr Ser Tyr Tyr Arg
   20 25 30
   Leu Leu His Asp Asn Pro Arg Tyr Tyr Met Asp Phe Thr Gly Cys Gly
   35 40 45
   Asn Thr Leu Asn Met Gln Cys Pro Gln Val Leu Gln Leu Ile Met Asp
   50 55 60
   Ser Leu Arg Tyr Trp Val Leu Glu Met His Val Asp Gly Phe Arg Ile
   65 70 75 80
   Asp Ala
<210> 131
   <211> 79
   <212> PRT
   <213> Unknown
<220>
   <223> Metagenom
<400> 131
   Val Tyr Ala Asp Val Val Phe Asn His Thr Ser Glu Gly Asn Glu Thr
   1 5 10 15
   Gly Pro Thr Phe Ser Phe Lys Gly Leu Glu Asn Arg Val Tyr Tyr Met
   20 25 30
   Leu Asn Asp Asp Gly Thr Tyr Arg Asn Tyr Ser Gly Cys Gly Asn Thr
   35 40 45
   Val Asn Gly Asn His Pro Ile Val Arg Glu Met Ile Phe His Cys Leu
   50 55 60
   Arg His Trp Val His Asn Tyr His Val Asp Gly Phe Arg Thr Ala
   65 70 75
<210> 132
   <211> 82
   <212> PRT
   <213> Unknown
<220>
   <223> Metagenom
<400> 132
   Val Tyr Ala Asp Val Val Phe Asn His Thr Ala Glu Gly Asn His Leu
   1 5 10 15
   Gly Pro Thr Leu Ser Leu Arg Gly Ile Asp Asn Thr Ser Ser Tyr Arg
   20 25 30
   Leu Gln Pro His Asp Pro Arg Phe Tyr Gln Asp Phe Thr Gly Cys Gly
   35 40 45
   Asn Thr Leu Asn Met Arg Ser Pro Arg Met Leu Gln Leu Leu Met Asp
   50 55 60
   Ser Leu Arg Tyr Trp Val Gln Glu Met His Val Asp Gly Phe Arg Ile
   65 70 75 80
   Asp Ala
<210> 133
   <211> 70
   <212> PRT
   <213> Unknown
<220>
   <223> Metagenom
<400> 133
   Val Tyr Ala Asp Val Val Phe Asn His Leu Gly Pro Ala Gly Asn Tyr
   1 5 10 15
   Leu Asn Arg Phe Gly Pro Tyr Phe Thr Asp Arg Tyr Ala Thr Pro Trp
   20 25 30
   Gly Ala Ala Val Asn Leu Asp Gly Pro Gly Ser Asp Glu Val Arg Ala
   35 40 45
   Phe Phe Val Asp Asn Ala Leu Met Trp Leu Arg Asp Tyr His Ile Asp
   50 55 60
   Gly Phe Arg Ile Asp Ala
   65 70
<210> 134
   <211> 82
   <212> PRT
   <213> Unknown
<220>
   <223> Metagenom
<400> 134
   Val Tyr Ala Asp Val Val Ile Asn His Thr Ala Glu Gly Asn His Leu
   1 5 10 15
   Gly Pro Thr Leu Ser Leu Arg Gly Val Asp Asn Ala Thr Tyr Tyr Arg
   20 25 30
   Leu Met Pro Asp Asp Arg Arg His Tyr Leu Asp Phe Thr Gly Cys Gly
   35 40 45
   Asn Thr Leu Asn Met Gln Ser Pro Gln Val Leu Gln Leu Ile Met Asp
   50 55 60
   Ser Leu Arg Tyr Trp Val Leu Glu Met His Val Asp Gly Phe Arg Ile
   65 70 75 80
   Asp Ala
<210> 135
   <211> 81
   <212> PRT
   <213> Unknown
<220>
   <223> Metagenom
<400> 135
   Val Tyr Ala Asp Val Val Ile Asn His Thr Ala Glu Gly Asn His Leu
   1 5 10 15
   Gly Pro Thr Leu Ser Phe Lys Gly Leu Asp Asn Pro Ser Tyr Tyr Arg
   20 25 30
   Leu Ala Asp Asp Pro Arg Tyr Tyr Thr Asp Thr Thr Gly Thr Gly Asn
   35 40 45
   Ser Leu Leu Met Arg Ser Pro His Val Leu Gln Met Ile Met Asp Ser
   50 55 60
   Leu Arg Tyr Trp Val Thr Glu Met His Val Asp Gly Phe Arg Ile Asp
   65 70 75 80
   Ala
<210> 136
   <211> 81
   <212> PRT
   <213> Unknown
<220>
   <223> Metagenom
<400> 136
   Val Tyr Ala Asp Val Val Ile Asn His Ser Arg Glu Gly Asn His Tyr
   1 5 10 15
   Gly Pro Thr Val Ser Phe Lys Gly Leu Asp Asn Asn Ile Tyr Tyr Met
   20 25 30
   Leu Glu Ser Ala Pro Glu Phe Tyr Asn Asp Phe Thr Gly Cys Gly Asn
   35 40 45
   Thr Leu Asn Cys Asn His Pro Val Val Arg Lys Phe Ile Leu Asp Cys
   50 55 60
   Leu Arg Tyr Trp Val Thr Glu Met His Val Asp Gly Phe Arg Ile Asp
   65 70 75 80
   Ala
<210> 137
   <211> 80
   <212> PRT
   <213> Unknown
<220>
   <223> Metagenom
<400> 137
   Val Tyr Ala Asp Val Val Ile Asn His Ser Ala Glu Leu Asp Lys Asp
   1 5 10 15
   Gly Pro Thr Leu Ser Leu Ser Gly Ile Asp Asn Arg Ser Tyr Tyr Trp
   20 25 30
   Leu Met Asp Asn Gly Asp Tyr Gln Asn Trp Thr Gly Cys Gly Asn Thr
   35 40 45
   Leu Asn Leu Ser Thr Pro Gly Val Val Ala Gln Met Met Asp Cys Leu
   50 55 60
   Arg Tyr Trp Val Glu Ser Phe His Ile Asp Gly Phe Arg Ile Asp Ala
   65 70 75 80
<210> 138
   <211> 82
   <212> PRT
   <213> Unknown
<220>
   <223> Metagenom
<400> 138
   Val Tyr Ala Asp Val Val Phe Asn His Thr Ala Glu Gly Asn His Met
   1 5 10 15
   Gly Pro Val Leu Ser Met Lys Gly Ile Asp Asn Ala Gly Tyr Tyr Arg
   20 25 30
   Leu Val Asp Ser Asp Arg Arg Tyr Tyr Tyr Asp Thr Thr Gly Thr Gly
   35 40 45
   Asn Ser Leu Asp Met Arg His Pro His Val Leu Gln Leu Ile Met Asp
   50 55 60
   Ser Leu Arg Tyr Trp Val Thr Asp Met His Val Asp Gly Phe Arg Ile
   65 70 75 80
   Asp Ala
<210> 139
   <211> 82
   <212> PRT
   <213> Unknown
<220>
   <223> Metagenom
<400> 139
   Val Tyr Ala Asp Val Val Phe Asn His Thr Ala Glu Gly Asn His Leu
   1 5 10 15
   Gly Pro Thr Leu Ser Phe Arg Gly Ile Asp Asn Pro Ala Tyr Tyr Arg
   20 25 30
   Leu Val Pro Asp Asp Lys Gln His Tyr Tyr Asp Thr Thr Gly Thr Gly
   35 40 45
   Asn Ser Phe Asn Met Ala His Pro His Ala Leu Gln Leu Ile Met Asp
   50 55 60
   Ser Leu Arg Tyr Trp Val Thr Glu Cys His Val Asp Gly Phe Arg Ile
   65 70 75 80
   Asp Ala
<210> 140
   <211> 81
   <212> PRT
   <213> Unknown
<220>
   <223> Metagenom
<400> 140
   Val Tyr Ala Asp Val Val Phe Asn His Thr Cys Glu Gly Asn Glu Gln
   1 5 10 15
   Gly Pro Ile Leu Ser Xaa Lys Gly Leu Glu Asn Arg Val Tyr Tyr Met
   20 25 30
   Met Ala Asn Gly Gly Ser His Tyr Met Asn Tyr Ser Gly Cys Gly Asn
   35 40 45
   Thr Val Asn Gly Asn His Pro Ile Val Arg Glu Met Ile Phe His Cys
   50 55 60
   Leu Arg His Trp Val His Asn Tyr His Ile Asp Gly Phe Arg Ile Asp
   65 70 75 80
   Ala
<210> 141
   <211> 82
   <212> PRT
   <213> Unknown
<220>
   <223> Metagenom
<400> 141
   Val Tyr Ala Asp Val Val Asn Asn His Thr Cys Glu Gly Asn Gln Met
   1               5                   10                  15
   Gly Pro Thr Leu Ser Trp Arg Gly Ile Asp Asn Ala Ser Tyr Tyr Arg
               20                  25                  30
   Leu Thr Asp Asp Asp Lys Arg Tyr Tyr Met Asp Tyr Thr Gly Cys Gly
           35                  40                  45
   Asn Thr Leu Asn Met Thr His Pro Arg Val Leu Gln Leu Ile Met Asp
       50                  55                  60
   Ser Leu Arg Tyr Trp Val Thr Glu Met His Val Asp Gly Phe Arg Ile
   65                  70                  75                  80
   Asp Ala
<210> 142
   <211> 82
   <212> PRT
   <213> Unknown
<220>
   <223> Metagenom
<400> 142
   Val Tyr Ala Asp Val Val Ile Asn His Thr Gly Glu Gly Asn His Gln
   1               5                   10                  15
   Gly Pro Thr Ile Ser Phe Lys Gly Leu Glu Asn Glu Ala Tyr Tyr Met
               20                  25                  30
   Leu Ser Pro Gly Asp Gly Ala Tyr Tyr Met Asn Tyr Ser Gly Cys Gly
           35                  40                  45
   Asn Thr Val Asn Ala Asn His Pro Val Val Glu Lys Phe Ile Ala Asp
       50                  55                  60
   Cys Leu Gly Tyr Trp Val Glu Glu Met His Val Asp Gly Phe Arg Ile
   65                  70                  75                  80
   Asp Ala
<210> 143
   <211> 82
   <212> PRT
   <213> Unknown
<220>
   <223> Metagenom
<400> 143
   Val Tyr Ala Asp Val Val Ile Asn His Thr Ser Glu Gly Asn His Leu
   1 5 10 15
   Gly Pro Thr Leu Ser Leu Arg Gly Ile Asp Asn Glu Thr Tyr Tyr Arg
   20 25 30
   Leu Thr Pro Arg Thr Ser Ala Thr Thr Ser Ile Ser Pro Ala Ala Gly
   35 40 45
   Thr Arg Ser Asn Met Xaa Ser Pro Gln Val Leu Gln Leu Ile Met Asp
   50 55 60
   Ser Xaa Arg Tyr Trp Val Xaa Xaa Met His Val Asp Gly Phe Arg Ile
   65 70 75 80
   Asp Ala
<210> 144
   <211> 82
   <212> PRT
   <213> Unknown
<220>
   <223> Metagenom
<400> 144
   Val Tyr Ala Asp Val Val Phe Asn His Thr Gly Glu Gly Asn His Gln
   1 5 10 15
   Gly Pro Thr Ile Ser Phe Lys Gly Leu Glu Xaa Arg Gly Val Leu His
   20 25 30
   Ala Leu Ala Gly Arg Arg Gly Val Leu His Glu Leu Leu Arg Leu Arg
   35 40 45
   Glu His Gly Gln Arg Glu Pro Pro Arg Arg Arg Glu Val His Arg Arg
   50 55 60
   Leu Pro Gly Leu Leu Gly Arg Xaa Met His Val Asp Gly Phe Arg Ile
   65 70 75 80
   Asp Ala
<210> 145
   <211> 82
   <212> PRT
   <213> Unknown
<220>
   <223> Metagenom
<400> 145
   Val Tyr Ala Asp Val Val Phe Asn His Thr Ala Glu Gly Asn Gln Leu
   1 5 10 15
   Gly Pro Thr Leu Ser Phe Arg Gly Ile Asp Asn Ala Asn Tyr Tyr Arg
   20 25 30
   Leu Val Asp Glu Asp Lys Lys His Tyr Tyr Asp Thr Thr Gly Thr Gly
   35 40 45
   Asn Thr Leu Leu Met Lys Ser Pro His Val Leu Gln Leu Ile Met Asp
   50 55 60
   Ser Leu Arg Tyr Trp Val Thr Glu Met His Val Asp Gly Phe Arg Ile
   65 70 75 80
   Asp Ala
<210> 146
   <211> 107
   <212> PRT
   <213> Unknown
<220>
   <223> Metagenom
<400> 146
   Val Tyr Ala Asp Val Val Ile Asn His Thr Ser Asp Gln His Pro Xaa
   1 5 10 15
   Phe Gln Glu Ser Arg Gln Asp Arg Thr Asn Pro Lys Ala Asp Trp Tyr
   20 25 30
   Val Trp Asp Asp Asp Gly Thr Lys Trp Ser Glu Ala Arg Val Ile Phe
   35 40 45
   Val Asp Thr Glu Pro Ser Asn Trp Thr Phe Asp Pro Gln Arg Glu Gln
   50 55 60
   Phe Tyr Trp His Arg Phe Phe Ser His Gln Pro Asp Leu Asn Phe Asp
   65 70 75 80
   Asn Pro Glu Val Glu Glu Ala Met Phe Asp Val Met Arg Phe Trp Leu
   85 90 95
   Asp Leu Gly Leu Asp Gly Phe Arg Ile Asp Ala
   100 105
<210> 147
   <211> 82
   <212> PRT
   <213> Unknown
<220>
   <223> Metagenom
<400> 147
   Val Tyr Ala Asp Val Val Phe Asn His Thr Gly Glu Gly Asn His Leu
   1 5 10 15
   Gly Leu Thr Leu Ser Phe Lys Gly Leu Asp Asn Pro Thr Tyr Tyr Gln
   20 25 30
   Leu Lys Arg Xaa Xaa Gln Arg Tyr Tyr Val Asp Tyr Thr Gly Thr Gly
   35 40 45
   Asn Ser Leu Asn Val His His Pro Gln Val Leu Gln Leu Val Leu Asp
   50 55 60
   Ser Leu Arg Tyr Trp Val Lys Val Met His Val Asp Gly Phe Arg Ile
   65 70 75 80
   Asp Ala
<210> 148
   <211> 107
   <212> PRT
   <213> Unknown
<220>
   <223> Metagenom
<400> 148
   Val Tyr Ala Asp Val Val Phe Asn His Ser Ser Asp Arg His Pro Trp
   1 5 10 15
   Phe Val Glu Ser Arg Ser Ser Arg Ala Asn Ala Lys Arg Asp Trp Tyr
   20 25 30
   Ile Trp Arg Asp Pro Ala Pro Gly Gly Gly Pro Pro Asn Asn Trp Met
   35 40 45
   Ser Asp Phe Gly Gly Pro Ala Trp Thr Leu Asp Pro Ala Thr Gly Gln
   50 55 60
   Tyr Tyr Leu His Ala Phe Leu Pro Gln Gln Pro Asp Leu Asn Trp Arg
   65 70 75 80
   Asn Pro Asp Val Arg Ala Ala Met Met Gly Val Leu Arg Phe Trp Leu
   85 90 95
   Asp Arg Gly Val Asp Gly Phe Arg Ile Asp Ala
   100 105
<210> 149
   <211> 71
   <212> PRT
   <213> Unknown
<220>
   <223> Metagenom
<400> 149
   Val Tyr Ala Asp Val Val Phe Asn His Leu Gly Pro Ala Gly Asn Tyr
   1 5 10 15
   Leu Pro Arg Phe Ser Arg Ser Tyr Leu Ser His Arg Tyr Ala Asn Glu
   20 25 30
   Trp Gly Asp Ala Leu Asn Tyr Asp Asp Thr Gly Cys Glu Gly Leu Arg
   35 40 45
   Glu Leu Val Leu Ser Asn Ile Ala Tyr Trp Val Arg Glu Phe His Leu
   50 55 60
   Asp Gly Phe Arg Ile Asp Ala
   65 70
<210> 150
   <211> 82
   <212> PRT
   <213> Unknown
<220>
   <223> Metagenom
<400> 150
   Val Tyr Ala Asp Val Val Phe Asn His Thr Gly Glu Ser Asp Ala Leu
   1 5 10 15
   Gly Pro Thr Leu Ser Leu Arg Gly Leu Asp Asn Arg Ala Tyr Tyr Arg
   20 25 30
   His Leu Pro Gly Glu Ala Gly Ser Leu Val Asn Asp Thr Gly Thr Gly
   35 40 45
   Asn Thr Val Ala Cys Asn Tyr Pro Val Val Arg Gly Leu Val Leu Asp
   50 55 60
   Thr Leu Arg His Phe Val Leu Asn Ala Gly Val Asp Gly Phe Arg Ile
   65 70 75 80
   Asp Ala
<210> 151
   <211> 81
   <212> PRT
   <213> Unknown
<220>
   <223> Metagenom
<400> 151
   Val Tyr Ala Asp Val Val Phe Asn His Thr Gly Glu Gly Asn His Leu
   1 5 10 15
   Gly His Pro Arg Val Gln Gly His Arg Gln Gln Gly Val Leu Pro Val
   20 25 30
   Gly Ala Gly Gln Pro Ala Leu Val His Arg Phe His Gly His Arg Lys
   35 40 45
   Gln Pro Glu His Ala Ala Ser Ala His Asp Pro Ala His His Gly Leu
   50 55 60
   Ala Ala Leu Leu Gly Thr Glu Met His Val Asp Gly Phe Arg Ile Asp
   65 70 75 80
   Ala
<210> 152
   <211> 82
   <212> PRT
   <213> Unknown
<220>
   <223> Metagenom
<400> 152
   Val Tyr Ala Asp Val Val Phe Asn His Thr Thr Glu Gly Asn His Gln
   1 5 10 15
   Gly Pro Thr Ile Asn Phe Arg Gly Phe Asp Asn Ser Val Tyr Tyr Phe
   20 25 30
   Thr Val Pro Asp Asp Lys Gln Tyr Tyr Met Asp Tyr Ser Gly Cys Gly
   35 40 45
   Asn Thr Leu Asn Cys Asn His Pro Val Val Glu Lys Met Ile Leu Asp
   50 55 60
   Cys Leu Glu Phe Trp Val Arg Asp Met His Ile Asp Gly Phe Arg Ile
   65 70 75 80
   Asp Ala
<210> 153
   <211> 82
   <212> PRT
   <213> Unknown
<220>
   <223> Metagenom
<400> 153
   Val Tyr Ala Asp Val Val Tyr Asn His Thr Ala Glu Gly Asn His Leu
   1 5 10 15
   Gly Pro Thr Leu Ser Phe Arg Gly Ile Asp Asn Ala Thr Tyr Tyr Trp
   20 25 30
   Leu Lys Pro Asp Gln Pro Arg Tyr Tyr Glu Asp Phe Thr Gly Cys Gly
   35 40 45
   Asn Ser Leu Asn Leu Thr His Pro His Val Leu Gln Leu Val Met Asp
   50 55 60
   Ser Leu Arg Tyr Trp Val Glu Gln Cys His Val Asp Gly Phe Arg Ile
   65 70 75 80
   Asp Ala
<210> 154
   <211> 70
   <212> PRT
   <213> Unknown
<220>
   <223> Metagenom
<400> 154
   Val Tyr Ala Asp Val Val Phe Asn His Leu Gly Pro Ala Gly Asn Tyr
   1 5 10 15
   Leu Arg Glu Phe Gly Pro Tyr Phe Thr Asp Arg Tyr Gly Thr Pro Trp
   20 25 30
   Gly Asp Ala Val Asn Leu Asp Asp Arg Gly Ser Thr Glu Val Arg Asn
   35 40 45
   Tyr Phe Phe Asp Asn Ala Leu Gly Trp Leu Glu His Tyr His Leu Asp
   50 55 60
   Gly Phe Arg Ile Asp Ala
   65 70
<210> 155
   <211> 82
   <212> PRT
   <213> Unknown
<220>
   <223> Metagenom
<400> 155
   Val Tyr Ala Asp Val Val Phe Asn His Thr Ala Glu Gly Asn His Leu
   1               5                   10                  15
   Gly Pro Met Leu Ser Phe Lys Gly Ile Asp Asn Leu Ser Tyr Tyr Arg
               20                  25                  30
   Thr Leu Pro Gly Asp Pro Arg Phe Tyr Met Asp Tyr Thr Gly Thr Gly
           35                  40                  45
   Asn Ser Leu Asn Val Arg His Pro His Ser Val Gln Leu Ile Met Asp
       50                  55                  60
   Ser Leu Arg Tyr Trp Val Thr Glu Met His Val Asp Gly Phe Arg Ile
   65                  70                  75                  80
   Asp Ala
<210> 156
   <211> 70
   <212> PRT
   <213> Unknown
<220>
   <223> Metagenom
<400> 156
   Val Tyr Ala Asp Val Val Phe Asn His Phe Gly Pro Asp Gly Cys Trp
   1               5                   10                  15
   Ile Glu Lys Phe Ser Pro His Phe Phe Ser Glu Arg Ala Thr Glu Trp
               20                  25                  30
   Gly Arg Ala Ile Asn Phe Asp Gly Glu His Ala Ala Glu Val Arg Ala
           35                  40                  45
   Phe Phe Val Ala Asn Ala Ser Tyr Trp Ile Glu Glu Phe His Leu Asp
       50                  55                  60
   Gly Phe Arg Ile Asp Ala
   65                  70
<210> 157
   <211> 82
   <212> PRT
   <213> Unknown
<220>
   <223> Metagenom
<400> 157
   Val Tyr Ala Asp Val Val Ile Asn His Thr Gly Glu Gly Ser His Thr
   1               5                   10                  15
   Gly Pro Thr Leu Cys Phe Arg Gly Ile Asp Asn Ala Ser Tyr Tyr Arg
               20                  25                  30
   Leu Asn Ala Gln Asn Pro Arg Leu Tyr Val Asp Tyr Thr Gly Cys Gly
           35                  40                  45
   Asn Thr Leu Asn Met Gln Asn Pro Arg Val Leu Gln Leu Ile Met Asp
       50                  55                  60
   Ser Leu Arg Tyr Trp Val Thr Glu Met His Val Asp Gly Phe Arg Ile
   65                  70                  75                  80
   Asp Ala
<210> 158
   <211> 81
   <212> PRT
   <213> Unknown
<220>
   <223> Metagenom
<400> 158
   Val Tyr Ala Asp Val Val Phe Asn His Thr Gly Glu Gly Asn His Leu
   1               5                   10                  15
   Gly Pro Thr Val Ser Leu Lys Gly Ile Asp Asn Thr Ala Tyr Tyr Arg
               20                  25                  30
   Leu Glu Glu Asp Lys Arg Phe Tyr Thr Asp Phe Thr Gly Thr Gly Asn
           35                  40                  45
   Ser Leu Asn Met Arg His Pro Arg Thr Ile Gln Leu Ile Met Asp Ser
       50                  55                  60
   Leu Arg Tyr Trp Val Thr Glu Met His Val Asp Gly Phe Arg Ile Asp
   65                  70                  75                  80
   Ala
<210> 159
   <211> 82
   <212> PRT
   <213> Unknown
<220>
   <223> Metagenom
<400> 159
   Val Tyr Ala Asp Val Val Phe Asn His Thr Ser Glu Gly His His Leu
   1 5 10 15
   Gly Pro Thr Leu Ser Tyr Lys Gly Ile Asp Asn Pro Thr Tyr Tyr Arg
   20 25 30
   Leu Leu Pro Glu Asp Pro Arg Phe Tyr Arg Asp Tyr Thr Gly Thr Gly
   35 40 45
   Asn Ser Leu Asn Met Arg His Pro Gln Thr Leu Lys Leu Val Met Asp
   50 55 60
   Ser Leu Arg Tyr Trp Val Leu Glu Met His Val Asp Gly Phe Arg Ile
   65 70 75 80
   Asp Ala
<210> 160
   <211> 80
   <212> PRT
   <213> Unknown
<220>
   <223> Metagenom
<400> 160
   Val Tyr Ala Asp Val Val Phe Asn His Thr Ala Glu Gly Asn Glu Thr
   1 5 10 15
   Gly Pro Thr Phe Cys Phe Lys Gly Leu Glu Asn Lys Val His Tyr Met
   20 25 30
   Leu Asn Glu Asn Gly Thr Tyr Lys Asn Phe Ser Gly Cys Gly Asn Thr
   35 40 45
   Val Asn Gly Asn His Pro Val Met Arg Glu Met Ile Phe His Cys Leu
   50 55 60
   Arg His Trp Val His Asn Tyr His Ile Asp Gly Phe Arg Ile Asp Ala
   65 70 75 80
<210> 161
   <211> 67
   <212> PRT
   <213> Unknown
<220>
   <223> Metagenom
<400> 161
   Val Tyr Ala Asp Val Val Ile Asn His Phe Gly Pro Ala Gly Asn Ser
   1 5 10 15
   His Tyr Ala Phe Ala Pro Glu Ile Lys Ala Pro Thr Met Thr Glu Trp
   20 25 30
   Gly Asp Ala Leu Asp Tyr Ser Arg Pro Gly Ala Arg Glu Leu Phe Ile
   35 40 45
   Thr Asn Ala Ala Tyr Trp Ile Arg Glu Phe His Phe Asp Gly Phe Arg
   50 55 60
   Ile Asp Ala
   65
<210> 162
   <211> 82
   <212> PRT
   <213> Unknown
<220>
   <223> Metagenom
<400> 162
   Val Tyr Ala Asp Val Val Asn Asn His Thr Ala Glu Gly Asn His Leu
   1 5 10 15
   Gly Pro Met Leu Ser Met Lys Gly Ile Asp Asn Ala Gly Tyr Tyr Arg
   20 25 30
   Leu Val Asp Asp Asp Lys Arg Phe Tyr Tyr Asp Thr Thr Gly Thr Gly
   35 40 45
   Asn Ser Leu Asp Met Arg His Pro His Val Leu Gln Leu Ile Met Asp
   50 55 60
   Ser Leu Arg Tyr Trp Val Thr Glu Met His Val Asp Gly Phe Arg Ile
   65 70 75 80
   Asp Ala
<210> 163
   <211> 80
   <212> PRT
   <213> Unknown
<220>
   <223> Metagenom
<400> 163
   Val Tyr Ala Asp Val Val Phe Asn His Thr Ala Glu Gly Asp Gln Arg
   1 5 10 15
   Gly Pro Thr Leu Ser Trp Arg Gly Leu Asp Asn Ala Ser Ser Tyr Ala
   20 25 30
   Leu Asp Gly Ser Gly Gln Tyr Leu Asn Phe Thr Gly Cys Gly Asn Ala
   35 40 45
   Ile Asn Ala Gly Glu Pro Arg Met Val Gln Phe Val Met Asp Ser Leu
   50 55 60
   Arg Trp Trp Val Gln Ala Phe Gly Val Asp Gly Phe Arg Ile Asp Ala
   65 70 75 80
<210> 164
   <211> 82
   <212> PRT
   <213> Unknown
<220>
   <223> Metagenom
<400> 164
   Val Tyr Ala Asp Val Val Phe Asn His Thr Ala Glu Gly Asn His Leu
   1 5 10 15
   Gly Pro Thr Leu Ser Phe Arg Gly Ile Asp Asn Ala Val Tyr Tyr Lys
   20 25 30
   Leu Pro Ala Asn Asp Arg Arg His Tyr Trp Asp Ser Thr Gly Thr Gly
   35 40 45
   Asn Thr Leu Asp Val Asn His Pro Gln Val Leu Lys Met Val Leu Asp
   50 55 60
   Ser Leu Arg His Trp Val Glu Asp Tyr His Val Asp Gly Phe Arg Ile
   65 70 75 80
   Asp Ala
<210> 165
   <211> 82
   <212> PRT
   <213> Unknown
<220>
   <223> Metagenom
<400> 165
   Val Tyr Ala Asp Val Val Phe Asn His Thr Ala Glu Gly Asn His Leu
   1 5 10 15
   Gly Pro Thr Leu Ser Phe Arg Gly Ile Asp Asn Ala Ala Tyr Tyr Arg
   20 25 30
   Leu Val Asp Ser Asp Arg Arg His Tyr Met Asp Tyr Thr Gly Thr Gly
   35 40 45
   Asn Thr Leu Asn Met Arg Asn Pro Phe Pro Leu Gln Leu Leu Met Asp
   50 55 60
   Ser Leu Arg Tyr Trp Val Thr Glu Cys His Val Asp Gly Phe Arg Ile
   65 70 75 80
   Asp Ala
<210> 166
   <211> 82
   <212> PRT
   <213> Unknown
<220>
   <223> Metagenom
<400> 166
   Val Tyr Ala Asp Val Val Phe Asn His Thr Ala Glu Gly Asn Gln Leu
   1 5 10 15
   Gly Pro Thr Leu Ser Phe Arg Gly Ile Asp Asn Thr Ser Tyr Tyr Trp
   20 25 30
   Leu Leu Pro Asp Glu Pro Arg Tyr Tyr Asp Asp Phe Thr Gly Cys Gly
   35 40 45
   Asn Ala Leu Asn Leu Thr His Pro Arg Val Leu Gln Met Val Met Asp
   50 55 60
   Ser Leu Arg Tyr Trp Val Glu Met Cys Leu Val Asp Gly Phe Arg Ile
   65 70 75 80
   Asp Ala
<210> 167
   <211> 82
   <212> PRT
   <213> Unknown
<220>
   <223> Metagenom
<400> 167
   Val Tyr Ala Asp Val Val Phe Asn His Thr Gly Glu Gly Ser His Thr
   1               5                   10                  15
   Gly Pro Thr Leu Cys Phe Arg Gly Ile Asp Asn Ala Ser Tyr Tyr Arg
               20                  25                  30
   Leu Asn Pro Gln Asn Pro Arg Leu Tyr Val Asp Tyr Thr Gly Cys Gly
           35                  40                  45
   Asn Thr Leu Asn Met Gln Asn Pro Arg Val Leu Gln Leu Ile Met Asp
       50                  55                  60
   Ser Leu Arg Tyr Trp Val Thr Glu Met His Val Asp Gly Phe Arg Ile
   65                  70                  75                  80
   Asp Ala
<210> 168
   <211> 80
   <212> PRT
   <213> Unknown
<220>
   <223> Metagenom
<400> 168
   Val Tyr Ala Asp Val Val Phe Asn His Thr Ala Glu Gly Asn Glu Thr
   1               5                   10                  15
   Gly Pro Thr Phe Cys Phe Lys Gly Leu Glu Asn Lys Val Tyr Tyr Met
               20                  25                  30
   Leu Asn Glu Asn Gly Thr Tyr Lys Asn Phe Ser Gly Cys Gly Asn Thr
           35                  40                  45
   Val Asn Gly Asn His Pro Val Met Arg Glu Met Ile Phe His Cys Leu
       50                  55                  60
   Arg His Trp Val His Asn Tyr His Ile Asp Gly Phe Arg Ile Asp Ala
   65                  70                  75                  80
<210> 169
   <211> 80
   <212> PRT
   <213> Unknown
<220>
   <223> Metagenom
<400> 169
   Val Tyr Ala Asp Val Val Phe Asn His Thr Ala Glu Gly Pro Gln Gly
   1 5 10 15
   Gly Pro Ala Leu Cys Trp Arg Gly Leu Ser Glu Met Lys Tyr Tyr Arg
   20 25 30
   His His Asp Asp Gly Arg Tyr Leu Asp Thr Thr Gly Cys Gly Asn Thr
   35 40 45
   Val Asn Phe Thr Glu Pro Arg Val Ile Gln Phe Ala Leu Asp Ser Leu
   50 55 60
   Arg Tyr Trp Val Asp Glu Phe Gly Ile Asp Gly Phe Arg Ile Asp Ala
   65 70 75 80
<210> 170
   <211> 71
   <212> PRT
   <213> Unknown
<220>
   <223> Metagenom
<400> 170
   Val Tyr Ala Asp Val Val Phe Asn His Phe Gly Pro Asp Gly Asn Tyr
   1 5 10 15
   Leu Arg Ala Tyr Ser Asp Asp Tyr Phe Thr Asp Arg Tyr Ala Thr Pro
   20 25 30
   Trp Gly Glu Ala Ile Asp Tyr Asp Gly Pro Gly Ser Arg Trp Val Arg
   35 40 45
   Gln Phe Ile Arg Glu Asn Ala Arg Tyr Trp Leu Asp Glu Tyr Arg Leu
   50 55 60
   Asp Gly Phe Arg Ile Asp Ala
   65 70
<210> 171
   <211> 82
   <212> PRT
   <213> Unknown
<220>
   <223> Metagenom
<400> 171
   Val Tyr Ala Asp Val Val Tyr Asn His Thr Ala Glu Gly Asn Gln Met
   1 5 10 15
   Gly Pro Thr Leu Ser Leu His Gly Val Asp Asn Val Thr Tyr Tyr Arg
   20 25 30
   Leu Val Pro His Asp Arg Arg Tyr Tyr Gln Asp Phe Thr Gly Cys Gly
   35 40 45
   Asn Thr Leu Asn Met Gln Ser Pro Gln Val Leu Gln Leu Ile Met Asp
   50 55 60
   Ser Leu Arg Tyr Trp Val Leu Glu Met His Val Asp Gly Phe Arg Ile
   65 70 75 80
   Asp Ala
<210> 172
   <211> 82
   <212> PRT
   <213> Unknown
<220>
   <223> Metagenom
<400> 172
   Val Tyr Ala Asp Val Val Phe Asn His Thr Cys Glu Gly Asn Glu Lys
   1 5 10 15
   Gly Pro Thr Leu Ser Trp Arg Gly Ile Asp Asn Val Ala Tyr Tyr Arg
   20 25 30
   Leu Ser Lys Glu Asp Pro Arg Tyr Tyr Thr Asp Phe Thr Gly Cys Gly
   35 40 45
   Asn Thr Leu Asn Met Thr His Pro Arg Val Leu Gln Leu Ile Met Asp
   50 55 60
   Ser Leu Arg Tyr Trp Val Thr Glu Met His Ile Asp Gly Phe Arg Ile
   65 70 75 80
   Asp Ala
<210> 173
   <211> 78
   <212> PRT
   <213> Unknown
<220>
   <223> Metagenom
<400> 173
   Val Tyr Ala Asp Val Val Ile Asn His Thr Ala Ala Ser Gly Gln Ala
   1               5                   10                  15
   Gly Thr Ser Val Leu Asp Arg Ile Val Pro Gly Tyr Tyr Gln Arg Leu
               20                  25                  30
   Leu Ala Asp Gly Ser Val Ala Asn Ser Thr Cys Cys Ser Asn Thr Ala
           35                  40                  45
   Thr Glu Asn Ala Met Met Gly Lys Leu Val Val Asp Ser Val Val Thr
       50                  55                  60
   Trp Ala Lys Glu Tyr Lys Val Asp Gly Phe Arg Ile Asp Ala
   65                  70                  75
<210> 174
   <211> 72
   <212> PRT
   <213> Unknown
<220>
   <223> Metagenom
<400> 174
   Val Tyr Ala Asp Val Val Tyr Asn His Leu Gly Pro Asp Gly Cys Tyr
   1               5                   10                  15
   Leu Thr Lys Phe Ala Thr Glu Tyr Phe Thr Asp Arg Tyr Lys Asn Glu
               20                  25                  30
   Trp Gly Glu Ala Val Lys Leu Arg Arg Arg Glu Leu Cys Ala Gly Ala
           35                  40                  45
   Gly Val Leu Leu Arg Arg Thr Ala Ala Tyr Trp Ile Asp Glu Phe His
       50                  55                  60
   Leu Asp Gly Phe Arg Ile Asp Ala
   65                  70
<210> 175
   <211> 82
   <212> PRT
   <213> Unknown
<220>
   <223> Metagenom
<400> 175
   Val Tyr Ala Asp Val Val Phe Asn His Thr Ala Glu Gly Asn His Leu
   1 5 10 15
   Gly Pro Met Leu Ser Phe Lys Gly Ile Asp Asn Ala Ala Tyr Tyr Arg
   20 25 30
   Val Ile Gly Asp Asn Arg Arg Tyr Tyr Met Asp Tyr Thr Gly Thr Gly
   35 40 45
   Asn Thr Leu Asn Met Arg His Pro His Val Leu Gln Leu Met Met Asp
   50 55 60
   Ser Leu Arg Tyr Phe Val Leu Asp Met His Val Asp Gly Phe Arg Ile
   65 70 75 80
   Asp Ala
<210> 176
   <211> 82
   <212> PRT
   <213> Unknown
<220>
   <223> Metagenom
<400> 176
   Val Tyr Ala Asp Val Val Phe Asn His Thr Ser Glu Gly Asn Ala Glu
   1 5 10 15
   Ser Gly Pro Ala Phe Cys Leu Lys Gly Phe Asp Asn Ser Ile Tyr Tyr
   20 25 30
   Ile Leu Asp Lys Asp Lys Ser Arg Tyr Ala Asp Tyr Thr Gly Cys Gly
   35 40 45
   Asn Thr Leu Asn Ser Asn His Pro Ile Val Arg Arg Met Ile Val Asp
   50 55 60
   Ser Leu Arg Tyr Trp Val Gln Glu Met His Val Asp Gly Phe Arg Ile
   65 70 75 80
   Asp Ala
<210> 177
   <211> 80
   <212> PRT
   <213> Unknown
<220>
   <223> Metagenom
<400> 177
   Val Tyr Ala Asp Val Val Phe Asn His Thr Ala Glu Gly Asn Glu Arg
   1 5 10 15
   Gly Pro Thr Ile Ser Phe Arg Gly Leu Asp Asn Lys Thr Tyr Tyr Met
   20 25 30
   Leu Thr Pro Glu Gly Tyr Tyr Phe Asn Phe Ser Gly Thr Gly Asn Thr
   35 40 45
   Leu Asn Cys Asn Asn Pro Ile Val Arg Asp Met Val Leu Asp Cys Leu
   50 55 60
   Arg Tyr Trp Ala Ala Glu Tyr His Val Asp Gly Phe Arg Ile Asp Ala
   65 70 75 80
<210> 178
   <211> 82
   <212> PRT
   <213> Unknown
<220>
   <223> Metagenom
<400> 178
   Val Tyr Ala Asp Val Val Ile Asn His Thr Thr Glu Gly Asn His Gln
   1 5 10 15
   Gly Pro Ile Val Asn Phe Arg Gly Phe Asp Asn Ser Val Tyr Tyr His
   20 25 30
   Leu Val Gln Asp Asp Lys Gln Tyr Tyr Met Asp Tyr Ser Gly Cys Gly
   35 40 45
   Asn Thr Val Asn Cys Asn His Pro Val Val Glu Lys Met Ile Leu Asp
   50 55 60
   Cys Leu Glu Phe Trp Val Arg Asp Met His Val Asp Gly Phe Arg Ile
   65 70 75 80
   Asp Ala
<210> 179
   <211> 82
   <212> PRT
   <213> Unknown
<220>
   <223> Metagenom
<400> 179
   Val Tyr Ala Asp Val Val Phe Asn His Thr Thr Glu Gly Asn His Gln
   1 5 10 15
   Gly Pro Ile Val Asn Phe Arg Gly Phe Asp Asn Ser Val Tyr Tyr His
   20 25 30
   Leu Val Gln Asp Asp Lys Gln Tyr Tyr Met Asp Tyr Ser Gly Cys Gly
   35 40 45
   Asn Thr Val Asn Cys Asn His Pro Val Val Glu Lys Met Ile Leu Asp
   50 55 60
   Cys Leu Glu Phe Trp Val Arg Asp Met His Val Asp Gly Phe Arg Ile
   65 70 75 80
   Asp Ala
<210> 180
   <211> 80
   <212> PRT
   <213> Unknown
<220>
   <223> Metagenom
<400> 180
   Val Tyr Ala Asp Val Val Phe Asn His Thr Gly Glu Asn Met Glu Val
   1 5 10 15
   Ala Asp Arg Gly Phe Thr Phe Ser Gly Ile Asp Arg Trp Tyr Tyr Tyr
   20 25 30
   Arg Met Asn Gln Glu Gly Glu Leu Ile Gly Pro Tyr Gly Asn Glu Ile
   35 40 45
   Arg Ser Glu Asp Arg Pro Met Val Gln Arg Trp Leu Ile Asp Gln Leu
   50 55 60
   Arg His Leu Val Asp Ile Phe Gly Val Asp Gly Phe Arg Ile Asp Ala
   65 70 75 80
<210> 181
   <211> 82
   <212> PRT
   <213> Unknown
<220>
   <223> Metagenom
<400> 181
   Val Tyr Ala Asp Val Val Tyr Asn His Thr Cys Glu Gly Asn His Leu
   1 5 10 15
   Gly Pro Thr Leu Ser Leu Arg Gly Ile Asp Asn Val Ser Tyr Tyr Arg
   20 25 30
   Leu Ala Glu Asn Glu Arg Arg His Tyr Ala Asp Phe Ser Gly Cys Gly
   35 40 45
   Asn Thr Leu Asn Leu Glu His Pro Asn Val Leu Gln Leu Val Thr Asp
   50 55 60
   Ser Leu Arg Tyr Trp Val Glu Val Met His Val Asp Gly Phe Arg Ile
   65 70 75 80
   Asp Ala
<210> 182
   <211> 82
   <212> PRT
   <213> Unknown
<220>
   <223> Metagenom
<400> 182
   Val Tyr Ala Asp Val Val Tyr Asn His Thr Cys Glu Gly Asn His Leu
   1 5 10 15
   Gly Pro Thr Leu Ser Leu Arg Gly Ile Asp Asn Val Ser Tyr Tyr Arg
   20 25 30
   Leu Ala Glu Asn Glu Arg Arg His Tyr Ala Asp Phe Ser Gly Cys Gly
   35 40 45
   Asn Thr Leu Asn Leu Glu His Pro Asn Val Leu Gln Leu Val Thr Asp
   50 55 60
   Ser Leu Arg Tyr Trp Val Glu Val Met His Val Asp Gly Phe Arg Ile
   65 70 75 80
   Asp Ala
<210> 183
   <211> 80
   <212> PRT
   <213> Unknown
<220>
   <223> Metagenom
<400> 183
   Val Tyr Ala Asp Val Val Phe Asn His Thr Ala Glu Gly Asn Glu Arg
   1 5 10 15
   Gly Pro His Ile Ser Phe Arg Gly Leu Asp Asn Lys Thr Tyr Tyr Met
   20 25 30
   Leu Thr Pro Glu Gly Tyr Tyr Phe Asn Phe Ser Gly Thr Gly Asn Thr
   35 40 45
   Leu Asn Cys Asn Asn Pro Ile Val Arg Ser Met Val Leu Asp Cys Leu
   50 55 60
   Arg Tyr Trp Ala Ser Glu Tyr His Val Asp Gly Phe Arg Ile Asp Ala
   65 70 75 80
<210> 184
   <211> 82
   <212> PRT
   <213> Unknown
<220>
   <223> Metagenom
<400> 184
   Val Tyr Ala Asp Val Val Ile Asn His Thr Ala Glu Gly Asn His Leu
   1 5 10 15
   Gly Pro Thr Leu Ser Phe Arg Gly Ile Asp Asn Pro Ala Tyr Tyr Arg
   20 25 30
   Leu Met Glu Asp Asp Ala Arg Phe Tyr Met Asp Tyr Thr Gly Thr Gly
   35 40 45
   Asn Thr Leu Asn Val Arg Gln Pro His Ser Leu Gln Leu Ile Met Asp
   50 55 60
   Ser Leu Arg Tyr Trp Val Thr Glu Met His Val Asp Gly Phe Arg Ile
   65 70 75 80
   Asp Ala
<210> 185
   <211> 82
   <212> PRT
   <213> Unknown
<220>
   <223> Metagenom
<400> 185
   Val Tyr Ala Asp Val Val Ile Asn His Thr Ala Glu Gly Asn Glu Phe
   1 5 10 15
   Gly Pro Thr Leu Ser Phe Arg Gly Leu Asp Asn Ala Ser Tyr Tyr Arg
   20 25 30
   Leu Leu Pro Asp Asn Pro Arg His Tyr Ile Asn Asp Thr Gly Thr Gly
   35 40 45
   Asn Thr Val Asn Leu Ser His Pro Arg Val Leu Gln Thr Val Met Asp
   50 55 60
   Ser Leu Arg Tyr Trp Val Glu Glu Cys His Val Asp Gly Phe Arg Ile
   65 70 75 80
   Asp Ala
<210> 186
   <211> 82
   <212> PRT
   <213> Unknown
<220>
   <223> Metagenom
<400> 186
   Val Tyr Ala Asp Val Val Tyr Asn His Thr Ala Glu Gly Asp Gln Arg
   1 5 10 15
   Gly Pro Thr Leu Ser Leu Arg Gly Phe Asp Asn Ala Asn Tyr Tyr Arg
   20 25 30
   Leu Ser Pro His Asp Arg Ser Leu Tyr Glu Asn Phe Ser Gly Thr Gly
   35 40 45
   Asn Thr Val Ser Phe Asp His Pro Ala Val Arg Ser Leu Val Ile Glu
   50 55 60
   Cys Leu Arg Tyr Trp Val Ser Asp Met Gly Leu Asp Gly Phe Arg Ile
   65 70 75 80
   Asp Ala
<210> 187
   <211> 82
   <212> PRT
   <213> Unknown
<220>
   <223> Metagenom
<400> 187
   Val Tyr Ala Asp Val Val Tyr Asn His Thr Gly Glu Gly Asn Glu Arg
   1 5 10 15
   Gly Pro Thr Leu Ser Leu Arg Gly Ile Asp Asn Lys Ser Tyr Tyr Arg
   20 25 30
   Leu Asn Pro Glu Asn Gly Arg His Tyr Val Asp Phe Thr Gly Thr Gly
   35 40 45
   Asn Thr Leu Asn Met Met Gln Pro Arg Ser Leu Gln Leu Val Thr Asp
   50 55 60
   Ser Leu Arg Tyr Trp Val Gln Glu Met His Val Asp Gly Phe Arg Ile
   65 70 75 80
   Asp Ala
<210> 188
   <211> 82
   <212> PRT
   <213> Unknown
<220>
   <223> Metagenom
<400> 188
   Val Tyr Ala Asp Val Val Phe Asn His Thr Val Glu Gly Asn His Met
   1 5 10 15
   Gly Pro Val Leu Ala Met Lys Gly Leu Asp Asn Thr Ala Tyr Tyr Arg
   20 25 30
   Thr Met Pro Gly Gln Ala Arg Tyr Tyr Met Asp Tyr Thr Gly Thr Gly
   35 40 45
   Asn Ser Leu His Met Arg His Pro His Val Leu Gln Met Ile Met Asp
   50 55 60
   Ser Leu Arg Tyr Trp Ile Thr Glu Met His Val Asp Gly Phe Arg Ile
   65 70 75 80
   Asp Ala
<210> 189
   <211> 82
   <212> PRT
   <213> Unknown
<220>
   <223> Metagenom
<400> 189
   Val Tyr Ala Asp Val Val Phe Asn His Thr Ala Glu Gly Asn His Leu
   1 5 10 15
   Gly Pro Thr Leu Ser Leu Arg Gly Ile Asp Asn Ser Ser Tyr Tyr Arg
   20 25 30
   Leu Leu Pro Asn Asn Pro Arg Tyr Tyr Gln Asp Phe Thr Gly Thr Gly
   35 40 45
   Asn Thr Leu Asn Met Arg Ser Pro Arg Val Leu Gln Leu Ile Met Asp
   50 55 60
   Ser Leu Arg Tyr Trp Val Leu Glu Met His Val Asp Gly Phe Arg Ile
   65 70 75 80
   Asp Ala
<210> 190
   <211> 81
   <212> PRT
   <213> Unknown
<220>
   <223> Metagenom
<400> 190
   Val Tyr Ala Asp Val Val Phe Asn His Thr Cys Glu Gly Asn Glu His
   1 5 10 15
   Gly Pro Thr Leu Ser Phe Lys Gly Leu Glu Asn Arg Val Tyr Tyr Met
   20 25 30
   Leu Ala Gly Gln Gly Glu His Tyr Lys Asn Tyr Ser Gly Cys Gly Asn
   35 40 45
   Thr Val Asn Gly Asn His Pro Ile Val Arg Glu Met Ile Phe His Cys
   50 55 60
   Leu Arg His Trp Val His Asn Tyr His Val Asp Gly Phe Arg Ile Asp
   65 70 75 80
   Ala
<210> 191
   <211> 69
   <212> PRT
   <213> Unknown
<220>
   <223> Metagenom
<400> 191
   Ala Asp Val Val Phe Asn His Leu Gly Pro Ser Gly Ala Leu Phe Asp
   1 5 10 15
   Arg Phe Ser Pro His Tyr Phe Thr Asp Arg His Asp Asn Asp Trp Gly
   20 25 30
   Gln Ala Leu Asn Phe Asp Asp Pro Glu Ser Gly Pro Val Arg Glu Phe
   35 40 45
   Phe Ile Thr Asp Ala Val His Trp Ile Gly Glu Tyr His Leu Asp Gly
   50 55 60
   Phe Arg Ile Asp Ala
   65
<210> 192
   <211> 69
   <212> PRT
   <213> Unknown
<220>
   <223> Metagenom
<400> 192
   Ala Asp Val Val Phe Asn His Leu Gly Pro Asp Gly Asn Tyr Leu His
   1 5 10 15
   Ala Phe Thr Gly Ser Tyr Phe Thr Asp Lys His Glu Asn Asp Trp Gly
   20 25 30
   Arg Ser Ile Asn Phe Asp Asp Leu His Ser Gly Gly Val Arg Glu Phe
   35 40 45
   Phe Val Ser Asn Ala Arg His Trp Ile Glu Glu Tyr His Phe Asp Gly
   50 55 60
   Phe Arg Ile Asp Ala
   65
<210> 193
   <211> 69
   <212> PRT
   <213> Unknown
<220>
   <223> Metagenom
<400> 193
   Ala Asp Val Val Phe Asn His Leu Gly Pro Glu Gly Asn Tyr Leu His
   1 5 10 15
   Ala Tyr Ala Cys Glu Phe Phe Thr Asp Arg His Lys Thr Asp Trp Gly
   20 25 30
   Ala Ala Ile Asn Phe Asp Gly Pro Asp Ser Arg Thr Val Arg Glu Phe
   35 40 45
   Phe Ile His Asn Ala Leu Tyr Trp Leu Glu Glu Tyr His Phe Asp Gly
   50 55 60
   Phe Arg Ile Asp Ala
   65
<210> 194
   <211> 68
   <212> PRT
   <213> Unknown
<220>
   <223> Metagenom
<400> 194
   Ala Asp Val Val Phe Asn His Leu Gly Pro Glu Ser Asn Phe Ala His
   1 5 10 15
   Glu Phe Gly Pro Tyr Phe Thr Asp Lys Tyr Lys Thr Pro Trp Gly Gln
   20 25 30
   Ala Ile Asn Tyr Asp Asp Lys Gly Cys Asp Gly Val Arg Asp Trp Val
   35 40 45
   Leu Asp Asn Val Arg Met Trp Leu Ala Glu Phe His Leu Asp Gly Phe
   50 55 60
   Arg Ile Asp Ala
   65
<210> 195
   <211> 66
   <212> PRT
   <213> Unknown
<220>
   <223> Metagenom
<400> 195
   Ala Asp Val Val Phe Asn His Leu Gly Pro Glu Gly Asn Cys Leu Pro
   1 5 10 15
   Arg Tyr Ala Pro Glu Phe Phe Thr Ser Arg His Ala Thr Pro Trp Gly
   20 25 30
   Asp Ala Ile Asn Phe Ala His Pro Ile Val Arg Arg Phe Phe Ile Ala
   35 40 45
   Asn Ala Leu Tyr Trp Leu Glu Glu Tyr Gln Leu Asp Gly Phe Arg Ile
   50 55 60
   Asp Ala
   65
<210> 196
   <211> 69
   <212> PRT
   <213> Unknown
<220>
   <223> Metagenom
<400> 196
   Ala Asp Val Val Phe Asn His Leu Gly Pro Ser Gly Asn Tyr Leu Ala
   1 5 10 15
   Arg Phe Ala Arg Asp Tyr Phe Ser Asp Arg His Ala Thr Asp Trp Gly
   20 25 30
   Ala Gly Ile Asn Phe Asp Gly Pro Arg Ala Gln His Val Arg Glu Leu
   35 40 45
   Val Leu Ala Asn Val Glu His Trp Val Arg Glu Phe His Val Asp Gly
   50 55 60
   Phe Arg Ile Asp Ala
   65
<210> 197
   <211> 68
   <212> PRT
   <213> Unknown
<220>
   <223> Metagenom
<400> 197
   Ala Asp Val Val Phe Asn His Phe Gly Pro Asp Gly Asn Tyr Leu Ala
   1 5 10 15
   Gln Phe Ser Arg His Tyr Phe Ala Ala Gln Gln Ser Pro Trp Gly Pro
   20 25 30
   Ala Ile Asn Leu Asp Gly Pro His Ser Ala Pro Val Arg Glu Phe Leu
   35 40 45
   Ile Glu Asn Ala Leu His Trp Val His Glu Tyr His Leu Asp Gly Phe
   50 55 60
   Arg Ile Asp Ala
   65
<210> 198
   <211> 74
   <212> PRT
   <213> Unknown
<220>
   <223> Metagenom
<400> 198
   Ala Asp Val Val Phe Asn His Leu Gly Pro Thr Leu Ser Phe Lys Gly
   1 5 10 15
   Ile Asp Asn Gln Ser Tyr Tyr Arg Leu Met Glu Gly Asp Glu Lys His
   20 25 30
   Tyr Met Asp Tyr Thr Gly Thr Gly Asn Ser Leu Asn Val Arg Gln Pro
   35 40 45
   His Ser Leu Gln Leu Leu Met Asp Ser Leu Arg Tyr Trp Val Thr Glu
   50 55 60
   Met His Val Asp Gly Phe Arg Ile Asp Ala
   65 70
<210> 199
   <211> 68
   <212> PRT
   <213> Unknown
<220>
   <223> Metagenom
<400> 199
   Ala Asp Val Val Phe Asn His Phe Gly Pro Glu Gly Asn Tyr Leu Gly
   1 5 10 15
   Gln Tyr Gly Pro Tyr Phe Thr Asp Arg Tyr Lys Thr Pro Trp Gly Leu
   20 25 30
   Ala Leu Asn Phe Asp Gly Pro Arg Ser Asp Asp Val Arg Trp Phe Phe
   35 40 45
   Ile His Asn Ala Leu His Trp Val Asp Glu Phe His Val Asp Gly Phe
   50 55 60
   Arg Ile Asp Ala
   65
<210> 200
   <211> 69
   <212> PRT
   <213> Unknown
<220>
   <223> Metagenom
<400> 200
   Ala Asp Val Val Phe Asn His Phe Gly Pro Thr Gly Asn Tyr Leu Xaa
   1 5 10 15
   Ala Phe Ser Pro His Tyr Val Ser Lys Lys His Ala Thr Glu Trp Gly
   20 25 30
   Glu Ser Leu Asn Phe Asp Gly Glu Ser Ala Gly Pro Val Arg Glu Phe
   35 40 45
   Val Thr Thr Asn Ala Ala His Trp Ile Arg Glu Tyr His Leu Asp Gly
   50 55 60
   Phe Arg Ile Asp Ala
   65
<210> 201
   <211> 90
   <212> PRT
   <213> Unknown
<220>
   <223> Metagenom
<400> 201
   Tyr Ala Asp Ala Val Leu Asn His Ser Gly Phe Gln Phe Ala Pro Phe
   1               5                   10                  15
   Gln Asp Val Ile Ala Arg Gly Thr Ala Ser Pro Tyr Trp Ser Trp Phe
               20                  25                  30
   Leu Val His Gly Asp Arg Val Asp Val Glu Ser Val Asn Tyr Glu Thr
           35                  40                  45
   Phe Ala Thr Arg Leu Arg His Met Pro Lys Leu Asn Leu Ala Glu Pro
       50                  55                  60
   Ala Ala Glu Glu Tyr Phe Leu His Val Ala Lys His Tyr Val Leu Glu
   65                  70                  75                  80
   Cys Asp Ile Asp Gly Phe Arg Ile Asp Ala
                   85                  90
<210> 202
   <211> 91
   <212> PRT
   <213> Unknown
<220>
   <223> Metagenom
<400> 202
   Tyr Ala Asp Ala Val Phe Asn His Ser Gly Phe Gln Phe Ala Pro Phe
   1               5                   10                  15
   Gln Asp Val Ile Ala Arg Gly Thr Ala Ser Pro Tyr Trp Ser Trp Phe
               20                  25                  30
   Phe Val His Gly Asp Arg Val Asp Val Glu Ser Val Asn Tyr Glu Thr
           35                  40                  45
   Phe Ala Thr Arg Leu Arg His Met Pro Lys Leu Asn Leu Ala Glu Pro
       50                  55                  60
   Ala Ala Glu Glu Tyr Phe Leu His Val Ala Lys His Tyr Val Leu Glu
   65                  70                  75                  80
   Cys Asp Ile Asp Gly Phe Arg Ile Asp Ala Ala
                   85                  90
<210> 203
   <211> 82
   <212> PRT
   <213> Unknown
<220>
   <223> Metagenom
<400> 203
   Tyr Ala Asp Ala Val Phe Asn His Thr Cys Glu Gly Asn Glu Lys Gly
   1               5                   10                  15
   Pro Thr Leu Ser Trp Arg Gly Ile Asp Asn Val Ala Tyr Tyr Arg Leu
               20                  25                  30
   Ser Lys Glu Asp Pro Arg Tyr Tyr Thr Asp Phe Thr Gly Cys Gly Asn
           35                  40                  45
   Thr Leu Asn Met Thr Arg Pro Arg Val Leu Gln Leu Ile Met Asp Ser
       50                  55                  60
   Leu Arg Tyr Trp Val Thr Glu Met His Ile Asp Gly Phe Arg Ile Asp
   65                  70                  75                  80
   Ala Ala
<210> 204
   <211> 70
   <212> PRT
   <213> Unknown
<220>
   <223> Metagenom
<400> 204
   Tyr Ala Asp Ala Val Phe Asn His Leu Gly Pro Ala Gly Asn Tyr Leu
   1               5                   10                  15
   Asn Arg Phe Gly Pro Tyr Phe Thr Asp Arg Tyr Val Thr Pro Trp Gly
               20                  25                  30
   Ala Ala Val Asn Leu Asp Gly Pro Gly Ser Asp Glu Val Arg Ala Phe
           35                  40                  45
   Phe Val Asp Asn Ala Leu Met Trp Leu Arg Asp Tyr His Ile Asp Gly
       50                  55                  60
   Phe Arg Ile Asp Ala Ala
   65                  70
<210> 205
   <211> 91
   <212> PRT
   <213> Unknown
<220>
   <223> Metagenom
<400> 205
   Tyr Ala Asp Ala Val Leu Asn His Ser Gly Phe Gln Phe Ala Pro Ser
   1               5                   10                  15
   Gln Asp Val Ile Ala Arg Gly Thr Ala Ser Pro Tyr Trp Ser Trp Phe
               20                  25                  30
   Phe Val His Gly Asp Arg Val Asp Val Glu Ser Val Asn Tyr Glu Thr
           35                  40                  45
   Phe Ala Thr Arg Leu Arg His Met Pro Lys Leu Asn Leu Ala Glu Pro
       50                  55                  60
   Ala Ala Glu Glu Tyr Phe Leu His Val Ala Lys His Tyr Val Leu Glu
   65                  70                  75                  80
   Cys Asp Ile Asp Gly Phe Arg Ile Asp Ala Ala
                   85                  90
<210> 206
   <211> 272
   <212> DNA
   <213> Unknown
<220>
   <223> Metagenom
<400> 206
   gtacgccgac gccgttttaa atcactcggg ctttcaattt gccccctttc aggacgtcat    60
   tgcgcgcggt acggcgtctc cctattggtc ttggttcctc gtgcatgggg accgcgttga   120
   cgtcgaatcc gtgaattatg agactttcgc gacgcgcctt cgacatatgc caaaactgaa   180
   cttggcagag cctgctgccg aagaatactt tctgcatgtg gccaaacact atgttctcga   240
   gtgcgatatc gacggattta ggatcgacgc cg                                 272
<210> 207
   <211> 274
   <212> DNA
   <213> Unknown
<220>
   <223> Metagenom
<400> 207
   gtacgccgac gccgtcttta atcactcggg ctttcaattt gccccctttc aggacgtcat    60
   tgcgcgcggt acggcgtctc cctattggtc ttggttcttc gtgcatgggg accgcgttga   120
   cgtcgaatcc gtgaattatg agactttcgc gacgcgcctt cgacatatgc caaaactgaa   180
   cttggcagag cctgctgccg aagaatactt tctgcatgtg gccaaacact atgttctcga   240
   gtgcgatatc gacgggttca tgatcgacgc cgcc                               274
<210> 208
   <211> 247
   <212> DNA
   <213> Unknown
<220>
   <223> Metagenom
<400> 208
   gtacgccgac gccgtgttta accacacgtg cgaagggaac gagaaaggcc cgacgctctc    60
   gtggcgcggg atcgacaacg tcgcgtatta ccgtctgtcg aaggaagatc cgcgctacta   120
   cacggatttc accggctgcg gcaacacgct gaacatgacg cgcccgcgcg tgctccagct   180
   catcatggac tccctgcgct actgggtgac ggagatgcac atcgatggat tcaggatcga   240
   cgccgcc                                                             247
<210> 209
   <211> 211
   <212> DNA
   <213> Unknown
<220>
   <223> Metagenom
<400> 209
   gtacgccgac gccgttttta atcacctcgg ccctgccggc aactacctga accgcttcgg    60
   gccctacttc accgatcgct acgtcacccc ttggggagcg gcggtgaacc tcgacgggcc   120
   gggtagcgac gaggtgcggg ccttcttcgt cgacaacgcc ctcatgtggc tgcgggacta   180
   ccacatcgac ggattccgga tcgacgccgc c                                  211
<210> 210
   <211> 274
   <212> DNA
   <213> Unknown
<220>
   <223> Metagenom
<400> 210
   gtacgccgac gccgttttaa atcactcggg ctttcaattt gccccctctc aggacgtcat    60
   tgcgcgcggt acggcgtctc cctattggtc ttggttcttc gtgcatgggg accgcgttga   120
   cgtcgaatcc gtgaattatg agactttcgc gacgcgcctt cgacatatgc caaaactgaa   180
   cttggcagag cctgctgccg aagaatactt tctgcatgtg gccaaacact atgttctcga   240
   gtgcgatatc gacggcttcc ggatcgacgc cgcc                               274
<210> 211
   <211> 205
   <212> DNA
   <213> Unknown
<220>
   <223> Metagenom
<400> 211
   cgacgtggtg ttcaaccatc ttggtccctc aggcgctctc ttcgaccgct tctccccgca    60
   ctacttcacc gaccggcatg acaacgactg ggggcaggcg ctcaacttcg acgaccccga    120
   atccggtccc gtccgggagt tcttcatcac cgacgccgtc cactggatcg gcgaatacca   180
   cctcgacggg ttccgcatcg acgcc                                         205
<210> 212
   <211> 207
   <212> DNA
   <213> Unknown
<220>
   <223> Metagenom
<400> 212
   gccgacgtgg tgttcaatca tctaggcccg gacggcaact acctccacgc ctttaccggg   60
   tcctacttca ccgacaagca cgagaacgac tggggccggt cgatcaactt cgacgatctg  120
   cactccggcg gcgtgcgcga gttcttcgtg tcgaacgcca ggcactggat cgaggagtat  180
   cacttcgacg ggttccgcat cgacgcc                                      207
<210> 213
   <211> 207
   <212> DNA
   <213> Unknown
<220>
   <223> Metagenom
<400> 213
   gccgacgtgg tgttcaatca cttggggccg gaggggaact acctgcacgc gtacgcgtgc   60
   gagttcttca ccgaccgcca caagaccgac tggggcgcgg cgatcaactt cgacgggccc  120
   gactcgcgca ccgtgcgcga gttcttcatc cataacgcgc tctactggct cgaggagtat  180
   cacttcgacg gcttccgcat cgacgcc                                      207
<210> 214
   <211> 204
   <212> DNA
   <213> Unknown
<220>
   <223> Metagenom
<400> 214
   ggcgtcgatg cggaacccgt cgaggtgaaa ctcggcgagc cacatgcgaa cattgtcgag   60
   gacccaatca cgaacgccat cgcagccttt gtcgtcgtag ttgatcgcct gcccccaagg  120
   cgttttgtat ttgtcggtga agtacggacc gaattcgtga gcgaaattcg attcgggccc  180
   taagtggttg aacaccacgt cggc                                         204
<210> 215
   <211> 198
   <212> DNA
   <213> Unknown
<220>
   <223> Metagenom
<400> 215
   gccgacgtgg tgttcaatca cctaggtccg gaaggcaact gcctgccgcg ttatgcaccc    60
   gagttcttca cctcacgcca tgccacaccc tggggcgacg ccatcaattt cgcgcacccg   120
   atcgtgcggc gttttttcat tgcgaatgcg ctctattggc tcgaggaata ccaattggac   180
   ggcttccgca tcgacgcc                                                  198
<210> 216
   <211> 205
   <212> DNA
   <213> Unknown
<220>
   <223> Metagenom
<400> 216
   cgacgtggtg ttcaaccatc ttggtccgag cgggaactat ctcgcccggt tcgcgcgcga    60
   ctacttcagc gatcgccacg ccaccgactg gggcgcgggc atcaacttcg atgggccgcg    120
   tgcgcagcac gtgcgcgagc tcgtgctggc caacgtggag cattgggtgc gcgagttcca   180
   cgtcgacggc ttccgcatcg acgcc                                          205
<210> 217
   <211> 202
   <212> DNA
   <213> Unknown
<220>
   <223> Metagenom
<400> 217
   cgacgtggtg ttcaaccact ttggtcccga cggcaactac ctggcgcagt tcagccgcca    60
   ctacttcgcg gcgcagcaga gcccgtgggg accggccatc aacctcgacg gcccccacag   120
   cgcgccggtg cgcgagttcc tcatcgagaa cgcgctgcac tgggtccacg agtaccacct   180
   cgacgggttc cgcatcgacg cc                                             202
<210> 218
   <211> 225
   <212> DNA
   <213> Unknown
<220>
   <223> Metagenom
<400> 218
   cgacgtggtg ttcaaccacc ttggtcccac cctgtccttc aagggcatcg acaaccagtc    60
   ctactaccgg ctgatggagg gcgacgaaaa gcactacatg gactacacgg gaacgggcaa   120
   ctcgctcaac gtccgccagc cgcactcgct gcagctgctc atggattcct tgcggtactg   180
   ggtcaccgaa atgcatgtgg acgggttccg catcgacgcc aaggg                    225
<210> 219
   <211> 201
   <212> DNA
   <213> Unknown
<220>
   <223> Metagenom
<400> 219
   cgacgtggtg ttcaaccact ttggacccga ggggaactac ctcggccagt acggtcccta   60
   cttcacggac cgctacaaga cgccgtgggg gctggcactc aacttcgacg gccctcgcag  120
   cgacgacgtg cgctggttct tcatccacaa cgcgctgcat tgggtcgacg agttccacgt  180
   cgacggcttc cgcatcgacg c                                              201
<210> 220
   <211> 204
   <212> DNA
   <213> Unknown
<220>
   <223> Metagenom
<220>
   <221> unsure
   <222> 43
<400> 220
   gacgtggtgt tcaaccattt cgggccgacg ggaaattacc tgncggcgtt ctcgccccac   60
   tatgtgtcca aaaagcacgc caccgaatgg ggcgaatcgc tcaacttcga cggcgaatcg  120
   gccggccccg tgcgcgagtt cgtcacgaca aatgcggccc actggattcg cgagtaccat  180
   ctcgacggct tccgcatcga cgcc                                           204
<210> 221
   <211> 235
   <212> DNA
   <213> Unknown
<220>
   <223> Metagenom
<400> 221
   ggtctacgcc gacgtcgtgt tcaaccacac cggcgagggc gacgcgacga aaccgacgtg   60
   gacgttccgc ggtctcgacg atcggctgta cctccacgac cgcgacggta cgtacaacga  120
   cgacgcgggc tgcggcaacg tcgccgaccc tggtgccgag gaggtgcggc ggctcgtgct  180
   cgaggccctc cagcgcttca ctgacctcgg catcgacggg ttccgcatcg acgcc        235
<210> 222
   <211> 243
   <212> DNA
   <213> Unknown
<220>
   <223> Metagenom
<400> 222
   ggtctacgcc gacgtcgtct tcaaccacac ggccgaggga aaccgcatgg gcccgacgct   60
   gtcgtggcgc gggatcgaca atacccacta ctaccggctg accgaggaca agcgctatca  120
   cctggactac acgggcacgg gcaacgccct gaacatgacg agcccgcgcg tgctccagat  180
   gatcatggac agcctgcgct actgggtcat ggagatgcac gtcgacggct tccgcacgac  240
   gcc                                                                243
<210> 223
   <211> 246
   <212> DNA
   <213> Unknown
<220>
   <223> Metagenom
<400> 223
   ggtctacgcc gacgtcgtgt tcaaccacac cggcgagggc aaccacaacg gcccgacgat   60
   cagcttccgg ggccaggcca acgaggccta ctaccacctc gtcgacgagg acaggaagta  120
   ctacatggac tactcgggct gcggcaacac gatcaacgcc aaccacccac tcgtcaccaa  180
   gctgatcatc gagagcctgg agtactgggt caccgagcac cacgtcgacg ggtccgcatc  240
   gacgcc                                                             246
<210> 224
   <211> 240
   <212> DNA
   <213> Unknown
<220>
   <223> Metagenom
<400> 224
   ggtctacgcc gacgtcgtgt tcaaccacac ggccgaaggc aacgaaacag gccccacgtt   60
   ctgcttcaag ggcttggaga acgaagttta ttacatgctg aatgagaacg gcacgtacaa  120
   aaacttctcc ggctgcggca acacggtgaa cggcaaccac cccgtcatgc gcgaaatgat  180
   cttccattgc ctccgtcatt gggtgcacaa ctaccacatc gacggcttcc gcacgacgcc  240
<210> 225
   <211> 247
   <212> DNA
   <213> Unknown
<220>
   <223> Metagenom
<400> 225
   ggtctacgcc gacgtcgtgt tcaaccacac ctgcgaagga aacgagcgcg gcccgacgct   60
   ctgttaccgc gggctcgacg cgccggtctg gtactgggtg gaccctgcgc agccgtcgcg  120
   ccacctcgac ttcaccggct gcggcaacag cttcaacatg cgccaccccc aggtgctgaa  180
   gctggcgatg gactcgctgc gctactgggt caccgagatg catgtcgacg ggttccgcat  240
   cgacgcc                                                            247
<210> 226
   <211> 241
   <212> DNA
   <213> Unknown
<220>
   <223> Metagenom
<400> 226
   ggtctacgcc gacgtcgtgt tcaaccacac ggcagaggag ggtctcggcg gacccaccag   60
   cagcctgcgc ggcatcgaca accggaacta ctaccgccag acccccgacg gtgaatacat    120
   cgacgtcacg ggatgcggca actcggtgaa caccgcgacg gaggcgtcgc gacggctcat   180
   cctcgactcc ctcaggtact gggcgaacga ggtgcaggtg gacggcttcc gcatcgacgc   240
   c                                                                   241
<210> 227
   <211> 247
   <212> DNA
   <213> Unknown
<220>
   <223> Metagenom
<400> 227
   ggtctacgcc gacgtcgtgt tcaaccacac ggcggaaggc aatcacatgg ggccgacgct   60
   gtccttccgc ggcatcgaca acgtttcgta ctaccgcgtc gatcccgcaa acccgcgtct   120
   ctacgtcgac tacaccggct gcggcaacac gctcaacatg caaaatccgc gcgtgctgca   180
   gctcatcatg gattcgctgc gctactgggt gactgagatg cacgtcgacg gcttccgcat   240
   cgacgcc                                                             247
<210> 228
   <211> 247
   <212> DNA
   <213> Unknown
<220>
   <223> Metagenom
<400> 228
   ggtctacgcc gacgtcgtga tcaaccacac cgccgagggc aacgagcggg gccccaccct   60
   ctccttccgc ggcatcgaca acgcctccta ctaccgtctg gtggacgggg actggcagca   120
   ctactacgac accaccggca ccggcaacag cctgctgatg cgccacccct acgtgctcca   180
   gctcatcatg gactcgctgc gctactgggt caccgagatg cacgtcgacg gcttccgcat   240
   cgacgcc                                                             247
<210> 229
   <211> 244
   <212> DNA
   <213> Unknown
<220>
   <223> Metagenom
<400> 229
   ggtctacgcc gacgtcgtgt acaaccacac cgccgagggc aaccagatgg ggccgacgct    60
   gtcgtttcgc ggcatcgaca atgccagcta ttacctgctc ggcgacgacc cgcgctatta   120
   tttcgacacg accggatgcg gcaacgcggt caatctgcgc caccagcgcg tgctgcagat   180
   ggtcatggat tcgctgcgct actgggtgca ggaatgccat gtcgacgggt tccgcatcga   240
   cgcc                                                                244
<210> 230
   <211> 247
   <212> DNA
   <213> Unknown
<220>
   <223> Metagenom
<400> 230
   ggtctacgcc gacgtcgtct tcaaccactc ggccgagggc aaccacatgg gccccacgct    60
   cagcttcaag gggttcgaca accccctcta ctaccggctg gtggccgacg agcaggagca   120
   ctacttcgac tacaccggca ccggcaacac catgaacatg cgcagcccgc acacgctgca   180
   gctggtcatg gacagcctcc ggtggtgggc cacggagatg cacgtcgacg gcttccgcat   240
   cgacgcc                                                             247
<210> 231
   <211> 244
   <212> DNA
   <213> Unknown
<220>
   <223> Metagenom
<400> 231
   ggtctacgcc gacgtcgtct tcaaccacac ctgcgaaggg aacgaggaag gcccgatcct    60
   cagcttcaaa ggactggaga accgcgtcta ctacatgctg gccaacggcg gcagccagta   120
   catgaactac tcaggctgcg gcaacacggt caacggcaat cacccgatcg tccgcgagat   180
   gatcttccat tgtttgcggc actgggtaca caactaccat atcgacgact tccgcatcga   240
   cgcc                                                                244
<210> 232
   <211> 247
   <212> DNA
   <213> Unknown
<220>
   <223> Metagenom
<400> 232
   ggtctacgcc gacgtcgtgt tcaaccacac cgccgagggc aatcacacgg gcccgatgct    60
   gagcttcaag gggatcgaca accaggcgta ctaccgcctg atggaggacg agcccaagta   120
   ctacagggac tacacgggca ccggtagctc cctcaacgtg cgccaccccc actcgctgca   180
   gctgatcatg gactcgctgc gctactgggt gaccgagatg cacgtcgacg gcttccgcat   240
   cgacgcc                                                             247
<210> 233
   <211> 246
   <212> DNA
   <213> Unknown
<220>
   <223> Metagenom
<400> 233
   gtctacgccg acgtcgtgtt caaccatacc gccgaaggca accacctcgg gccgagcttc   60
   aacctcaagg gcatcgacaa cccgacctac tatcgtctgg ttccgaacga gcctcgctat  120
   tacttcgact acacgggcac cggcaacacg ctcaacgtgc ggcaccctca ggtgctggcg  180
   ctcatcatgg actcgctgcg ctactgggcg atcgagatgc acgtcgacgg gttccgcatc  240
   gacgcc                                                             246
<210> 234
   <211> 246
   <212> DNA
   <213> Unknown
<220>
   <223> Metagenom
<400> 234
   gtctacgccg acgtcgtgta caaccacgcg gccgaaggga accacctcgg cccgatgctg   60
   tcgttcaagg gctacgacaa cgaggcgtac taccggctcg gccccgacga cccccgctac  120
   tacgtcgact acaccggctg cggcaacagc ctcaacatgc gcaacccgca cgtgctgcaa  180
   ctgatcatgg acagcctgcg gtactgggtc gaagagatgc acgtcgacgg gttccgcatc  240
   gacgcc                                                             246
<210> 235
   <211> 247
   <212> DNA
   <213> Unknown
<220>
   <223> Metagenom
<400> 235
   ggtctacgcc gacgtcgtct tcaaccacac gagcgagggc aatcacgacg gcccgacgat   60
   cagcttcaag ggattcgaga acagcgtcta ctaccatctc acaccgcggg atcgccagta  120
   ctacatggac tattcgggtt gcggcaacac ggtcaactgc aaccacccgc tcgtggacaa  180
   gttcatcctg gacagcctcg agttctgggt gcacgagatg cacgtcgacg gcttccgcat  240
   cgacgcc                                                            247
<210> 236
   <211> 244
   <212> DNA
   <213> Unknown
<220>
   <223> Metagenom
<400> 236
   ggtctacgcc gacgtcgtgt tcaaccacac cgccgagggc aaccacctag gcccggtgct 60
   cgccttcaag ggcatcgaca acgcgtcgta ctatcggctg gcggacgacg cccgttatta 120
   catggacttc accggctgcg gaaatacgtt gaacatgcgc agccctcagg tgttgcagct 180
   gatcatggac agcctgcgct attggcacct tgacatgcac gtcgacgggt tccgcatcga 240
   cgcc 244
<210> 237
   <211> 247
   <212> DNA
   <213> Unknown
<220>
   <223> Metagenom
<400> 237
   ggtctacgcc gacgtcgtgt tcaaccacac gaccgagggc gacgagcacg gaccgacgct 60
   ttcgtttcgc ggcatcgata accccacgca ttacctgctg aaccccgacg accgcgcgcg 120
   gtacatcgac tacaccggct gcggcaacac tatcagcggc aatcacccca tcgtgcggcg 180
   aatgattctc gacgccctgc gctattgggt cgagcacatg cacgtcgacg gcttccgcat 240
   cgacgcc 247

<210> 238 <211> 247 <212> DNA <213> Unknown <220>
   <223> Metagenom <400> 238
<210> 239 <211> 247 <212> DNA <213> Unknown <220>
   <223> Metagenom <400> 239
<210> 240 <211> 247 <212> DNA <213> Unknown <220>
   <223> Metagenom <400> 240
<210> 241 <211> 241 <212> DNA <213> Unknown <220>
   <223> Metagenom <400> 241
<210> 242
   <211> 240
   <212> DNA
   <213> Unknown
<220>
   <223> Metagenom
<400> 242
   ggcgtcgatg cggaagccgt cgatgtggta gttgtgcacc caatggcgga ggcaatggaa   60
   gatcatctcg cgcatcacgg gatgattgcc gttcactgtg ttgccgcagc cggagaagtt   120
   tttgtacgtg ccgttgtcgt tcagcatgta atagactttg ttctccaggc ccttgaagca   180
   gaaagtggga cctgtctcgt tgccttcggc ggtgtggttg aacacgacgt cggcgtagac   240
<210> 243
   <211> 247
   <212> DNA
   <213> Unknown
<220>
   <223> Metagenom
<400> 243
   ggtctacgcc gacgtcgtct tcaaccacac cgccgagggc aaccatctcg gacccgtgct   60
   gtcgctcaag ggaatcgaca acgcggcgta ctaccggctg gtcgccgacc accgccggta   120
   ctacatggac tacacgggca ccggcaacag cctgaacatg cgccacccgc acgtgctgca   180
   gctcttgatg gattcgctgc ggtactgggt gaccgagatg cacgtggacg ggttccgcat   240
   cgacgcc                                                             247
<210> 244
   <211> 247
   <212> DNA
   <213> Unknown
<220>
   <223> Metagenom <400> 244
<210> 245
   <211> 240
   <212> DNA
   <213> Unknown
<220>
   <223> Metagenom
<400> 245
   ggtctacgcc gacgtcgtgt tcaaccacac gtcggaaggc aacgaaacgg gccccacgtt   60
   cagcttcaaa ggcctggaga accgcgtcta ctacatgctc aacgacgacg gcacctaccg  120
   gaactacagc ggctgcggca acaccgtcaa cggcaatcac ccgatcgtgc gcgagatgat  180
   cttccattgc ctccgccatt gggtgcacaa ctaccacgtc gacggcttcc gcacggcgcc  240
<210> 246
   <211> 246
   <212> DNA
   <213> Unknown
<220>
   <223> Metagenom
<400> 246
   ggtctacgcc gacgtcgtct tcaaccacac cgcggaaggg aatcatctcg gaccgacgtt   60
   gtccctgcgc ggcatcgaca acacctcgtc ctatcggctg cagcctcacg acccgcggtt  120
   ctaccaggac ttcaccggct gcggcaacac gctgaacatg cgcagcccgc gcatgctgca  180
   gctgctgatg gacagcctgc gctactgggt gcaggagatg cacgtggacg gcttccgcat  240
   cgacgc                                                             246
<210> 247
   <211> 211
   <212> DNA
   <213> Unknown
<220>
   <223> Metagenom
<400> 247
   ggtctacgcc gacgtcgtgt tcaaccacct cggccctgcc ggcaactacc tgaaccgctt   60
   cgggccctac ttcaccgatc gctacgccac cccttgggga gcggcggtga acctcgacgg  120
   gccgggtagc gacgaggtgc gggccttctt cgtcgacaac gccctcatgt ggctgcggga  180
   ctaccacatc gacggcttcc gcatcgacgc c                                 211
<210> 248
   <211> 247
   <212> DNA
   <213> Unknown
<220>
   <223> Metagenom
<400> 248
   ggtctacgcc gacgtcgtca tcaaccacac cgccgagggc aaccatctcg gcccgaccct   60
   gtcgctgcgc ggcgtcgata acgccaccta ctaccggttg atgccggacg accggcgcca  120
   ctacctggat ttcaccggat gcgggaacac gctcaacatg cagagcccgc aggtgctgca  180
   gctgatcatg gacagcctgc ggtactgggt gctcgagatg cacgtcgacg ggttccgcat  240
   cgacgcc                                                            247
<210> 249
   <211> 244
   <212> DNA
   <213> Unknown
<220>
   <223> Metagenom
<400> 249
   ggtctacgcc gacgtcgtca tcaaccacac ggccgagggc aaccacctgg gcccgacgct   60
   gtccttcaag ggcctcgaca acccctcgta ctaccggctg gccgacgatc cccgttacta  120
   cacggacacc acggggaccg ggaactccct gctcatgcgg tccccgcacg tactccagat  180
   gatcatggac tcgctgcggt actgggtcac cgagatgcac gtggacgggt tccgcatcga  240
   cgcc                                                                244
<210> 250
   <211> 244
   <212> DNA
   <213> Unknown
<220>
   <223> Metagenom
<400> 250
   ggtctacgcc gacgtcgtca tcaaccactc gcgcgagggg aaccactacg ggccgacggt   60
   cagcttcaag ggtttggata ataatatcta ttacatgctg gagtcggccc cggaattcta  120
   caacgacttc accggctgcg gcaacacgct gaattgcaac catccggtcg tccgcaagtt  180
   catcctcgac tgcctccggt actgggtgac cgagatgcac gtcgacgggt tccgcatcga  240
   cgcc                                                                244
<210> 251 <211> 241 <212> DNA <213> Unknown <220>
   <223> Metagenom <400> 251
<210> 252 <211> 247 <212> DNA <213> Unknown <220>
   <223> Metagenom <400> 252
<210> 253 <211> 247 <212> DNA <213> Unknown <220>
   <223> Metagenom <400> 253
<210> 254 <211> 244 <212> DNA <213> Unknown <220>
   <223> Metagenom <220>
   <221> unsure <222> 65-67
<400> 254
   ggtctacgcc gacgtcgtct tcaaccacac ctgcgaaggg aacgagcagg gaccgatcct    60
   cagcnnnaaa gggctggaaa accgcgtcta ttacatgatg gctaacggcg gcagccatta   120
   catgaactac tcaggctgcg gcaacacggt caacggcaat cacccgatcg tccgcgagat   180
   gatcttccat tgcttgcggc actgggtgca taactaccac atcgacggct tccgcatcga   240
   cgcc                                                                244
<210> 255
   <211> 247
   <212> DNA
   <213> Unknown
<220>
   <223> Metagenom
<400> 255
   ggtctacgcc gacgtcgtga acaaccacac ctgcgaaggc aaccagatgg ggccgacgct    60
   ttcctggcgc ggcatcgaca acgccagcta ttaccgcctg accgacgacg ataaacgcta   120
   ctacatggat tacaccggct gcggcaatac gctcaacatg acccacccgc gcgtgctcca   180
   gctcatcatg gacagcctgc ggtattgggt gacagagatg cacgttgacg ggttccgcat   240
   cgacgcc                                                             247
<210> 256
   <211> 246
   <212> DNA
   <213> Unknown
<220>
   <223> Metagenom
<400> 256
   ggtctacgcc gacgtcgtga tcaaccacac gggcgagggc aaccaccagg gcccgacgat    60
   cagcttcaag ggtctcgaga acgaggcgta ctacatgctc tcgccgggcg acggggcgta   120
   ctacatgaac tactccggct gcgggaacac ggtcaacgcg aaccaccccg tcgtcgagaa   180
   gttcatcgcc gactgcctgg gctactgggt cgaggagatg cacgtcgacg ggttccgcat   240
   cgcgcc                                                              246
<210> 257
   <211> 247
   <212> DNA
   <213> Unknown
<220>
   <223> Metagenom
<220>
   <221> unsure
   <222> 163, 199, 214, 215
<400> 257
   ggtctacgcc gacgtcgtca tcaaccacac ctcggaaggg aatcacctcg ggccgacgtt   60
   gtcgctgcgc gggatcgaca acgagaccta ttaccgcctg acgccgagga caagcgctac  120
   tacctcgatt tcaccggctg cgggaacacg ctcaaacatg canagcccgc aggtgctgca  180
   gctgatcatg gacagcatnc ggtactgggt gctnnacatg cacgtcgacg gcttccgcat  240
   cgacgcc                                                            247
<210> 258
   <211> 247
   <212> DNA
   <213> Unknown
<220>
   <223> Metagenom
<220>
   <221> unsure
   <222> 80, 214, 215
<400> 258
   ggtctacgcc gacgtcgtct tcaaccacac gggcgagggg aaccaccagg gcccgacgat   60
   cagcttcaag ggtctcgagn gacgaggcgt actacatgct ctcgccgggc gacggggcgt  120
   actacatgaa ctactccggc tgcgggaaca cggtcaacgc gaaccacccc gtcgtcgaga  180
   agttcatcgc cgactgcctg ggctactggg tcgnnagatg cacgtcgacg gcttccgcat  240
   cgacgcc                                                            247
<210> 259
   <211> 247
   <212> DNA
   <213> Unknown
<220>
   <223> Metagenom
<400> 259
   ggtctacgcc gacgtcgtgt tcaaccacac cgccgagggc aaccagctcg gcccgacgct   60
   gtcgttccgc gggatcgaca acgccaacta ctaccgcctc gtggacgagg acaagaagca  120
   ctactacgac accacgggca cgggcaacac gctgctcatg aagagccccc acgtgctgca  180
   gctgatcatg gactcgctgc gctactgggt gaccgagatg cacgtagacg ggttccgcat  240
   cgacgcc                                                            247
<210> 260
   <211> 322
   <212> DNA
   <213> Unknown
<220>
   <223> Metagenom
<220>
   <221> unsure
   <222> 47
<400> 260
   ggtctacgcc gacgtcgtga tcaaccacac cagcgaccag cacccgnggt tccaggagtc   60
   gcgccaggac cgcaccaacc ccaaggccga ctggtacgtg tgggacgacg acggcaccaa  120
   gtggtccgag gcccgcgtga tcttcgtcga caccgagccc tcgaactgga cgttcgaccc  180
   gcagcgcgag cagttctact ggcaccgctt cttctcgcat cagcccgacc tcaacttcga  240
   caaccccgag gtcgaggagg cgatgttcga cgtcatgcgg ttctggctcg acctcggcct  300
   cgacggcttc cgcatcgacg cc                                           322
<210> 261
   <211> 247
   <212> DNA
   <213> Unknown
<220>
   <223> Metagenom
<220>
   <221> unsure
   <222> 108-110
<400> 261
   ggtctacgcc gacgtcgtgt tcaaccacac cggcgagggc aatcacctcg ggctgacgtt   60
   gtccttcaag ggtctagaca acccgaccta ctaccagctg aagcgcgnnn agcagcgcta  120
   ctacgtcgac tacaccggca ccggcaacag tctgaacgtg caccaccctc aggtgttgca  180
   gctcgtgctc gacagcttgc gctactgggt gaaggtgatg catgtggacg ggttccgcat  240
   cgacgcc                                                            247
<210> 262
   <211> 322
   <212> DNA
   <213> Unknown
<220>
   <223> Metagenom
<400> 262
   ggtctacgcc gacgtcgtct tcaaccacag ctccgaccgg cacccatggt tcgtggagag   60
   ccgctcgtca cgcgcgaacg ccaaacgcga ctggtatatc tggcgcgacc cggctcccgg  120
   gggcgggccg cccaacaatt ggatgagcga tttcggcggg ccggcgtgga cgctcgatcc  180
   cgcgaccggg caatattatc tccacgcctt cctgccgcaa cagcccgacc tcaactggcg  240
   caatccggac gtgcgcgcgg cgatgatggg cgtgctgcga ttctggctcg accgcggagt  300
   cgacgggttc cgcatcgacg cc                                           322
<210> 263
   <211> 214
   <212> DNA
   <213> Unknown
<220>
   <223> Metagenom
<400> 263
   ggtctacgcc gacgtcgtgt tcaaccacct cggcccggcc gggaactacc tgccccggtt   60
   ctcgcggtcg tacctcagcc atcgctacgc caacgaatgg ggcgacgcac tcaactacga  120
   cgacacggga tgcgaggggc tgcgcgaact ggtgctgtcg aacatcgcct actgggtgcg  180
   cgagtttcac ctcgacgggt tccgcatcga cgcc                              214
<210> 264
   <211> 247
   <212> DNA
   <213> Unknown
<220>
   <223> Metagenom
<400> 264
   ggtctacgcc gacgtcgtgt tcaaccacac cggcgagagc gatgcgctcg gcccgaccct   60
   gtcgctgcgc gggctcgaca accgggccta ttaccgccac cttcccggcg aagccggaag  120
   cctggtcaat gatacgggaa ccggcaacac ggtcgcctgc aactacccgg tcgtccgggg  180
   actcgttctc gatacgctgc gccattttgt gctgaacgcc ggcgtcgacg gcttccgcat  240
   cgacgcc                                                            247
<210> 265
   <211> 246
   <212> DNA
   <213> Unknown
<220>
   <223> Metagenom
<400> 265
   ggtctacgcc gacgtcgtct tcaaccacac gggcgaagga aaccacctcg gccccaccct   60
   cgcgttcaag ggcatcgaca acaaggcgta ctaccggttg gagccggaca acccgcactt  120
   gtacaccgat ttcacgggca ccggaaacag cctgaacatg cagcatccgc gcacgatcca  180
   gctcatcatg gactcgctgc gctactgggt accgagatgc acgtagacgg cttccgcatc  240
   gacgcc                                                             246
<210> 266
   <211> 247
   <212> DNA
   <213> Unknown
<220>
   <223> Metagenom
<400> 266
   ggtctacgcc gacgtcgtgt tcaaccacac gaccgagggg aaccatcagg ggccgacgat   60
   caacttcaga ggcttcgaca acagcgtcta ctacttcacg gttcccgacg acaagcagta  120
   ctacatggac tacagcgggt gcgggaacac gctgaactgc aaccacccgg tcgtcgagaa  180
   gatgatcctg gattgtctcg agttctgggt gcgggacatg cacatcgacg gcttccgcat  240
   cgacgcc                                                            247
<210> 267
   <211> 247
   <212> DNA
   <213> Unknown
<220>
   <223> Metagenom
<400> 267
   ggtctacgcc gacgtcgtgt acatccatac cgccgaaggc aaccacctcg ggcccacgct   60
   gtcgttccgc ggtatcgaca acgcgaccta ctactggctg aaaccggacc agccgcgtta  120
   ttacgaggat ttcaccggat gcggcaattc gctgaacctg acgcatcctc atgtgttgca  180
   gcttgtcatg gattcgctgc gctactgggt ggagcagtgc catgtcgacg gcttccgcat  240
   cgacgcc                                                            247
<210> 268
   <211> 211
   <212> DNA
   <213> Unknown
<220>
   <223> Metagenom
<400> 268
   ggtctacgcc gacgtcgtct tcaaccatct cggacccgcc ggcaactacc tgcgcgaatt   60
   cggtccctat ttcaccgaca ggtacgggac gccgtggggc gatgccgtga atctcgacga  120
   cagagggtct accgaagtac gcaactactt tttcgacaac gctcttggat ggttggagca  180
   ctaccacctc gacggcttcc gcatcgacgc c                                 211
<210> 269
   <211> 247
   <212> DNA
   <213> Unknown
<220>
   <223> Metagenom
<400> 269
   ggtctacgcc gacgtcgtgt tcaaccacac ggcggagggg aaccacctcg gcccgatgct   60
   ctcgttcaag ggatcgacaa acctctccta ctaccggacg ctgcccggcg acccgcgctt  120
   ctacatggac tacaccggca ccggcaacag cctcaacgtc cgccaccccc actcggtgca  180
   gctgatcatg gactcgttgc gctactgggt caccgagatg cacgtcgacg gcttccgcat  240
   cgacgcc                                                            247
<210> 270
   <211> 211
   <212> DNA
   <213> Unknown
<220>
   <223> Metagenom
<400> 270
   ggtctacgcc gacgtcgtgt tcaaccattt cggtccggac ggctgctgga tcgagaagtt    60
   ttcgccacac ttcttttcgg aacgagccac cgaatggggg cgcgccatca acttcgatgg   120
   cgaacacgcc gctgaggtgc gcgcgttctt cgtcgcgaac gcgtcctatt ggatcgagga   180
   atttcatctc gacgggttcc gcatcgacgc c                                  211
<210> 271
   <211> 247
   <212> DNA
   <213> Unknown
<220>
   <223> Metagenom
<400> 271
   ggtctacgcc gacgtcgtga tcaaccacac gggcgaaggc agccataccg gccccacgct    60
   gtgctttcgg ggcatcgaca acgcgagcta ttaccgcctg aacgcacaga acccgcgcct   120
   ctacgtcgat tacaccggct gcggcaatac gctcaacatg cagaacccgc gggtgctcca   180
   gctcatcatg gattcgctgc gctactgggt caccgaaatg cacgtcgacg ggttccgcat   240
   cgacgcc                                                             247
<210> 272
   <211> 244
   <212> DNA
   <213> Unknown
<220>
   <223> Metagenom
<400> 272
   ggtctacgcc gacgtcgtct tcaaccacac cggcgaggga aaccatctgg gtccgacggt    60
   ttcgctcaag ggcatcgaca acacggcgta ttaccggctc gaggaagaca agcggttcta   120
   tacggatttc accggcaccg gtaactcgct caacatgcgc cacccgcgca cgatccagtt   180
   gatcatggac tcgctgcgct actgggtgac ggagatgcac gtcgacggct tccgcatcga   240
   cgcc                                                                244
<210> 273
   <211> 247
   <212> DNA
   <213> Unknown
<220>
   <223> Metagenom
<400> 273
   ggtctacgcc gacgtcgtct tcaaccacac ctccgagggg caccacctgg ggccgacgct   60
   gtcgtacaag gggatcgaca atccgaccta ctatcggctt ctgcccgagg acccgcgctt  120
   ctaccgcgac tacaccggca cggggaactc gctgaacatg cggcacccgc aaacgctgaa  180
   gctggtgatg gattcgcttc gctactgggt gctcgagatg cacgtggacg ggttccgcat  240
   cgacgcc                                                            247
<210> 274
   <211> 241
   <212> DNA
   <213> Unknown
<220>
   <223> Metagenom
<400> 274
   ggtctacgcc gacgtcgtct tcaaccacac ggccgaaggc aacgaaacag gccccacgtt   60
   ctgcttcaag ggcttggaga acaaagttca ttacatgctg aatgaaaacg gcacgtacaa  120
   aaacttctcc ggctgcggca acacggtgaa cggcaaccac cccgtcatgc gcgaaatgat  180
   cttccattgc ctccgtcatt gggtgcacaa ctaccacatc gacgggttcc gcatcgacgc  240
   c                                                                  241
<210> 275
   <211> 202
   <212> DNA
   <213> Unknown
<220>
   <223> Metagenom
<400> 275
   ggtctacgcc gacgtcgtca tcaaccactt cggtcctgcc ggtaactcgc actacgcgtt   60
   cgcaccggag atcaaggccc cgacgatgac cgagtggggc gacgccctcg actacagccg  120
   ccccggtgca cgcgagctgt tcatcacgaa tgcggcgtac tggatccgcg agttccactt  180
   cgacggcttc cgcatcgacg cc                                           202
<210> 276
   <211> 247
   <212> DNA
   <213> Unknown
<220>
   <223> Metagenom
<400> 276
   ggtctacgcc gacgtcgtga acaaccacac ggcggagggg aaccacctgg gcccgatgct   60
   ctccatgaag gggatcgaca acgccggcta ttaccggctg gtcgacgacg acaaacgctt  120
   ctactacgac accacaggaa ccggcaacag cctggacatg cgccaccccc acgtactgca  180
   gctgatcatg gacagcctgc gttactgggt cacggagatg catgtcgacg ggttccgcat  240
   cgacgcc                                                            247
<210> 277
   <211> 241
   <212> DNA
   <213> Unknown
<220>
   <223> Metagenom
<400> 277
   ggtctacgcc gacgtcgtct tcaaccacac cgcggaaggg gaccagcgag gtccgactct   60
   cagctggcgc ggcctcgaca acgccagcag ctacgccctg gatggcagcg ggcaatatct  120
   gaacttcacc ggctgcggca atgcgatcaa tgccggcgag ccgcgcatgg tgcagttcgt  180
   gatggacagc ctgcgctggt gggtgcaggc attcggcgtc gacgggttcc gcatcgacgc  240
   c                                                                  241
<210> 278
   <211> 247
   <212> DNA
   <213> Unknown
<220>
   <223> Metagenom
<400> 278
   ggtctacgcc gacgtcgtgt tcaaccatac cgccgagggc aatcatctcg ggccgacgct   60
   gtccttccgc ggcatcgaca acgccgtcta ctacaagttg ccggccaacg accgccgcca  120
   ctattgggac agcaccggga cgggcaacac gctcgacgtg aaccatccgc aggtgttgaa  180
   gatggtgctc gattcactcc gccactgggt cgaggactac catgtcgacg gcttccgcat  240
   cgacgcc                                                            247
<210> 279
   <211> 247
   <212> DNA
   <213> Unknown
<220>
   <223> Metagenom
<400> 279
   ggtctacgcc gacgtcgtct tcaaccacac cgccgaggga aaccatctcg ggccaacact   60
   gtcgttccgg ggcatcgaca atgccgccta ttaccgcctc gtcgactccg accgccgcca  120
   ctacatggac tacaccggta ccggcaacac cctgaacatg cgcaacccgt tcccgctgca  180
   actgctgatg gacagcctgc gctactgggt caccgaatgc cacgtcgacg gcttccgcat  240
   cgacgcc                                                            247
<210> 280
   <211> 247
   <212> DNA
   <213> Unknown
<220>
   <223> Metagenom
<400> 280
   ggtctacgcc gacgtcgtct tcaaccatac cgccgaaggc aatcagctcg gccccacgct   60
   gagctttcgc ggcatcgaca acacctccta ttactggctc ctgcccgacg agccgcgcta  120
   ctacgacgac ttcaccggct gcggcaacgc gctcaacctg acacatccgc gcgtgctgca  180
   gatggtgatg gattcgctcc gttactgggt cgagatgtgc ctggtcgacg ggttccgcat  240
   cgacgcc                                                             247
<210> 281
   <211> 248
   <212> DNA
   <213> Unknown
<220>
   <223> Metagenom
<400> 281
   gggtctacgc cgacgtcgtg ttcaaccaca cgggcgaagg cagccatacc ggcccgacgc   60
   tgtgctttcg aggcatcgac aacgcgagtt attaccgcct gaacccgcag aacccgcgcc  120
   tctatgtcga ttacaccggc tgcggcaaca cgctcaacat gcagaacccg cgggtgctcc  180
   agctcatcat ggattcgctg cgctactggg tcaccgaaat gcacgtcgac ggcttccgca  240
   tcgacgcc                                                            248
<210> 282
   <211> 242
   <212> DNA
   <213> Unknown
<220>
   <223> Metagenom
<400> 282
   cggtctacgc cgacgtcgtg ttcaaccaca cggccgaagg caacgaaaca ggccccacgt   60
   tctgcttcaa gggcttggag aacaaagttt attacatgct gaatgaaaac ggcacgtaca  120
   aaaacttctc cggctgcggc aacacggtga acggcaacca ccccgtcatg cgcgaaatga  180
   tcttccattg cctccgtcat tgggtgcaca actaccacat cgacggcttc cgcatcgacg  240
   cc                                                                  242
<210> 283
   <211> 241
   <212> DNA
   <213> Unknown
<220>
   <223> Metagenom
<400> 283
   ggtctacgcc gacgtcgtgt tcaaccacac cgccgagggc ccgcagggcg gtcccgcact   60
   ctgctggcgc ggactgagcg aaatgaagta ctaccgccat cacgacgacg gccggtacct  120
   ggacaccacc ggttgtggaa acacggtcaa cttcactgag ccgcgcgtaa tccagttcgc  180
   gctggattcc ctgcggtact gggtcgacga gttcggtatc gacggcttcc gcatcgacgc  240
   c                                                                   241
<210> 284
   <211> 214
   <212> DNA
   <213> Unknown
<220>
   <223> Metagenom
<400> 284
   ggtctacgcc gacgtcgtgt tcaaccactt cggtcccgac ggcaactacc tgcgcgccta   60
   ttcggacgac tacttcaccg accgctacgc caccccctgg ggcgaggcga tcgactacga   120
   tggacccggt agtcgctggg tgcgccagtt catccgcgag aacgcccgct actggctcga   180
   cgagtaccgg ctcgacgggt tccgcatcga cgcc                               214
<210> 285
   <211> 247
   <212> DNA
   <213> Unknown
<220>
   <223> Metagenom
<400> 285
   ggtctacgcc gacgtcgtgt acaaccacac cgcggaaggc aatcagatgg ggccgacgct   60
   gtcgctgcac ggcgtcgaca acgtcaccta ctaccgcctc gtgccgcacg acaggcgcta   120
   ctaccaggac ttcaccggct gcggcaacac cctcaacatg cagagcccgc aggtgctgca   180
   gctgatcatg gacagcctgc gttactgggt gctcgagatg cacgtcgacg ggttccgcat   240
   cgacgcc                                                             247
<210> 286
   <211> 247
   <212> DNA
   <213> Unknown
<220>
   <223> Metagenom
<400> 286
   ggtctacgcc gacgtcgtgt tcaaccacac gtgcgaaggg aacgagaaag gcccgacgct   60
   ctcgtggcgc gggatcgaca acgtcgcgta ttaccgtctg tcgaaggaag atccgcgcta   120
   ctacacggat ttcaccggct gcggcaacac gctgaacatg acgcacccgc gcgtgctcca   180
   gctcatcatg gactccctgc gctactgggt gacggagatg cacatcgacg ggttccgcat   240
   cgacgcc                                                             247
<210> 287
   <211> 235
   <212> DNA
   <213> Unknown
<220>
   <223> Metagenom
<400> 287
   ggtctacgcc gacgtcgtga tcaaccacac ggccgccagc ggccaggccg ggaccagcgt    60
   cctcgaccgg atcgtgcccg gctactacca gcggctgctc gccgacggct cggtggccaa   120
   cagcacctgc tgctcgaaca ccgccaccga gaacgccatg atgggcaagc tcgtcgtcga   180
   ctcggtcgtc acctgggcca aggagtacaa ggtcgacggc ttccgcatcg acgcc        235
<210> 288
   <211> 215
   <212> DNA
   <213> Unknown
<220>
   <223> Metagenom
<400> 288
   ggtctacgcc gacgtcgtct acaaccacct cggtcctgac ggctgctacc tcacgaagtt    60
   cgcagactga gtacttcacc gaccggtaca agaacgaatg gggcgaggcc gtgaacttcg   120
   acggcgagag ctctgcgccg gtgcgggagt tttttgcgga gaacgccgcg tactggatcg   180
   acgagtttca cctggacggg ttccgcatcg acgcc                              215
<210> 289
   <211> 247
   <212> DNA
   <213> Unknown
<220>
   <223> Metagenom
<400> 289
   ggtctacgcc gacgtcgtgt tcaaccacac cgccgaaggc aatcatctcg ggcccatgct    60
   gtcgttcaag gggatcgaca acgcggcgta ctaccgcgtc attggggaca accgtcgcta   120
   ctacatggac tacaccggca cgggcaacac gctgaacatg cgccatcctc acgtgctgca   180
   gctgatgatg gacagccttc gctacttcgt gctcgacatg cacgtggacg ggttccgcat   240
   cgacgcc                                                             247
<210> 290
   <211> 247
   <212> DNA
   <213> Unknown
<220>
   <223> Metagenom
<400> 290
   ggtctacgcc gacgtcgtgt tcaaccacac gtcggagggg aacgccgagt ccggtcccgc    60
   cttctgcctc aaggggttcg acaacagcat ctactacatc ctcgacaagg acaagtcgcg   120
   gtacgccgac tacaccgggt gcgggaacac gctcaactcc aaccatccga tcgtgcgccg   180
   catgatcgtg gacagcctgc gctactgggt gcaggagatg cacgtcgacg gcttccgcat   240
   cgacgcc                                                             247
<210> 291
   <211> 241
   <212> DNA
   <213> Unknown
<220>
   <223> Metagenom
<400> 291
   ggtctacgcc gacgtcgtgt tcaaccacac cgccgaggga aacgaacgcg ggccgacgat   60
   ctcgttccgc ggcctcgaca acaagacgta ctacatgctg acgccggagg ggtattactt  120
   caacttcagc ggcaccggca acacgctgaa ctgcaacaat cccatcgtgc gcgacatggt  180
   gctcgactgc ctgcgctact gggcggcgga gtatcacgtg gacggcttcc gcatcgacgc  240
   c                                                                  241
<210> 292
   <211> 247
   <212> DNA
   <213> Unknown
<220>
   <223> Metagenom
<400> 292
   ggtctacgcc gacgtcgtga tcaaccacac gaccgagggg aaccatcagg ggccgatcgt   60
   caacttccgc ggcttcgaca acagcgtcta ctaccacctg gtccaggacg acaagcagta  120
   ctacatggac tacagcggtt gcggcaacac ggtgaactgc aaccacccgg tcgtcgagaa  180
   gatgatcctg gactgcctcg agttctgggt gcgggacatg cacgtcgacg gcttccgcat  240
   cgacgcc                                                            247
<210> 293
   <211> 247
   <212> DNA
   <213> Unknown
<220>
   <223> Metagenom
<400> 293
   ggtctacgcc gacgtcgtgt tcaaccacac gaccgagggg aaccatcagg ggccgattgt   60
   caacttccgc ggcttcgaca acagcgtcta ctaccacctg gtccaggacg acaagcagta  120
   ctacatggac tacagcggtt gcggcaacac ggtgaactgc aaccacccgg tcgtcgagaa  180
   gatgatcctg gactgcctcg agttctgggt gcgcgacatg cacgtcgacg gcttccgcat  240
   cgacgcc                                                            247
<210> 294
   <211> 241
   <212> DNA
   <213> Unknown
<220>
   <223> Metagenom
<400> 294
   ggtctacgcc gacgtcgtgt tcaaccacac gggcgaaaac atggaggtag cggatcgcgg   60
   cttcaccttc agcgggatcg accggtggta ctactaccgg atgaaccagg agggggagct  120
   gatcggaccc tacggcaacg agatccgcag cgaggatcgg ccgatggttc agcgttggct  180
   gatcgaccag ctgcgacatc tggtggacat tttcggagtc gacggcttcc gcgtcgacgc  240
   c                                                                  241
<210> 295
   <211> 247
   <212> DNA
   <213> Unknown
<220>
   <223> Metagenom
<400> 295
   ggtctacgcc gacgtcgtgt acaaccacac gtgcgagggc aatcatctcg ggccgacgct   60
   gtcgctgcgg gggatcgaca acgtctcgta ttaccgcttg gccgagaacg agcgcaggca  120
   ctatgccgac ttcagcggct gcggcaacac gctgaacctc gagcacccga atgtgctgca  180
   gctggtgacg gattcgctgc gctactgggt cgaggtgatg cacgtggacg ggttccgcat  240
   cgacgcc                                                            247
<210> 296
   <211> 247
   <212> DNA
   <213> Unknown
<220>
   <223> Metagenom
<400> 296
   ggtctacgcc gacgtcgtgt acaaccacac gtgcgagggc aatcatctcg ggccgacgct   60
   gtcgctgcgg gggatcgaca acgtctcgta ttaccgcttg gccgagaacg agcgcaggca  120
   ctatgccgac ttcagcggct gcggcaacac gctgaacctc gagcacccga atgtgctgca  180
   gctggtgacg gattcgctgc gctactgggt cgaggtgatg cacgtggacg ggttccgcat  240
   cgacgcc                                                            247
<210> 297
   <211> 241
   <212> DNA
   <213> Unknown
<220>
   <223> Metagenom
<400> 297
   ggtctacgcc gacgtcgtct tcaaccacac ggccgaaggc aacgaacgcg gcccgcacat   60
   ctcgttccgc ggcctcgaca acaagaccta ctacatgctc acgccggagg gctactactt  120
   caacttctcc ggcacgggca acacgctcaa ctgcaacaac ccgatcgtgc gcagcatggt  180
   gctcgattgc ctgcgctact gggcgtcgga gtatcacgtc gacggcttcc gcatcgacgc  240
   c                                                                  241
<210> 298
   <211> 247
   <212> DNA
   <213> Unknown
<220>
   <223> Metagenom
<400> 298
   ggtctacgcc gacgtcgtca tcaaccacac cgcggagggc aaccacctcg gcccgacgct   60
   gtcgttccgg ggcatcgaca acccggcgta ctaccggctg atggaggacg acgcccgctt  120
   ctacatggac tacacgggca cgggcaacac cctcaacgtg cgtcagccgc actcgctgca  180
   gctgatcatg gactctctgc gctactgggt caccgagatg cacgtggacg gcttccgcat  240
   cgacgcc                                                            247
<210> 299
   <211> 247
   <212> DNA
   <213> Unknown
<220>
   <223> Metagenom
<400> 299
   ggtctacgcc gacgtcgtga tcaaccacac cgccgaaggc aacgaattcg gtccgacgct   60
   gtcgttccgc ggcctcgata acgccagtta ttaccgactg ctgcccgaca atccgcgcca  120
   ctacatcaac gacaccggca ccggcaacac ggtgaacctg tcccacccgc gcgtgctgca  180
   gacggtgatg gattcgctgc gctactgggt cgaggaatgc catgtcgacg gcttccgcat  240
   cgacgcc                                                            247
<210> 300
   <211> 247
   <212> DNA
   <213> Unknown
<220>
   <223> Metagenom
<400> 300
   ggtctacgcc gacgtcgtgt acaaccacac cgccgagggc gatcagcgcg gcccgacgct   60
   gtcgctgcgc ggcttcgaca acgccaacta ctaccgcctg tcgccgcacg accggtcgct  120
   gtacgagaac ttctccggga cgggcaacac cgtgagcttc gatcatccgg cggtgcgctc  180
   gctcgtgatc gagtgtctgc gctactgggt gagcgacatg ggcttggacg ggttccgcat  240
   cgacgcc                                                            247
<210> 301
   <211> 247
   <212> DNA
   <213> Unknown
<220>
   <223> Metagenom
<400> 301
   ggtctacgcc gacgtcgtgt acaaccacac cggtgagggc aacgagcggg gaccgacgct 60
   gtctttgcgt ggcatcgaca acaagtcgta ctaccggctc aaccctgaga acggtcggca 120
   ctacgtcgac ttcaccggaa ccggcaacac gctcaacatg atgcagccgc gctcgctgca 180
   actcgtcacg gacagcctcc gctactgggt gcaagagatg cacgtcgacg gcttccgcat 240
   cgacgcc 247
<210> 302
   <211> 247
   <212> DNA
   <213> Unknown
<220>
   <223> Metagenom
<400> 302
   ggtctacgcc gacgtcgtgt tcaaccacac ggtcgagggg aatcacatgg ggccggtgct 60
   ggcgatgaaa ggcctcgaca acaccgccta ctaccgcacg atgccgggcc aggcgcggta 120
   ctacatggac tacaccggca ccggaaacag cctgcacatg cgccatccgc acgtgctgca 180
   gatgatcatg gacagcctgc gctactggat cacggagatg cacgtcgacg gcttccgcat 240
   cgacgcc 247
<210> 303
   <211> 247
   <212> DNA
   <213> Unknown
<220>
   <223> Metagenom
<400> 303
   ggtctacgcc gacgtcgtgt tcaaccacac cgccgagggc aatcacctcg ggccgacgct 60
   gtcgctgcgc ggcatcgaca acagctccta ctatcgcctg ctgccgaaca acccgcgcta 120
   ctaccaggac ttcaccggca cgggcaacac gctcaacatg cgcagcccgc gcgtgctgca 180
   gctcatcatg gacagcctgc gctactgggt cctcgagatg cacgtggacg gcttccgcat 240
   cgacgcc 247
<210> 304
   <211> 244
   <212> DNA
   <213> Unknown
<220>
   <223> Metagenom
<400> 304
   ggtctacgcc gacgtcgtgt tcaaccacac gtgcgaaggg aacgagcacg gcccgacgct 60
   cagcttcaag ggcctcgaga accgcgtgta ctacatgctc gccggccagg gcgagcacta 120
   caagaactac tcgggctgcg gcaacacggt caacggaaac catcccatcg tgcgggagat 180
   gatcttccat tgcctccgcc actgggtgca caactaccac gtcgacgggt tccgcatcga 240
   cgcc 244

<210> 305
   <211> 123
   <212> PRT
   <213> Artificial Sequence
<220>
   <223> Konsensus A
<220>
   <221> VARIANT
   <222> 8
   <223> X = A oder T
<220>
   <221> VARIANT
   <222> 9
   <223> X = G oder I oder V oder T oder L oder A oder S
<220>
   <221> VARIANT
   <222> 10
   <223> X = K oder E oder R
<220>
   <221> VARIANT
   <222> 11
   <223> X = H oder A oder R
<220>
   <221> VARIANT
   <222> 12
   <223> X = I oder M oder V oder L
<220>
   <221> VARIANT
   <222> 13
   <223> X = H oder D oder R oder T oder P oder S oder A oder Y oder N oder K
<220>
   <221> VARIANT
   <222> 14
   <223> X = P oder T oder N oder S oder A oder R oder H oder Q
<220>
   <221> VARIANT
   <222> 15
   <223> X = P oder R oder T oder S oder G oder A oder D oder E oder F
<220>
   <221> VARIANT
   <222> 16
   <223> X = F oder Y oder D
<220>
   <221> VARIANT
   <222> 17
   <223> X = L oder V oder F oder Y oder I oder W oder T
<220>
   <221> VARIANT
   <222> 18
   <223> X = S oder N oder A oder K oder R oder E oder C odert Q oder H oder L
<220>
   <221> VARIANT
   <222> 19
   <223> X = R oder A oder H oder E oder D oder W oder N oder G oder K
<220>
   <221> VARIANT
   <222> 20
   <223> X = W oder I oder V oder F oder L
<220>
   <221> VARIANT
   <222> 21
   <223> X = F oder L oder M oder K oder R oder V oder N
<220>
   <221> VARIANT
   <222> 22
   <223> X = Q oder D oder G oder Y oder C oder T oder N oder S oder A oder R
<220>
   <221> VARIANT
   <222> 23
   <223> X = E oder A oder K oder H oder L oder R oder G oder D
<220>
   <221> VARIANT
   <222> 24
   <223> X = V oder L oder M oder A oder I
   Position Aminosäure
<220>
   <221> VARIANT
   <222> 25
   <223> X = K oder R oder T oder S oder G oder N oder H oder A
<220>
   <221> VARIANT
   <222> 26
   <223> X = G oder A oder Q oder E oder S oder T oder V oder N oder D oder K
   oder R oder Y
<220>
   <221> VARIANT
   <222> 27
   <223> X = T oder P oder E oder A oder K oder N oder H oder S oder G oder R
<220>
   <221> VARIANT
   <222> 28
   <223> X = S oder N oder T oder F oder Y oder G oder D oder V oder H oder A oder K oder -
<220>
   <221> VARIANT
   <222> 29
   <223> X = P oder G oder K oder S oder R oder I oder -
<220>
   <221> VARIANT
   <222> 30
   <223> X = G oder K oder D oder A oder S oder H oder E oder -
<220>
   <221> VARIANT
   <222> 31
   <223> X = R oder P oder E oder A oder L oder D oder -
<220>
   <221> VARIANT
   <222> 32
   <223> X = G oder -
<220>
   <221> VARIANT
   <222> 33
   <223> X = R oder -
<220>
   <221> VARIANT
   <222> 34
   <223> X = P oder K oder -
<220>
   <221> VARIANT
   <222> 35
   <223> X = E oder N oder A oder R oder D oder P oder -
<220>
   <221> VARIANT
   <222> 36
   <223> X = R oder L oder A oder P oder V oder F oder -
<220>
   <221> VARIANT
   <222> 37
   <223> X = F oder Y oder I
<220>
   <221> VARIANT
   <222> 38
   <223> X = A oder G oder L oder I oder W oder V oder N oder Q oder M oder T
<220>
   <221> VARIANT
   <222> 39
   <223> X = V oder H oder K oder F oder T oder I
<220>
   <221> VARIANT
   <222> 40
   <223> X = T oder S oder G oder A oder Q oder H oder L oder M
<220>
   <221> VARIANT
   <222> 41
   <223> E
<220>
   <221> VARIANT
   <222> 42
   <223> X = F oder A oder Y oder H oder V oder T oder I
<220>
   <221> VARIANT
   <222> 43
   <223> X = Y oder W oder I oder F oder V
<220>
   <221> VARIANT
   <222> 44
   <223> X = D oder F oder S oder E oder Q oder Y oder H oder G oder P oder N
<220>
   <221> VARIANT
   <222> 45
   <223> X = G oder A oder D oder R oder S oder T oder E oder -
<220>
   <221> VARIANT
   <222> 46
   <223> X = N oder K oder D oder S oder H oder A oder T oder -
<220>
   <221> VARIANT
   <222> 47
   <223> X = P oder L oder V oder I oder M oder A oder N oder G oder D
<220>
   <221> VARIANT
   <222> 48
   <223> X = A oder D oder Q oder N oder E oder T
<220>
   <221> VARIANT
   <222> 49
   <223> X = H oder Q oder L oder A oder E oder D oder V oder P oder N
<220>
   <221> VARIANT
   <222> 50
   <223> X = L oder I oder V oder P oder T
<220>
   <221> VARIANT
   <222> 51
   <223> X = A oder T oder H oder N oder K oder D oder Q oder R oder S oder G
<220>
   <221> VARIANT
   <222> 52
   <223> X = T oder A oder D oder N oder G oder K oder S oder R oder P oder Q oder V
<220>
   <221> VARIANT
   <222> 53
   <223> X = V oder Y oder G oder S oder T oder D oder A oder Q oder N oder L oder F
<220>
   <221> VARIANT
   <222> 54
   <223> X = I oder A oder L oder V oder Q oder W oder E oder D oder S oder T oder R
<220>
   <221> VARIANT
   <222> 55
   <223> X = D oder K oder T oder N oder A oder S oder Y oder E oder W oder G
<220>
   <221> VARIANT
   <222> 56
   <223> X = L oder E oder A oder S oder D oder K oder T oder I oder Y oder F oder R
<220>
   <221> VARIANT
   <222> 57
   <223> X = Y oder L oder S oder V oder T oder G oder E oder P
<220>
   <221> VARIANT
   <222> 58
   <223> X = A oder G oder N oder D oder E oder I oder S oder T oder V oder L oder M
<220>
   <221> VARIANT
   <222> 59
   <223> X = R oder D oder S oder G oder N oder A
<220>
   <221> VARIANT
   <222> 60
   <223> X = T oder N oder -
<220>
   <221> VARIANT
   <222> 61
   <223> X = A oder -
<220>
   <221> VARIANT
   <222> 62
   <223> X = S oder M oder -
<220>
   <221> VARIANT
   <222> 63
   <223> X = D oder P oder A oder - Position Aminosäure
<220>
   <221> VARIANT
   <222> 64
   <223> X = Q oder R oder A oder K oder V oder G oder T oder M oder -
<220>
   <221> VARIANT
   <222> 65
   <223> X = S oder T oder M oder V oder L oder D oder W oder -
<220>
   <221> VARIANT
   <222> 66
   <223> X = H oder R oder S oder K oder Q oder D oder T
<220>
   <221> VARIANT
   <222> 67
   <223> X = L oder A oder V oder T oder D
<220>
   <221> VARIANT
   <222> 68
   <223> X = F oder H oder D oder Q oder T oder L oder Y
<220>
   <221> VARIANT
   <222> 69
   <223> X = D oder E oder F
<220>
   <221> VARIANT
   <222> 70
   <223> X = F oder V oder G
<220>
   <221> VARIANT
   <222> 71
   <223> X = A oder P oder V oder R oder G oder T oder H
<220>
   <221> VARIANT
   <222> 72
   <223> X = L oder Y oder F oder V
<220>
   <221> VARIANT
   <222> 73
   <223> X = H oder G oder A oder S oder F oder K
<220>
   <221> VARIANT
   <222> 74
   <223> X = F oder Y oder A oder R oder K oder S oder D oder N oder E
<220>
   <221> VARIANT
   <222> 75
   <223> X = L oder K oder R oder N oder G oder D oder S oder H oder Q oder A
<220>
   <221> VARIANT
   <222> 76
   <223> X = L oder F oder I oder V oder A
<220>
   <221> VARIANT
   <222> 77
   <223> X = Q oder Y oder E oder M oder H oder R oder L oder F oder K oder A oder S oder G oder I oder V
<220>
   <221> VARIANT
   <222> 78
   <223> X = R oder G oder Q oder E oder D oder N oder A oder H oder S oder T
<220>
   <221> VARIANT
   <222> 79
   <223> X = M oder A oder Q oder V oder R oder F oder E
<220>
   <221> VARIANT
   <222> 80
   <223> X = S oder A oder F oder L oder V
<220>
   <221> VARIANT
   <222> 81
   <223> X = A oder N oder R oder K oder Q oder S oder L oder H oder T oder G
<220>
   <221> VARIANT
   <222> 82
   <223> X = G oder N oder A oder R oder K oder Q oder S oder Y oder F
<220>
   <221> VARIANT
   <222> 83
   <223> X = N oder G oder E oder Q oder D oder K oder -
<220>
   <221> VARIANT
   <222> 84
   <223> X = R oder -
<220>
   <221> VARIANT
   <222> 85
   <223> X = G oder R oder K oder E oder N oder Q oder D oder P oder A
<220>
   <221> VARIANT
   <222> 86
   <223> X = G oder A oder M oder D oder E oder S oder I oder L oder P
<220>
   <221> VARIANT
   <222> 87
   <223> X = F oder S oder W oder Y oder K oder A oder H
<220>
   <221> VARIANT
   <222> 88
   <223> X = D oder W oder Y oder N oder E oder P
<220>
   <221> VARIANT
   <222> 89
   <223> X = I oder H oder -
<220>
   <221> VARIANT
   <222> 90
   <223> X = L oder D oder -
<220>
   <221> VARIANT
   <222> 91
   <223> X = M oder L oder I
<220>
   <221> VARIANT
   <222> 92
   <223> X = R oder G oder S oder P oder T oder Q oder K oder A oder N
<220>
   <221> VARIANT
   <222> 93
   <223> X = S oder N oder K oder T oder R oder A
<220>
   <221> VARIANT
   <222> 94
   <223> X = L oder I oder V oder F oder Y
<220>
   <221> VARIANT
   <222> 95
   <223> X = R oder K oder L oder F oder G oder A oder Y
<220>
   <221> VARIANT
   <222> 96
   <223> X = F oder Q oder T oder D oder A oder E oder S oder P oder R oder L
<220>
   <221> VARIANT
   <222> 97
   <223> X = G oder A oder R oder N oder S oder T oder -
<220>
   <221> VARIANT
   <222> 98
   <223> X = S oder G oder T oder W oder D oder L oder -
<220>
   <221> VARIANT
   <222> 99
   <223> X = S oder G oder D oder -
<220>
   <221> VARIANT
   <222> 100
   <223> X = D oder L oder P oder -
<220>
   <221> VARIANT
   <222> 101
   <223> X = D oder Y oder K oder -
<220>
   <221> VARIANT
   <222> 102
   <223> X = G oder T oder -
<220>
   <221> VARIANT
   <222> 103
   <223> X = S oder N oder -
<220>
   <221> VARIANT
   <222> 104
   <223> X = R oder -
<220>
   <221> VARIANT
   <222> 105
   <223> X = F oder L oder D oder -
<220>
   <221> VARIANT
   <222> 106
   <223> X = L oder T oder M oder V oder G oder H oder P
<220>
   <221> VARIANT
   <222> 107
   <223> X = E oder K oder A oder Q oder R oder G oder S oder Y oder F oder H oder L oder D
<220>
   <221> VARIANT
   <222> 108
   <223> X = Q oder A oder E oder W oder D oder R oder G oder V oder M oder L oder I oder T oder S
<220>
   <221> VARIANT
   <222> 109
   <223> X = H oder N oder Q oder R oder P oder G oder S oder E oder T
<220>
   <221> VARIANT
   <222> 110
   <223> X = P oder G oder S oder N oder E oder A oder I
<220>
   <221> VARIANT
   <222> 111
   <223> X = A oder K oder L oder Q oder T oder D oder S oder G oder H oder Y
<220>
   <221> VARIANT
   <222> 112
   <223> X = F oder L oder R oder M oder S oder V oder K oder Q oder G oder N oder A oder E
<220>
   <221> VARIANT
   <222> 113
   <223> X = A oder S oder T oder L
<220>
   <221> VARIANT <222> 114 <223> X = V oder A oder M oder G oder S oder I oder N oder L
<220>
   <221> VARIANT
   <222> 115
   <223> X = T oder V oder A oder S
<220>
   <221> VARIANT
   <222> 116
   <223> X = Y oder F
<400> 305
   Val Asp Gly Phe Arg Ile Asp Xaa Xaa Xaa Xaa Xaa Xaa Xaa Xaa Xaa
   1               5                   10                  15
   Xaa Xaa Xaa Xaa Xaa Xaa Xaa Xaa Xaa Xaa Xaa Xaa Xaa Xaa Xaa Xaa
               20                  25                  30
   Xaa Xaa Xaa Xaa Xaa Xaa Xaa Xaa Glu Xaa Xaa Xaa Xaa Xaa Xaa Xaa
           35                  40                  45
   Xaa Xaa Xaa Xaa Xaa Xaa Xaa Xaa Xaa Xaa Xaa Xaa Xaa Xaa Xaa Xaa
       50                  55                  60
   Xaa Xaa Xaa Xaa Xaa Xaa Xaa Xaa Xaa Xaa Xaa Xaa Xaa Xaa Xaa Xaa
   65                  70                  75                  80
   Xaa Xaa Xaa Xaa Xaa Xaa Xaa Xaa Xaa Xaa Xaa Xaa Xaa Xaa Xaa Xaa
                   85                  90                  95
   Xaa Xaa Xaa Xaa Xaa Xaa Xaa Xaa Xaa Xaa Xaa Xaa Xaa Xaa Xaa Xaa
               100                 105                 110
   Xaa Xaa Xaa Xaa Val Asp Asn His Asp Thr Glu
           115                 120
<210> 306
   <211> 121
   <212> PRT
   <213> Artificial Sequence
<220>
   <223> Konsensus D
<220>
   <221> VARIANT
   <222> 8
   <223> X = A oder T
<220>
   <221> VARIANT
   <222> 9
   <223> X = G oder I oder V oder T oder L oder A oder S
<220>
   <221> VARIANT
   <222> 10
   <223> X = K oder E oder R
<220>
   <221> VARIANT
   <222> 11
   <223> X = H oder R
<220>
   <221> VARIANT
   <222> 12
   <223> X = I oder M oder V oder L
<220>
   <221> VARIANT
   <222> 13
   <223> X = H oder D oder R oder T oder P oder S oder A oder N
<220>
   <221> VARIANT
   <222> 14
   <223> X = P oder T oder N oder S oder A oder R
<220>
   <221> VARIANT
   <222> 15
   <223> X = P oder R oder T oder S oder G oder A oder E oder D
<220>
   <221> VARIANT
   <222> 16
   <223> X = F oder Y oder D
<220>
   <221> VARIANT
   <222> 17
   <223> X = L oder V oder F oder Y oder I oder W
<220>
   <221> VARIANT
   <222> 18
   <223> X = S oder N oder A oder K oder R oder E oder C oder H
<220>
   <221> VARIANT
   <222> 19
   <223> X = R oder A oder H oder E oder D oder N
<220>
   <221> VARIANT
   <222> 20
   <223> X = W oder V oder I oder F
<220>
   <221> VARIANT
   <222> 21
   <223> X = F oder L oder M oder V oder K oder R oder N
<220>
   <221> VARIANT
   <222> 22
   <223> X = Q oder D oder G oder Y oder C oder N oder T oder S oder A
<220>
   <221> VARIANT
   <222> 23
   <223> X = E oder A oder K oder H oder G oder R oder L
<220>
   <221> VARIANT
   <222> 24
   <223> X = V oder L oder M oder I
   Position Aminosäure
<220>
   <221> VARIANT
   <222> 25
   <223> X = K oder R oder H oder T oder S oder G oder N
<220>
   <221> VARIANT
   <222> 26
   <223> X = G oder A oder Q oder E oder T oder S oder V oder N oder D oder K oder R
<220>
   <221> VARIANT
   <222> 27
   <223> X = T oder P oder E oder A oder K oder H oder S oder G oder R
<220>
   <221> VARIANT
   <222> 28
   <223> X = S oder N oder T oder F oder V oder G oder D oder A oder -
<220>
   <221> VARIANT
   <222> 29
   <223> X = P oder G oder K oder S oder R oder -
<220>
   <221> VARIANT
   <222> 30
   <223> X = G oder K oder D oder A oder H oder -
<220>
   <221> VARIANT
   <222> 31
   <223> X = R oder P oder E oder A oder L oder -
<220>
   <221> VARIANT
   <222> 32
   <223> X = G oder -
<220>
   <221> VARIANT
   <222> 33
   <223> X = R oder -
<220>
   <221> VARIANT
   <222> 34
   <223> X = P oder -
<220>
   <221> VARIANT
   <222> 35
   <223> X = E oder N oder A oder R oder D oder -
<220>
   <221> VARIANT
   <222> 36
   <223> X = R oder L oder A oder P oder V oder F
<220>
   <221> VARIANT
   <222> 37
   <223> X = F oder Y oder I
<220>
   <221> VARIANT
   <222> 38
   <223> X = A oder G oder I oder V oder L oder W oder Q
<220>
   <221> VARIANT
   <222> 39
   <223> X = V oder T oder F oder H oder K
<220>
   <221> VARIANT
   <222> 40
   <223> X = T oder S oder G oder A oder Q oder H
<220>
   <221> VARIANT
   <222> 41
   <223> E
<220>
   <221> VARIANT
   <222> 42
   <223> X = F oder A oder Y oder H oder I oder V oder T
<220>
   <221> VARIANT
   <222> 43
   <223> X = Y oder W oder F oder I
<220>
   <221> VARIANT
   <222> 44
   <223> X = D oder F oder S oder E oder P oder Q oder Y oder H oder N
<220>
   <221> VARIANT
   <222> 45
   <223> X = G oder A oder D oder R oder S
<220>
   <221> VARIANT
   <222> 46
   <223> X = N oder K oder D oder S oder H oder A oder T oder -
<220>
   <221> VARIANT
   <222> 47
   <223> X = P oder L oder V oder I oder A oder N oder G oder D oder -
<220>
   <221> VARIANT
   <222> 48
   <223> X = A oder D oder Q oder N oder T oder E
<220>
   <221> VARIANT
   <222> 49
   <223> X = H oder Q oder L oder A oder E oder D oder P oder V
<220>
   <221> VARIANT
   <222> 50
   <223> X = L oder P oder I oder V
<220>
   <221> VARIANT
   <222> 51
   <223> X = A oder T oder H oder N oder K oder D oder R oder Q oder S oder G
<220>
   <221> VARIANT
   <222> 52
   <223> X = T oder A oder D oder N oder G oder K oder S oder R oder Q oder P oder V
<220>
   <221> VARIANT
   <222> 53
   <223> X = V oder Y oder D oder Q oder G oder S oder T oder A oder N oder L
<220>
   <221> VARIANT
   <222> 54
   <223> X = I oder A oder L oder V oder E oder D oder Q oder W oder S
<220>
   <221> VARIANT
   <222> 55
   <223> X = D oder K oder T oder N oder A oder S oder Y oder E
<220>
   <221> VARIANT
   <222> 56
   <223> X = L oder E oder A oder S oder D oder K oder Y oder F oder T oder I
<220>
   <221> VARIANT
   <222> 57
   <223> X = Y oder L oder S oder V oder T oder G
<220>
   <221> VARIANT
   <222> 58
   <223> X = A oder G oder N oder D oder E oder T oder I oder S oder V
<220>
   <221> VARIANT
   <222> 59
   <223> X = R oder D oder S oder G oder N
<220>
   <221> VARIANT
   <222> 60
   <223> X = T oder -
<220>
   <221> VARIANT
   <222> 61
   <223> X = A oder -
<220>
   <221> VARIANT
   <222> 62
   <223> X = S oder M oder -
<220>
   <221> VARIANT
   <222> 63
   <223> X = D oder P oder -
   Position Aminosäure
<220>
   <221> VARIANT
   <222> 64
   <223> X = Q oder R oder A oder K oder V oder G oder T oder M oder -
<220>
   <221> VARIANT
   <222> 65
   <223> X = S oder T oder M oder V oder L oder D oder -
<220>
   <221> VARIANT
   <222> 66
   <223> X = H oder R oder S oder K oder Q oder D oder T
<220>
   <221> VARIANT
   <222> 67
   <223> X = L oder A oder V oder T
<220>
   <221> VARIANT
   <222> 68
   <223> X = F oder L oder H oder D oder Q
<220>
   <221> VARIANT
   <222> 69
   <223> X = D oder E
<220>
   <221> VARIANT
   <222> 70
   <223> X = F oder V
<220>
   <221> VARIANT
   <222> 71
   <223> X = A oder P oder T oder V oder R oder G
<220>
   <221> VARIANT
   <222> 72
   <223> X = L oder F oder Y
<220>
   <221> VARIANT
   <222> 73
   <223> X = H oder A oder G oder F
<220>
   <221> VARIANT
   <222> 74
   <223> X = F oder Y oder A oder D oder R oder K oder N
<220>
   <221> VARIANT
   <222> 75
   <223> X = L oder K oder R oder N oder Q oder D oder G oder S oder A
<220>
   <221> VARIANT
   <222> 76
   <223> X = L oder F oder V oder I oder A
<220>
   <221> VARIANT
   <222> 77
   <223> X = Q oder Y oder E oder M oder H oder R oder L oder F oder K oder A oder S
<220>
   <221> VARIANT
   <222> 78
   <223> X = R oder G oder Q oder E oder N oder D oder A oder S oder T oder H
<220>
   <221> VARIANT
   <222> 79
   <223> X = M oder A oder Q oder V oder F
<220>
   <221> VARIANT
   <222> 80
   <223> X = S oder A oder F oder L oder V
<220>
   <221> VARIANT
   <222> 81
   <223> X = A oder N oder R oder K oder Q oder S oder L oder H oder T
<220>
   <221> VARIANT
   <222> 82
   <223> X = G oder N oder A oder R oder K oder Q oder S oder H
<220>
   <221> VARIANT
   <222> 83
   <223> X = N oder G oder E oder Q oder -
<220>
   <221> VARIANT
   <222> 84
   <223> X = R oder -
<220>
   <221> VARIANT
   <222> 85
   <223> X = G oder R oder K oder E oder N oder D oder Q oder P
<220>
   <221> VARIANT
   <222> 86
   <223> X = G oder A oder M oder D oder S oder I oder L oder P
<220>
   <221> VARIANT
   <222> 87
   <223> X = F oder S oder W oder Y oder A oder K
<220>
   <221> VARIANT
   <222> 88
   <223> X = D oder N oder W oder Y oder E
<220>
   <221> VARIANT
   <222> 89
   <223> X = M oder L
<220>
   <221> VARIANT
   <222> 90
   <223> X = R oder G oder S oder T oder Q oder K oder A
<220>
   <221> VARIANT
   <222> 91
   <223> X = S oder N oder K oder T oder A
<220>
   <221> VARIANT
   <222> 92
   <223> X = L oder I oder V oder F oder Y
<220>
   <221> VARIANT
   <222> 93
   <223> X = R oder K oder L oder F oder G
<220>
   <221> VARIANT
   <222> 94
   <223> X = F oder Q oder T oder D oder A oder E oder S oder P oder R
<220>
   <221> VARIANT
   <222> 95
   <223> X = G oder A oder R oder N oder S oder T
<220>
   <221> VARIANT
   <222> 96
   <223> X = S oder G oder T oder W oder D
<220>
   <221> VARIANT
   <222> 97
   <223> X = S oder G oder D oder -
<220>
   <221> VARIANT
   <222> 98
   <223> X = D oder L oder Y oder F oder n oder -
<220>
   <221> VARIANT
   <222> 99
   <223> X = D oder S oder Y oder -
<220>
   <221> VARIANT
   <222> 100
   <223> X = G oder T oder -
<220>
   <221> VARIANT
   <222> 101
   <223> X = S oder -
<220>
   <221> VARIANT
   <222> 102
   <223> X = R oder -
<220>
   <221> VARIANT
   <222> 103
   <223> X = F oder L oder D oder -
<220>
   <221> VARIANT
   <222> 104
   <223> X = L oder T oder M oder V oder H oder -
<220>
   <221> VARIANT
   <222> 105
   <223> X = E oder K oder A oder Q oder R oder G oder D oder -
<220>
   <221> VARIANT
   <222> 106
   <223> X = Q oder A oder E oder W oder D oder R oder G oder T oder L oder V oder M oder I oder S
<220>
   <221> VARIANT
   <222> 107
   <223> X = H oder N oder Q oder R oder T oder P oder G oder S oder K
<220>
   <221> VARIANT
   <222> 108
   <223> X = P oder G oder E oder A oder S oder N oder I
<220>
   <221> VARIANT
   <222> 109
   <223> X = A oder K oder L oder Q oder T oder D oder S oder G oder H
<220>
   <221> VARIANT
   <222> 110
   <223> X = F oder L oder R oder M oder S oder V oder K oder N oder D oder Q oder G oder A
<220>
   <221> VARIANT
   <222> 111
   <223> X = A oder S oder T
<220>
   <221> VARIANT
   <222> 112
   <223> X = V oder A oder N oder M oder G oder S oder L
<220>
   <221> VARIANT
   <222> 113
   <223> X = T oder S oder V oder A
<220>
   <221> VARIANT
   <222> 114
   <223> X = Y oder F
<400> 306
   Val Asp Gly Phe Arg Ile Asp Xaa Xaa Xaa Xaa Xaa Xaa Xaa Xaa Xaa
   1               5                   10                  15
   Xaa Xaa Xaa Xaa Xaa Xaa Xaa Xaa Xaa Xaa Xaa Xaa Xaa Xaa Xaa Xaa
               20                  25                  30
   Xaa Xaa Xaa Xaa Xaa Xaa Xaa Xaa Glu Xaa Xaa Xaa Xaa Xaa Xaa Xaa
           35                  40                  45
   Xaa Xaa Xaa Xaa Xaa Xaa Xaa Xaa Xaa Xaa Xaa Xaa Xaa Xaa Xaa Xaa
       50                  55                  60
   Xaa Xaa Xaa Xaa Xaa Xaa Xaa Xaa Xaa Xaa Xaa Xaa Xaa Xaa Xaa Xaa
   65                  70                  75                  80
   Xaa Xaa Xaa Xaa Xaa Xaa Xaa Xaa Xaa Xaa Xaa Xaa Xaa Xaa Xaa Xaa
                   85                  90                  95
   Xaa Xaa Xaa Xaa Xaa Xaa Xaa Xaa Xaa Xaa Xaa Xaa Xaa Xaa Xaa Xaa
               100                 105                 110
   Xaa Xaa Val Asp Asn His Asp Thr Glu
           115                 120
<210> 307
   <211> 109
   <212> PRT
   <213> Artificial Sequence
<220>
   <223> Konsensus B
<220>
   <221> VARIANT
   <222> 1
   <223> X = V oder -
<220>
   <221> VARIANT
   <222> 2
   <223> X = Y oder -
<220>
   <221> VARIANT
   <222> 5
   <223> X = V oder A
<220>
   <221> VARIANT
   <222> 7
   <223> X = F oder Y oder I oder N oder L
<220>
   <221> VARIANT
   <222> 8
   <223> X = N oder -
<220>
   <221> VARIANT
   <222> 9
   <223> X = H oder -
<220>
   <221> VARIANT
   <222> 10
   <223> X = L oder F oder T oder S oder A oder -
<220>
   <221> VARIANT
   <222> 11
   <223> X = G oder S oder A oder C oder R oder T oder V oder -
<220>
   <221> VARIANT
   <222> 12
   <223> X = P oder D oder E oder A oder F oder -
<220>
   <221> VARIANT
   <222> 13
   <223> X = A oder E oder D oder S oder T oder Q oder R oder L oder N oder G oder -
<220>
   <221> VARIANT
   <222> 14
   <223> X = G oder S oder H oder N oder P oder D oder M oder F
<220>
   <221> VARIANT
   <222> 15
   <223> X = P oder A oder E oder -
<220>
   <221> VARIANT
   <222> 16
   <223> X = W oder H oder A oder Q oder E oder R oder K oder L oder V oder P oder -
<220>
   <221> VARIANT
   <222> 17
   <223> X = F oder Q oder L oder T oder R oder G oder S oder E oder H oder Y oder N oder M oder K oder D oder A oder -
<220>
   <221> VARIANT
   <222> 18
   <223> X = N oder A oder C oder Q oder V oder G oder K oder D
<220>
   <221> VARIANT
   <222> 19
   <223> X = Y oder C oder F oder L oder S oder W oder E oder P oder R oder D oder -
<220>
   <221> VARIANT
   <222> 20
   <223> X = L oder A oder F oder H oder I oder S oder T oder M oder G oder V
<220>
   <221> VARIANT
   <222> 21
   <223> X = N oder R oder H oder P oder G oder D oder A oder T oder Y oder E oder I oder W oder L oder F oder V oder S
<220>
   <221> VARIANT
   <222> 22
   <223> X = R oder E oder A oder Q oder K oder S oder T oder C oder G oder N oder V
<220>
   <221> VARIANT
   <222> 23
   <223> X = F oder Y oder D oder S oder V oder W oder L oder M oder R
<220>
   <221> VARIANT
   <222> 24
   <223> X = G oder A oder S oder T oder R oder K oder Q oder H oder D Position Aminosäure
<220>
   <221> VARIANT
   <222> 25
   <223> X = C oder P oder D oder G oder R oder T oder A oder -
<220>
   <221> VARIANT
   <222> 26
   <223> X = P oder E oder D oder H oder S oder N oder L oder F oder Q oder I oder V oder Y oder A
<220>
   <221> VARIANT
   <222> 27
   <223> X = Y oder F oder E oder H oder P oder A oder R oder D oder S oder V
<220>
   <221> VARIANT
   <222> 28
   <223> X = F oder V oder L oder I oder K oder Q oder D oder N oder E oder A oder P oder R
<220>
   <221> VARIANT
   <222> 29
   <223> X = T oder S oder A oder K oder F oder R oder Q oder M oder E oder N oder P oder T oder V oder G oder W oder Y oder L
<220>
   <221> VARIANT
   <222> 30
   <223> X = D oder S oder K oder A oder H oder G oder L oder V oder T oder I oder Y oder W oder N
<220>
   <221> VARIANT
   <222> 31
   <223> X = R oder K oder Q oder P oder E oder W oder V oder Y oder S oder H
<220>
   <221> VARIANT
   <222> 32
   <223> X = Y oder H oder Q oder T oder R oder L oder W
<220>
   <221> VARIANT
   <222> 33
   <223> X = A oder V oder G oder K oder D oder E oder S oder M oder I oder P oder R oder F oder L oder Q oder -
<220>
   <221> VARIANT
   <222> 34
   <223> X = T oder N oder W oder A oder V oder D oder L oder H oder M oder Q oder F oder -
<220>
   <221> VARIANT
   <222> 35
   <223> X = P oder D oder E oder R oder L oder G oder H oder N oder A oder T oder V oder S oder N oder Q oder M oder I
<220>
   <221> VARIANT
   <222> 36
   <223> X = W oder D oder A oder G oder E oder N oder P oder S oder Q oder K oder H oder R oder -
<220>
   <221> VARIANT
   <222> 37
   <223> X = G oder D oder P oder S oder N oder Q oder E oder A oder R oder H
<220>
   <221> VARIANT
   <222> 38
   <223> X = A oder D oder L oder E oder Q oder R oder P oder G oder T oder H oder N oder -
<220>
   <221> VARIANT
   <222> 39
   <223> X = T oder P oder R oder Q oder S oder E oder Y oder K oder G oder A oder W oder D oder -
<220>
   <221> VARIANT
   <222> 40
   <223> X = K oder G oder A oder Q oder H oder F oder S oder R oder E oder V oder -
<220>
   <221> VARIANT
   <222> 41
   <223> X = W oder G oder V oder L oder D oder -
<220>
   <221> VARIANT
   <222> 42
   <223> X = S oder G oder L oder V oder -
<220>
   <221> VARIANT
   <222> 43
   <223> X = E oder P oder H oder -
<220>
   <221> VARIANT
   <222> 44
   <223> X = A oder P oder -
<220>
   <221> VARIANT
   <222> 45
   <223> X = R oder N oder -
<220>
   <221> VARIANT
   <222> 46
   <223> X = V oder N oder -
<220>
   <221> VARIANT
   <222> 47
   <223> X = I oder W oder E oder R oder T oder Y oder F oder H oder L oder S oder -
<220>
   <221> VARIANT
   <222> 48
   <223> X = A oder S oder G oder F oder M oder L oder Y oder T oder H oder V oder -
<220>
   <221> VARIANT
   <222> 49
   <223> X = V oder I oder L oder S oder H oder N oder K oder R oder M oder F oder A oder Q oder T oder D oder E oder W oder Y oder -
<220>
   <221> VARIANT
   <222> 50
   <223> X = N oder D oder K oder R oder G oder S oder L
<220>
   <221> VARIANT
   <222> 51
   <223> X = L oder F oder Y oder T oder H oder D oder I oder S oder W oder V
<220>
   <221> VARIANT
   <222> 52
   <223> X = D oder A oder R oder S oder E oder G oder T
<220>
   <221> VARIANT
   <222> 53
   <223> X = G oder D oder H oder R oder P oder E oder K oder C oder T
<220>
   <221> VARIANT
   <222> 54
   <223> X = P oder R oder K oder L oder E oder T oder S oder H oder Q oder C oder A oder Y oder F
<220>
   <221> VARIANT
   <222> 55
   <223> X = G oder D oder R oder E oder H oder S oder N oder A oder P oder -
<220>
   <221> VARIANT
   <222> 56
   <223> X = S oder G oder C oder A oder L oder H oder W oder Q oder E oder N oder T oder -
<220>
   <221> VARIANT
   <222> 57
   <223> X = R oder D oder S oder G oder A oder Q oder C oder T oder R oder H oder V oder E oder -
<220>
   <221> VARIANT
   <222> 58
   <223> X = F oder L oder E oder A oder I oder V oder R oder M oder -
<220>
   <221> VARIANT
   <222> 59
   <223> X = D oder S oder R oder -
<220>
   <221> VARIANT
   <222> 60
   <223> X = P oder -
<220>
   <221> VARIANT
   <222> 61
   <223> X = Q oder A oder -
<220>
   <221> VARIANT
   <222> 62
   <223> X = R oder T oder -
<220>
   <221> VARIANT
   <222> 63
   <223> X = E oder G oder -
<220>
   <221> VARIANT
   <222> 64
   <223> X = Q oder -
<220>
   <221> VARIANT
   <222> 65
   <223> X = F oder Y oder -
<220>
   <221> VARIANT
   <222> 66
   <223> X = Y oder -
<220>
   <221> VARIANT
   <222> 67
   <223> X = W oder L oder - Position Aminosäure
<220>
   <221> VARIANT
   <222> 68
   <223> X = H oder -
<220>
   <221> VARIANT
   <222> 69
   <223> X = R oder A oder -
<220>
   <221> VARIANT
   <222> 70
   <223> X = F oder -
<220>
   <221> VARIANT
   <222> 71
   <223> X = F oder L oder -
<220>
   <221> VARIANT
   <222> 72
   <223> X = S oder P oder -
<220>
   <221> VARIANT
   <222> 73
   <223> X = H oder Q oder -
<220>
   <221> VARIANT
   <222> 74
   <223> X = Q oder M oder -
<220>
   <221> VARIANT
   <222> 75
   <223> X = P oder -
<220>
   <221> VARIANT <222> 76
   <223> X = D oder K oder -
<220>
   <221> VARIANT
   <222> 77
   <223> X = L oder -
<220>
   <221> VARIANT
   <222> 78
   <223> X = N oder P oder A oder D oder S oder L oder H oder -
<220>
   <221> VARIANT
   <222> 79
   <223> X = F oder W oder P oder S oder A oder G oder C oder M oder L oder V oder T oder E oder -
<220>
   <221> VARIANT
   <222> 80
   <223> X = D oder R oder A oder G oder T oder N oder Q oder S oder E oder M oder K oder -
<220>
   <221> VARIANT
   <222> 81
   <223> X = N oder R oder H oder A oder E oder C oder S oder Q oder T oder Y oder -
<220>
   <221> VARIANT
   <222> 82
   <223> X = D oder T oder R oder E oder A oder P oder Q oder -
<220>
   <221> VARIANT
   <222> 83
   <223> X = E oder T oder I oder D oder W oder G oder P oder H oder R oder V oder L oder Q oder N oder Y oder F oder A oder M
<220>
   <221> VARIANT
   <222> 84
   <223> X = V oder L oder A oder M oder S oder T oder P
<220>
   <221> VARIANT
   <222> 85
   <223> X = R oder G oder E oder H oder V oder I oder D oder T oder L oder Q
<220>
   <221> VARIANT
   <222> 86
   <223> X = A oder N oder E oder R oder W oder Q oder D oder V oder H oder S oder K
<220>
   <221> VARIANT
   <222> 87
   <223> X = F oder Y oder W oder L oder A oder R oder G oder M oder Q
<220>
   <221> VARIANT
   <222> 88
   <223> X = F oder I oder V oder L oder M oder A
<220>
   <221> VARIANT
   <222> 89
   <223> X = V oder F oder I oder R oder L oder T oder M oder P oder A
<220>
   <221> VARIANT
   <222> 90
   <223> X = D oder H oder A oder E oder T oder S oder R oder G
<220>
   <221> VARIANT
   <222> 91
   <223> X = N oder D oder T oder V oder L oder A oder S oder C oder Q
<220>
   <221> VARIANT
   <222> 92
   <223> X = A oder V oder I oder M oder L
<220>
   <221> VARIANT
   <222> 93
   <223> X = L oder R oder V oder A oder E oder S oder G oder Q oder K
<220>
   <221> VARIANT
   <222> 94
   <223> X = M oder G oder Y oder H oder F oder R oder W oder T oder -
<220>
   <221> VARIANT
   <222> 95
   <223> X = W oder F oder L oder Y oder -
<220>
   <221> VARIANT
   <222> 96
   <223> X = L oder V oder I oder A oder H oder -
<220>
   <221> VARIANT
   <222> 97
   <223> X = R oder E oder D oder A oder G oder H oder L oder T oder Q oder S oder K oder I oder M
<220>
   <221> VARIANT
   <222> 98
   <223> X = D oder H oder E oder A oder N oder M oder Q oder V oder S oder I
<220>
   <221> VARIANT
   <222> 99
   <223> X = Y oder F oder L oder R oder M oder C oder A oder V
<220>
   <221> VARIANT
   <222> 100
   <223> X = H oder Q oder R oder G oder L oder K oder D
<220>
   <221> VARIANT
   <222> 101
   <223> X = I oder L oder F oder V
<220>
   <221> VARIANT
   <222> 109
   <223> X = A oder -
<400> 307
   Xaa Xaa Ala Asp Xaa Val Xaa Xaa Xaa Xaa Xaa Xaa Xaa Xaa Xaa Xaa
   1               5                   10                  15
   Xaa Xaa Xaa Xaa Xaa Xaa Xaa Xaa Xaa Xaa Xaa Xaa Xaa Xaa Xaa Xaa
               20                  25                  30
   Xaa Xaa Xaa Xaa Xaa Xaa Xaa Xaa Xaa Xaa Xaa Xaa Xaa Xaa Xaa Xaa
           35                  40                  45
   Xaa Xaa Xaa Xaa Xaa Xaa Xaa Xaa Xaa Xaa Xaa Xaa Xaa Xaa Xaa Xaa
       50                  55                  60
   Xaa Xaa Xaa Xaa Xaa Xaa Xaa Xaa Xaa Xaa Xaa Xaa Xaa Xaa Xaa Xaa
   65                  70                  75                  80
   Xaa Xaa Xaa Xaa Xaa Xaa Xaa Xaa Xaa Xaa Xaa Xaa Xaa Xaa Xaa Xaa
                   85                  90                  95
   Xaa Xaa Xaa Xaa Xaa Asp Gly Phe Arg Ile Asp Ala Xaa
               100                 105
<210> 308
   <211> 78
   <212> PRT
   <213> Artificial Sequence
<220>
   <223> Konsensus C
<220>
   <221> VARIANT
   <222> 1
   <223> X = V oder -
<220>
   <221> VARIANT
   <222> 2
   <223> X = Y oder -
<220>
   <221> VARIANT
   <222> 7
   <223> X = F oder I
<220>
   <221> VARIANT
   <222> 10
   <223> X = F oder L oder T
<220>
   <221> VARIANT
   <222> 11
   <223> X = G oder A
<220>
   <221> VARIANT
   <222> 12
   <223> X = P oder A
<220>
   <221> VARIANT
   <222> 13
   <223> X = T oder D oder A oder E oder S
<220>
   <221> VARIANT
   <222> 14
   <223> X = G oder S
<220>
   <221> VARIANT
   <222> 15
   <223> X = Q oder -
<220>
   <221> VARIANT
   <222> 16
   <223> X = A oder -
<220>
   <221> VARIANT
   <222> 17
   <223> X = G oder -
<220>
   <221> VARIANT
   <222> 18
   <223> X = T oder -
<220>
   <221> VARIANT
   <222> 19
   <223> X = N oder C oder S
<220>
   <221> VARIANT
   <222> 20
   <223> X = Y oder W oder F oder V
<220>
   <221> VARIANT
   <222> 21
   <223> X = L oder I oder A
<220>
   <221> VARIANT
   <222> 22
   <223> X = E oder A oder R oder H oder G oder D
<220>
   <221> VARIANT
   <222> 23
   <223> X = A oder K oder Q oder E oder R
<220>
   <221> VARIANT
   <222> 24
   <223> X = F oder Y oder I Position Aminosäure
<220>
   <221> VARIANT
   <222> 25
   <223> X = S oder G oder T oder A oder V
<220>
   <221> VARIANT
   <222> 26
   <223> X = P oder R oder G oder C oder -
<220>
   <221> VARIANT
   <222> 27
   <223> X = H oder P oder S oder E oder D oder G
<220>
   <221> VARIANT
   <222> 28
   <223> X = Y oder F
<220>
   <221> VARIANT
   <222> 29
   <223> X = V oder F oder Y
<220>
   <221> VARIANT
   <222> 30
   <223> X = S oder A oder T oder Q
<220>
   <221> VARIANT
   <222> 31
   <223> X = K oder E oder A oder D oder R
<220>
   <221> VARIANT
   <222> 32
   <223> X = K oder R oder Q oder L
<220>
   <221> VARIANT
   <222> 33
   <223> X = H oder Y oder L oder -
<220>
   <221> VARIANT
   <222> 34
   <223> X = A oder Q oder G oder K oder E
<220>
   <221> VARIANT
   <222> 35
   <223> X = T oder S oder N oder D
<220>
   <221> VARIANT
   <222> 36
   <223> X = E oder P oder D oder G
<220>
   <221> VARIANT
   <222> 37
   <223> X = W oder S
<220>
   <221> VARIANT
   <222> 38
   <223> X = G oder V
<220>
   <221> VARIANT
   <222> 39
   <223> X = E oder R oder P oder D oder Q oder L oder A
<220>
   <221> VARIANT
   <222> 40
   <223> X = S oder A oder G oder N
<220>
   <221> VARIANT
   <222> 41
   <223> X = L oder I oder V oder S
<220>
   <221> VARIANT
   <222> 42
   <223> X = N oder T
<220>
   <221> VARIANT
   <222> 43
   <223> X = F oder L oder Y oder C
<220>
   <221> VARIANT
   <222> 44
   <223> X = D oder C
<220>
   <221> VARIANT
   <222> 45
   <223> X = G oder D oder S
<220>
   <221> VARIANT
   <222> 46
   <223> X = E oder P oder R oder K oder L oder N
<220>
   <221> VARIANT
   <222> 47
   <223> X = S oder H oder G oder R oder D oder T
<220>
   <221> VARIANT
   <222> 48
   <223> X = A oder S oder C
<220>
   <221> VARIANT
   <222> 49
   <223> X = G oder A oder T oder D oder R oder Q
<220>
   <221> VARIANT
   <222> 50
   <223> X = P oder G oder D oder E oder T oder H
<220>
   <221> VARIANT
   <222> 51
   <223> X = N oder -
<220>
   <221> VARIANT
   <222> 52
   <223> X = A oder -
<220>
   <221> VARIANT
   <222> 53
   <223> X = M oder -
<220>
   <221> VARIANT
   <222> 54
   <223> X = V oder M
<220>
   <221> VARIANT
   <222> 55
   <223> X = R oder G
<220>
   <221> VARIANT
   <222> 56
   <223> X = E oder A oder N oder D oder W oder K
<220>
   <221> VARIANT
   <222> 57
   <223> X = F oder Y oder W oder L
<220>
   <221> VARIANT
   <222> 58
   <223> X = V oder F oder L
<220>
   <221> VARIANT
   <222> 59
   <223> X = T oder V oder I oder F oder L
<220>
   <221> VARIANT
   <222> 60
   <223> X = T oder A oder E oder D oder H oder S
<220>
   <221> VARIANT
   <222> 61
   <223> X = N oder S
<220>
   <221> VARIANT
   <222> 62
   <223> X = A oder V
<220>
   <221> VARIANT
   <222> 63
   <223> X = A oder S oder L oder R oder E oder V
<220>
   <221> VARIANT
   <222> 64
   <223> X = H oder Y oder G oder M oder T
<220>
   <221> VARIANT
   <222> 66
   <223> X = I oder V oder L oder A
<220>
   <221> VARIANT
   <222> 67
   <223> X = R oder E oder H oder A oder D oder K
<220>
   <221> VARIANT
   <222> 68
   <223> X = E oder H
<220>
   <221> VARIANT
   <222> 69
   <223> X = Y oder F
<220>
   <221> VARIANT
   <222> 70
   <223> X = H oder K
<220>
   <221> VARIANT
   <222> 71
   <223> X = L oder V oder F
<400> 308
   Xaa Xaa Ala Asp Val Val Xaa Asn His Xaa Xaa Xaa Xaa Xaa Xaa Xaa
   1               5                   10                  15
   Xaa Xaa Xaa Xaa Xaa Xaa Xaa Xaa Xaa Xaa Xaa Xaa Xaa Xaa Xaa Xaa
               20                  25                  30
   Xaa Xaa Xaa Xaa Xaa Xaa Xaa Xaa Xaa Xaa Xaa Xaa Xaa Xaa Xaa Xaa
           35                  40                  45
   Xaa Xaa Xaa Xaa Xaa Xaa Xaa Xaa Xaa Xaa Xaa Xaa Xaa Xaa Xaa Xaa
       50                  55                  60
   Trp Xaa Xaa Xaa Xaa Xaa Xaa Asp Gly Phe Arg Ile Asp Ala
   65                  70                  75
<210> 309
   <211> 27
   <212> DNA
   <213> Artificial Sequence
<220>
   <223> GEX 023
<220>
   <223> Symbole für degenerierte Basenpositionen (Mischungen verschiedener Nukleotide): R=AG; Y=CT; M=AC; K=GT; W=AT; S=CG; B=CGT; D=AGT; H=ACT; V=ACG; N=ACGT
<400> 309
   ggtctacgcc gacgtcgtsw wcaacca 27
<210> 310
   <211> 24
   <212> DNA
   <213> Artificial Sequence
<220>
   <223> GEX 024
<220>
   <223> Symbole für degenerierte Basenpositionen (Mischungen verschiedener Nukleotide): R=AG; Y=CT; M=AC; K=GT; W=AT; S=CG; B=CGT; D=AGT; H=ACT; V=ACG; N=ACGT
<400> 310
   cgtcgacggc ttccgsatcg acrc 24
<210> 311
   <211> 21
   <212> DNA
   <213> Artificial Sequence
<220>
   <223> GEX 025
<220>
   <223> Symbole für degenerierte Basenpositionen (Mischungen verschiedener Nukleotide): R=AG; Y=CT; M=AC; K=GT; W=AT; S=CG; B=CGT; D=AGT; H=ACT; V=ACG; N=ACGT
<400> 311
   ggcgtcgatg cggaasccgt c 21
<210> 312
   <211> 25
   <212> DNA
   <213> Artificial Sequence
<220>
   <223> GEX 026
<220>
   <223> Symbole für degenerierte Basenpositionen (Mischungen verschiedener Nukleotide): R=AG; Y=CT; M=AC; K=GT; W=AT; S=CG; B=CGT; D=AGT; H=ACT; V=ACG; N=ACGT
<400> 312
   gctcggtgtc gtggttgtcs acgwa 25
<210> 313
   <211> 24
   <212> DNA
   <213> Artificial Sequence
<220>
   <223> GEX 036
<220>
   <223> Symbole für degenerierte Basenpositionen (Mischungen verschiedener Nukleotide): R=AG; Y=CT; M=AC; K=GT; W=AT; S=CG; B=CGT; D=AGT; H=ACT; V=ACG; N=ACGT
<400> 313
   gtacgccgac gccgtnwtha ayca 24
<210> 314
   <211> 29
   <212> DNA
   <213> Artificial Sequence
<220>
   <223> GEX 038
<220>
   <223> Symbole für degenerierte Basenpositionen (Mischungen verschiedener Nukleotide): R=AG; Y=CT; M=AC; K=GT; W=AT; S=CG; B=CGT; D=AGT; H=ACT; V=ACG; N=ACGT
<400> 314
   gggcggcgtc gatcckraan ccrtcrvrs 29
<210> 315
   <211> 27
   <212> DNA
   <213> Artificial Sequence
<220>
   <223> GEX 210
<220>
   <223> Symbole für degenerierte Basenpositionen (Mischungen verschiedener Nukleotide): R=AG; Y=CT; M=AC; K=GT; W=AT; S=CG; B=CGT; D=AGT; H=ACT; V=ACG; N=ACGT
<400> 315
   cgacgtggtg ttcaaccayy tnggncc 27
<210> 316
   <211> 26
   <212> DNA
   <213> Artificial Sequence
<220>
   <223> GEX 211
<220>
   <223> Symbole für degenerierte Basenpositionen (Mischungen verschiedener Nukleotide): R=AG; Y=CT; M=AC; K=GT; W=AT; S=CG; B=CGT; D=AGT; H=ACT; V=ACG; N=ACGT
<400> 316
   gccgacgtgg tgttcaayca yytngg 26

## Patentansprüche

1. Glykosylhydrolase, deren Aminosäuresequenz mit der in Seq. 1 angegebenen Aminosäuresequenz zu 100 % übereinstimmt.

2. Nukleinsäure, kodierend für eine Glykosylhydrolase, deren Aminosäuresequenz mit der in Seq. 1 angegebenen Aminosäuresequenz zu 100 % identisch ist.

3. Für eine Glykosylhydrolase kodierende Nukleinsäure, deren Nukleotidsequenz mit der in Seq. 4 angegebenen Nukleotidsequenz zu 100 % übereinstimmt.

4. Verwendung einer Nukleinsäure nach einem der Ansprüche 2 bis 3 zur Identifizierung und/oder Gewinnung einer neuen Glykosylhydrolase.

5. Verwendung einer Glykosylhydrolase nach Anspruch 1 zur Fusion oder Verknüpfung mit einem anderen Protein zur Entwicklung eines neuen Enzyms.

6. Verwendung einer Nukleinsäure nach einem der Ansprüche 2 bis 3 zur Fusion mit einer anderen Nukleinsäure zur Entwicklung eines neuen Enzyms.

7. Vektor, der eine der in den Ansprüchen 2 bis 3 bezeichneten Nukleinsäuren enthält.

8. Wasch- oder Reinigungsmittel, **dadurch gekennzeichnet, dass** es eine Glykosylhydrolase gemäß Anspruch 1 enthält.

9. Mittel gemäß Anspruch 8, **dadurch gekennzeichnet, dass** es 0,000001 Gewichts-Prozent bis 5 Gew.-% der Glykosylhydrolase enthält.

10. Verfahren zur Reinigung von Textilien oder von harten Oberflächen, **dadurch gekennzeichnet, dass** in wenigstens einem der Verfahrensschritte eine Glykosylhydrolase gemäß Anspruch 1 zum Einsatz kommt.

11. Verfahren nach Anspruch 10, **dadurch gekennzeichnet, dass** die Glykosylhydrolase in einer Menge von 0,01 mg bis 400 mg in dem durch dieses Enzym gekennzeichneten Verfahrensschritt zum Einsatz kommt.

12. Verfahren nach Anspruch 10 oder 11, **dadurch gekennzeichnet, dass** die Glykosylhydrolase in einer Konzentration von 0,0005 bis 20 mg pro I Waschflotte zum Einsatz kommt.

13. Verwendung einer Glykosylhydrolase gemäß Anspruch 1 in einem Wasch- oder Reinigungsmittel nach einem der Ansprüche 8 oder 9 zur Reinigung von Textilien oder von harten Oberflächen.

14. Verwendung nach Anspruch 13, **dadurch gekennzeichnet, dass** die Glykosylhydrolase in einer Menge von 0,01 mg bis 400 mg zum Einsatz kommt.

15. Verwendung nach Anspruch 13 oder 14, **dadurch gekennzeichnet, dass** die Glykosylhydrolase in einer Konzentration von 0,0005 bis 20 mg pro I Waschflotte zum Einsatz kommt.

16. Verwendung einer Glykosylhydrolase gemäß Anspruch 1 zur Behandlung von Rohmaterialien oder Zwischenprodukten in der Textilherstellung.

17. Verfahren zur Stärkeverflüssigung und zur Ethanolproduktion, **dadurch gekennzeichnet, dass** darin eine Glykosylhydrolase gemäß Anspruch 1 eingesetzt wird.

18. Verwendung einer Glykösylhydrolase gemäß Anspruch 1 zur Herstellung von linearen und/oder kurzkettigen Oligosacchariden.

19. Verwendung einer Glykosylhydrolase gemäß Anspruch1 zur Hydrolyse von Cyclodextrinen.

20. Verwendung einer Glykosylhydrolase gemäß Anspruch 1 zur Freisetzung von niedermolekularen Verbindungen aus Polysaccharid-trägern oder Cyclodextrinen.

21. Verwendung einer Glykosylhydrolase gemäß Anspruch 1 zur Herstellung von Lebensmitteln und/oder Lebensmittelbestandteilen.

22. Verwendung einer Glykosylhydrolase gemäß Anspruch 1 zur Herstellung von Tierfutter und/oder Tierfutterbestandteilen.

23. Verwendung einer Glykosylhydrolase gemäß Anspruch 1 zur Auflösung stärkehaltiger Klebeverbindungen.

24. Verfahren zum temporären Kleben, **dadurch gekennzeichnet, dass** darin eine Glykosylhydrolase gemäß Anspruch 1 eingesetzt wird.

## Claims

1. A glycosil hydrolase the amino acid sequence of which corresponds to 100% to the amino acid sequence depicted in SEQ ID NO: 1.

2. A nucleic acid coding for a glycosil hydrolase the amino acid sequence of which is 100% identical to the amino acid sequence depicted in SEQ ID NO: 1.

3. A nucleic acid coding for a glycosil hydrolase the nucleotide sequence of which corresponds to 100% to the nucleotide sequence depicted in SEQ ID NO: 4.

4. Use of a nucleic acid according to any one of claims 2 to 3 for identifying and/or recovering a new glycosil hydrolase.

5. Use of a glycosil hydrolase according to claim 1 for fusion or linkage with another protein for the development of a new enzyme.

6. Use of a nucleic acid according to any one of claims 2 to 3 for fusion with another nucleic acid for the development of a new enzyme.

7. A vector containing one of the nucleic acids described in claims 2 to 3.

8. A washing or cleaning agent, **characterised in that** it contains a glycosil hydrolase according to claim 1.

9. The agent according to claim 8, **characterised in that** it contains 0.000001 wt-% to 5 wt-% of glycosil hydrolase.

10. A method for cleaning of textiles or hard surfaces, **characterised in that** a glycosil hydrolase according to claim 1 is used in at least one of the method steps.

11. The method according to claim 10, **characterised in that** the glycosil hydrolase is used in the method step, which is **characterised by** this enzyme, in an amount of 0.01 mg to 400 mg.

12. The method according to claim 10 or 11, **characterised in that** the glycosil hydrolase is used in a concentration of 0.0005 to 20 mg per I wash liquor.

13. Use of a glycosil hydrolase according to claim 1 in a washing or cleaning agent according to any one of claims 8 or 9 for the cleaning of textiles or hard surfaces.

14. Use according to claim 13, **characterised in that** the glycosil hydrolase is used in an amount of 0.01 mg to 400 mg.

15. Use according to claim 13 or 14, **characterised in that** the glycosil hydrolase is used in a concentration of 0.0005 to 20 mg per I wash liquor.

16. Use of a glycosil hydrolase according to claim 1 for the treatment of raw materials or intermediate products in the production of textiles.

17. A method for starch liquefaction and for the production of ethanol, **characterised in that** a glycosil hydrolase according to claim 1 is used.

18. Use of a glycosil hydrolase according to claim 1 for the production of linear and/or short chain oligosaccharides.

19. Use of a glycosil hydrolase according to claim 1 for the hydrolysis of cyclodextrins.

20. Use of a glycosil hydrolase according to claim 1 for the release of low molecular compounds from polysaccharide carriers or cyclodextrins.

21. Use of a glycosil hydrolase according to claim 1 for the production of foodstuff and/or foodstuff ingredients.

22. Use of a glycosil hydrolase according to claim 1 for the production of animal feed and/or animal feed ingredients.

23. Use of a glycosil hydrolase according to claim 1 for disintegrating starch-containing adhesive bonds.

24. A method for temporary adhesive bonding, **characterised in that** a glycosil hydrolase according to claim 1 is used.

## Revendications

1. Glycosylhydrolase dont la séquence d'acides aminés correspond à 100% à la séquence d'acides aminés indiquée dans la Séq. 1.

2. Acide nucléique codant pour une glycosylhydrolase dont la séquence d'acides aminés est identique à 100% à la séquence d'acides aminés indiquée dans la Séq. 1.

3. Acide nucléique codant pour une glycosylhydrolase dont la séquence de nucléotides correspond à 100% à la séquence de nucléotides indiquée dans la Séq. 4.

4. Utilisation d'un acide nucléique selon l'une des revendications 2 à 3 pour l'identification et/ou l'obtention d'une nouvelle glycosylhydrolase.

5. Utilisation d'une glycosylhydrolase selon la revendication 1 pour la fusion ou la liaison à une autre protéine pour le développement d'une nouvelle enzyme.

6. Utilisation d'un acide nucléique selon l'une des revendications 2 à 3 pour la fusion à un autre acide nucléique pour le développement d'une nouvelle enzyme.

7. Vecteur qui comprend un des acides nucléiques décrits dans les revendications 2 à 3.

8. Agent de lavage ou de nettoyage, **caractérisé en ce qu'**il comprend une glycosylhydrolase selon la revendication 1.

9. Agent selon la revendication 8, **caractérisé en ce qu'**il comprend de 0,000001 % en poids à 5 % en poids de glycosylhydrolase.

10. Procédé de nettoyage de textiles ou de surfaces dures, **caractérisé en ce qu'**une glycosylhydrolase selon la revendication 1 est mise en oeuvre dans au moins une des étapes de procédé.

11. Procédé selon la revendication 10, **caractérisé en ce que** la glycosylhydrolase est mise en oeuvre en une quantité de 0,01 mg à 400 mg dans l'étape de procédé **caractérisée par** cette enzyme.

12. Procédé selon la revendication 10 ou 11, **caractérisé en ce que** la glycosylhydrolase est mise en oeuvre en une concentration de 0,0005 à 20 mg par litre de bain aqueux.

13. Utilisation d'une glycosylhydrolase selon la revendication 1 dans un agent de lavage ou de nettoyage selon l'une des revendications 8 à 9 pour le nettoyage de textiles ou de surfaces dures.

14. Utilisation selon la revendication 13, **caractérisée en ce que** la glycosylhydrolase est mise en oeuvre en une quantité de 0,01 mg à 400 mg.

15. Utilisation selon la revendication 13 ou 14, **caractérisée en ce que** la glycosylhydrolase est mise en oeuvre en une concentration de 0,0005 à 20 mg par litre de bain aqueux.

16. Utilisation d'une glycosylhydrolase selon la revendication 1 pour le traitement des matériaux bruts ou des produits intermédiaires dans la fabrication du textile.

17. Procédé de liquéfaction de l'amidon et de production d'éthanol, **caractérisé en ce qu'**une glycosylhydrolase selon la revendication 1 y est mise en oeuvre.

18. Utilisation d'une glycosylhydrolase selon la revendication 1 pour la fabrication d'oligosaccharides linéaires et/ou à chaînes courtes.

19. Utilisation d'une glycosylhydrolase selon la revendication 1 pour l'hydrolyse des cyclodextrines.

20. Utilisation d'une glycosylhydrolase selon la revendication 1 pour la libération de composés de faible poids moléculaire à partir de supports en polysaccharides ou de cyclodextrines.

21. Utilisation d'une glycosylhydrolase selon la revendication 1 pour la fabrication d'aliments et/ou de composants alimentaires.

22. Utilisation d'une glycosylhydrolase selon la revendication 1 pour la fabrication d'aliments pour animaux et/ou de composants d'aliments pour animaux.

23. Utilisation d'une glycosylhydrolase selon la revendication 1 pour la dissolution des composés collants contenant de l'amidon.

24. Procédé de collage temporaire, **caractérisé en ce qu'**une glycosylhydrolase selon la revendication 1 y est mise en oeuvre.
